# EUROPÄISCHE PATENTSCHRIFT

(11) **EP 0 773 955 B1**
(45) Veröffentlichungstag und Bekanntmachung des Hinweises auf die Patenterteilung: **16.04.2003**
(21) Anmeldenummer: 95923231.5
(22) Anmeldetag: 06.06.1995
(51) Int. Cl.: C07K 5/06, C07D 207/16, C07D 207/48, C07D 205/04, C07D 211/60, A61K 31/40, A61K 38/05

(54) **NEUE THROMBININHIBITOREN, IHRE HERSTELLUNG UND VERWENDUNG**
NEW THROMBIN INHIBITORS, THEIR PREPARATION AND USE
NOUVEAUX INHIBITEURS DE THROMBINE, LEUR PREPARATION ET LEUR UTILISATION

(30) Priorität: 17.06.1994 DE 4421052
(43) Veröffentlichungstag der Anmeldung: 21.05.1997
(73) Patentinhaber: BASF AKTIENGESELLSCHAFT, 67056 Ludwigshafen (DE)
(72) Erfinder: BÖHM, Hans-Joachim, D-67117 Limburgerhof (DE); KOSER, Stefan, D-67063 Ludwigshafen (DE); MACK, Helmut, D-67067 Ludwigshafen (DE); PFEIFFER, Thomas, D-67459 Böhl-Iggelheim (DE); SEITZ, Werner, D-68723 Plankstadt (DE); HÖFFKEN, Hans, Wolfgang, D-67069 Ludwigshafen (DE); HORNBERGER, Wilfried, D-67434 Neustadt (DE)
(74) Vertreter: Grünecker, Kinkeldey, Stockmair & Schwanhäusser Anwaltssozietät
(86) Internationale Anmeldenummer: EP9502135
(87) Internationale Veröffentlichungsnummer: WO95035309

(56) Entgegenhaltungen:
- EP-A- 0 513 543
- EP-A- 0 601 459
- WO-A-92/08709
- WO-A-94/29336
- PHARMAZIE, Bd.43, Nr.11, November 1988, BERLIN DD Seiten 782 - 783 J STÜRZEBECHER ET AL. 'Synthetische Inhibitoren der Serinprotease'
- PHARMAZIE, Bd.43, Nr.6, Juni 1988, BERLIN DD Seiten 412 - 414 B VOIGT ET AL. 'Sythese von N-alpha(Arylsu lphonyl)-4-amidino-phenylalanyl-prolinen und von N-alpha-(Arylsulphonylglycyl)-4-am idino-phenylalanyl-prolinen und deren Prüfung als Inhibitoren von Serinproteinasen'
- PHARMAZIE, Bd.39, Nr.5, Mai 1984, BERLIN DD Seiten 315 - 317 G WAGNER ET AL. 'Synthese von N-alpha-(Tosyl-Beta-alanyl-) und N-epsilon- (Tosyl-epsilon-aminocapronyl)am idinophenylalaninamiden als stark wirksame Thrombininhibitoren'
- PHARMAZIE, Bd.39, Nr.6, Juni 1984, BERLIN DD Seiten 379 - 381 B VOIGT & G WAGNER 'Synthese von N-alpha-( arylsulphonylglycyl)-4-amidinophenylalanin amiden als Thrombininhibitoren'
- PHARMAZIE, Bd.40, Nr.8, August 1985, BERLIN DD Seiten 527 - 529 B VOIGT & G WAGNER 'Synthese von N-alpha-(Benzoylglycyl) und N-alpha-(benzy loxycarbonylglycyö)-4-amidinophenylalanina miden als Thrombininhibitoren'
- PHARMAZIE, Bd.41, Nr.6, 1986, BERLIN DD Seiten 378 - 381 B VOIGT & G WAGNER 'Synthese von N-alpha-(Tosylprolylglycyl) und N-alpha-(T osylglycylproly)-4-amidinophenylalaninamid en als Thrombininhibitoren'
- PHARMAZIE, Bd.42, Nr.2, Februar 1987, BERLIN DD Seiten 114 - 116 J STÜRZEBECHER ET AL. 'Synthetische Inhibitoren von Serinproteasen'
- PHARMAZIE, Bd.41, Nr.4, April 1986, BERLIN DD Seiten 233 - 235 B VOIGT & G WAGNER 'Synthese von N-alpha-(Arylsulphonyl-L-prolyl) und N-alpha-(benzyloxycarbonyl-L-prolyl)-D, L-4-amidinophenylalaninamiden als Thrombininhibitoren'
- CHEMICAL ABSTRACTS, vol. 103, no. 11, 16. September 1985, Columbus, Ohio, US; abstract no. 84003j, H TSUNEMATSU ET AL. 'A new serine protease which preferentially recognizes p-guanidino-L-phenylalanine residue in ascitic plasma from Ehrlich ascites tumor-bearing mice' Seite 276 ; & BIOCHEMICAL AND BIOPHYSICAL RESEARCH COMMUNICATIONS., Bd.128, Nr.3, 1985, DULUTH, MINNESOTA US Seiten 1233 - 1238

## Beschreibung

Die Erfindung betrifft neue Thrombininhibitoren, deren Herstellung sowie deren Verwendung zur Bekämpfung von Krankheiten.

Thrombin gehört zur Gruppe der Serinproteasen und spielt als terminales Enzym in der Blutgerinnungskaskade eine zentrale Rolle. Sowohl die intrinsische als auch die extrinsische Gerinnungskaskade führen über mehrere Verstärkungsstufen zur Entstehung von Thrombin aus Prothrombin. Die thrombinkatalysierte Spaltung von Fibrinogen zu Fibrin leitet dann die Blutgerinnung und die Aggregation der Thrombozyten ein, die ihrerseits durch die Bindung von Plättchenfaktor 3 und Gerinnungsfaktor XIII sowie eine ganze Reihe von hochaktiven Mediatoren die Thrombinbildung verstärken.

Thrombinbildung und -wirkung sind zentrale Ereignisse bei der Entstehung sowohl von weißen, arteriellen als auch von roten, venösen Thromben und daher potentiell wirksame Angriffspunkte für Pharmaka. Thrombin-Inhibitoren sind im Gegensatz zu Heparin in der Lage, unabhängig von Kofaktoren gleichzeitig die Wirkungen von Thrombin sowohl in der Gerinnungskaskade als auch auf Thrombozyten vollständig zu hemmen. Sie können in der Akutphase thromboembolische Ereignisse nach perkutaner transluminaler koronarer Angioplastie (PTCA) und Lyse verhindern und als Antikoagulantien in der extrakorporalen Zirkulation (Herz-Lungen-Maschine, Hämodialyse) dienen. Sie können auch allgemein zur Thromboseprophylaxe, beispielsweise nach chirurgischen Eingriffen dienen.

Es ist bekannt, daß synthetische Argininderivate die Enzymaktivität des Thrombins beeinflussen, indem sie mit dem aktiven Serinrest der Protease Thrombin in Wechselwirkung treten. Peptide auf der Basis Phe-Pro-Arg, in denen die N-terminale Aminosäure in der D-Form vorliegt, haben sich als besonders günstig erwiesen. D-Phe-Pro-Arg-isopropylester ist als kompetitiv wirkender Thrombininhibitor beschrieben (C. Mattson u.a., Folia Haematol. 109, 43-51, 1982).

Die Derivatisierung des C-Terminus Arginin zum Aldehyd führt zu einer Verstärkung der Inhibitorwirkung. So sind eine Vielzahl von Arginalen beschrieben, die die Hydroxylgruppe des "aktiven" Serins halbacetalisch zu binden vermögen (EP 185.390, 479.489, 526.877, 542.525; WO 93 15 756, 93 18 060).

Die thombininhibitorische Wirksamkeit peptidischer Ketone, fluorierter Alkylketone, sowie von Ketoestern, Borsäurederivaten, Phosphorsäureestern und α-Ketocarbonsäureamiden ist ebenfalls mit dieser Serin-Wechselwirkung erklärbar (EP 118.280, 195.212, 362.002, 364.344, 410.411, 471.651, 589.741, 293.881, 503.203, 504.064, 530.167; WO 92 07 869; 94 08 941).

DE 31 08 810 und WO 93 11 152 beschreiben dipeptidische ω-Aminoalkylguanidine.

Bei den von J. Oleksyszyn u.a. in J. Med. Chem. 37, 226-231 (1994) beschriebenen peptidischen 4-Amidinophenyl-glycinphosphonat-diphenylestern handelt es sich um irreversible Thrombininhibitoren mit unzureichender Selektivität gegenüber anderen Serinproteasen.

Die nicht vorveröffentlichten EP 601.459 und WO 94/29336 beschreiben peptidische Thrombininhibitoren.

Gegenstand der Erfindung sind Verbindungen der Formel 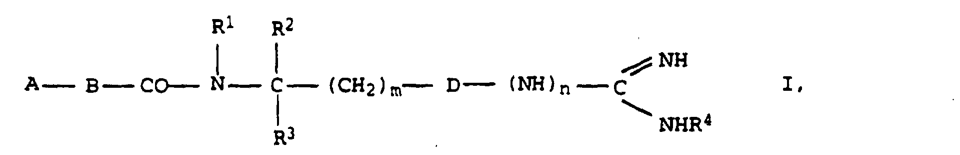 sowie deren Salze mit physiologisch verträglichen Säuren und deren Stereoisomeren, worin die Substituenten folgende Bedeutungen besitzen:
- A:: 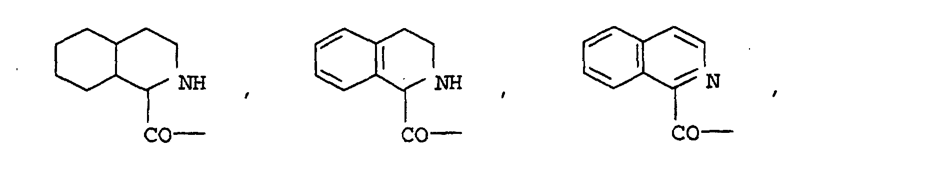 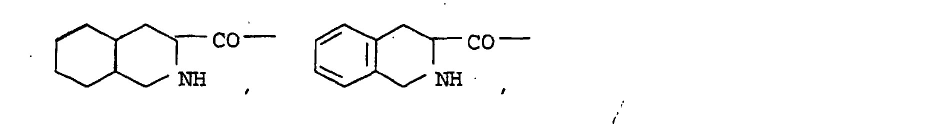 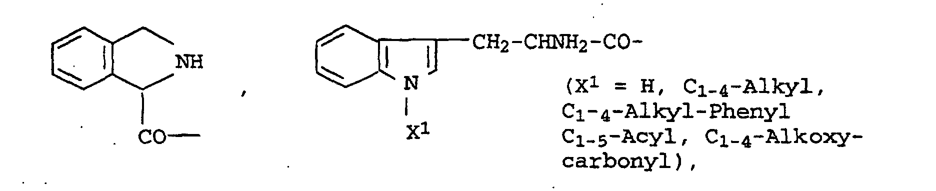 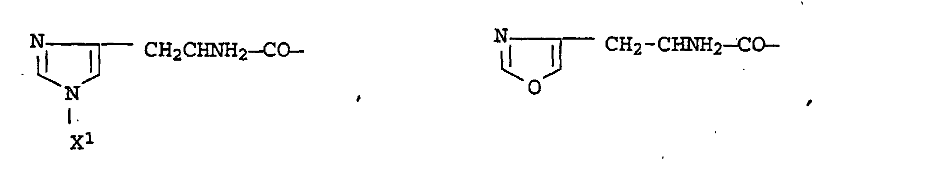 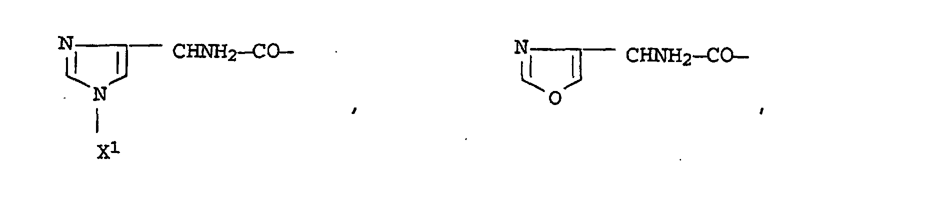 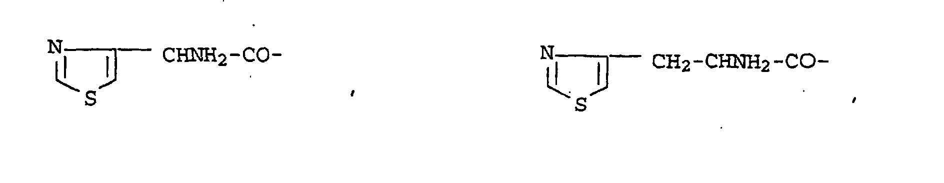 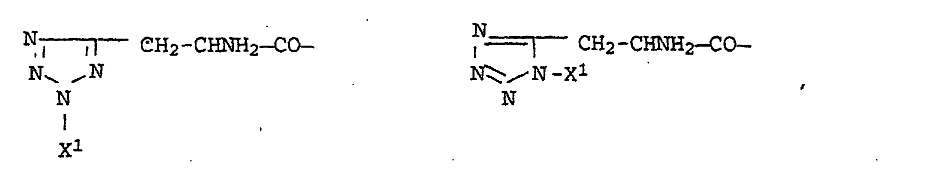 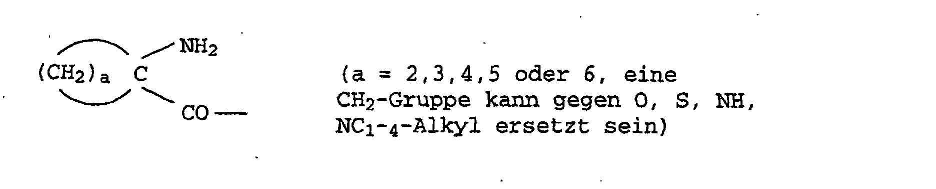 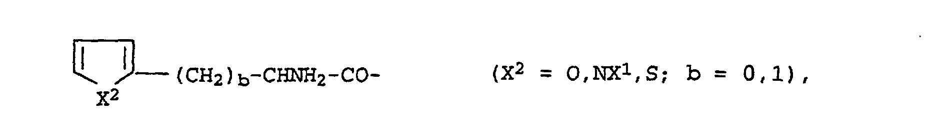 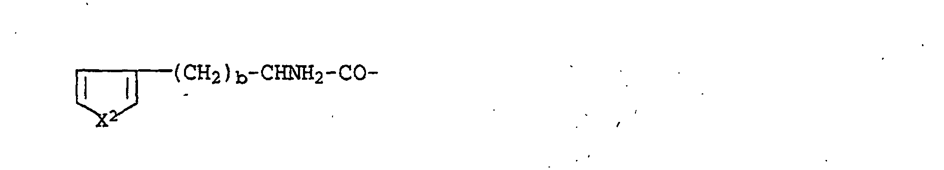 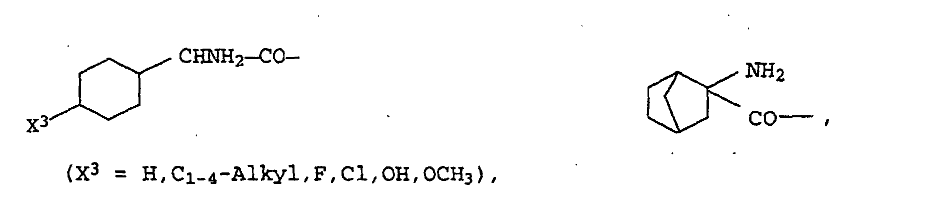 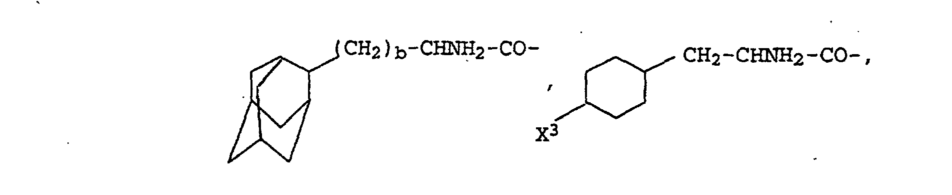 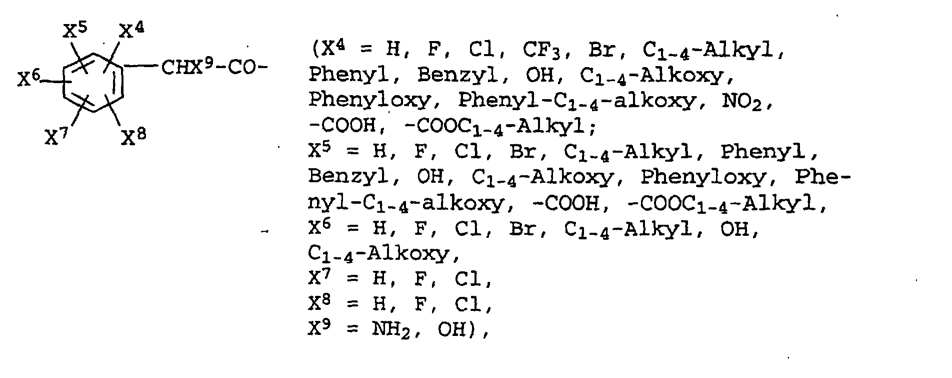 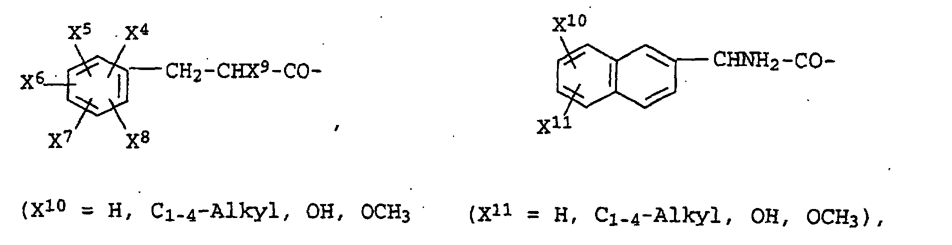 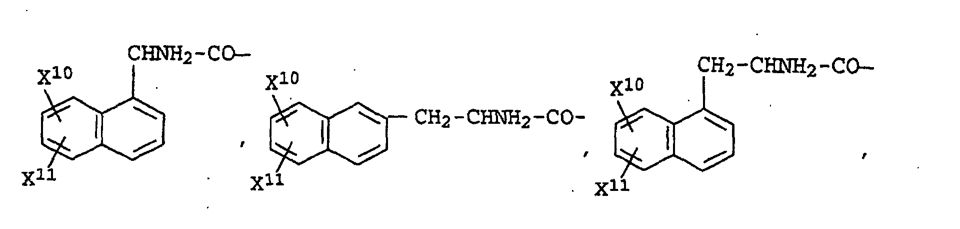 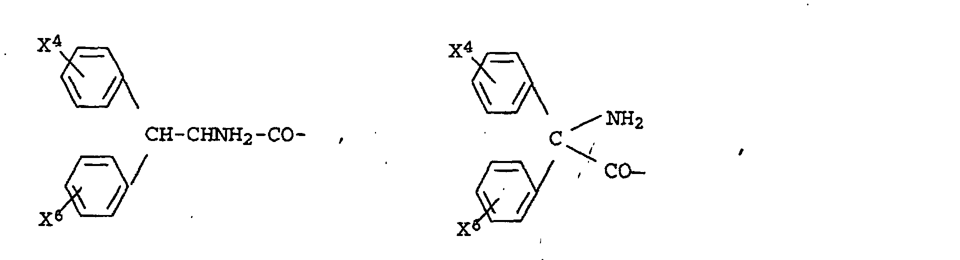 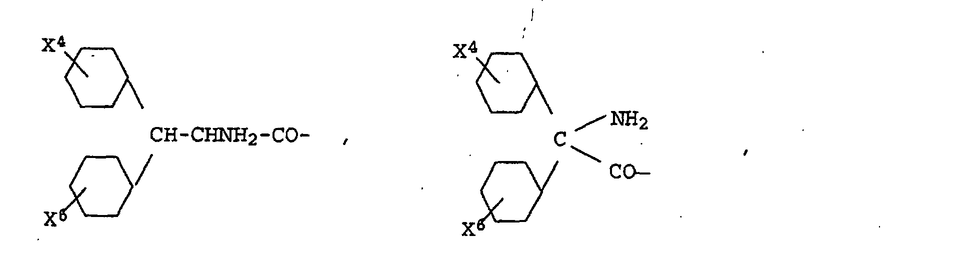 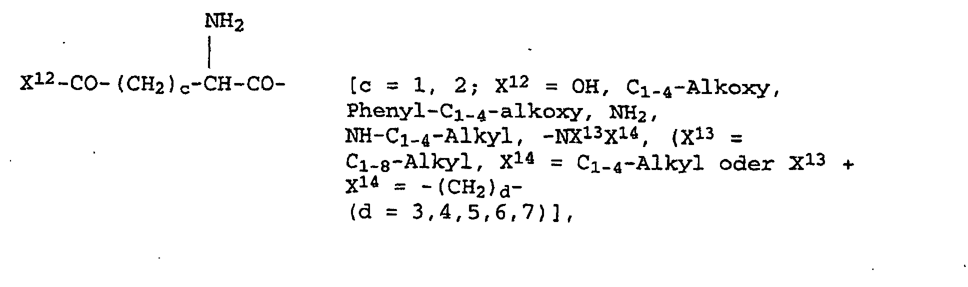 X¹³ ― SO₂ ― CH₂ ― CH₂ ― CHNH₂ ― CO ― ,

H₂N-CH₂-CO-, H₂N-CHX¹³-CO-

- wobei in allen vorstehend genannten A-Resten die α-NH- oder α-NH₂-Gruppe mono- bzw. disubstituiert sein kann durch C₁₋₁₂-Alkyl, Phenyl-C₁₋₄-alkylen, X¹²OC-C₁₋₆-alkylen, X¹²OC-C₁₋₆-alkylcarbonyl, -α- bzw. β-Naphthyl-C₁₋₄-alkylen, C₁₋₁₂-Alkylcarbonyl, Phenyl-C₁₋₄-alkylcarbonyl, C₁₋₇-Alkoxycarbonyl, Phenyl-C₁₋₅-alkoxycarbonyl, -α- bzw. β-Naphthyl-C₁₋₄-alkylcarbonyl-, C₁₋₆-Alkylaminocarbonyl oder Phenyl-C₁₋₄-alkylaminocarbonyl -
- noch A:: X¹-NH-CH₂-CH₂-CO-, X¹-NH-CH₂-CH₂-CH₂-CO- 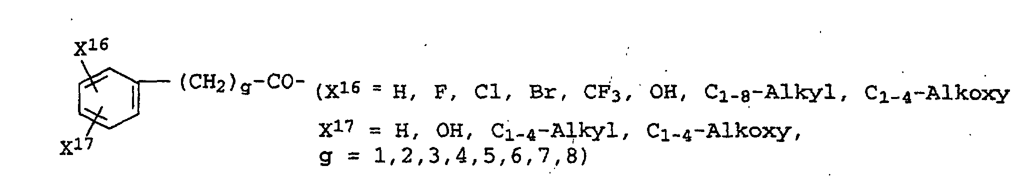 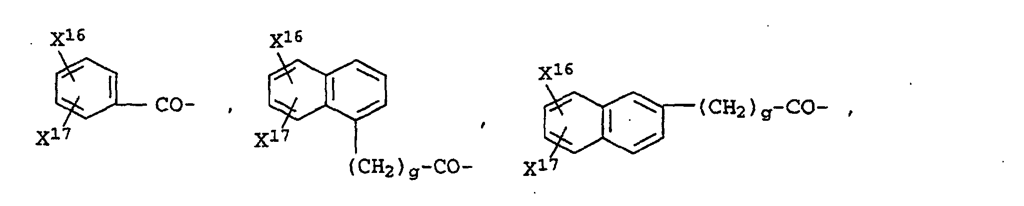 X¹⁸-O-CO-C₁₋₄-alkylen-CO- (X¹⁸ = H, C₁₋₄-Alkyl),
C₁₋₁₂-Alkyl-CO-, C₁₋₁₀-Alkyl-NH-CO-, Phenyl-C₁₋₄-alkylen-NH-CO-, α- oder β-Naphthyl-CO- oder C₃₋₇-Cycloalkyl-CO-,
- B:: 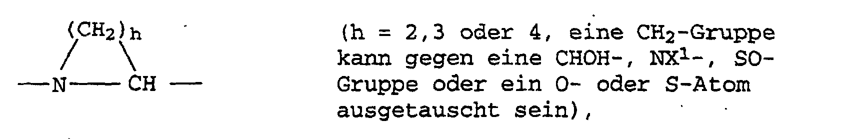 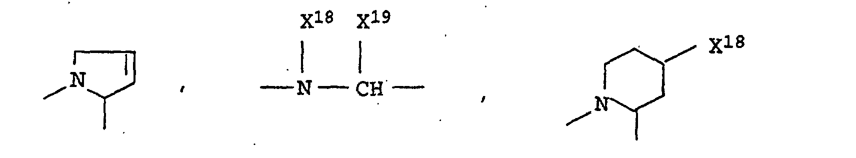 (X¹⁸ = H oder C₁₋₄-Alkyl,
X¹⁹ = H, C₁₋₆-Alkyl, Phenyl, Benzyl, Cyclohexyl oder Cyclohexylmethyl)
- R¹:: H oder C₁₋₄-Alkyl,
- R²:: H,
- R³:: H,
- m:: 0,
- D:: Phenylen, an welchem (CH₂)ₘ und (NH)ₙ para- oder meta-ständig zueinander gebunden sind und welches in der ortho-Stellung zu (CH₂)ₘ durch F, Cl, Br, HO-CH₂-, OH, NH₂, NO₂, C₁₋₄-Alkoxy, C₁₋₆-Alkyl oder COX²¹ (X²¹ = H, C₁₋₄-Alkyl, C₁₋₄-Alkoxy, OH, NH₂, NH-C₁₋₄-Alkyl) -O-(CH₂)₁₋₃-CO-X²¹ oder -(CH₂)₁₋₃-CO-X²¹ substituiert sein kann,
Pyridinylen, Pyrimidinylen, Pyrazinylen oder Pyridazinylen, an welchen (CH₂)ₘ und (NH)ₙ para- oder meta-ständig zueinander gebunden sind und welche in der ortho-Stellung zu (CH₂)ₘ durch F, Cl, Br, OH, NH₂, C₁₋₄-Alkoxy oder C₁₋₆-Alkyl substituiert sein können,
1,4- oder 1,3-Cyclohexylen, in dem eine CH₂-Gruppe in ortho-Stellung zu (CH₂)ₘ durch NH, O, S oder SO ersetzt sein kann oder
Piperidinylen, welches in 3- oder 4-Position zum Stickstoff mit (CH₂)ₘ verbunden ist, und in welchem das Stickstoffatom selbst die C(=NH)NHR⁴-Gruppe trägt,
- n:: 0 oder 1,
- R⁴:: H, -CO-C₁₋₂₀-Alkyl, -CO-O-C₁₋₂₀-Alkyl, OH oder NH₂,
wobei A, B, D, R¹, R⁴, n nicht folgende Bedeutungen gleichzeitig annehmen darf:

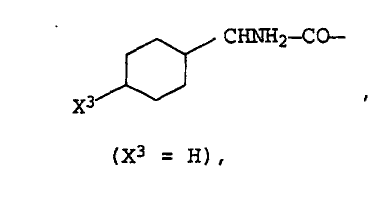

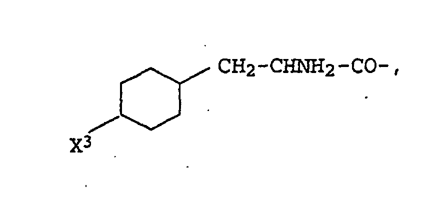

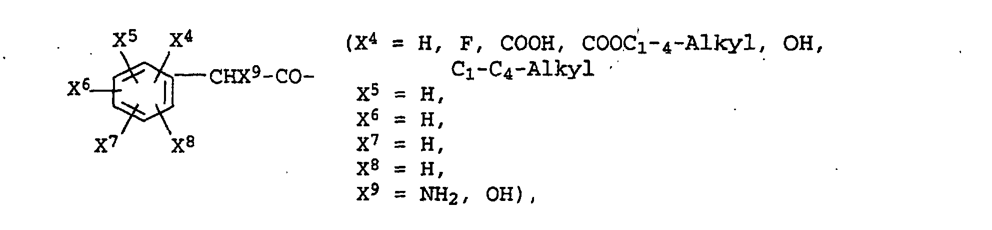

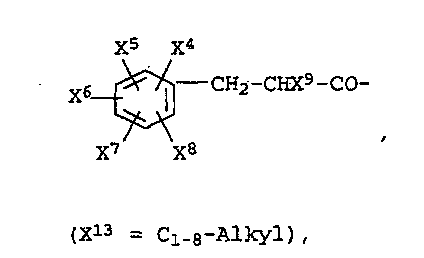

H₂N-CH₂-CO-, H₂N-CHX¹³-CO-

- wobei in allen vorstehend genannten A-Resten die α-NHoder α-NH₂-Gruppe monosubstituiert sein kann durch C₁₋₆-Alkyl, Phenyl-C₁₋₄-alkylen, C₁₋₆-Alkylcarbonyl, C₁₋₆-Alkoxycarbonyl, Phenyl-C₁₋₅-alkoxycarbonyl, 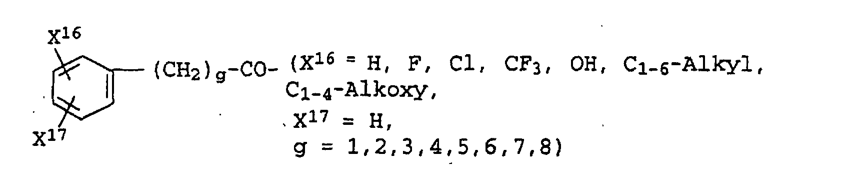 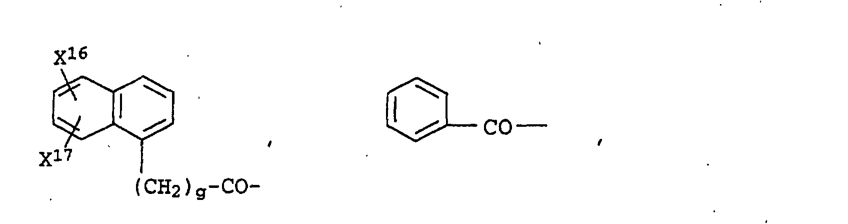 X¹⁸-O-CO-C₁₋₄-alkylen-CO- (X¹⁸ = H, C₁₋₄-Alkyl),
   C₁₋₁₂-Alkyl-CO-, C₃₋₇-Cycloalkyl-CO-, 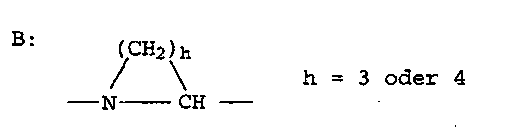
   - R¹:: H oder C₁₋₄-Alkyl
   - D:: Phenylen, an welchem (CH₂)ₘ und (NH)ₙ paraständig zueinander gebunden sind
   1,4-Cyclohexylen
   oder
   Piperidinylen, welches in 4-Position zum Stickstoff mit (CH₂)ₘ verbunden ist, und in welchem das Stickstoffatom selbst die C(=NH)NHR⁴-Gruppe trägt,
   - n:: 0 oder 1,
   - R⁴:: H, -CO-C₁₋₆-Alkyl, -CO-O-C₁₋₆-Alkyl oder OH
   und auch nicht folgende Bedeutung annehmen darf:
   A: 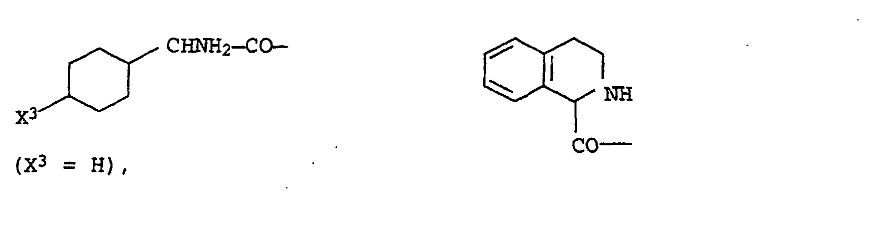 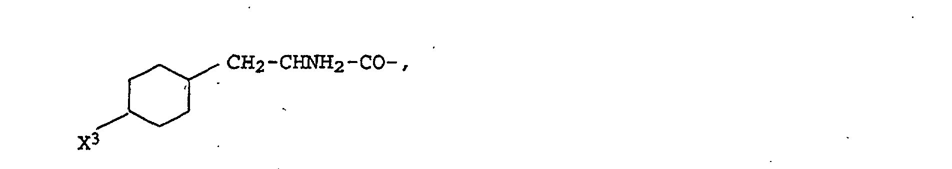 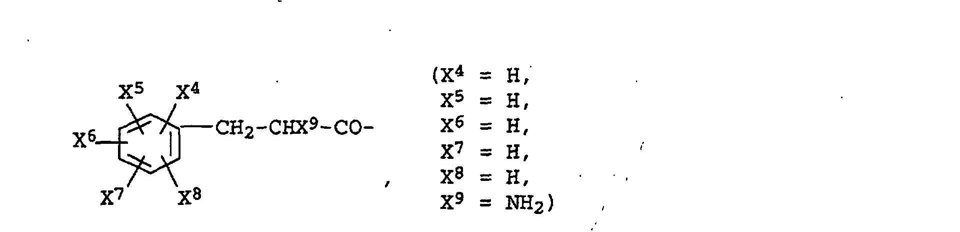 X¹² = OH, C₁₋₄-Alkoxy, NH₂,
- wobei in allen vorstehend genannten A-Resten die α-NH- oder α-NH₂-Gruppe mono- bzw, disubstituiert sein kann durch C₁₋₄-Alkyl, X¹²OC-C₁₋₄-alkylen, X¹²OC-C₁₋₂-alkylcarbonyl, C₁₋₄-Alkylcarbonyl, C₁₋₄-Alkoxycarbonyl,
   B: 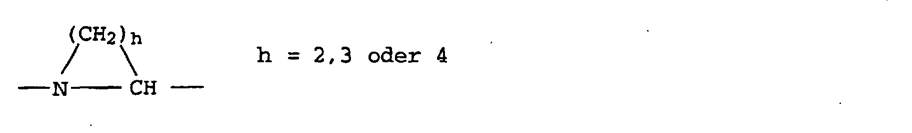 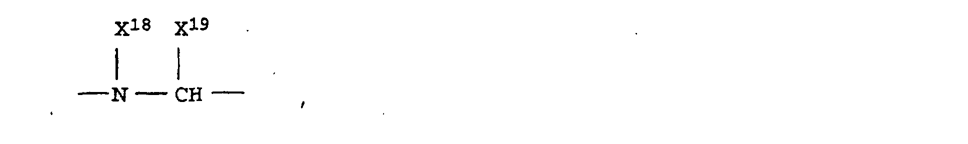
   X¹⁸ = H
   X¹⁹ = C₁₋₆-Alkyl, Benzyl)

   - R¹:: H
   - D:: Phenylen, an welchem (CH₂)ₘ und (NH)ₙ paraständig zueinander gebunden sind
   1,4-Cyclohexylen,
   oder
   Piperidinylen, welches in 4-Position zum Stickstoff mit (CH₂)ₘ verbunden ist, und in welchem das Stickstoffatom selbst die C(=NH)NHR⁴-Gruppe trägt,
   - n:: 0
   - R⁴:: H
   und auch nicht folgende Bedeutung annehmen darf:
   A: 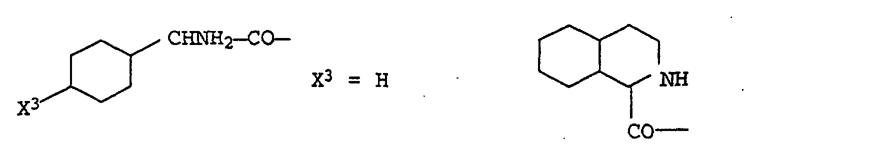 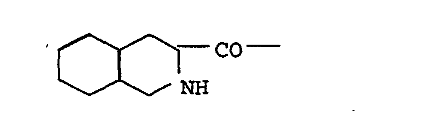 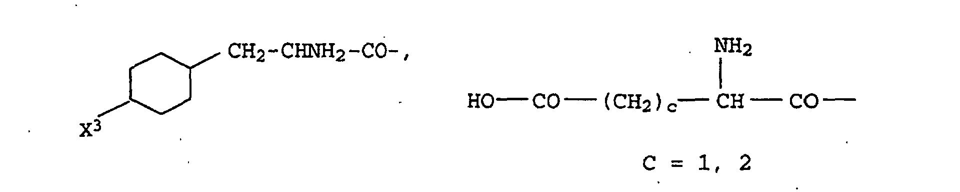 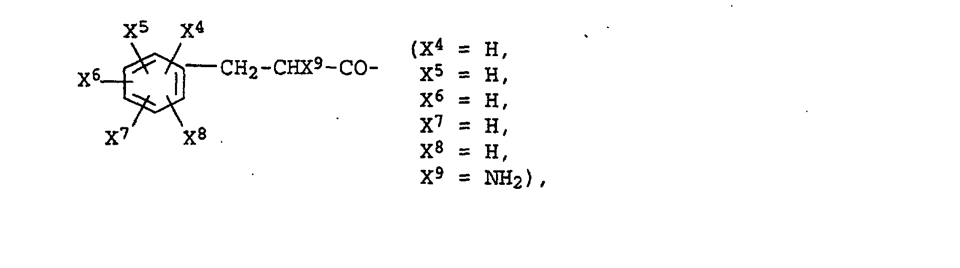
- wobei in allen vorstehend genannten A-Resten die α-NH₂-Gruppe monosubstituiert sein kann durch X¹²OC-C₁₋₃-alkylen, C₁₋₅-Alkylcarbonyl, mit X¹² = OH
   B: 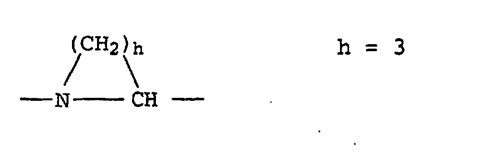
   - R¹:: H
   - D:: Phenylen, an welchem (CH₂)ₘ und (NH)ₙ meta- oder paraständig zueinander gebunden sind und welches in der ortho-Stellung zu (CH₂)ₘ durch F substituiert sein kann,
   Pyridinylen mit der Amidingruppe in 3-Stellung oder Pyridazinylen, an welchen (CH₂)ₘ und (NH)ₙ paraständig zueinander gebunden sind
   Piperidinylen, welches in 4-Position zum Stickstoff mit (CH₂)ₘ verbunden ist, und in welchem das Stickstoffatom selbst die C(=NH)NHR⁴-Gruppe trägt,
   - n:: 0
   - R⁴:: H
   und nicht N-[[1-(Aminoiminomethyl)-3-piperidinyl]methyl]-1-Dphenylalanyl-L-prolinamid bedeutet

Die in der Formel I enthaltenen Alkylreste können gerade oder verzweigtkettig sein.

Bevorzugt sind die Verbindungen der Formel I in denen die Substituenten folgende Bedeutungen besitzen:
A: 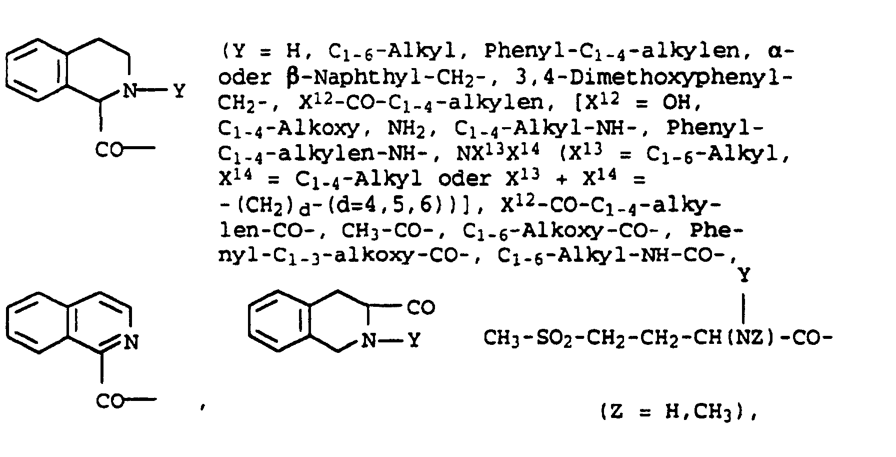 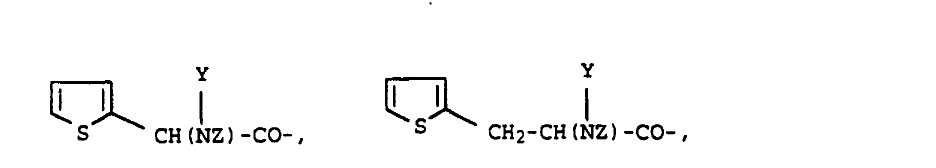 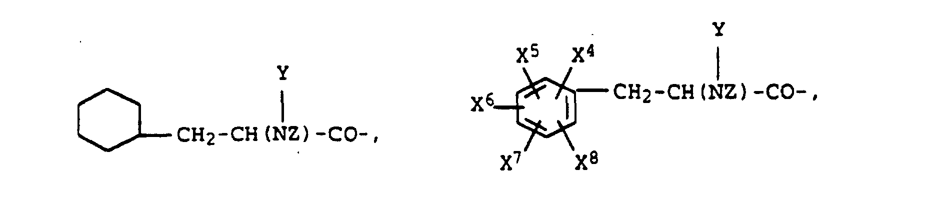 (X⁴ = H, F, Cl, Br, CF₃, C₁₋₄-Alkyl, OH, OCH₃, NO₂, Phenyl, vorzugsweise H, F, Cl, Br, CH₃, t-Butyl, OH, OCH₃, NO₂, X⁵ = H, F, Cl, Br, CH₃, OH, OCH₃, Phenyl, vorzugsweise H, F, OH, OCH₃, Phenyl, X⁶ = H, F, Cl, Br, CH₃, OH, OCH₃, vorzugsweise H, F, OH, OCH₃, X⁷ = H, F, X⁸ = H, F), 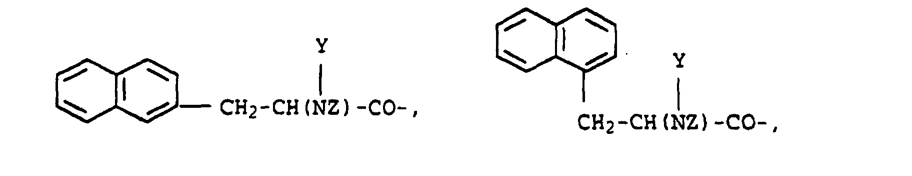 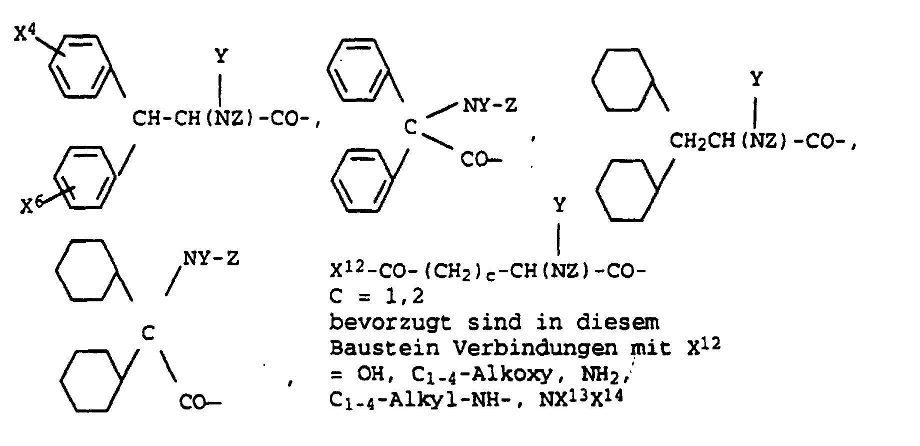 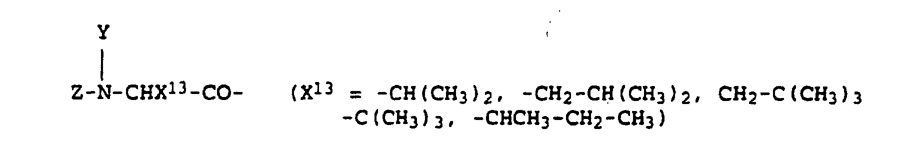 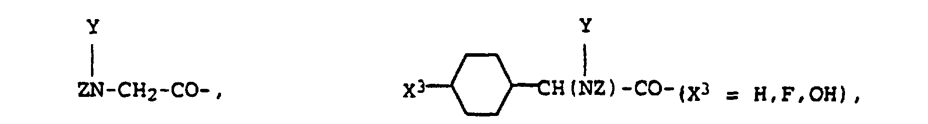 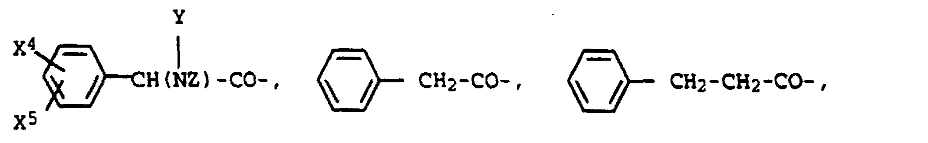 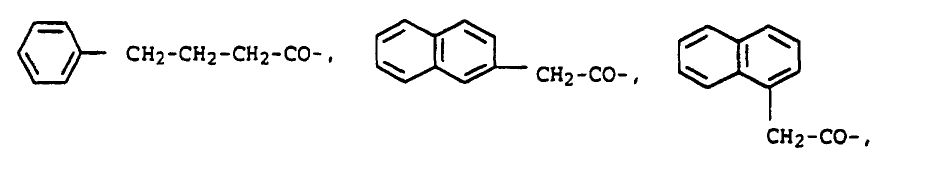 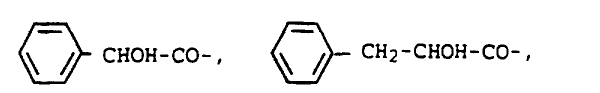
B:

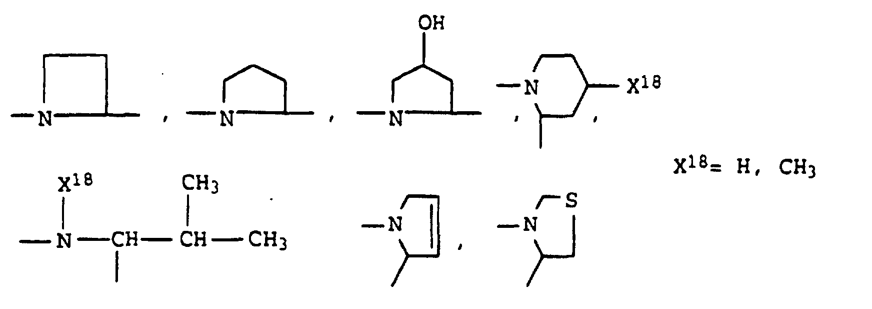

- R¹:: H, CH₃
- R²:: H
- R³:: H, CH₃, CHO, COCF₃, COC₂F₅, CO-CH₂OH, CO-CH₃, CO-CH₂-Phenyl, CH₂OH,
- R⁴:: H, OH, NH₂
- m:: 0,
eine bevorzugte Gruppe von D-Bausteinen ist 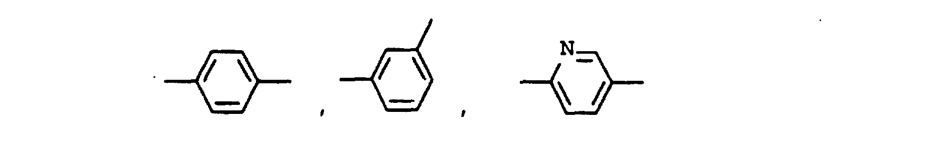 bevorzugte Bausteine der Kombination -(CH₂)ₘ-D-(NH)ₙ- in der allgemeinen Formel I sind:
für -(CH₂)ₘ-D-(NH)ₙ- mit m = 0, n = 0 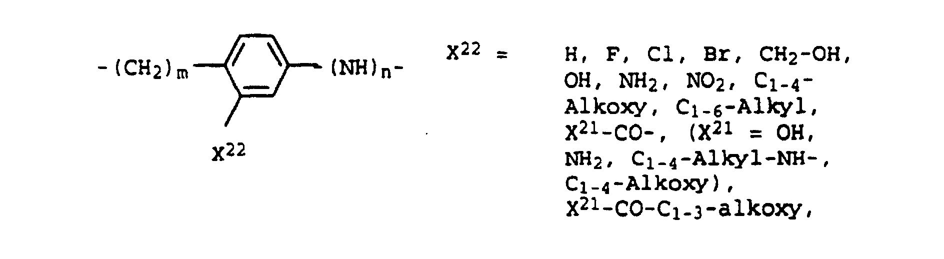 für -(CH₂)ₘ-D-(NH)ₙ- mit m = 0, n = 0,1 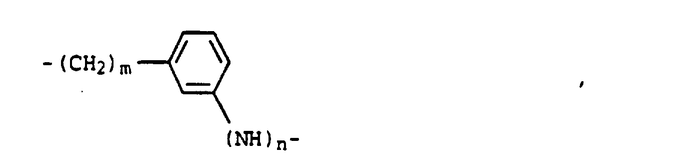 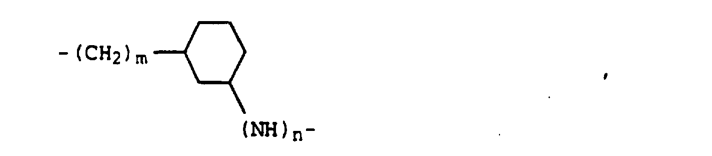 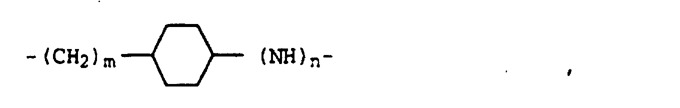 für -(CH₂)ₘ-D-(NH)ₙ- mit m = 0, n = 0,1 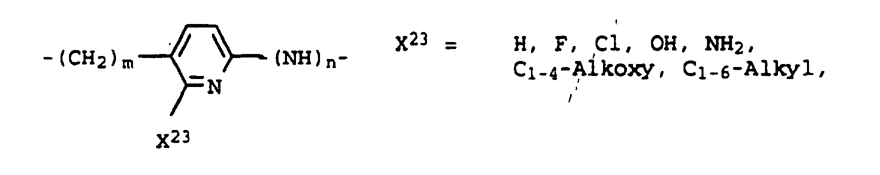 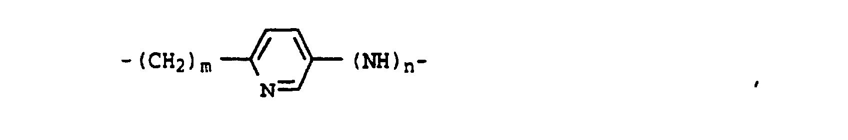 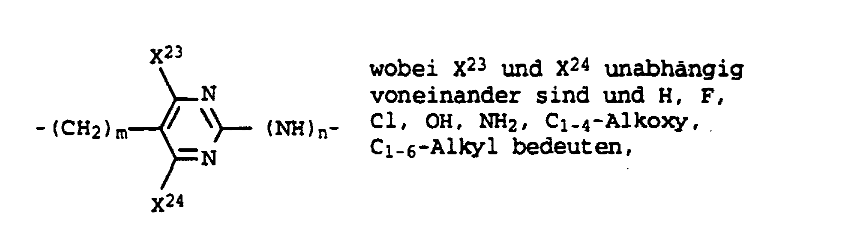 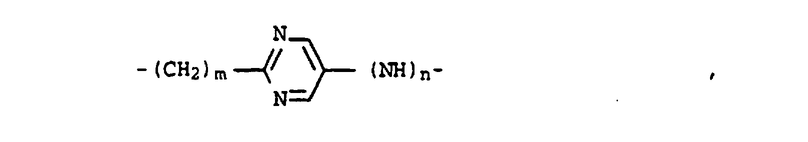 für -(CH₂)ₘ-D-(NH)ₙ- mit m = 0, n = 0: 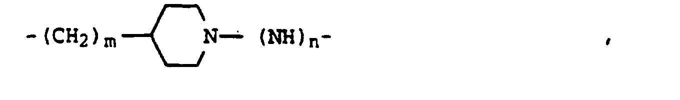 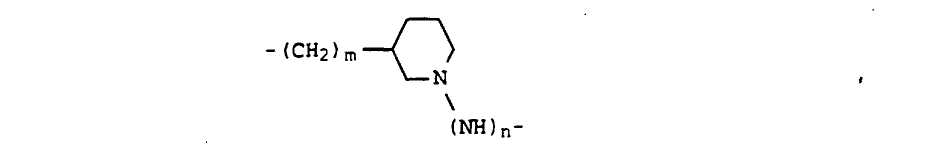 für -(CH₂)ₘ-D-(NH)ₙ- mit m = 0, n = 0

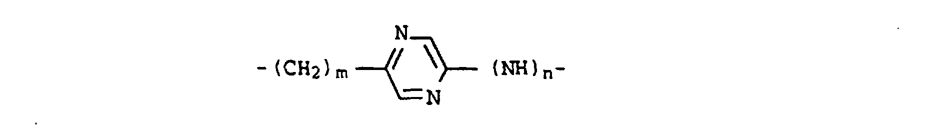

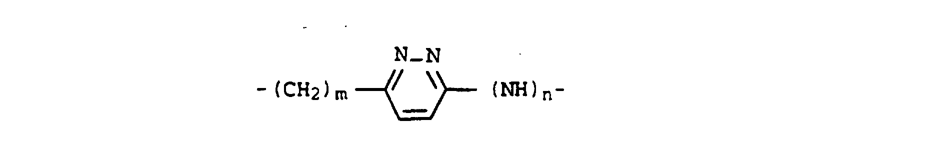

Besonders bevorzugt sind die Verbindungen der Formel I in denen die Substituenten folgende Bedeutung besitzen:
A: 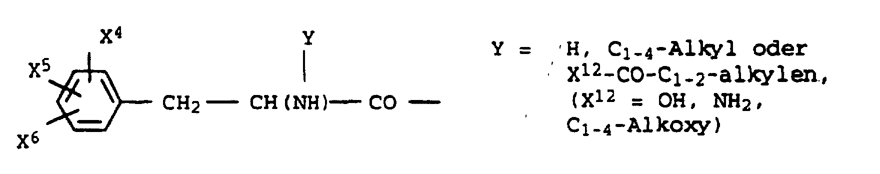
   - X⁴ =: H, F, Cl, CH₃, CF₃, OCH₃ oder Br
   - X⁵ =: H, Cl,, CH₃, oder OCH₃, OC₂H₅
   - X⁶ =: H oder OCH₃, 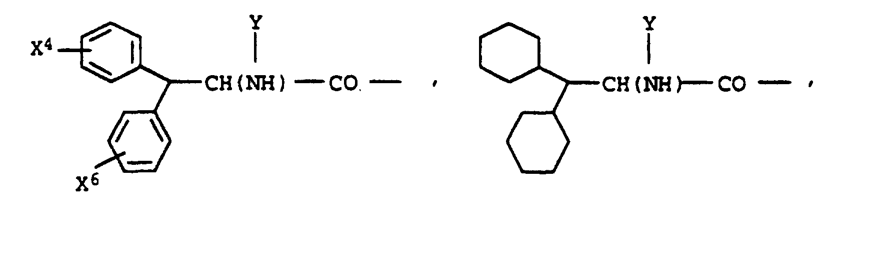 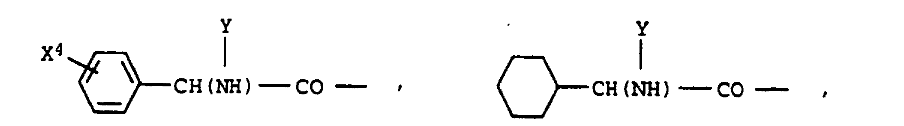 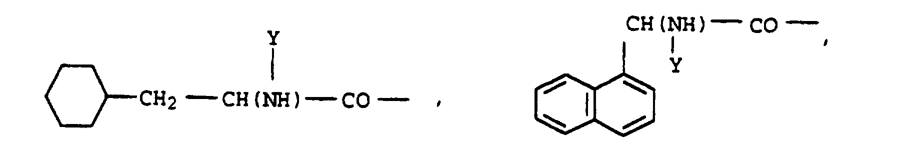 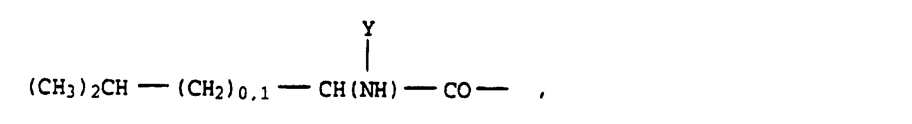 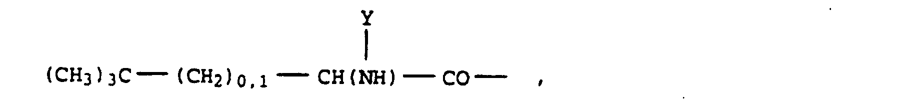 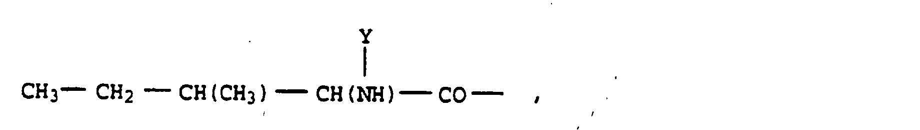 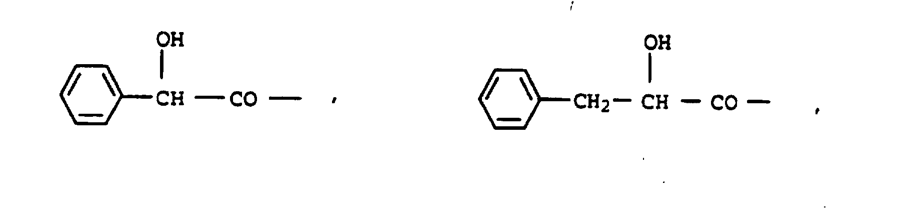
B: 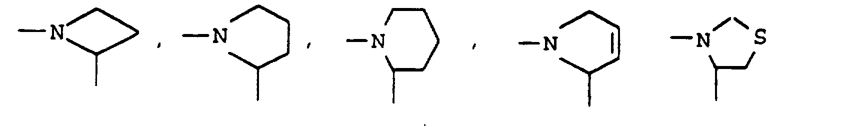
   - R¹ =: H
   - R², R³ =: H
für -(CH₂)ₘ-D-(NH)ₙ- mit m = 0, n = 0 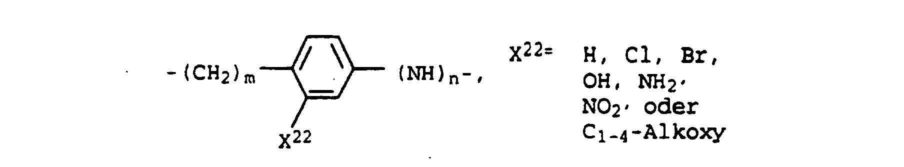 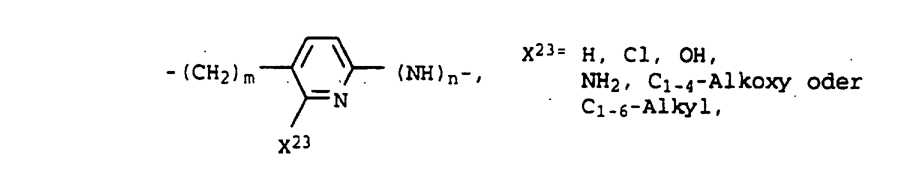 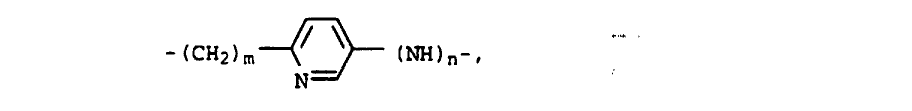 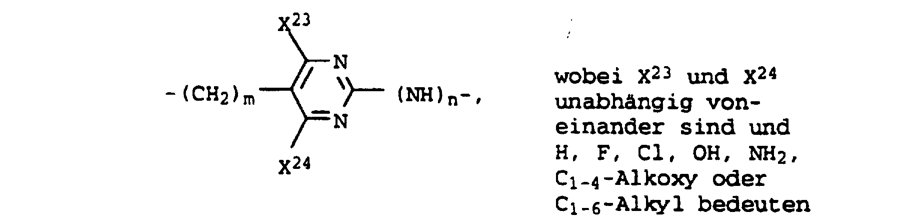 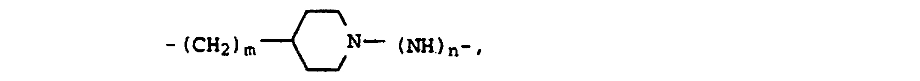 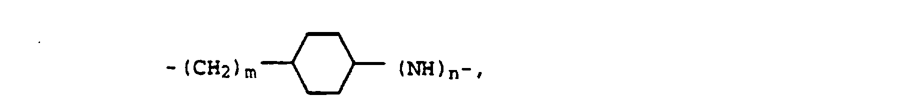 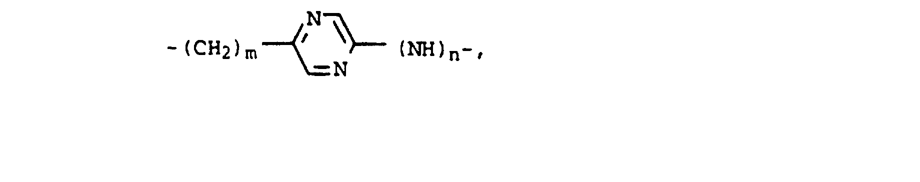 für -(CH₂)ₘ-D-(NH)ₙ- mit m = 0, n = 1 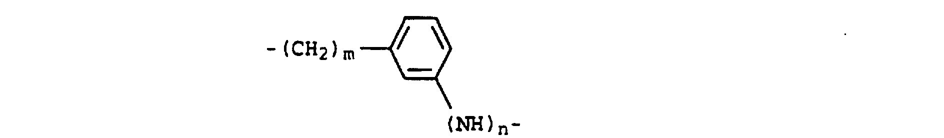 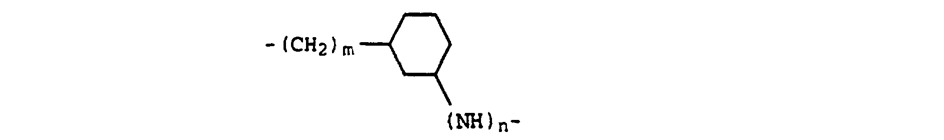 R⁴: H, OH.

Unter den besonders bevorzugten Verbindungen sind folgende Kombinationen hervorzuheben, wobei A und B die als besonders bevorzugt beschriebene Bedeutung besitzen:

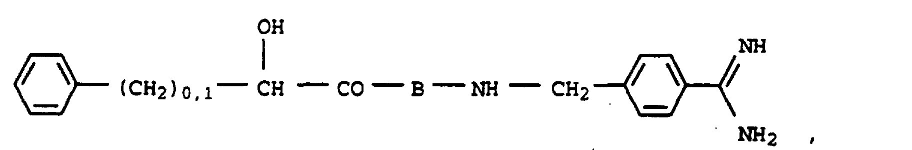

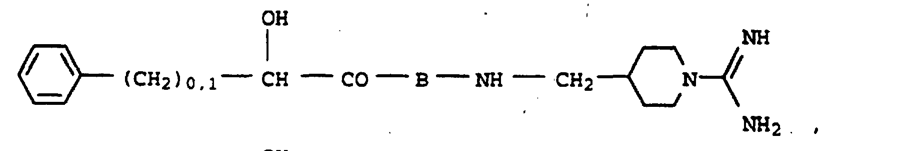

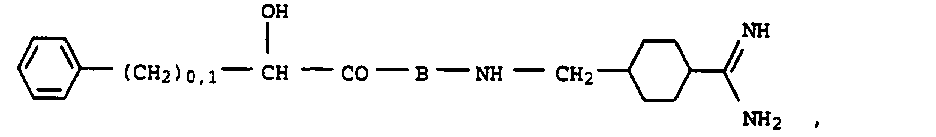

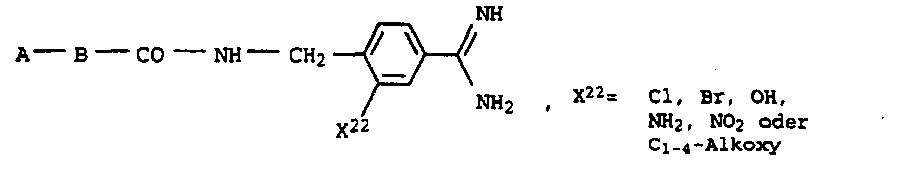

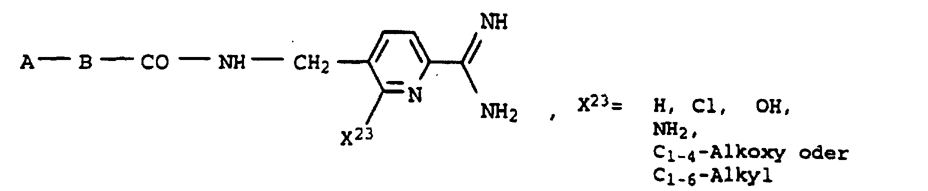

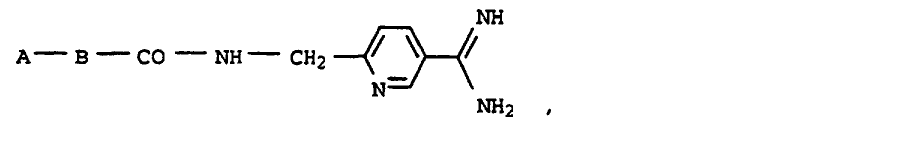

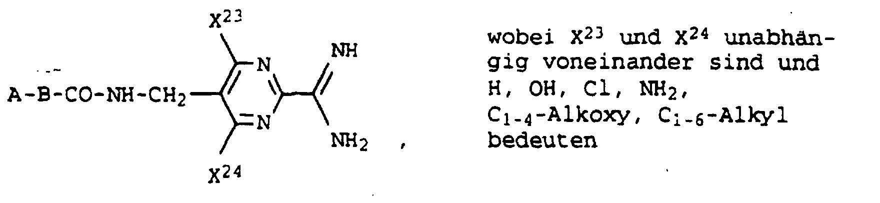

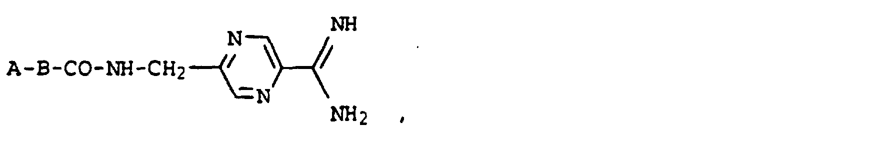

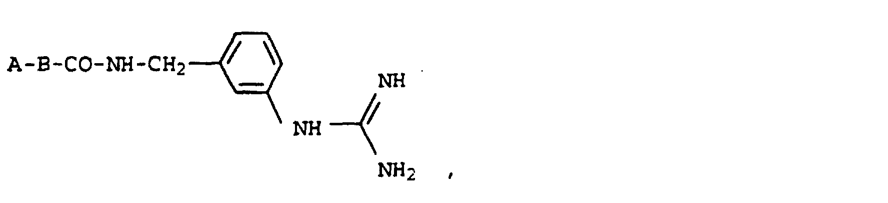

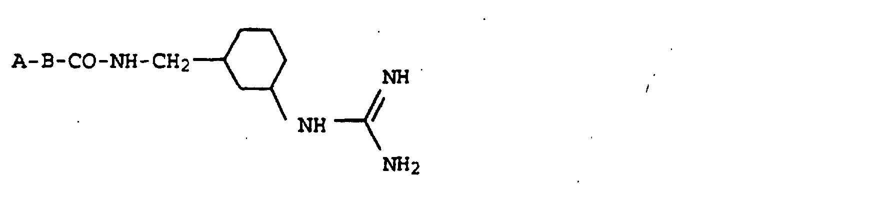

Folgende Substanzen seien beispielhaft genannt:
1. Boc-(D)-Phe-Pro-NH-(4-Am)-2-phenethyl
2. H-(D)-Phe-Pro-NH-(4-Am)-2-phenethyl
3. Boc-Phe-Pro-NH-pAmb
4. H-Phe-Pro-NH-pAmb
5. Boc-(D)-Phe-Pro-NH-pAmb
6. Ac-(D)-Phe-Pro-NH-pAmb
7. H-(D)-Phe-Pro-NH-pAmb
8. H-(D)-Phe-Pro-N(Me)-pAmb
9. Me-(D)-Phe-Pro-NH-pAmb
10. Z-Me-(D)-Phe-Pro-NH-pAmb
11. HOOC-CH₂-(D)-Phe-Pro-NH-pAmb
12. MeOOC-CH₂-(D)-Phe-Pro-NH-pAmb
13. t-BuOOC-CH₂-(Boc)-(D)-Phe-Pro-NH-pAmb
14. EtOOC-(D)-Phe-Pro-NH-pAmb
15. Boc-(D)-Phe-Pro-NH-mAmb
16. H-(D)-Phe-Pro-NH-mAmb
17. Z-(D)-Phe-Pro-(D,L)(4-Am)-PhgOH
18. Z-(D)-Phe-Pro-(D,L)(4-Am)-PhgOMe
19. H-(D)-Phe-Pro-(D,L)(4-Am)-Phg-OH
20. Boc-(D)-Phe-Pro-(4-Am)-PhgCH₂Ph
21. H-(D)-Phe-Pro-(4-Am)-PhgCH₂Ph
22. H-(D)-Phe-Pro-NH-pAm-[(D,L)-α-Me]-benzyl
23. Me-(D)-Phe-Pro-(D bzw. L)(4-Am)-Phgψ[CH₂-OH]/a
24. Me-(D)-Phe-Pro-(D bzw. L)(4-Am)-Phgψ[CH₂-OH]/b
25. Boc-(D)-Phe(4-F)-Pro-NH-pAmb
26. H-(D)-Phe(4-F)-Pro-NH-pAmb
27. Boc-(D)-Phe(4-Cl)-Pro-NH-pAmb
28. H-(D)-Phe(4-Cl)-Pro-NH-pAmb
29. Boc-(D,L)-Phe(4-Br)-Pro-NH-pAmb
30. H-(D,L)-Phe(4-Br)-Pro-NH-pAmb
31. H-(D)-Phe(4-OH)-Pro-NH-pAmb
32. Boc-(D)-Phe(4-MeO)-Pro-NH-pAmb
33. H-(D)-Phe(4-MeO)-Pro-NH-pAmb
34. Boc-(D,L)-Phe(4-EtO)-Pro-NH-pAmb
35. H-(D,L)-Phe(4-EtO)-Pro-NH-pAmb
36. Boc-(D)-Phe(4-BzlO)-Pro-NH-pAmb
37. H-(D)-Phe(4-BzlO)-Pro-NH-pAmb
38. Boc-(D,L)-Phe(4-Et)-Pro-NH-pAmb
39. H-(D,L)-Phe(4-Et)-Pro-NH-pAmb
40. Boc-(D,L)-Phe(4-iPr)-Pro-NH-pAmb
41. H-(D,L)-Phe(4-iPr)-Pro-NH-pAmb
42. Z-(D)-Phe(4-tBuO)-Pro-NH-pAmb
43. H-(D)-Phe(4-tBuO)-Pro-NH-pAmb
44. Boc-(D,L)-Phe(4-tBu)-Pro-NH-pAmb
45. H-(D,L)-Phe(4-tBu)-Pro-NH-pAmb
46. H-(D,L)-Phe(4-Ph)-Pro-NH-pAmb
47. Boc-(D,L)-Phe(4-n-Bu)-Pro-NH-pAmb
48. H-(D,L)-Phe(4-n-Bu)-Pro-NH-pAmb
49. Boc-(D)-Phe(4-COOMe)-Pro-NH-pAmb
50. H-(D)-Phe(4-COOMe)-Pro-NH-pAmb
51. H-(D)-Phe(4-NO₂)-Pro-NH-pAmb
52. Boc-(D,L)-Phe(3-F)-Pro-NH-pAmb
53. H-(D,L)-Phe(3-F)-Pro-NH-pAmb
54. Boc-(D,L)-Phe(3-Cl)-Pro-NH-pAmb
55. H-(D,L)-Phe(3-Cl)-Pro-NH-pAmb
56. H-(D,L)-Phe(3-OH)-Pro-NH-pAmb
57. Boc-(D,L)-Phe(3-MeO)-Pro-NH-pAmb
58. H-(D,L)-Phe(3-MeO)-Pro-NH-pAmb
59. Boc-(D,L)-Phe(3-PhO)-Pro-NH-pAmb
60. H-(D,L)-Phe(3-PhO)-Pro-NH-pAmb
61. Boc-(D,L)-Phe(3-Me)-Pro-NH-pAmb
62. H-(D,L)-Phe(3-Me)-Pro-NH-pAmb
63. H-(D,L)-Phe(3-Ph)-Pro-NH-pAmb
64. Boc-(D,L)-Phe(3-CF₃)-Pro-NH-pAmb
65. H-(D,L)-Phe(3-CF₃)-Pro-NH-pAmb
66. Boc-(D,L)-Phe(2-F)-Pro-NH-pAmb
67. H-(D,L)-Phe(2-F)-Pro-NH-pAmb
68. Boc-(D,L)-Phe(2-Cl)-Pro-NH-pAmb
69. H-(D,L)-Phe(2-Cl)-Pro-NH-pAmb
70. Boc-(D,L)-Phe(2-OH)-Pro-NH-pAmb
71. H-(D,L)-Phe(2-OH)-Pro-NH-pAmb
72. Boc-(D,L)-Phe(2-Meo)-Pro-NH-pAmb
73. H-(D,L)-Phe(2-MeO)-Pro-NH-pAmb
74. Boc-(D,L)-Phe(2-Me)-Pro-NH-pAmb
75. H-(D,L)-Phe(2-Me)-Pro-NH-pAmb
76. Boc-(D,L)-Phe(2-iPr)-Pro-NH-pAmb
77. H-(D,L)-Phe(2-iPr)-Pro-NH-pAmb
78. Boc-(D,L)-Phe(2-Ph)-Pro-NH-pAmb
79. H-(D,L)-Phe(2-Ph)-Pro-NH-pAmb
80. Boc-(D,L)-Phe(3,4-(F)₂)-Pro-NH-pAmb
81. H-(D,L)-Phe(3,4-(F)₂)-Pro-NH-pAmb
82. Boc-(D,L)-Phe(3,4-(Cl)₂)-Pro-NH-pAmb
83. H-(D,L)-Phe(3,4-(Cl)₂)-Pro-NH-pAmb
84. Boc-(D,L)-Phe(3-Cl-4-MeO)-Pro-NH-pAmb
85. H-(D,L)-Phe(3-Cl-4-MeO)-Pro-NH-pAmb
86. Boc-(D,L)-Phe(3-Cl-4-EtO)-Pro-NH-pAmb
87. H-(D,L)-Phe(3-Cl-4-EtO)-Pro-NH-pAmb
88. H-(D,L)-Phe(3,4-(MeO)₂)-Pro-NH-pAmb
89. Boc-(D,L)-Phe(3,4-(Me)₂)-Pro-NH-pAmb
90. H-(D,L)-Phe(3,4-(Me)₂)-Pro-NH-pAmb
91. Boc-(D,L)-Phe(3-Me-4-iPr)-Pro-NH-pAmb
92. H-(D,L)-Phe(3-Me-4-iPr)-Pro-NH-pAmb
93. Boc-(D,L)-Phe(2,3-(MeO)₂)-Pro-NH-pAmb
94. H-(D,L)-Phe(2,3-(MeO)₂)-Pro-NH-pAmb
95. Boc-(D,L)-Phe(2,5-(MeO)₂)-Pro-NH-pAmb
96. H-(D,L)-Phe(2,5-(MeO)₂)-Pro-NH-pAmb
97. Boc-(D,L)-Phe(3,5-(MeO)₂)-Pro-NH-pAmb
98. H-(D,L)-Phe(3,5-(MeO)₂)-Pro-NH-pAmb
99. Boc-(D,L)-Phe(3,4,5-(MeO)₃)-Pro-NH-pAmb
100.H-(D,L)-Phe(3,4,5-(MeO)₃)-Pro-NH-pAmb
101.Hoc-(D,L)-Phe(2,4,6-(Me)₃)-Pro-NH-pAmb
102.H-(D,L)-Phe(2,4,6-(Me)₃)-Pro-NH-pAmb
103.Boc-(D)-αNal-Pro-NH-pAmb
104.H-(D)-αNal-Pro-NH-pAmb
105.H-(D)-ßNal-Pro-NH-pAmb
106.Boc-(D,L)-αNgl-Pro-NH-pAmb
107.H-(D,L)-αNgl-Pro-NH-pAmb
108.Boc-(D,L)-βNgl-Pro-NH-pAmb
109.H-(D,L)-βNgl-Pro-NH-pAmb
110.H-(D,L)-1-Tic-Pro-NH-pAmb
111.Boc-(D)-3-Tic-Pro-NH-pAmb
112.H-(D)-3-Tic-Pro-NH-pAmb
113.1-Icc-Pro-NH-pAmb
114.Boc-(D,L)-2-Tgl-Pro-NH-pAmb
115.H-(D,L)-2-Tgl-Pro-NH-pAmb
116.Boc-(D,L)-2-Tal-Pro-NH-pAmb
117.H-(D,L)-2-Tal-Pro-NH-pAmb
118.Boc-(D)-Phg-Pro-NH-pAmb
119.H-(D)-Phg-Pro-NH-pAmb
120.Boc-(D,L)-Phg(4-MeO)-Pro-NH-pAmb
121.H-(D,L)-Phg(4-MeO)-Pro-NH-pAmb
122.Boc-(D)-Chg-Pro-NH-pAmb
123.H-(D)-Chg-Pro-NH-pAmb
124.EtOOC-(D)-Chg-Pro-NH-pAmb
125.HOOC-CH₂-(D)-Chg-Pro-NH-pAmb
126.tBuOOC-CH₂-(D)-Chg-Pro-NH-pAmb
127.Boc-(D)-Cha-Pro-NH-pAmb
128.Me-(D)-Cha-Pro-NH-pAmb
129.Me-(Z)-(D)-Cha-Pro-NH-pAmb
130.N,N-Me₂-(D)-Cha-Pro-NH-pAmb
131.Boc-(D)-Trp(Boc)-Pro-NH-pAmb
132.H-(D)-Trp-Pro-NH-pAmb
133.Boc-(D,L)-Dpa-Pro-NH-pAmb
134.H-(D bzw. L)-Dpa-Pro-NH-pAmb/a
135.H-(D bzw. L)-Dpa-Pro-NH-pAmb/b
136.EtOOC-(D bzw. L)-Dpa-Pro-NH-pAmb/a
137.EtOOC-(D bzw. L)-Dpa-Pro-NH-pAmb/b
138.HOOC-CH₂-(D bzw. L)-Dpa-Pro-NH-pAmb/a
139.HOOC-CH₂-(D bzw. L)-Dpa-Pro-NH-pAmb/b
140.Boc-(D bzw. L)-Dpa(4,4'-(Cl)₂)-Pro-NH-pAmb/a
141.Boc-(D bzw. L)-Dpa(4,4'-(Cl)₂)-Pro-NH-pAmb/b
142.H-(D bzw. L)-Dpa(4,4'-(Cl)₂)-Pro-NH-pAmb/a
143.H-(D bzw. L)-Dpa(4,4'-(Cl)₂)-Pro-NH-pAmb/b
144.EtOOC-(D bzw. L)-Dpa(4,4'-(Cl)₂)-Pro-NH-pAmb/a
145.EtOOC-(D bzw. L)-Dpa(4,4'-(Cl)₂)-Pro-NH-pAmb/b
146.HOOC-CH₂-(D bzw. L)-Dpa(4,4'-(Cl)₂)-Pro-NH-pAmb/a
147.HOOC-CH₂-(D bzw. L)-Dpa(4,4'-(Cl)₂)-Pro-NH-pAmb/b
148.H-(D bzw. L)-Dch-Pro-NH-pAmb/a
149.H-(D bzw. L)-Dch-Pro-NH-pAmb/b
150.Boc-(D)-Val-Pro-NH-pAmb
151. H-(D)-Val-Pro-NH-pAmb
152. Boc-(D)-Leu-Pro-NH-pAmb
153. H-(D)-Leu-Pro-NH-pAmb
154. Boc-(D)-Gly(α-tBu)-Pro-NH-pAmb
155. H-(D)-Gly(α-tBu)-Pro-NH-pAmb
156. Boc-(D)-Ala(β-tBu)-Pro-NH-pAmb
157. H-(D)-Ala(β-tBu)-Pro-NH-pAmb
158. H-(D bzw. L)-Msu-Pro-NH-pAmb/a
159. H-(D bzw. L)-Msu-Pro-NH-pAmb/b
160. Boc-(Cyclo)Leu-Pro-NH-pAmb
161.H-(Cyclo)Leu-Pro-NH-pAmb
162. Boc-Gly-Pro-NH-pAmb
163. H-Gly-Pro-NH-pAmb
164. Ph-CH₂-CO-Gly-Pro-NH-pAmb
165. Ph-CH₂-CH₂-CO-Gly-Pro-NH-pAmb
166. Ph-CH₂-Gly-Pro-NH-pAmb
167. β-Naphthyl-CH₂-Gly-Pro-NH-pAmb
168. [3,4-(MeO)₂-phenyl]-Ch₂-Gly-Pro-NH-pAmb
169. Ph-CH₂-CO-Pro-NH-pAmb
170. Ph-CH₂-CH₂-CO-Pro-NH-pAmb
171. Ph-CH₂-CH₂-CH₂-CO-Pro-NH-pAmb
172. α-Naphthyl-CO-Pro-NH-pAmb
173. β-Naphthyl-CO-Pro-NH-pAmb
174. α-Naphthyl-CH₂-CO-Pro-NH-pRmb
175. β-Naphthyl-CH₂-CO-Pro-NH-pAmb
176. β-Naphthyl-SO₂-Pro-NH-pAmb
177. p-Tol-SO₂-Pro-NH-pAmb
178. Ph-CH₂-CH₂-SO₂-Pro-NH-pAmb
179. H-Asp-Pro-NH-pAmb
180. Boc-Asp(OMe)-Pro-NH-pAmb
181. H-Asp(OMe)-Pro-NH-pAmb
182.Ph-CH₂-CO-Asp(OMe)-Pro-NH-pAmb
183.Ph-CH₂-CH₂-CO-Asp(OMe)-Pro-NH-pAmb
184.(n-Pr)₂CH-CO-Asp-Pro-NH-pAmb
185.H-Asp(OBzl)-Pro-NH-pAmb
186.(n-Pr)₂CH-CO-Asp(OHzl)-Pro-NH-pAmb
187.Ph-CH₂-CO-Asp-Pro-NH-pAmb
188.Ph-CH₂-CH₂-CO-Asp-Pro-NH-pAmb
189.(n-Pr)₂CH-CO-Asp(OMe)-Pro-NH-pAmb
190.Z-(D)-Asp(OMe)-Pro-NH-pAmb
191.H-(D)-Asp-Pro-NH-pAmb
192. Z-(D)-Asp(OtBu)-Pro-NH-pAmb
193.H-(D)-Asp(OtBu)-Pro-NH-pAmb
194. Boc-(D)-Asp(OBzl)-Pro-NH-pAmb
195.H-(D)-Asp(OBzl)-Pro-NH-pAmb
196. Z-(D)-Glu(OtHu)-Pro-NH-pAmb
197. H-(D)-Glu(OtBu)-Pro-NH-pAmb
198.H-(D)-Glu-Pro-NH-pAmb
199. (D)-Ph-CH₂-CHOH-CO-Pro-NH-pAmb
200. (D)-Man-Pro-NH-pAmb
201. Boc-(D)-Phe-Aze-NH-pAmb
202.H-(D)-Phe-Aze-NH-pAmb
203.Boc-(D)-Phe-(D,L)-Pic-NH-pAmb
204.H-(D)-Phe-(D bzw. L)-Pic-NH-pAmb/a
205.H-(D)-Phe-(D bzw. L)-Pic-NH-pAmb/b
206.Boc-(D)-Phe-(D,L/trans)-Pic(4-Me)-NH-pAmb
207.H-(D)-Phe-(D,L/trans)-Pic(4-Me)-NH-pAmb
208.Boc-(D)-Phe-Pyr-NH-pAmb
209.H-(D)-Phe-Pyr-NH-pAmb
210.Hoc-(D)-Phe-Hyp(O-tBu)-NH-pAmb
211.H-(D)-Phe-Hyp-NH-pAmb
212.Boc-(D)-Phe-(Me)Val-NH-pAmb
213.H-(D)-Phe-(Me)Val-NH-pAmb
214.Boc-(D)-Phe-Val-NH-pAmb
215.H-(D)-Phe-Val-NH-pAmb
216.Boc-(D)-Phe-Tia-NH-pAmb
217.H-(D)-Phe-Tia-NH-pAmb
218.H-(D)-Phe-Pro-NH-3-(6-am)-pico
219.Boc-(D)-Chg-Pro-NH-3-(6-Am)-pico
220.H-(D)-Chg-Pro-NH-3-(6-Am)-pico
221.HOOC-CH₂-(D)-Chg-Pro-NH-3-(6-Am)-pico
222.HOOC-CH₂-(D)-Chg-Pyr-NH-3-(6-Am)-pico
223.HOOC-CH₂-(D)-Chg-2-Phi-NH-3-(6-Am)-pico
224.HOOC-CH(Me)-(D)-Chg-Pro-NH-3-(6-Am)-pico
225.Boc-(D)-Phe-Pro-NH-3-(2-Me-6-Am)-pico
226.H-(D)-Phe-Pro-NH-3-(2-Me-6-Am)-pico
227.Boc-(D)-Chg-Pro-NH-3-(2-Me-6-Am)-pico
228.H-(D)-Chg-Pro-NH-3-(2-Me-6-Am)-pico
229. tBuOOC-CH₂-(D)-Chg-Pro-NH-3-(2-Me-6-Am)-pico
230. HOOC-CH₂-(D)-Chg-Pro-NH-3-(2-Me-6-Am)-pico
231. MeOOC-CH₂-(D)-Chg-Pro-NH-3-(2-Me-6-Am)-pico
232. Boc-(D)-Chg-Pro-NH-2-(5-Am)-pico
233. H-(D)-Chg-Pro-NH-2-(5-Am)-pico
234. HOOC-CH₂-(D)-Chg-Pro-NH-2-(5-Am)-pico
235. HOOC-CH₂-(D)-Chg-Pro-NH-5-(2-Am)-pym
236. (D)-Man-Pro-NH-4-(1-Am)-pip
237. Boc-(D)-Phe-Pro-NH-pHamb
238. H-(D)-Phe-Pro-NH-pHamb
239. Boc-(D)-Phe-Pro-NH-(2-MeO)-pAmb
240. H-(D)-Phe-Pro-NH-(2-MeO)-pAmb
241. Boc-(D)-Phe(4-Meo)-Pro-NH-(2-MeO)-pAmb
242. H-(D)-Phe(4-MeO)-Pro-NH-(2-MeO)-pAmb
243. HOOC-CH₂-(D)-Phe(4-MeO)-Pro-NH-(2-MeO)-pAmb
244. Boc-(D)-Chg-Pro-NH-(2-MeO)-pAmb
245. H-(D)-Chg-Pro-NH-(2-MeO)-pAmb
246. HOOC-CH₂-(D)-Chg-Pro-NH-(2-MeO)-pAmb
247. Boc-(D)-Chg-Aze-NH-(2-MeO)-pAmb
248.H-(D)-Chg-Aze-NH-(2-MeO)-pAmb
249.Boc-(D)-Chg-Pro-NH-(2-iPrO)-pAmb
250.H-(D)-Chg-Pro-NH-(2-iPrO)-pAmb
251.Boc-(D)-Chg-Pro-NH-(2-Cl)-pAmb
252.H-(D)-Chg-Pro-NH-(2-Cl)-pAmb
253.H-(D)-Phe-Pro-(D,L)(4-Am)-PhgOMe
254.Boc-(D,L)-Phe(3-OH)-Pro-NH-pAmb
255.BOC-(D,L)-1-Tic-Pro-NH-pAmb
256.H-(D)-Chg-Pro-NH-3-(2-MeO-6-Am)-pico

Die Verbindungen der Formel I können als solche oder in Form ihrer Salze mit physiologisch verträglichen Säuren vorliegen. Beispiele für solche Säuren sind: Salzsäure, Zitronensäure, Weinsäure, Milchsäure, Phosphorsäure, Methansulfonsäure, Essigsäure, Ameisensäure, Maleinsäure, Fumarsäure, Malonsäure, Bernsteinsäure, Hydroxybernsteinsäure, Schwefelsäure, Glutarsäure, Asparaginsäure, Brenztraubensäure, Benzoesäure, Glucuronsäure, Oxalsäure, Ascorbinsäure und Acetylglycin.

Die neuen Verbindungen lassen sich zur Therapie und Prophylaxe aller Krankheiten verwenden, bei denen Thrombin eine Rolle spielt. Dies sind besonders thromboembolische Erkrankungen wie Myokardinfarkt, periphere arterielle Verschlußkrankheit, tiefe Venenthrombose, Lungenembolie und Schlaganfall. Darüber hinaus können sie zur Verhinderung der Reocclusion nach Öffnung arterieller Gefäße durch mechanische Methoden oder Lyse verwendet werden.

Ferner sind die Substanzen geeignet, die Thrombinbildung durch direkte Hemmung von Kallikrein zu verhindern.

Ihr besonderer Vorteil liegt darin, daß sie auch nach oraler Gabe wirksam sind.

Gegenstand der Erfindung sind weiter folgende Substanzen der Formel II, die wertvolle Zwischenprodukte zur Herstellung der Verbindungen I darstellen: 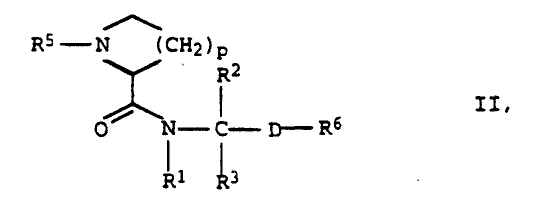 worin R¹, R², R³ und D die für Formel I angegebene Bedeutungen besitzen, und
- R⁵:: H, C₁₋₄-Alkoxy-CO- oder Phenyl-C₁₋₃-alkoxy-CO-,
- R⁶:: ein Cyano-, Amidino- oder Guanidino-Rest in der m- oder p-Stellung zu C(R², R³) und
- p:: 1,2 oder 3
bedeuten.

Die in der Beschreibung und in den Beispielen verwendeten Abkürzungen haben folgende Bedeutungen:
- Ala: = Alanin
- Am: = amidino
- (m bzw. p) Amb: = (meta- bzw. para-)Amidinobenzyl
- Asp: = Asparaginsäure
- Aze: = Azetidin-2-carbonsäure
- Boc: = t-Butyloxycarbonyl
- Bzl: = Benzyl
- Cbz: = Benzyloxycarbonyl
- Cha: = Cyclohexylalanin
- Chg: = Cyclohexylglycin
- DCC: = Dicyclohexylcarbodiimid
- Dch: = Dicyclohexylalanin
- DCM: = Dichlormethan
- DIPEA: = Diisopropylethylamin
- DMF: = Dimethylformamid
- Dpa: = Diphenylalanin
- Dpg: = Diphenylglycin
- EDC: = N'-(3-Dimethylaminopropyl)-N-ethylcarbodiimid
- Glu: = Glutaminsäure
- Gly: = Glycin
- pHamb: = para-Hydroxyamidinobenzyl; (Ham = Hydroxyamidono)
- HOBT: = Hydroxybenzotriazol
- HoSu: = Hydroxysuccinimid
- Hyp: = Hydroxyprolin
- Icc: = Isochinolincarbonsaure
- iPr: = iso-Propyl
- Leu: = Leucin
- Man: = Mandelsäure
- (Me)Val: = N-Methyl-valin
- Msu: = Methioninsulfon
- (α bzw. β)Nal: = ((α- bzw. β-)Naphthylalanin
- NBS: = N-Bromsuccinimid
- Ngl: = Naphthylglycin
- Ph: = Phenyl
- Phe: = Phenylalanin
- Phg: = Phenylglycin
- 2-Phi: = 2-Perhydroindol-carbonsäure
- Pic: = Pipecolinsäure (Piperidin-2-carbonsäure)
- pico: = picolyl 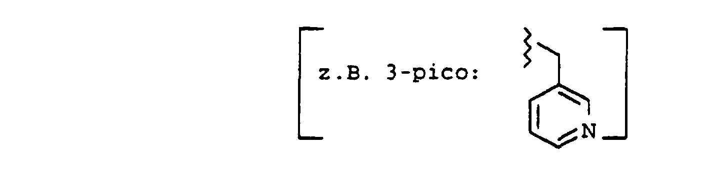
- pip: piperidinyl-methyl 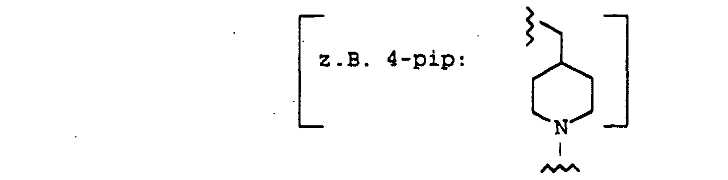
- Pro: = Prolin
- pym: pyrimidyl-methyl 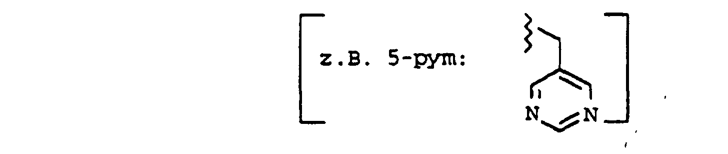
- PyBrop: = Bromo-tris-pyrrolidino-phosphoniumhexafluoro-phosphat
- Pyr: = 3,4-Pyrrolin-2-carbonsäure
- RT: = Raumtemperatur
- Tal: = Thienylalanin
- TBAB: = Tetrabutylammoniumbromid
- tBu: = tertiär-Butyl
- TEA: = Triethylamin
- TEACl: = Tetraethylammoniumchlorid
- TFA: = Trifluoressigsäure
- Tgl: = Thienylglycin
- Tia: = Thiazolidin-4-carbonsäure
- Tic: = Tetrahydroisochinolincarbonsäure
- Tol: = Tolyl
- Trp: = Tryptophan
- Val: = Valin
- Z: = Benzyloxycarbonyl (=Cbz)

### Beispiele:

A. Allgemeine Vorschriften
   A.I. Entfernung und Einführung von Schutzgruppen
      A.I.a. Schutzgruppen werden entsprechend den von Gross und Meienhofer (E. Gross, J. Meienhofer " The Peptides; Analysis, Synthesis, Biology"; 1st. ed. Vol. 3, Academic Press, New York 1981) beschrieben Methoden abgespalten.
      A.I.b. Cbz-Schutzgruppen werden entweder hydrogenolytisch nach Standardreaktionsbedingungen oder mit HF nach der in Stewart, J. M.; Young, J. D. "Solid Phase Peptide Synthesis" 2. Auflage; Pierce Chemical Company 1984) beschriebenen Methode abgespalten.
      A.I.c. Enthält das geschützte Molekül ausschließlich Boc-Schutzgruppen, so werden diese mit HCl/Dioxan bzw. HCl/Methylenchlorid bzw. CF₃COOH/Methylenchlorid nach Standardreaktionsbedingungen abgespalten (siehe Bodansky, M. & Bodansky, A. "The Practice of Peptide Synthesis", Springer-Verlag, 1984).
A.II. Allgemeine Vorschriften zur Hydrolyse von Estergruppen
   A.II.a. 1 mMol des Esters wird in THF (4 ml/mMol) bei 0°C vorgelegt. Anschließend werden 1,2 Eq. LiOH (1M Lösung) zugefügt und das Gemisch über Nacht bei RT gerührt. Nach wäßriger Aufarbeitung erhält man die entsprechende Säure.
   A.II.b. 1 mMol des Esters wird in MeOH (4 ml/mMol) bei 0°C vorgelegt. Anschließend werden 1,2 Eq. LiOH (1M Lösung) zugefügt und das Gemisch über Nacht bei RT gerührt. Nach wäßriger Aufarbeitung erhält man die entsprechende Säure.
   A.II.c. 1 mMol des Esters wird in 2ml 2N HCl über Nacht bei RT gerührt. Das Produkt wird wäßrig aufgearbeitet.
A.III Allgemeine Vorschrift zur Amidierung
   A.III.1. In Anlehnung an eine Vorschrift von Vieweg et al. (H. Vieweg et al. Pharmazie 1984, 39, 226) werden aus Nitrilen die Amidine, N-Hydroxy-amidine und N-Amino-amidine wie folgt dargestellt:

1 Eq. des Nitrils wird in Pyridin/ Triethylamin (10/1; ca. 20-30ml/g Substanz) gelöst. Die Lösung wird dann mit H₂S-Gas gesättigt und über Nacht verschlossen bei RT stehengelassen. Anschließend wird die Reaktionsmischung in salzsaures Eiswasser eingerührt, der entstandene Niederschlag abgesaugt, mit viel Wasser nachgewaschen und dann getrocknet. Die Substanz wird in Aceton (ca. 20-30ml/g Substanz) gelost. Nach Zugabe von MeI (1ml/g Substanz) läßt man die Lösung über Nacht stehen. Durch Zugabe von Diethylether wird das S-Methyl-Thioimidsaureesterhydroiodid gefällt und nochmals zur Reinigung aus MeOH/Diethylether umgefällt.

Das Salz wird in abs. MeOH (ca. 30ml/g Substanz) vorgelegt. Nach Zugabe von Ammoniumacetat (zur Synthese von N-Hydroxyamidinen wird Hydroxylammoniumacetat bz. -chlorid und zur Synthese von N-Amino-Amidinen Hydraziniumacetat bzw. -chlorid verwendet) läßt man das Gemisch über Nacht bei RT rühren. Nach Filtration der Suspension wird ein Teil des Solvens im Vakuum entfernt und das Amidinohydroiodid durch Zugabe von Ether gefällt und abgesaugt. Anschließend wird das Rohprodukt mittels RP-HPLC gereinigt.
A.III.2. Wahlweise wird die Amidierung auch mittels einer Pinner-Reaktion durchgeführt (D. Neilson in Patai The Chemistry of Amidines and Imidates" S.385-489, John Wiley & Sons, New York, 1975; R. Roger, D. Neilson Chem. Rev. 1961, 61, 179; ferner siehe Beispiel 2)
A.III.3 Eine weitere Möglichkeit zur Amidierung besteht in der Umwandlung einer Nitrilgruppe in eine Hydroxyamidingruppe mit Hydroxylaminhydrochlorid und anschließender Hydrierung mit H₂/Raney-Nickel (bzw. H₂/Pd-C) zum Amidin.

10 mMol des Nitrilderivates werden in 100 ml MeOH gelöst und nach Zugabe von 3 Eq. Hydroxylaminhydrochlorid und 4,5 Eq. TEA bis zum vollständigen Umsatz bei Raumtemperatur gerührt. Anschließend wird das Reaktionsgemisch eingeengt und in DCM aufgenommen. Die organische Phase wird mit Wasser (pH 5-6) gewaschen, mit Na₂SO₄ getrocknet und eingeengt.

Der Rückstand wird in 100 ml 5%iger methanolischer HOAc gelöst und nach Zugabe von Raney-Nickel (wahlweise auch Pd/C 10 %) unter einer Wasserstoffatmosphäre hydriert. Nach vollständigem Umsatz des Eduktes wird der Katalysator abfiltriert und das Filtrat eingeengt. Das Produkt wird nach Bedarf entweder mittels einer säulenchromatographischen Trennung über Kieselgel oder einer reversed Phase HPLC Trennung gereinigt.
A.IV Allgemeine Vorschrift zur Guanidierung von Aminen
A.IV.1. Darstellung freier Guanidino-Verbindungen

Freie Guanidinoverbindungen werden ausgehend von den entsprechenden Aminen als Vorläufer analog einer Vorschrift von Miller et al. bzw. Mosher et al. (A. E. Miller, J. J. Bischoff Synthesis 1986, 777; K. Kim, Y.-T. Lin, H. S. Mosher Tetrahedron Letters 1988, 29, 3183) synthetisiert.
A.IV.1.a. 1Eq. K₂CO₃ und 1Eq. Amin werden in 10ml Wasser gelöst vorgelegt. 1Eq. Aminoiminomethansulfonsäure wird dann portionsweise unter starkem Rühren zugegeben. Nach 24stündigem Rühren wird die Reaktionsmischung filtriert. Der abfiltrierte Feststoff ist das Guanidin.
A.IV.1.b. Äquimolare Mengen eines Amins und der Aminoiminomethansulfonsäure werden bei Raumtemperatur in absolutem MeOH (1ml/ mMol) bis zur Bildung einer klaren Lösung gerührt. Dann wird das Lösungsmittel im Vakuum entfernt und das Rohprodukt mittels RP-HPLC gereinigt.
A.IV.2. Darstellung von Alkoxycarbonylguanidinen

Die Umsetzungen zu Alkoxycarbonylguanidinen werden in Analogie zu den folgenden Literaturvorschriften durchgeführt:
1. R. J. Bergeron, J. S. McManis J. Org. Chem. 1987, 52, 1700
2. R. Dubey, S. Abuzar, S. Sharma, R.K. Chatterjee J. Med. Cem. 1985, 28, 1748
3. S. Shawkat, S. Sharma Synthesis 1992, 664
4. A. S. Vendrini, P. Lucietto, G. Fossati, C. Giordani Tetrahedron Lett. 1992, 33, 6541
5. Z. P. Tian, P. Edwards, R. W. Roeske Int. J. Pept. Prot. Res. 1192, 40, 119

A. V. Allgemeine Veresterungsmethoden
A. V. 1. 1 Eq. der Carbonsäure wird zusammen mit 1.1Eq. N'-(3-Dimethylaminopropyl)-N-ethylcarbodiimid- hydrochlorid, 2Eq. Alkohol und katalytischen Mengen Dimethylaminopyridin bei Raumtemperatur über Nacht in Methylenchlorid gerührt. Anschließend wird die Lösung mit Methylenchlorid verdünnt, mit 20%iger NaHSO4-Lsg. extrahiert, getrocknet und im Vakuum eingeengt.
A. V. 2. 1Eq. der Carbonsäure wird mit dem entsprechenden Alkohol und katalytischen Mengen p-Toluolsulfonsäure in Chloroform (wahlweise auch Toluol) zum Sieden erwärmt. Nach vollständigem Umsatz (DC-Kontrolle) wird die Lösung mit ges. NaHCO₃-Lsg. und Kochsalzlösung gewaschen, getrocknet und einrotiert.
B. Allgemeine Synthese-Strategien

Die Darstellung der beanspruchten Verbindungen kann auf mehreren Wegen erfolgen.
1. Ausgehend von entsprechenden geschützten A-Derivaten W-A-OH lassen sich nacheinander nach bekannten Synthesemethoden die Bausteine H-B-COOW' (W'=Alkyl) und H-N(R1)-C(R2R3)- (CH2)m-D-(NH)n-C(NH)NHR4, welche jeweils in geeignet geschützter Form vorliegen, ankuppeln (s. Schema I). Wie in der Peptidchemie üblich, sind zwischen den einzelnen Kupplungsschritten gezielt die Schutzgruppen der Reaktionszentren der nachfolgenden Kupplung abzuspalten. Sämtliche eingesetzten Bausteine sind entweder käuflich oder lassen sich nach bzw. in Analogie zu bekannten Literaturvorschriften synthetisieren.
2. Die Synthesen können auch in umgekehrter Reihenfolge durch Kuppeln von H-N(R1)-C(R2R3)-(CH2)m-D-(NH)n-C(NH)NHR4 (R4 ist eine geegnete Schutzgruppe) mit geeignet geschützten W-B-COOH- und anschließend A-Derivaten W-A-OH erfolgen (s. Schema II)
3. Die Guanidin-, Amidin-, N-Hydroxyamidin- bzw. N-Aminoamidinfunktionen werden entweder in geschützter Form (protoniert, oder mit geeigneten Schutzgruppen versehen) mit dem Baustein H-N(R1)-C(R2R3)-(CH2)m-D-(NH)n-C(NH)NHR4 in die Darstellung der Wirksubstanzen eingeführt und anschließend entschützt, oder aber nach der Kupplung der Bausteine auf der Stufe W-A-B-CO-N(R1)-C(R2R3)-(CH2)m-D-NH2 durch Guanylierung bzw. W-A-B-CO-N(R1)-C(R2R3)-(CH2)m-D-CN durch Amidierung, N-Hydroxyamidierung oder N-Aminamidierung hergestellt. 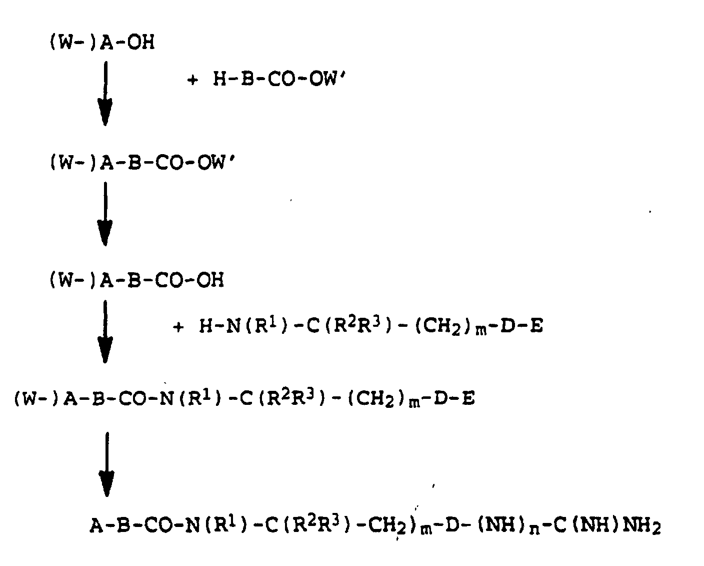 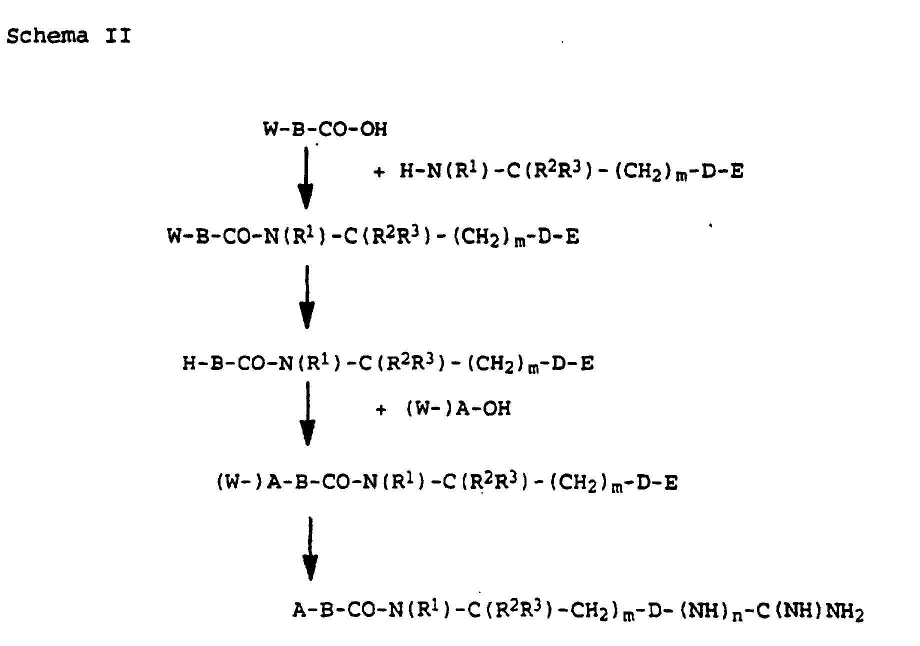 W ist eine der üblichen, N-terminalen Schutzgruppen (vorzugsweise Boc bzw. Cbz) oder eine Hydroxylschutzgruppe und W' bedeutet Methyl, Ethyl, tert. Butyl oder Benzyl.

E bedeutet -CN oder -(NH)ₙ-C(NH)NHR⁴, wobei R⁴ eine Schutzgruppe darstellt. Für die Synthese der Guanidine (n=1) kann E auch -NH-R⁴ bzw. NH₂ bedeuten.

Für die Synthese von N-Amidino-piperidinen (n=O) kann E auch R⁴ bzw. H bedeuten.

Literatur für Peptidchemie:
1. E. Gross, J. Meienhofer " The Peptides; Analysis, Synthesis, Biology"; 1st. ed. Vol. 1; Academic Press, New York 1979
2. E. Gross, J. Meienhofer " The Peptides; Analysis, Synthesis, Biology"; 1st. ed. Vol. 2, Academic Press, New York 1980
3. E. Gross, J. Meienhofer "The Peptides; Analysis, Synthesis, Biology"; ist. ed. Vol. 3, Academic Press, New York 1981
4. M. Deffner, E. Jaeger, P. Stelzel, P. Thamm, G. Wendenberg, E. Wünsch in Houben-Weyl "Methoden der Chemie", 4. Aufl. Vol. XV/1, Herausg. E. Wünsch Georg Thieme Verlag Stuttgart, 1974
5. M. Deffner, E. Jaeger, P. Stelzel, P. Thamm, G. Wendenberg, E. Wünsch in Houben-Weyl "Methoden der Chemie", 4. Aufl. Vol. XV/2, Herausg. E. Wünsch Georg Thieme Verlag Stuttgart, 1974
6. Bodansky, M. & Bodansky, A. "The Practice of Peptide Syn-, thesis", Springer-Verlag, 1984

B.I. Verknüpfung der Bausteine (W-)A-OH und H-B-CO-N(R1)-C(R2R3)- (CH2)m-D-(NH)nC(NH)NHR4 bzw. H-B-CO-N(R1)-C(R2R3)-CH2)m-D-CN entsprechend der allgemeinen Formel I (R1, R2, R3 = H, Alkyl) und Schema II
   Das Hydrochlorid HClxH-B-CO-N(R1)-C(R2R3)-(CH2)m-D-(NH)nC(NH)NHR4 bzw. HClxH-B-CO-N(R1)-C(R2R3)-(CH2)m-D-CN wird zunächst nach Standard-Peptidkupplungsbedingungen (siehe Bodansky, M. & Bodansky, A. "The Practice of Peptide Synthesis", Springer-Verlag, 1984) aus w-B-CO-OH (W = eine Schutzgruppe, vorzugsweise Boc oder Cbz) und dem Amin H-N(R1)-C(R2R3)-(CH2)m-D- (NH)nC(NH)NHR4 bzw. N-N(R1)-C(R2R3)-(CH2)m-D-CN mit anschließender Schutzgruppenabspaltung dargestellt. Das Hydrochlorid wird dann wie folgt zu den Substanzen (entsprechend der allgemeinen Formel) umgesetzt:
   B.I.a. W-A-OH = geschützte Aminosäure (entsprechend der allgemeinen Formel)
      1Eq. der geschützten Aminosäure W-A-OH und 1.1Eq. des Hydrochlorids HClxH-B-CO-N(R1)-C(R2R3)-(CH2)m-D-(NH)nC(NH)NHR4 bzw. HClxH-H-CO-N(R1)-C(R2R3)-(CH2)m-D-CN (ensprechend der allgemeinen Formel I) werden nach Standard-Peptidkupplungsvorschriften zu dem gewünschten Produkt umgesetzt. Falls E = CN ist, wird die Nitrilfunktion entsprechend A.III.1-3 in die Amidino- bzw. Hydroxyamidinogruppe überführt. Anschließend werden vorhandene Schutzgruppen nach Standardmethoden abgespalten.
   B.I.b. A-OH = N-Acyl-AS (AS sind die unter A in der allgemeinen Formel I genannten Aminosäuren Acyl = HOOC-C₁₋₆-Alkylcarbonyl, C₁₋₁₂-Alkylcarbonyl, Phenyl-C₁₋₄-alkylcarbonyl, α- bzw. β-Naphthyl-C₁₋₄-alkylcarbonyl
   B.I.b.1. Zunächst wird eine geschützte Aminosäure W-A-OH wie unter B.I.a. beschrieben an das dort beschriebene Hydrochlorid gekuppelt (R4 muß eine übliche Schutzgruppe sein). Anschließend wird die N-terminale Schutzgruppe der Aminosäure entfernt (die Schutzgruppe muß orthogonal zu R4 abspaltbar sein) und diese mit Carbonsäuren AcylOH (entsprechend der allgemeinen Formel) unter Standard-Peptidkupplungsbedingungen zu dem gewünschten Produkt umgesetzt. Zur Freisetzung der Amidino- , N-Amino-Amidino-, N-Hydroxy-Amidino oder Guanidinoguppe wird diese (falls gewünscht) unter Standardbedingungen abgespalten (siehe Kap. A.I.). Falls E = CN ist, wird die Nitrilfunktion entsprechend A.III.1-3 in die Amidino- bzw. Hydroxyamidinogruppe überführt.
   B.I.b.2. Zunächst wird die N-terminale Aminosäure H-A-OCH3 am N-Terminus mit einer Carbonsäure AcylOH (entsprechend der allgemeinen Formel I) unter Standard-Peptidkupplungsbedingungen zu dem N - acylierten Aminosäureester umgesetzt und anschließend die Estergruppe hydrolysiert. Dann wird die acylierte Aminosäure wie unter B.I.b.1 beschrieben mit dem Hydrochlorid HClxH-B-CO-N(R1)-C(R2R3)-(CH2)m-D-(NH)nC(NH)NHR4 bzw. HClxH-B-CO-N(R1)-C(R2R3)-CH2)m-D-CN unter Standardbedingungen gekuppelt.
      Falls E = CN ist, wird die Nitrilfunktion entsprechend A.III.1-3 in die Amidino- bzw. Hydroxyamidinogruppe überführt. Abschließend werden die Schutzgruppen abgespalten.
   B.I.c. A-OH = N-Alkyl-AS (AS sind die für A in der allgemeinen Formel I beschriebenen Aminosauren, Alkyl = C₁₋₁₂-Alkyl, Phenyl-C₁₋₄-alkylen, HOOC-C₁₋₆-alkylen, α-bzw. β-Naphthyl-C₁₋₄-alkylen)

Ein Syntheseweg (Syntheseweg 1) ist der Aufbau des alkylierten Bausteins A-OH (bzw. A-B-CO-OH) mit anschließender Kupplung des Bausteins H-B-CO-N(R1)-C(R2R3)-(CH2)m-D-(NH)nC(NH)NHR4 (bzw. H-N(R1)-C(R2R3)-(CH2)m-D-(NH)nC(NH)NHR4) bzw. H-B-CO-N(R1)-C(R2R3)-(CH2)m-D-CN (bzw. H-N(R1)-C(R2R3)-(CH2)m-D-CN). Ein alternativer Weg (Syntheseweg 2) ist die Synthese des Bausteins H-AS-B-CO-N(R1)-C(R2R3)-(CH2)m-D-(NH)nC(NH)NHR4 bzw. H-AS-B-CO-N(R1)-C(R2R3)-(CH2)m-D-CN mit anschließender N-terminaler Alkylierung. Eine vorhandene Amidino- bzw. Guanidinogruppe muß geeignet geschützt sein.
Falls E = CN ist, wird die Nitrilfunktion entsprechend A.III.1-3 in die Amidino- bzw. Hydroxyamidinogruppe überführt.

### Syntheseweg 2:

B.I.c.1 1 mmol H-AS-B-CO-N(R1)-C(R2R3)-(CH2)m-D-(NH)nC(NH)NHR4 bzw. H-AS-B-CO-N(R1)-C(R2R3)-(CH2)m-D-CN werden in MeOH (10ml) gelöst. Nach Zugabe von TEACl (1mmol), NaBH₃CN (0,7mmol) und RCHO (1,05mmol) wird das Reaktionsgemisch über Nacht gerührt. Das Lösungsmittel wird im Vakuum entfernt und der Rückstand in Essigester aufgenommen. Die organische Phase wird mit Wasser (2x) und ges. Kochsalzlösung (1x) gewaschen und mit Na₂SO₄ getrocknet. Nach Entfernung des Lösungsmittels wird das Rohprodukt mittels RP-HPLC gereinigt.
   Falls E = CN ist, wird die Nitrilfunktion entsprechend A.III.1-3 in die Amidino- bzw. Hydroxyamidinogruppe überführt
B.I.c.2 1mmol H-AS-B-CO-N(R1)-C(R2R3)-(CH2)m-D-(NH)nC(NH)NHR4 bzw. H-AS-B-CO(R1)-C(R2R3)-(CH2)m-D-CN werden in Acetonitril zusammen mit K₂CO₃ (2.5Eq.) vorgelegt. Nach Zugabe des Alkylierungsreagenzes wird das Reaktionsgemisch bis zu vollstandigem Umsatz des Eduktes bei 60°C gerührt. Nach Abkühlen arbeitet man wäßrig auf und reinigt das Produkt mittels RP-HPLC.
   Falls E = CN ist, wird die Nitrilfunktion entsprechend A.III.1-3 in die Amidino- bzw. Hydroxyamidinogruppe überführt.

### Syntheseweg 1:

Die Darstellung des alkylierten Bausteins A-OH (A = N-Alkyl-AS) erfolgt analog der Literaturvorschrift von G. Iwasaki et al. (G. Iwasaki et al. Chem. Pharm. Bull 1989, 37, 280 und Chem. Lett. 1988, 1691). Die folgende Vorschrift lehnt sich an diese Literaturvorschrift an:
B.I.c.3. 1.5Eq. H-AS-OCH₃oder H-AS-B-COOCH₃ werden zusammen mit 1Eq. Alkylierungsreagenz und 2Eq. Ammoniumcarbonat in Nitromethan/Wasser 4Tage bei 60°C gerührt. Der Ansatz wird wässrig aufgearbeitet und das Produkt mittels Chromatographie gereinigt. Nach Schützen der N-Alkylaminofunktion mittels geeigneter Schutzgruppe wird die Aminosäureesterfunktion verseift und das Produkt ananlog B.I.a. umgesetzt.
B.I.c.4. Die Darstellung des N-Alkyl-AS-OCH₃ oder N-Alkyl-AS-B-COOCH₃ Bausteins kann auch mittels reduktiver Aminierung aus H-AS-OCH₃ oder H-AS-B-COOCH₃ und Aldehyd erfolgen.
B.I.d. A-OH = N-subst. Aminocarbonyl-AS (AS sind die unter A in der allgemeinen Formel beschriebenen Aminosäuren; subst. Aminocarbonyl = C₁₋₆-Alkylaminocarbonyl, Phenyl-C₁₋₄-alkylaminocarbonyl)
   1Eq. H-AS-B-CO-N(R1)-C(R2R3)-(CH2)m-D-(NH)nC(NH)NHR4 bzw. H-AS-B-CO-N(R1)-C(R2R3)-(CH2)m-D-CN wird mit verschiedenen Isocyanaten unter Standardreaktionsbedingungen (Arnold et al. Chem. Rev. 1957, 57, 47) zu den entsprechenden Harnstoffderivaten umgesetzt. Falls E = CN ist, wird die Nitrilfunktion entsprechend A.III.1-3 in die Amidino- bzw. Hydroxyamidinogruppe überführt.
B.I.e. A = X¹⁵-(CH₂)_{f}-SO2 (X¹⁵ und f entsprechen den in der allgemeinen Formel I unter A beschriebenen Variationen)
1.1Eq. des Hydrochlorids HClxH-B-CO-N(R1)-C(R2R3)-(CH2)m-D-(NH)nC(NH)NHR4 bzw. HClxH-B-CON(R1)-C(R2R3)-(CH2)m-D-CN wird zusammen mit 1.5Eq. Diisopropylethylamin, katalytischen Mengen Dimethylaminopyridin und 1Eq. eines substituierten Sulfonylchlorids in Methylenchlorid umgesetzt. Nach wäßriger Aufarbeitung wird das Rohprodukt mittels RP-HPLC gereinigt.
Falls E = CN ist, wird die Nitrilfunktion entsprechend A.III.1-3 in die Amidino- bzw. Hydroxyamidinogruppe überführt.
B.II. Verknüpfung des Amins H-N(R1)-C(R2R3)-(CH2)m-D-(NH)nC(NH)NHR4 bzw. H-N(R1)-C(R2R3)-(CH2)m-D-CN mit dem Baustein A-B-CO-OH (entsprechend der allgemeinen Formel I und Schema I.

Der Baustein A-B-CO-OH wird zunächst analog den allgemeinen Vorschriften in Kap. B.I. synthetisiert. Anschließend wird das Amin H-N(R1)-C(R2R3)-(CH2)m-D-(NH)nC(NH)NHR4 wie in den folgenden Beschreibungen mit dem Baustein A-B-CO-OH gekuppelt:
B.II.a. R1 = H, R2, R3 = H, C₁₋₄Alkyl, Phenyl, Phenyl-C₁₋₄-alkylen, n = 0
B.II.a.1 Der (falls nötig geschützte) Baustein A-B-CO-OH wird unter Standard-Peptidkupplungsbedingungen mit dem Amin H-N(R1)-C(R2R3)-(CH2)m-D-(NH)nC(NH)NHR4 (R4 = -CO-C₁₋₂₀-Alkyl, -CO-O-C₁₋₂₀-Alkyl) umgesetzt. Nach wäßriger Aufarbeitung wird das Rohprodukt mittels RP-HPLC gereinigt. Substanzen mit R4 = H können durch Standard-Entschützungsmethoden erhalten werden.
B.II.a.2. Der (falls nötig geschützte) Baustein A-B-COOH wird unter Standard-Peptidkupplungsbedingungen mit dem Amin H-N(R1)-C(R2R3)-(CH2)m-D-CN (R1, R2, R3, D, m entspricht der allgemeinen Formel) umgesetzt. Nach wäßriger Aufarbeitung wird das Rohprodukt mittels RP-HPLC gereinigt. Das erhaltene Nitril wird wie unter Kap. A. III. beschrieben in das Amidin (R4 = H), N-Hydroxy-Amidin (R4 = OH), N-Amino-Amidin (R4 = NH2) überführt.
B.II.b. R1 = H; R2 = H, C₁₋₄-Alkyl, R3 = H, C₁₋₄Alkyl, Phenyl, Phenyl-C₁₋₄-alkylen, n = 1
B.II.b.1 Der (falls nötig geschützte) Baustein A-B-CO-OH wird unter Standard-Peptidkupplungsbedingungen mit dem Amin H-N(R1)-C(R2R3)-(CH2)m-D-(NH)nC(NH)NHR4 (R4 = -CO-C₁₋₂₀-Alkyl, -CO-O-C₁₋₂₀-Alkyl) umgesetzt. Nach wäßriger Aufarbeitung wird das Rohprodukt mittels RP-HPLC gereinigt. Substanzen mit R4= H können durch Standard Entschützungsmethoden erhalten werden.
B.II.b.2. Der (falls nötig geschützte) Baustein A-B-COOH wird unter Standard-Peptidkupplungsbedingungen mit dem Amin H-N(R1)-C(R2R3)-(CH2)m-D-NHW (W ist eine zu gleichzeitig vorhandenen Schutzgruppen orthogonale Schutzgruppe) umgesetzt. Nach wäßriger Aufarbeitung wird das Rohprodukt mittels RP-HPLC gereinigt. Das erhaltene geschützte Amin wird mittels Standard Entschützungsmethoden freigesetzt und wie unter Kap. A. IV. beschrieben in das Guanidin (R4 = H) überführt.
B.II.c. R1 = C₁₋₄-Alkyl

Diese Substanzen werden wie unter B.II.b. beschrieben dargestellt. Die Kupplungsreaktion erfolgt mit den Kupplungsreagenzien Pivaloylchlorid, PyBrop (Castro et al. Tetrahedron Lett. 1990, 31,669; Castro et al. Tetrahedron Lett. 1991, 32, 1967 ; E. Frerot et al. Tetrahedron 1990, 47, 259) oder BOPCl (M.J.O. Anteunis Int. J. Pept. Prot. Res. 1987, 29, 574).
B.II.d. R3 = -CO-X²⁰ bzw. CO-CO-X²⁰ entspricht der allgemeinen Formel)
B.II.d.1 R3 = -CO-X²⁰ (X²⁰ = OH, Alkoxy, Aryloxy, Aralkoxy)

Der Baustein A-B-CO-N(R1)-CR2(COON)-(CH2)m-D-(NH)nC(NH)NHR4 bzw. A-B-CO-N(R1)-C(R2(COOH)-CH2)m-D-CN wird nach Standard-Peptidkupplungsmethoden synthetisiert (vgl. Kap. B. II.a.1.). Für Produkte mit X²⁰ = Alkoxy, Aryloxy oder Aralkoxy wird die C-terminale Säure mittels Standard-Veresterungsmethoden (siehe A. VI.) zu den entsprechenden Estern umgesetzt. Falls D noch die Nitrilfunktion trägt, wird diese entsprechend AIII1-3 in die Amidino bzw. Hydroxyamidinogruppe überführt.
B.II.d.2. R3 = -CO-X²⁰ (X²⁰ = Alkyl, Aryl, Aralkyl, CF3, C2F5)

Der Baustein A-B-CO-N(R1)-CR2(COOH)-(CH2)m-D-(NH)nC(NH)NHR4 wird wie in B.II.d.1 beschrieben dargestellt. Die COX²⁰-Funktion wird anschließend in einer Dakin-West Reaktion unter Standardreaktionsbedingungen (W. Steglich, G. Höfle Angew. Chem. internat. Ed. 1969, 8, 981; w. Steglich, G. Höfle Chem. Ber. 1969, 102, 883) dargestellt. R⁴ muß für diese Umsetzung eine Alkoxycarbonylgruppe sein (vorzugsweise Boc oder Cbz). Alternativ dazu kann auch A-B-CO-N(R1)-CR2-COOH-(CH2)m-D-CN in der Dakin-West Reaktion eingesetzt werden. In diesem Fall wird anschließend die Nitrilfunktion entsprechend A.III.1-3 in die Amidino- bzw. Hydroxyamidinogruppe überführt.

Eine allgemeine Versuchsvorschrift für die Dakin-West Reaktion lautet wie folgt:

1Eq. des N-terminal geschützten Tripeptids wird zusammen mit 2.5Eq. Triethylamin, 5Eq. Anhydrid ([X²⁰-C(O)-O-(O)C-X²⁰), entsprechend der allgemeinen Formel I) und 0.1Eq. Dimethylaminopyridin bei 50-60°C gerührt bis keine CO₂-Entwicklung mehr zu beobachten ist. Hierauf wird das Reaktionsgemisch mit ges. Na₂CO₃-Lsg. bei 60°C für 2h gerührt. Das Gemisch wird zwischen Essigester und ges. NaHCO₃-Lsg. verteilt und die organische Phase anschließend mit ges. NaHCO3-Lsg. (2x) und 20% NaHSO₄-Lsg. extrahiert, mit Na₂SO₄ getrocknet und einrotiert. Das Rohprodukt wird mittels RP-HPLC gereinigt.
B.II.d.3. R3 = -CO-X²⁰ (X²⁰ = natürliche Aminosäure)

Der Baustein A-B-CO-N(R1)-CR2(COOH)-(CH2)m-D-(NH)nC(NH)NHR4 wird wie in B.II.d.1 beschrieben dargestellt. R4 muß für die folgende Umsetzung eine Alkoxycarbonylgruppe sein (vorzugsweise Boc oder Cbz). Alternativ dazu kann auch A-B-CO-N(R1)-CR2(COOH)-(CH2)m-D-CN in die nachfolgende Reaktion eingesetzt werden. Diese Bausteine werden anschließend unter Standard-Peptidkupplungsbedingungen mit einer C-terminal geschützten Aminosäure umgesetzt. Die gewünschten Produkte werden abschließend durch die Entfernung der Schutzgruppen freigesetzt (analog A.I. und A.II.), bzw. die Nitrilfunktion entsprechend A.III.1-3 in die Amidino-bzw. Hydroxyamidinogruppe überführt.
B.II.d.4. R3 = -CO-CO-X20

Die Vorgehensweise zum Aufbau dieser Substanzen ergibt sich aus der PCT-Anmeldung WO 94/08941. Entsprechend dieser Vorschrift wird zunächst der Baustein W-N(R1)-CR2(CO-CO-X20)-D-(NH)n-C(NH)NHR4 (W ist eine geeignete Schutzgruppe und muß orthogonal zu R4 sein) wie folgt synthetisiert:

Eine N-terminal geschützte Aminosäure W-NH-CR2(COOH)-(CH2)m-D-(NH)nC(NH)NHR4 (R4 muß eine zu W orthogonale Schutzgruppe sein) wird zunächst in das Cyanhydrin W-NH-C(U)(R²)-CH(OH)-CN (mit U = -(CH2)m-D-(NH)nC(NH)NHR4) überführt.

Anschließend wird die Nitril- in eine Carboxylfunktion überführt, die Carboxylgruppe unter geeigneten Veresterungsbedingungen (siehe A.V.) verestert, die N-terminale Aminoschutzgruppe W abgespalten und das erhaltene Amin mit dem Baustein A-B-CO-OH gekuppelt.
a. Für die Bildung von Produkten mit X²⁰ = C₁₋₄-Alkoxy, Phenyl-C₁₋₄-alkoxy wird die Gruppe X20 durch Umesterung eingeführt.
b. Für die Bildung von Produkten mit X²⁰ = Aminosäure folgt die C-terminale Esterhydrolyse mit anschließender Aminosäurekupplung.
c. Für die Bildung von Produkten mit X²⁰ = H, C₁₋₄-Alkyl, Phenyl, Phenyl-C₁₋₄-alkylen, -CF₃, -C₂F₅ wird die C-terminale Estergruppe zunächst hydrolysiert, dann in das Weinreb-Amid überführt und anschließend mit den X²⁰ entsprechenden Nukleophilen umgesetzt.

Die so gewonnene Baustein A-B-CO-NR1-C(U)(R²)-CH(OH)-C(O)-X²⁰ wird mittels Swern-Oxidationsbedingungen zum Ketoamid oxidiert. Abschließend werden noch vorhandene Schutzgruppen unter Standardbedingungen abgespalten.
C. Darstellung von Edukten
C.1. Darstellung von Boc-(D)PheOSu

1 Eq. Boc-(D)Phe-OH wurde mit 1.05 Eq. Hydroxysuccinimid und 1.05 Eq. Dicyclohexylcarbodiimid über Nacht bei RT in Acetonitril (2.5ml/mmol) gerührt. Anschließend wurde die Suspension filtriert und das Filtrat am Rotationsverdampfer eingeengt. Es verblieb das Produkt mit nahezu quantitativer Ausbeute.
C.2. Darstellung von Boc-(D,L)Dpa-OH

Boc-(D,L)Dpa-OH wurde entsprechend der Vorschrift von Kakkar et al. (L. Cheng, C. A. Goodwin, M. F. Schully, V. V. Kakkar J. Med. Chem. 1992, 35, 3364) dargestellt.
C.3. Darstellung von Boc-(D,L)Dch-OH

Boc-(D,L)Dpa-OH (1mmol) wurde in 12ml MeOH zusammen mit katalytischen Mengen von 5% Rh/Al₂O₃ bei 5bar hydriert. Nach Filtration und Entfernen des Solvens im Vakuum erhielt man das Produkt in quantitativer Ausbeute.
C.4. Darstellung von Boc-1-(D,L)Tic-OH

Boc-1(D,L)Tic-OH wurde nach einer Vorschrift von R. T. Shuman et al. dargestellt (R. T. Shuman et al. J. Med. Chem. 1993, 36, 314).
C.5. Darstellung von Cbz-(D)PhePro-OSu
   i. 30g Cbz-(D)PheOH, 11.54g Hydroxysuccinimid, 20.68g Dicyclohexylcarbodiimid und 300ml Dimethoxyethan wurden bei Raumtemperatur über Nacht gerührt. Anschließend wurde die Suspension filtriert, das Filtrat einrotiert und der Rückstand in 200ml Acetonitril gelõst. Der ausgefallene Dicyclohexylharnstoff wurde abfiltriert und das Filtrat eingeengt. Es verblieben 40g des Succinimidesters (weißer Feststoff).
   ii. 40g Cbz-(D)PheOSu, 17.31g Prolin, 12.63g NaHCO3, 225ml Wasser und 225ml Dimethoxyethan wurden bei Raumtemperatur über Nacht gerührt (Gasentwicklung). Anschließend wurde Dimethoxyethan im Vakuum entfernt und die verbliebene wäßrige Lösung mit 1N HCl auf pH 2 eingestellt. Das abgeschiedene Öl wurde mit Methylenchlorid extrahiert. Die vereinten Methylenchlorid-Extrakte wurden mit ges. Kochsalzlösung gewaschen und nach Trocknung mit Na2SO4 einrotiert. Es verblieben 39.7g Cbz-(D)PheProOH (weißer Feststoff).
   iii.39.7g Cbz-(D)PheProOH, 11.53g Hydroxysuccinimid, 20.66g Dicyclohexylcarbodiimid und 400ml Dimethoxyethan wurden bei Raumtemperatur über Nacht gerührt. Am nächsten Tag wurde Dicyclohexylharnstoff abfiltriert, das Filtrat eingeengt und der Rückstand in 300ml Acetonitril aufgenommen. Nochmals ausgefallener Dicyclohexylharnstoff wurde abfiltriert und das Filtrat im Vakuum eingeengt. Es verblieben 48.92g des Hydoxysuccinimid-Esters.
C.6 Darstellung von Boc-geschützten Phenylalaninderivaten

Sofern die Aminosäuren H-A-OH bzw. Boc-A-OH nicht käuflich waren wurden sie in Analogie zu bekannten Literaturvorschriften hergestellt (Übersicht: Houben-Weyl, Band E 16d/Teil 1 S 406 ff)

Häufig eingesetzte Edukte für die Alaninderivate waren Benzophenoniminoessigsäureethylester, Acetamidomalonsäurediethylester und Isonitrilessigsäureethylester.

### Beispielhaft sei erwähnt

1 Eq. Diphenylglycinimin, 3 Eq. K₂CO₃ und das entsprechende Benzylbromid (wahlweise auch -chlorid bzw. iodid) wurden in Acetonitril über Nacht zum Sieden erhitzt. Nach Abkühlen wurde das Reaktionsgemisch filtriert und das Filtrat eingeengt. Dann wurde der Rückstand in 1N HCl bis zur vollständigen Spaltung des Imins gerührt. Anschließend wurde die wäßrige Phase mit Essigester extrahiert, mit Na₂CO₃ basisch gestellt und mit Essigester extrahiert. Die vereinten organischen Extrakte wurden mit Na2SO4 getrocknet und einrotiert. Der Rückstand, das entsprechende Phenylalaninderivat, wurde unter Standardbedingungen mit einer Schutzgruppe N-terminal geschützt (vorzugsweise Boc oder Cbz).
C.7 Darstellung von Boc-geschützten Glycinderivaten
   Die Darstellung verschiedener Glycinderivate erfolgte z.B. ausgehend von Isonitrilessigsäureethylester und einem entsprechenden Keton (siehe H.-J. Prätorius, J. Flossdorf, M.-R.Kula Chem.Ber. 195, 108, 3079).
   Boc-Suberylglycin wurde analog der Literatur (O.P. Goel et al. Tetrahedron Lett. 1993, 34, 953) synthetisiert.
   Boc-(3-Ph)-Pro-OH wurde analog einer Vorschrift von J.Y.L. Chung et al. (J.Y.L.Chung et al. J.Org.Chem. 1990, 55, 270) synthetisiert.
   Tetralinylglycin wurde ausgehend von 1,2-Dihydronaphthalin dargestellt, 1,2-Dihydronaphthalin wurde zunächst mit HBr in 1-Tetralylbromid überführt (analog J.Med.Chem. 1994, 37, 1586). Anschließend wurde das Bromid mit Acetamidomalonsäurediethylester umgesetzt, hydrolytisch gespalten und die erhaltene α-Aminosäure unter Standardbedingungen in die Boc-geschützte Form überführt.
C.8 Darstellung von Boc-(D)-(α-Methyl)-Cha-OH
   Boc-(D)-(α-Methyl)-Cha-OH wurde durch Hydrierung von (D)-(α-Methyl)-Phe-OH und anschließende Einführung der Boc-Schutzgruppe hergestellt. Weitere Möglichkeiten zur Synthese α-substituierter Aminosäuren sind die Bucherer Synthese ausgehend von Ketonen sowie die α-Alkylierung von α-Aminosäuren.
C.9 Darstellung von Hydroxyessigsäurederivaten
   Hydroxyessigsäurederivate wurden entweder analog S.Bajusz (WO93/18060) oder ausgehend von entsprechenden Essigsäuremethylesterderivaten durch α-Hydroxylierung mittels Davis-Reagenz (F.A.Davis, L.C. Vishwakarma, J.M.Billmers J.Org.Chem. 1984, 49, 3241) dargestellt.
C.10. p-(2-Aminoethyl)-benzonitril:
   Die Herstellung erfolgte nach EP 445796
C.11. p-Cyanobenzylamin:
C.11.a. 200 g 4-Cyanobenzylbromid (1.02 Mol), 700 ml Toluol, 200 g Natriumazid (3.07 Mol), 32.9 g TBAB und 700 ml Wasser wurden bei Raumtemperatur über Nacht gerührt. Anschließend wurden die beiden Phasen getrennt und die Toluolphase nochmal mit Wasser nachgewaschen. Das Lösungsmittelvolumen wurde auf 1/5 im Vakuum reduziert.
C.11.b. 267.6 g Triphenylphosphin (1.02 Mol) wurden bei 10 °C in 500 ml Tetrahydrofuran vorgelegt. Zu dieser Lösung wurde das in 165 ml Tetrahydrofuran gelöste Azid langsam zugetropft (Stickstoffentwicklung). Nach Ende der Zugabe wurden 27.6 ml Wasser (1.53 Mol) langsam zugegeben und das Reaktionsgemisch für 48 h bei Raumtemperatur gerührt. Die Lösung wurde dann einrotiert und der Rückstand in kalter 3N HCl (1L) aufgenommen. Der ausgefallene Feststoff wurde abgesaugt und das Filtrat bis zur vollständigen Entfernung des Triphenylphosphinoxids mit Toluol gewaschen. Anschließend wurde die saure wäßrige Phase mit Na₂CO₃ (fest) auf pH = 9 eingestellt, der ausgefallene Feststoff abfiltriert und das Filtrat mit Diethylether extrahiert. Der Feststoff wurde in Diethylether gelöst und zusammen mit den etherischen Extrakten getrocknet. Das Ethervolumen wurde dann reduziert und das Hydrochlorid durch HCl-Gaseinleitung gefällt. Das Salz wurde abfiltriert, mit Diethylether gewaschen und an der Luft getrocknet (Salz sublimiert im Hochvakuum). Ausbeute: 137,6 g
C.11.c. Die Herstellung des p-Cyanobenzylamins aus p-Cyanobenzylbromid wurde auch über das Phthalimid mit anschließender Spaltung durch Hydrazinhydrat in guten Ausbeuten hergestellt.
   Ebenfalls geeignet ist die Synthese über das Urotropiniumsalz (W. Walter u.a., Ann. 1962, 660, 60).
C.12. m-Cyanobenzylamin:
   Die Herstellung erfolgte nach Literaturangabe (Pharmazie 1978, 33, 15)
C.13.a. N-(p-Cyanobenzyl)-benzophenonimin
   Zu einer Lösung von 150 g (0,8 Mol) 97 %igem Benzophenonimin und 144,8 g (0.74 Mol) p-Cyano-benzylbromid in 450 ml Acetonitril gab man 270 g (2,0 Mol) wasserfreies K₂CO₃ und ließ bei Raumtemperatur 6 h rühren. Nach Absaugen der anorganischen Salze wurde das Lösungsmittel weitgehend abdestilliert, der Rückstand mit 300 ml Wasser versetzt und mehrmals mit Essigester extrahiert. Die organische Phase wurde 2 x mit Wasser gewaschen, über Na₂SO₄ getrocknet und zur Trockene eingeengt. Nach digerieren mit Ether erhielt man 180 g weiße Kristalle, Fp 101-102°C.
C.13.b 1-(4-Cyanophenyl)ethylamin
   Zu einer Lösung von Lithiumdiisopropylamid, hergestellt aus 8,15 g (0,08 Mol) Diisopropylamin und 48,3 ml (0,08 Mol) 15%ige Lösung von Butyllithium in Hexan - in 100 ml abs. Tetrahydrofuran, tropfte man bei -70°C 20,7 g (0,07 Mol) N-(p-Cyanobenzyl)-benzophenonimin und ließ 15 Minuten nachrühren. Danach tropfte man 9,94 g (0,07 Mol) Methyljodid zu und ließ die Temperatur des Reaktionsgemisches auf Raumtemperatur ansteigen. Nach Zugabe von 100 ml Wasser wurde mehrmals mit Ether extrahiert, die Etherphase mit 5 %iger Zitronensäurelösung, 5 %iger NaHCO₃-Lösung und Wasser gewaschen, über Na₂SO₄ getrocknet und der Ether abdestilliert. Der Rückstand wurde in 150 ml Tetrahydrofuran gelöst, 100 ml 1N HCl zugegeben und über Nacht bei Raumtemperatur gerührt. Aus dem Reaktionsgemisch wurde im Vakuum das Tetrahydrofuran abdestilliert, die verbleibende Saurephase zur Entfernung des Benzophenons mehrmals mit Ether extrahiert, anschließend die Säurephase mit wäßriger K₂CO₃-Lösung unter Eiskühlung alkalisch gestellt und die ölige Base mit Methylenchlorid extrahiert. Der Extrakt wurde über K₂CO₃ getrocknet. Nach Abziehen des Methylenchlorids verblieben 9,7 g (95 %) eines gelblichen Öls, das ohne weitere Reinigung in die Folgereaktion einging.
C.13. (D,L)-1-(4-Cyanophenyl)-ethylamin:
C.14. 4-Aminomethyl-3-methoxy-benzonitril:
C.14.a. 3-Nitro-4-methyl-benzonitril
   Zu 1 1 rauchender Salpetersäure wurden bei -10°C innerhalb 90 min 399g (2,56 Mol) p-Tolunitril gegeben. 1h nach Zugabe wurde das Gemisch auf 2,5L Eis/H₂O gegossen, wobei ein Feststoff ausfiel, der über eine Filternutsche abgetrennt und mit Wasser pH-neutral gewaschen wurde. Die Ausbeute des Produktes betrug 363g (88%). ¹H-NMR (CDCl₃; δ in ppm): 8,3 (d, 1H); 7,8 (dd, 1H); 7,5 (dd, 1H); 2,7 (s, 3H)
C.14.b. 3-Amino-4-methyl-benzonitril:
   120 g 3-Nitro-4-methyl-benzonitril wurden in 1,2L EtOH suspendiert und in Gegenwart von 7g Pd/C (10%) mit 50 L Wasserstoff bei RT hydriert. Nach Abtrennung des Katalysators über Celite wurde das Lösungsmittel abgezogen und man erhielt 95g sauberes Produkt (97%). ¹H-NMR (DMSO-d₆; δ in ppm): 7,1 (dd, 1H); 6,90 (d, 1H); 6,85 (dd, 1H); 5,35 (s, 2H, NH2); 2,15 (s, 3H)
C.14.c. 3-Hydroxy-4-methyl-benzonitril:
   Zu 85 g (0,72 Mol) 3-Amino-4-methyl-benzonitril in 1,8 1 6N HCl wurde bei 0-5°C innerhalb 30 min eine Lösung aus 49,2 g (0,72 Mol) NaNO₂ in 217 ml Wasser getropft. Man rührte anschließend weitere 30 min bei 0-5°C und dann noch 1 h bei Siedetemperatur. Nach Erkalten der Lösung konnte das Produkt mit Essigester und daraus in Form des Phenolats, mit eiskalter 5N NaOH extrahiert werden. Die Wasserphase wurde dann mit 6N HCl auf pH 3 angesäuert und das Produkt mit Essigester extrahiert. Man erhielt 41 g (43%) des Phenols.
   ¹H-NMR (DMSO-d₆; δ in ppm): 10,3 (s, OH); 7,25 (dd, 1H); 7,15 (d, 1H); 7,1 (dd, 1H); 2,20 (s, 3H)
C.14.d 3-Methoxy-4-methyl-benzonitril:
   15 g (0,11 Mol ) 3-Hydroxy-4-methyl-benzonitril, gelöst in 30 ml DMF, wurden zu einer Suspension aus 0,11 Mol NaH und 30 ml DMF getropft und solange gerührt, bis keine H₂-Entwicklung mehr zu beobachten war. Dann tropfte man 10,6 ml (0,17 Mol) Methyljodid zu und rührte 1h bei RT. Die Lösung wurde auf Eiswasser gegossen und das Produkt mit Ether/Essigester 7:1 extrahiert. Nach Abziehen des Lösungsmittels begann das Produkt langsam zu kristallisieren. Man erhielt 14,8 g (89 %) des Produktes. ¹H-NMR (CDCl₃; **δ** in ppm): 7,2 (m, 2H); 7,02 (s, 1H); 3,85 (s, 3H); 2,25 (s, 3H)
C.14.e. 4-Brommethyl-3-methoxy-benzonitril:
   14,7 g (0,1 Mol) 3-Methoxy-4-methyl-benzonitril wurde in 210 ml 1,2-Dichlorethan gelöst, portionsweise innerhalb 1h mit 19,1 g (0,11 Mol) NBS in Gegenwart katalytischer Mengen AIBN bei 82°C bromiert und nach beendeter Zugabe weitere 30 min bei 82°C gerührt. Nach Zugabe von n-Heptan wurde ausgefallenes Succinimid abgetrennt und das Lösungsmittel abgezogen. Das Produkt enthielt neben kleiner Mengen Edukt noch Spuren des entsprechenden Benzalbromids. ¹H-NMR (DMSO-d₆ ; δ in ppm): 7,60 (dd, 1H); 7,50 (d, 1H); 7,40 (dd, 1H); 4,68 (s, 2H); 3,96 (s, 3H)
C.14.f. 4-Phthalimidomethyl-3-methoxy-benzonitril:
   24,4 g (108 Mol) 4-Brommethyl-3-methoxy-benzonitril, gelöst in 125 ml DMF und 20,0 g Kaliumphthalimid wurden 24 h bei RT und dann noch 1 h bei 50°C gerührt. Das Gemisch wurde auf Wasser gegossen, wobei das Produkt als Feststoff ausfiel. Man erhielt 21,5 g (68%) des Produktes. ¹H-NMR (DMSO-d₆ ; δ in ppm): 7,9 (m, 4H); 7,5 (d, 1H); 7,35-7,25 (m, 2H); 7,78 (s, 2H); 3,92 (s, 3H)
C.14.g 4-Aminomethyl-3-methoxy-benzonitril:
   21,2 g (73 mMol) 4-Phthalimidomethyl-3-methoxy-benzonitril, gelöst in 290ml THF, wurden 10,6 ml Hydrazinhydrat gegeben und 20 h bei RT gerührt. Dann tropfte man 180 ml 2N HCl zu und zog das Lösungsmittel nach 1,5 h vollständig ab. Der Rückstand wurde in MTBE aufgenommen, mit iN HCl extrahiert, mit 2N NaOH auf pH 9-10 eingestellt und mit DCM extrahiert. Man erhielt 8,0 g (68 %) es Produktes. ¹H-NMR (DMSO-d₆ ; δ in ppm): 7,55 (dd, 1H); 7,40 (dd, 1H); 7,37 (d, 1H); 3,85 (s, 3H); 3,70 (s, 2H); 2,5-1,6 (NH₂).
C.15 4-Aminomethyl-3-isopropoxy-benzonitril:
C.15.a. 3-i-Propoxy-4-methyl-benzonitril:
   7,0 g 3-Hydroxy-4-methyl-benzonitril (52,6 mmol) wurden mit 57,8 mol NaH in 100 ml DMF deprotoniert und mit 7,4 ml 2-Brompropan bei 0°C versetzt. Nach 45 min wurde die Temperatur auf 50°C erhöht und weitere 5 h gerührt. Die Reaktionsmischung wurde auf Wasser gegossen und das Produkt mit Ether extrahiert. Das Produkt wurde säulenchromatographisch über Kieselgel gereinigt (Laufmittel: Dichlormethan/10 % Heptan). Man erhielt 6,3 g (68 %); Schmp: 60-61°C
C.15.b. 4-Brommethyl-3-i-propoxy-benzonitril:
   6,1 g 3-i-Propoxy-4-methyl-benzonitril (33,4 mmol) wurden analog Beispiel (C.14.e.) mit NBS und AIBN bromiert. Das Produkt entstand in nahezu quantitativer Ausbeute.
   ¹-H-NMR (DMSO-d₆-, δ in ppm): 7,65-7,30 (3H, Aromaten-H); 4,85 (1H, CH), 4,63 (2H, CH₂), 1,40-1,25 (6H, 2xCH₃)
C.15.c. 4-Aminomethyl-3-i-propoxy-benzonitril (Hydrochlorid):
   8,8 g des Bromids (i) (33,4 mmol) wurden in 100 ml MeOH gelöst und bei 40°C zu 150 ml mit Ammoniak gesättigtem MeOH langsam hinzugetropft. Das Lösungsmittel wurde abgezogen, das Produkt in Dichlormethan aufgenommen, mit 1N Natronlauge gewaschen und mit etherischer HCl als Hydrochlorid ausgefällt. Man erhielt 2,6 g.
   ¹-H-NMR (DMSO-d⁶-, δ in ppm): 8,6 (3H, NH₃⁺), 7,65-7,40 (3H, Aromaten-H), 4,80 (1H, CH); 4,00 (2H, CH₂), 1,4-1,3 (6H, 2xCH₃)
C.16 4-Aminomethyl-3-chlor-benzonitril:
C.16.a. 4-Brommethyl-3-chlor-benzonitril:
   3-Chlor-4-methyl-benzonitril wurde analog Beispiel (C.14.e.) mit NBS und AIBN bromiert. ¹-H-NMR (DMSO-d₆, δ in ppm): 8,10 und 7,85 (3H, Aromaten-H), 4,80 (2H, CH₂)
C.16.b. 4-Aminomethyl-3-chlor-benzonitril:
   10,0 g des Bromids wurden analog Beispiel (C.14.f) mit Kaliumphthalimid umgesetzt. Man erhielt 9,6 g Phthalimidomethyl-3-chlor-benzonitril, welches mit Hydrazinhydrat analog Beispiel (C.14.g.) gespalten wurde. Das freie Amin (4,0 g) erhielt man durch Extraktion mit Dichlormethan aus der mit Natronlauge auf pH 9-19 eingestellten Wasserphase.
   ¹⁻H-NMR (DMSO-d₆, δ in ppm): 7,95-7,78 (3H, Aromaten-H); 3,85 (2H, CH₂), 2,1 (breites Signal, 2H, NH₂)

### D. Beispiele:

### Beispiel 1:

### Boc-(D)-Phe-Pro-NH-(4-Am)-2-phenethyl:

Zu einer Lösung von 10 mMol Boc-(D)-Phe-Pro-OH und 11 mMol N-Methylmorpholin in 10 ml DMF gab man bei -15°C innerhalb 2 Minuten 10 mMol Chlorameisensäure-isobutylester, rührte 10 Minuten nach und gab anschließend eine Lösung von 10 mMol p-Cyanobenzylamin und 11 mMol N-Methylmorpholin in 3 ml DMF zu. Nach 3-stündigem Nachrühren bei -15°C war laut DC-Kontrolle (DCM/MeOH, 9/1) keine Ausgangsverbindung mehr nachweisbar.

Zur Isolierung wurde das Reaktionsgemisch in 200 ml Wasser eingegossen, wobei sich ein Öl abschied, das nach kurzer Zeit erstarrte und nach Zerkleinern abgesaugt wurde. Der noch feuchte Rückstand wurde in einem Gemisch aus 250 ml Essigester und 50 ml Ether gelöst und nacheinander mit einer 5 %igen wäßrigen Zitronensäure-, Bicarbonat- und gesättigten Kochsalzlösung gewaschen. Nach Trocknen über Na₂SO₄ wurde das Lösungsmittel im Vakuum abdestilliert, der Rückstand mit n-Hexan versetzt und anschließend abgesaugt. Eine Umkristallisation aus 50 ml Essigester ergab 7,4 mMol DC-reines Produkt, welches analog A.III.1. nach der H₂S-Methode zum Amidin-Hydrojodid umgesetzt wurde.

Man erhielt gelbliche Kristalle; Fp: 158-165°C
FAB-MS: 508 (M+H⁺).

### Beispiel 2:

### H-(D)-Phe-Pro-NH-(4-Am)-2-phenethyl:

Die Abspaltung der Boc-Gruppe von Beispiel 1 wurde analog A.I.c. durchgeführt. Als Lösungsmittelgemisch wurde hier Dichlormethan/Essigester 1:1 eingesetzt. Man erhielt das Dihydrochlorid in Form weißer Kristalle; Fp: 203-206°C (Zersetzung); FAB-MS: 408 (M+H⁺)

### Beispiel 3:

### Boc-(L)-Phe-Pro-NH-pAmb:

Die Verbindung wurde ausgehend von Boc-(L)-PheOH und H-Pro-p-cyano-benzylamid x HCl analog B.I. und anschließender Umsetzung des Nitrils zum Amidin analog A.III.1. hergestellt. Das dabei erhaltene Amidin-Hydrojodid wurde über einen Acetationentauscher (IRA 420) zum Amidin-Hydroacetat umgewandelt.
¹-H-NMR (d₆-DMSO, δ in ppm):
8,4(m,1H,NH); 7,75(d,2H,Ar-H); 7,45(d,2H,Ar-H); 7,2(m,5H,Ar-H); 7,18/7,02(2d,1H,NH); 4,48-4,18(m,4H,CH₂/2-α-H); 3,6(m,2H,Pro); 3,0-2,7(m,2H,CH₂-Ph); 2,18-1,8(m,4H,Pro); 1,3-1,2(2s,9H,Boc)
MS: 494 (M+H⁺); 394 (-Boc); mp: 142°C

### Beispiel 4:

### H-(L)-Phe-Pro-NH-pAmb:

Die Verbindung wurde durch Boc-Abspaltung analog A.I.c. aus Beispiel 3 hergestellt. Das enthaltene Dihydrochlorid wurde säulenchromatographisch über Kieselgel durch Zusatz von Essigsäure zum Dihydroacetat umgesetzt; Fp: 69°C; FAB-MS: 394 (M+H⁺)

### Beispiel 5:

### Boc-(D)-Phe-Pro-NH-pAmb:

Zu einer Lösung von 5,1 g (14,2 mMol) Boc-D-Phe-Pro-OH und 1,53 g (15,2 mMol) N-Methylmorpholin in 15 ml DMF gab man bei -15°C innerhalb 2 Min. 2,0 g (14,6 mMol) Chlorameisensäure-isobutylester, rührte 10 Min. nach und gab anschließend eine Lösung von 1.9 g (14,2 mMol) p-Cyanobenzylamin und 1,53 g N-Methylmorpholin in 3 ml DMF zu. Nach 3-stündigem Nachrühren bei -15°C war laut DC-Kontrolle (CH₂Cl₂/MeOH, 9/1) keine Ausgangsverbindung mehr nachweisbar.

Zur Isolierung wurde das Reaktionsgemisch in 200 ml Wasser eingegossen, wobei sich ein Öl abschied, das nach kurzer Zeit erstarrte und nach Zerkleinern abgesaugt wurde. Der noch feuchte Rückstand wurde in einem Gemisch aus 250 ml Essigester und 50 ml Ether gelöst und nacheinander mit einer 5 %igen wäßrigen Zitronensäure, Bicarbonat- und gesättigten Kochsalzlösung gewaschen. Nach Trocknen über Na₂SO₄ wurde das Lösungsmittel im Vakuum abdestilliert, der Rückstand mit n-Hexan versetzt und anschließend abgesaugt. Eine Umkristallisation aus 50 ml Essigester ergab 5,6 g DC-reines Boc-(D)-Phe-Pro-p-cyano-benzylamid; Fp: 156-157°C Thioamidbildung: 4,1 g der vorstehenden Verbindung und 4 ml Triethylamin wurden in 40 ml Pyridin gelöst, bei 0°C mit H₂S gesättigt und über Nacht bei Raumtemperatur stehen gelassen. Gemäß DC-Kontrolle (CH₂Cl₂/MeOH, 9/1) war die Umsetzung zum Thioamid vollständig. Zur Isolierung wurde das Pyridin im Vakuum weitgehend abdestilliert, der Rückstand in 250 ml Essigester aufgenommen und mit Kochsalz-, 5 %iger Zitronensäure- und NaHCO₃-Lösung gewaschen. Nach Trocknen und Abdestillieren des Lösungsmittels erhielt man 4,1 g reines kristallines Thioamid.

Amidinbildung: Das Thioamid wurde in 150 ml Aceton gelöst und bei Raumtemperatur nach Zusatz von 7 ml Methyljodid über Nacht stehen gelassen. Nach Abziehen des Lösungsmittels wurde der amorphe Rückstand mit trockenem Ether ausgerührt und anschließend getrocknet. Das S-Methyl-thiomidsäuremethylester-hydrojodid wurde in 50 ml Ethanol gelöst, mit 15 ml 10 %iger Ammoniumacetatlösung versetzt und 3 Stunden auf 60°C erwärmt. Zur Isolierung wurde das Lösungsmittel abgezogen, der Rückstand in 100 ml CH₂Cl₂ gelöst, die unlöslichen Bestandteile abfiltriert und anschließend das CH₂Cl₂ abdestilliert. Durch Digerieren mit einem Essigester-Diethylether-Gemisch wurden die darin löslichen Verunreinigungen abgetrennt. Das verbliebene Jodid-Acetat-Mischsalz wurde in Aceton/Wasser (3/2) gelöst und mittels eines IRA-Acetat-Ionenaustauschers in das reine Acetat überführt. Die Lösung wurde zur Trokkene eingeengt und der Rückstand gefriergetrocknet. Es wurden 3.8 g DC-reines (CH₂Cl₂/MeOH/50 %iger Eisessig, 20/5/1) Boc-D-Phe-Pro-NH-pAmb in Form des Acetats, Fp. 195-200°C (Zersetzung), isoliert.

### Beispiel 6:

### Ac-(D)-Phe-Pro-NH-pAmb:

10.4 g (0,05 Mol) Ac-D-Phe-OH, 6.3 g (0,055 Mol) N-Hydroxysuccinimid und 11,4 g (0.055 Mol) Dicyclohexylcarbodiimid wurden in 150 ml Acetonitril gelöst und über Nacht bei Raumtemperatur gerührt. Der gebildete Niederschlag wurde abfiltriert, das Lösungsmittel abdestilliert und der Rückstand im Vakuum getrocknet und ohne weitere Reinigung für die Folgereaktion eingesetzt.

13,3 g (0.05 Mol) (4-Cyanobenzyl)prolylamid-hydrochlorid (s. Beispiel 10) wurden in 100 ml Methylenchlorid gelöst und nacheinander bei 0°C mit 15 ml Triethylamin und einer Lösung des vorstehenden Ac-D-Phe-O-Succinimids in 70 ml Methylenchlorid versetzt. Das Reaktionsgemisch wurde über Nacht bei Raumtemperatur gerührt und nacheinander mit Wasser, 5 %iger Zitronensäure-, 5 %iger NaHCO₃- und Kochsalzlösung gewaschen. Nach Trocknen und Abdestillieren des Lösungsmittel wurde der Rückstand über eine Kieselgelsäure (Eluent: CH₂Cl₂/MeOH, 50/2) gereinigt und anschließend analog Beispiel 5 in das Amidin überführt.
Acetat: Fp 220 - 224°C (Zersetzung), FAB-MS: 436 (M+H⁺)

### Beispiel 7:

### H-(D)-Phe-Pro-NH-pAmb:

4.9 g (10 mMol) der nach Beispiel 5 erhaltenen Verbindung wurden in einem Lösungsmittelgemisch aus 100 ml Chloroform und 100 ml Essigester gelöst und bei -15°C unter Feuchtigkeitsausschluß mit HCl-Gas gesättigt. Nach einer Stunde war laut DC (CH₂Cl₂/MeOH/50%iger Eisessig, 20/5/1) keine Boc-Verbindung mehr nachweisbar.

Durch Einleiten von Stickstoff wurde bei -15 °C das überschüssige HCl-Gas weitgehend entfernt, wobei sich das Dihydrochlorid als feines Kristallisat ausschied. Nach Zugabe von 50 ml Ether zur Vervollständigung der Abscheidung wurde der Niederschlag abgesaugt und mit einem Essigester-Ether-Gemisch (1/1) nachgewaschen. Der Rückstand wurde in Wasser gelöst, mit Aktivkohle behandelt und lyophilisiert. Man erhielt 3.7 g (95 % d. Th.) des Dihydrochlorids als weiße Kristalle, Fp 215 °C (Zersetzung); FAB-MS 394 (M+H⁺).

### Beispiel 8:

### H-(D)-Phe-Pro-N(Me)-pAmb:

Die Verbindung wurde analog Beispiel 5 durch Umsetzung von Boc-(D)-Phe-Pro-OH und N-Methyl-4-cyanobenzylamin hergestellt. Anschließend wurde die Boc-Gruppe analog A.I.c. abgespalten. Man erhielt das Dihydrochlorid in Form eines amorphen Feststoffs;
FAB-MS: 408 (M+H⁺)

### Beispiel 9:

### Me-(D)-Phe-Pro-NH-pAmb:

4,0 g der nachstehenden Verbindung (Beispiel 10) wurden in 25 ml EtOH gelöst, mit 1,55 g 32%iger HCl versetzt und nach Zugabe von 0,6 g 10%igem Pd/C hydriert. Nach 1 h war der Umsatz quantitativ (laut DC: Methylenchlorid/MeOH/50%ig HOAc; 35/15/5). Nach Absaugen des Katalysators und Abdestillieren des Lösungsmittels wurde der Rückstand mit 100 ml Essigester in ein weißes Pulver überführt und nach Lösen in Wasser lyophilisiert. Man isolierte 3,1 g Dihydrochlorid, das sich nach sintern bei 100°C oberhalb von 215°C zersetzte.

### Beispiel 10:

### Z-Me-(D)-Phe-Pro-NH-pAmb:

### (4-Cyanobenzyl)-prolylamid-hydrochlorid:

276 g BocPro-OSuccinimid (0.88 Mol) wurden in 2 1 Methylenchlorid bei 0 °C vorgelegt. Zu dieser Lösung wurden in Folge 163.9 g 4-Cyanobenzylaminhydrochlorid (0.97 Mol) und 230 ml Diisopropylethylamin (1.34 Mol) gegeben. Die Suspension wurde im auftauenden Eisbad 48 h gerührt und anschließend filtriert. Das Filtrat wurde mit 20 %iger NaSO₄-Lsg. (4 x), ges. Na₂HCO₃-Lsg. (3 x) und ges. Kochsalzlösung (2 x) extrahiert, getrocknet und einrotiert. Es verblieben 299 g Produkt, das nach Umkristallisation aus Methyltert.-butylether bei 124-125°C schmolz.

299 g der Boc-geschützten Verbindung wurden in 1 1 Diethylether gelöst. Nach Zugabe etherischer HCl-Lösung (HCl im Überschuß) wurde über Nacht gerührt. Das ausgefallene Salz wurde abfiltriert, mit Diethylether gewaschen und im Vakuum getrocknet. Das Rohprodukt wurde aus EtOH umkristallisiert.
Ausbeute: 200 g; Fp: 209-213°C (Zersetzung)

### Z-Me-(D)-Phe-Pro-p-cyano-benzylamid:

3.1g (0.01Mol) Z-Me-(D)-Phe-OH und 1.49g (0.011Mol) Hydroxybenzotrialzol wurden in 50ml DMF gelöst und bei 0°C mit 2.1g (0.01Mol) Dicyclohexylcarbodiimid versetzt. Nach 30min. gab man 2.7g (0.01Mol) (4-Cyanobenzyl)-prolylamid - hydrochlorid und 2.2ml N-Methylmorpholin zu. Das Reaktionsgemisch wurde über Nacht bei Raumtemperatur gerührt, der ausgefallene Harnstoff abfiltriert und das Lösungsmittel im Vakuum abdestilliert. Der Rückstand wurde in 100ml Essigester gelöst und nacheinander mit Wasser, 5%iger Zitronensäure, 5%iger NaHCO3- und Kochsalzlösung-Lösung gewaschen. Nach Trocknen und Abdestillieren des Lösungsmittels verblieben 4.7g (90% d. Th.) zähes Öl, das in der Folgereaktion eingesetzt wurde.

### Amidierung mittels Pinner-Reaktion

In eine Lösung von 8.9g abs. EtOH in 25ml Methylenchlorid wurden bei 0°C 12.3g Acetylchlorid eingetropft und 40min. nachgerührt. Anschließend tropfte man bei 0 °C eine Lösung von 4.7g der obigen Substanz in 30ml abs. Methylenchlorid zu. Das Reaktionsgemisch wurde 4Tage bei 0°C stehengelassen. Nach Einengen der Lösung im Vakuum wurde der Rückstand mit 100ml Methylenchlorid verdünnt und diese Lösung mit eiskalter 15%iger K₂CO₃-Lsg. geschüttelt. Trocknen und Abdestillieren des Lösungsmittels ergab die rohe Iminoetherbase, die in 30ml MeOH gelöst und mit 0.8g Ammoniumacetat versetzt wurde. Die Lösung blieb 2 Tage bei Raumtemperatur stehen.

Nach Abfiltrieren des Lösungsmittels wurde der Rückstand über eine Kieselgelsäule (Methylenchlorid/MeOH/50%ig HOAc; 40/10/2.5) gereinigt. Die eingedampften Eluate wurden in Toluol aufgenommen und erneut einrotiert. Der Rückstand wurde in Wasser gelöst, mit Aktivkohle behandelt und anschließend lyophilisiert. Es verblieben 4.1 g (76 % d. Th.) weiße Kristalle, die bei 83°C sinterten und bei 178-184°C schmolzen.

### Beispiel 11

### HOOC-CH₂-(D)-Phe-Pro-NH-pAmb:

2.4 g t-BuOOC-CH₂-(Boc)-(D)-Phe-Pro-NH-pAmb Hydroacetat (aus Beispiel 13) wurden in 80 ml absolutem DCM und 15 ml etherischer HCl über Nacht bei Raumtemperatur gerührt. Das Lösungsmittel wurde im Vakuum abgezogen, der Rückstand aus DCM/Aceton 2:1 ausgerührt und abfiltriert. Man erhielt 1,6 g des Produkts als Hydrochlorid bzw. Dihydrochlorid bzw. als Mischung beider Salzformen in Form eines weißen Feststoffs. Fp: 210-220°C.

### Beispiel 12:

### MeOOC-CH₂-(D)-Phe-Pro-NH-pAmb:

0,5 g der Verbindung aus Beispiel 11 wurden zusammen mit 2 ml etherischer HCl, 3 ml DCM und 3 ml Methanol 30 h bei Raumtemperatur gerührt. Das Lösungsmittel wurde eingeengt und der Rückstand mehrfach aus Ether ausgerührt. Man erhielt 0,5 g Produkt als Hydrochlorid, bzw. Dihydrochlorid bzw. Mischung beider Salzformen. Fp: 104-120°C

### Beispiel 13:

### t-BuOOC-CH₂-(Boc)-(D)-Phe-Pro-NH-pAmb:

a) H-(D)-Phe-Pro-p-cyano-benzylamid:
   5,6 g Boc-(D)-Phe-Pro-p-cyano-benzylamid (aus Beispiel 5) wurden analog A.I.c. gespalten. Man erhielt 4,6 g (95 %) des Produkts als Hydrochlorid in Form weißer Kristalle.
b) t-BuOOC-CH₂-(Boc)-(D)-Phe-Pro-p-cyano-benzylamid:
   6,19 g H-(D)-Phe-Pro-p-cyano-benzylamid (15 mMol) wurden zusammen mit 0,98 g Bromessigsäure-tert.-butylester (5 mMol) und 0,63 g Ammoniumcarbonat im Gemisch aus 35 ml Wasser und 8 ml Nitromethan 2 h bei 50°C erhitzt. Dann wurde mit Essigester extrahiert, die organische Phase mehrfach mit 0,1 N Salzsäure gewaschen, die Wasserphasen mit DCM extrahiert und die vereinigten organischen Phasen über MgSO₄ getrocknet. Nach Abziehen des Lösungsmittels wurde das Produkt als Hydrochlorid mit etherischer HCl ausgefällt. Man erhielt 2,6 g (98 %) des Hydrochlorids. Das überschüssige H-(D)-Phe-Pro-p-cyano-benzylamid wurde durch Extraktion der Wasserphasen bei pH 10 mit DCM zurückgewonnen.
c) t-BuOOC-CH₂-(Boc)-(D)-Phe-Pro-p-cyano-benzylamid:
   2,6 g des vorstehenden Hydrochlorids (4,9 mMol) wurden zusammen mit 1,2 g (Boc)₂O (5,5 mMol) und 1,87 ml DIPEA (11 mMol) in 95 ml absolutem DCM über Nacht bei Raumtemperatur gerührt. Anschließend wurde das Lösungsmittel eingeengt, der Rückstand in Ether aufgenommen, mit 0,1 N Salzsäure, dann mit Wasser gewaschen, das Lösungsmittel über MgSO₄ getrocknet und im Vakuum abgezogen. Nach Ausrühren des Rückstandes aus Hexan wurden 2,8 g Produkt als weißer Feststoff erhalten.
d) t-BuOOC-CH₂-(Boc)-(D)-Phe-Pro-NH-pAmb:
   Die Umsetzung der Nitrilfunktion zur Amidinofunktion erfolgte analog A.III.1. mit einer Gesamtausbeute von 96 %.
   Die Überführung des Hydrojodids in das Hydroacetat erfolgte mittels eines IRA-Acetationenaustauschers; Fp: 116-121°C

### Beispiel 14:

### EtOOC-(D)-Phe-Pro-NH-pAmb:

Die Verbindung wurde hergestellt durch Umsetzung von N-(Ethoxycarbonyl)-(D)-phenylalanin (J. Org. Chem. 1980, 45, 4519) mit (4-Cyanobenzyl)-prolylamidhydrochlorid (aus Beispiel 10) und nachfolgender Amidinbildung (analog Beispiel 5). Man erhielt weiße Kristalle des Hydroacetats; Fp: 105-107°C; FAB-MS: 466 (M+H⁺)

### Beispiel 15:

### Boc-(D)-Phe-Pro-NH-mAmb:

Die Verbindung erhielt man aus Hoc-(D)-Phe-Pro-OH mit m-Cyanobenzylamin (analog Heispiel 5). Das Hydroacetat erhielt man in Form weißer Kristalle; Fp: 130-133°C

### Beispiel 16:

### H-(D)-Phe-Pro-NH-mAmb:

Die Abspaltung der Boc-Gruppe aus Beispiel 15 erfolgte analog A.I.c. Die weißen Kristalle des Dihydrochlorids schmolzen bei 155-160°; FAB-MS: 394 (M+H⁺)

### Beispiel 17:

### Z-(D)-Phe-Pro-(D,L)-(4-Am)-PhgOH:

a) 39,7 g (100,1 mMol) Z-(D)-Phe-Pro-OH, 11,53 g (100,1 mMol) HOSu und 20,66 g (100,1 mMol) DCC wurden in 400 ml Dimethoxyethan 18 h bei RT gerührt. Anschließend wurde der Feststoff abfiltriert, das Filtrat eingeengt und der Rückstand in Acetonitril aufgenommen. Erneut ausgefallener Feststoff wurde abfiltriert und die organische Lösung im Vakuum zur Trockne eingedampft. Rohausbeute: 48,9 g Z-(D)-Phe-Pro-OSuccinimid.
b) 24,53 g H-Phg(4-CN)-OEt x HCl, 34,9 ml DIPEA und 41,9 g Z-(D)-Phe-Pro-OSuccinimid wurden in 200 ml DMF gelöst und 18 h bei RT gerührt. Zur Aufarbeitung wurde DMF im Vakuum entfernt und der verbliebene Rückstand in DCM aufgenommen. Die organische Phase wurde mit 1N HCl extrahiert, mit NaSO₄ getrocknet und im Vakuum eingeengt. Es verblieben 54,04 g Z-(D)-Phe-Pro-NH-Phg(4-CN)-OEt.
c) 54,04 g Z-(D)-Phe-Pro-NH-Phg(4-CN)-OEt wurden in 340 ml THF/EtOH/Wasser (3:1:1) gelöst und nach Zugabe von 3,05 g LiOH 18 h bei RT gerührt. Anschließend wurde das Reaktionsgemisch im Vakuum eingeengt, die verbliebene wäßrige Lösung auf pH 2 angesäuert und mit Essigester extrahiert. Die vereinigten organischen Extrakte wurden mit ges. NaCl-Lsg. gewaschen, mit NaSO₄ getrocknet und am Rotationsverdampfer zur Trockne eingeengt. Rohausbeute: 40,94 g Z-(D)-Phe-Pro-NH-Phg(4-CN)-OH.
d) 2,78 g Z-(D)-Phe-Pro-NH-Phg(4-CN)-OH, 18,9 ml Pyridin und 8,7 ml TEA wurden in einem Reaktionskolben vereinigt und mit H₂S-Gas gesattigt. Man ließ die Lösung 18 h bei Raumtemperatur stehen. Anschließend wurde das Reaktionsgemisch auf 2 L Eiswasser gegossen und die wäßrige Phase mit 1 N HCl auf pH 3 eingestellt. Das ausgefallene Produkt wurde abfiltriert, in Essigester gelöst und die Lösung mit NaSO₄ getrocknet. Nach Entfernen des Essigesters im Vakuum wurde der Rückstand mit 20 ml Aceton und 3,5 ml Mel vereinigt und 18 h bei RT gerührt. Hierauf wurden die flüchtigen Bestandteile im Vakuum entfernt und das rohe Thiomethyliminhydrojodid in 8 ml MeOH und 8 ml methanolischer Ammoniumacetatlösung (10%ig) 18 h gerührt. Anschließend wurde das Reaktionsgemisch eingeengt, der Rückstand in DCM aufgenommen und der ausgefallene Feststoff abfiltriert. Nach Einengen des Filtrates erhielt man 3,75 g Rohprodukt. Dieses wurde mittels Reversed Phase HPLC-Chromatographie gereinigt. Ausbeute: 1,5 g.
   ¹H-NMR (d₆-DMSO, δ in ppm): 8,4 - 8,0 (2m, 1H, NH); 7,7 - 7,4 (m, 4H, Ar-H); 7,3 - 7,1 (m, 10H, Ar-H); 7,0 (sb, 1H, NH); 5,2 - 4,8 (m, 3H, OCH2/α-Phg); 4,6 - 4,2 (m, 2H, α-Pro/α-Phe); 3,6 - 3,2 (2H, δ-Pro); 3,0 - 2,6 (m, 2H, CH2-Ph); 2,2 - 1,6 (m, 4H, α/β-Pro)
   FAB-MS: 572 (M+H⁺); Fp: 155 - 158°C

### Beispiel 18:

### Z-(D)-Phe-Pro-(D,L)-(4-Am)-Phg-OMe:

a) Zu 14 ml DCM wurden 5,14 ml abs. MeOH und 6,16 ml Acetylchlorid bei 0°C gegeben. Anschließend fügte man zu dieser Lösung 2,78 g Z-(D)-Phe-Pro-NH-Phg(4-CN)-OH in 10 ml DCM und ließ sie 48 h bei Raumtemperatur stehen. Zur Aufarbeitung wurde das Reaktionsgemisch eingeengt, der Rückstand in Essigester aufgenommen und mit kalter K₂CO₃-Lösung (5%ig) gewaschen. Nach Trocknen der organischen Phase mit NaSO₄ wurde das Lösungsmittel am Rotationsverdampfer entfernt und der rohe Iminomethylether in 6,5 ml MeOH und 6,5 ml methanolischer Ammoniumacetatlösung (10%ig) 18 h stehengelassen. Nach Eindampfen der Lösung erhielt man 2,4 g des Rohproduktes, welches durch eine Reversed Phase HPLC-Chromatographie gereinigt wurde.
   ¹H-NMR (d₆-DMSO, δ in ppm): 9,6 - 9,2 (b, N-H); 8,75/8,5 (2d, 1H, NH); 7,8 (m, 2H,Ar-H); 7,6 (m, 2H, Ar-H); 7,35 - 7,2 (m, 10H, Ar-H); 7,05 (sb, 1H, NH); 5,6 (2d, 2H, OCH2); 5,0 - 4,2 (3m, 4H, α-Pro/α-Phe/α-Phg); 3,6 (2s, 3H, OCH3); 3,5 - 3,2 (2H, δ-Pro); 3,0 - 2,6 (m, 2H, CH2-Ph); 2,2 - 1,6 (m, 4H, (β/γ-Pro)
   FAB-MS: 586 (M+H⁺); Fp: 129-131°C (Hydrochlorid)

### Beispiel 19:

### H-(D)-Phe-Pro-(D,L)-(4-Am)-Phg-OH:

Die Verbindung wurde durch Cbz-Abspaltung aus Beispiel 17 hergestellt. ¹H-NMR (d₆-DMSO, δ in ppm): 9,0 (b, NH); 8,7/8,4/8,05 (3d, 1H, NH); 7,8 - 7,0 (m, 9H, Ar-H); 5,1/4,9 (d, 1H, α-Phg); 4,45-4,0 (m, 2H, α-Pro/α-Phe); 3,8 - 3,0 (m, 2H, δ-Pro); 3,0 - 2,7 (m, 2H, CH2-Ph); 2,2 - 1,4 (m, 4H, β/γ-Pro)
FAB-MS: 438 (M+H⁺); mp: 149 - 150°C (Dihydrochlorid)

### Beispiel 20:

### Boc-(D)-Phe-Pro-(4-Am)-Phg-CH₂Ph

a) N-(Diphenylmethylen)-4-cyano-benzylamin:
   33,73 g (0,2 Mol) 4-Cyanobenzylamin und 36,25g (0,2 Mol) Benzophenonimin wurden in 540 ml DCM bei Raumtemperatur gelöst und über Nacht gerührt. Anschließend wurde das Reaktionsgemisch mit 2 x 90 ml Wasser gewaschen, mit Na₂SO₄ getrocknet und das Lösungsmittel am Rotationsverdampfer entfernt. Es verblieben 55,59 g (93,7 %) des Rohproduktes. Nach Umkristallisation aus 550 ml iPrOH wurden 97,67 g (80,4 %) reines Produkt gewonnen. ¹H-NMR (CDCl₃, δ in ppm): 7,7-7,15 (m, 9H, Ar-H); 4,65 (s, 2H, CH₂-N)
b) N-(Diphenylmethylen)-α-(β-Phenylacetyl)-4-cyano-benzylamin:
   63,3 mMol LDA wurden bei -30°C in 45 ml THF vorgelegt. Anschließend wurden 15 g (50,61 mMol) N-(Diphenylmethylen)-4-cyano-benzylamin in 75 ml THF langsam zugetropft. Nach weiteren 10 min. Rühren bei -30°C wurden 8,6 g (55,7 mMol) Säurechlorid (in 7,5 ml THF gelöst) bei -78°C langsam zugegeben. Nach 18 h Rühren des Reaktionsgemisches (man ließ die Reaktionstemperatur über Nacht auf Raumtemperatur steigen) wurde es auf -20°C abgekühlt, mit 3,6 ml HOAc und 17,25 ml Wasser versetzt. Nachdem das Reaktionsgemisch auf Raumtemperatur gekommen war, wurde das THF im Vakuum entfernt, der Rückstand wurde in Ether aufgenommen, die organische Phase mit ges. NaCl-Lsg. gewaschen, getrocknet und der Ether am Rotationsverdampfer entfernt. Es verblieben 26,18 g Rohprodukt, welches ohne weitere Reinigung im Folgeschritt eingesetzt wurde.
c) α-(β-Phenylacetyl)-4-cyano-benzylamin:
   26,18 g des synthetisierten Rohketons wurden über Nacht in 250 ml 0,25 N HCl bei Raumtemperatur gerührt. Anschließend wurde das Reaktionsgemisch mit DCM extrahiert und die waßrige Phase lyophilisiert. Nach einer Reversed Phase HPLC-Trennung verblieben 2,52 g α-(β-Phenylacetyl)-4-Cyano-benzylamin. ¹H-NMR (DMSO-d₆, δ in ppm): 9,0 (s, 3H, NH₃); 8,0/7,75 (2d, 4H); 7,25 (m, 3H); : 7,0 (dd, 2H); 5,7 (s, 1H, NCH); 3,9/3,6 (2d, Ph-CH₂-); FAB-MS (M⁺): 250
d) 2,51 g (6,92 mMol) Boc-D-Phe-Pro-OH wurden in 40 ml abs. DCM bei -20°C vorgelegt. Anschließend wurden 0,80 ml N-Methylmorpholin und 0,90 ml (6,92 mMol) Chlorameisensäureisobutylester zu der Lösung gegeben und diese 20 min. bei -20°C gerührt. Nach Zugabe weiterer 0,80 ml N-Methylmorpholin und 2,52 g (6,92 mMol) α,(β-Phenylacetyl)-4-Cyano-benzylamin wurde das Reaktionsgemisch eine weitere Stunde gerührt. Zur Aufarbeitung wurde das Reaktionsgemisch mit 50 ml DCM verdünnt und die organische Lösung mit 1 N HCl (3 x 40 ml), 5%iger NaHCO₃-Lsg. (2 x 40 ml) und ges. NaCl-Lsg. (1 x 40 ml) gewaschen. Nach Trocknen und Einengen der Lösung verblieben 3,97 g des Rohproduktes. Dieses wurde mit einer Säulenchromatographie (Hexan/Essigester) gereinigt. Ausbeute: 3,43 g.
e) 3,43 g (5,77 mMol) Boc-(D)-Phe-Pro-Phg(4-CN)-CH₂-Ph x HOAc wurden in 21,9 ml Pyridin und 9,85 ml TEA gelöst. Diese Lösung wurde bei Raumtemperatur mit H₂S-Gas gesättigt und über Nacht gerührt. Anschließend wurde mit Stickstoff H₂S weitestgehend entfernt und die Lösung auf 5%ige Zitronensäure gegossen. Die wäßrige Phase wurde mehrfach mit Essigester extrahiert. Die vereinigten organischen Phasen wurden mit ges. NaCl-Lsg. gewaschen und nach Trocknen mit Na₂SO₄ am Rotationsverdampfer eingeengt. Rohausbeute: 4,13 g.
f) 4,13 g des rohen Thioamids wurden 18 h zusammen mit 23,3 ml Aceton und 4,05 ml Mel gerührt. Danch wurde die Reaktionslösung am Rotationsverdampfer eingeengt. Der Rückstand wurde in 9,1 ml abs. MeOH und 9,1 ml abs NH₄OAc-Lsg. (10%ig in MeOH) gelöst und 18 h gerührt. Hierauf wurde das Reaktionsgemisch zur Trockne eingeengt und der Rückstand mittels einer Reversed-Phase HPLC-Chromatographie (Acetonitril/Wasser) gereinigt.Ausbeute: 680 mg (Hydroacetat).
   ¹H-NMR (d₆-DMSO, δ in ppm): 9,4/9,2/8,8/8,6 (4d, 1H, N-H); 7,9 - 7,5 (m, 4H, Ar-H); 7,3 - 7,0 (m, 11H, Ar-H/NH); 6,0/5,7 (2m, 1H, α-Phg); 4,4 - 2,6 (m, 8H); 2,2 - 1,6 m, 4H, β/γ-Pro); 1,3 - 1,2 (2sb, 9H, Boc)
   MS: 612 (M+H⁺), 512 (-Boc), 365, 161, 120

### Beispiel 21:

### H-(D)-Phe-Pro-(4-Am)-Phg-CH₂Ph:

Die Verbindung wurde durch eine Boc-Abspaltung (HCl/Dioxan) von Beispiel 20 hergestellt. ¹H-NMR (d₆-DMSO, δ in ppm): 9,5/9,4 (2d, 1 H, NH); 9,4/9,2 (2sb, 3H, N-H); 7,8 (d, 2H, Ar-H); 7,6 (d. 2H, Ar-H); 7,4 - 7,0 (m, 10H, Ar-H); 5,8/5,6(2d, 1H, α-Phg); 4,35 (m, 2H, α-Phe/α-Pro); 4,1/3,9 (4d, 2H, CH2-Ph); 3,7 (m, 2H, CH2); 3,2/3,0 (2m, 2H, CH2); 1,8 - 1,6 (2m , 4H, β/γ-Pro)
MS: 512 (M+H⁺), 393, 252, 161 (Dihydrochlorid)

### Beispiel 22:

### H-(D)-Phe-Pro-NH-pAm-[(D,L)-α-Me]-benzyl:

(a) N-(p-Cyanobenzyl)-benzophenonimin:
   Zu einer Lösung von 150 g (0,8 Mol) 97%igem Benzophenonimin und 144,8 g (0,74 Mol) p-Cyano-benzylbromid in 450 ml Acetonitril gab man 270 g (2,0 Mol) wasserfreies K₂CO₃ und ließ bei Raumtemperatur 6 h rühren. Nach Absaugen der anorganischen Salze wurde das Lösungsmittel weitgehend abdestilliert, der Rückstand mit 300 ml Wasser versetzt und mehrmals mit Essigester extrahiert. Die organische Phase wurde 2 x mit Wasser gewaschen, über Na₂SO₄ getrocknet und zur Trockene eingeengt. Nach digerieren mit Ether erhielt man 180 g weiße Kristalle, Fp 101-102°C.
(b) 1-(4-Cyanophenyl)ethylamin:
   Zu einer Lösung von Lithiumdiisopropylamid, hergestellt aus 8,15 g (0,08 Mol) Diisopropylamin und 48,3 ml (0,08 Mol) 15%ige Lösung von Butyllithium in Hexan - in 100 ml abs. Tetrahydrofuran tropfte man bei -70°C 20,7 g (0,07 Mol) N-(p-Cyanobenzyl)-benzophenonimin und ließ 15 Minuten nachrühren. Danach tropfte man 9,94 g (0,07 Mol) Methyljodid zu und ließ die Temperatur des Reaktionsgemisches auf Raumtemperatur ansteigen. Nach Zugabe von 100 ml Wasser wurde mehrmals mit Ether extrahiert, die Etherphase mit 5%iger Zitronensäurelösung, 5%iger NaHCO₃-Lösung und Wasser gewaschen, über Na₂SO₄ getrocknet und der Ether abdestilliert. Der Rückstand wurde in 150 ml Tetrahydrofuran gelöst, 100 ml 1N HCl zugegeben und über Nacht bei Raumtemperatur gerührt. Aus dem Reaktionsgemisch wurde in Vakuum das Tetrahydrofuran abdestilliert, die verbleibende Säurephase zur Entfernung des Benzophenons mehrmals mit Ether extrahiert, anschließend die Säurephase mit wässriger K₂CO₃-Lösung unter Eiskühlung alkalisch gestellt und die ölige Base mit Methylenchlorid extrahiert. Der Extrakt wurde über K₂CO₃ getrocknet. Nach Abziehen des Methylenchlorids verblieben 9,7 g (95 %) eines gelblichen Öls, das ohne weitere Reinigung in die Folgereaktion einging.
(c) Boc-D-phenylalanyl-prolin-(D,L)-α-methyl-4-cyano-benzylamid:
   Zu einer Lösung von 3,65 g (25 mMol) 1-(4-Cyanophenyl)-ethylamin und 9,1 g (25 mMol) Boc-D-Phe-Pro-OH in 150 ml Methylenchlorid tropfte man bei -5°C 16,2 g Diisopropylamin und 22 ml (30 mMol) Propan-phosphonsäureanhydrid (50%ige Lösung in Essigester). Es wurde 2 h nachgerührt, wobei man die Temperatur von -5° auf 20°C ansteigen ließ. Die organische Phase wurde mit Wasser, 5%iger Natriumbicarbonat- und 5%iger Zitronensäurelösung gewaschen, über Na₂SO₄ getrocknet und zur Trockene eingeengt. Man erhielt einen schwach gelblichen kristallinen Rückstand, der ohne weitere Reinigung in die Folgereaktion einging.
(d) (D)-Phenylalanyl-prolin-(D,L)-α-methyl-4-amidino-benzylamid:
   4,1 g der vorstehenden Verbindung und 4 ml Triethylamin wurden in 40 ml Pyridin gelöst, bei 0°C mit H₂S gesättigt und über Nacht bei Raumtemperatur stehen gelassen. Gemäß DC-Kontrolle (CH₂Cl₂/MeOH, 9/1) war die Umsetzung zum Thioamid vollständig. Zur Isolierung wurde das Pyridin im Vakuum weitgehend abdestilliert, der Rückstand in 250 ml Essigester aufgenommen und mit Kochsalz-, 5%iger Zitronensäure- und NaHCO₃-Lösung gewaschen. Nach Trocknen und Abdestillieren des Lösungsmittels erhielt man 4,1 g reines kristallines Thioamid.
   Das Thioamid wurde in 150 ml Aceton gelöst und bei Raumtemperatur nach Zusatz von 7 ml Methyliodid 6 h stehen gelassen. Nach Abziehen des Lösungsmittels wurde der amorphe Rückstand mit trockenem Ether ausgerührt und anschließend getrocknet. Das S-Methyl-thioimidsäuremethylester-hydroiodid wurde in 50 ml Ethanol gelöst, mit 15 ml 10%iger Ammoniumacetatlösung versetzt und 3 h auf 60°C erwärmt. Zur Isolierung wurde das Lösungsmittel abgezogen, der Rückstand in 100 ml CH₂Cl₂ gelöst, die unlöslichen Bestandteile abfiltriert und anschließend das CH₂Cl₂ abdestilliert. Durch Digerieren mit einem Essigester-Diethylether-Gemisch wurden die darin löslichen Verunreinigungen abgetrennt. Das verbliebene Iodid-Acetat-Mischsalz wurde in Aceton/Wasser (3/2) gelöst und mittels eines IRA-Acetat-Ionenaustausches in das reine Acetat überführt und anschließend gefriergetrocknet. Man isolierte ein weißes Pulver, Fp: 110-115°C; FAB-MS: 508 (M+H⁺)
(e) H-(D)-Phe-Pro-NH-pAm[(D,L)-α-Me]-benzyl:
   Die vorstehende Verbindung wurde in 70 ml CH₂Cl₂ gelöst und mit 80 ml HCl-gesättigtem Essigester versetzt. Nach kurzer Zeit schied sich ein Niederschlag aus, der durch Zugabe von Ether vervollständigt wurde. Dieser wurde abgesaugt, mit Ether HCl-frei gewaschen und im Vakuum getrocknet. Man erhielt weiße Kristalle, Fp 190 - 195°C (Dihydrochlorid),
   FAB-MS: 407 (M⁺).

### Beispiel 23:

### Me-(D)-Phe-Pro-(D bzw. L) (4 Am)-PhΨ[CH₂OH]/a:

N-(p-Cyanobenzyl)-benzophenonimin wurde in Acetonitril mit wasserfreiem Kaliumcarbonat, Tetrabutylammonium-jodid und Paraformaldehyd hydroxymethyliert. Weiße Kristalle: Fp 115-117°C. Anschließend wurde die Alkoholgruppe mit t-Butyldimethylsilylchlorid verethert und mittels 0,1 N Methansulfonsäure in THF zum geschützten (D,L)-α-Hydroxymethyl-p-cyanobenzylamin gespalten. Dieses Amin wurde nach Standardbedingungen mit Z-Me-(D)-Phe-Pro-OH gekuppelt und mit H₂S in das Thioamid überführt, das säulenchromatographisch in die Diastereomeren getrennt wurde. Das reine Diastereomer a wurde amidiert und anschließend säurekatlysiert bzw. hydrogenolytisch die Schutzgruppen abgespalten. Die nach Gefriertrocknung erhaltenen weißen Kristalle schmolzen bei 175-180°C, FAB-MS: 438 (M+H⁺).

### Beispiel 24:

### Me-(D)-Phe-Pro-(D bzw. L)(4 Am)-PhΨ[CH₂OH]/b:

Aus dem diastereomeren-reinen Thioamid b wurden durch Amidierung und Schutzgruppen-Abspaltung amorphe weiße Kristalle erhalten,
FAB-MS: 438 (M+H⁺).

### Beispiel 25:

### Boc-(D)-Phe(4-F)-Pro-NH-pAmb:

Hergestellt durch Umsetzung von Boc-D-Phe(4-F)-OH mit N-(4-Cyanobenzyl)prolin-amid und nachfolgender Amidierung. Weiße Kristalle, Fp: 184-187°C (Hydroacetat)

### Beispiel 26:

### H-(D)-Phe(4-F)-Pro-NH-pAmb:

Abspaltung der Boc-Gruppe nach Standardbedingungen aus Verbindung 25. Dihydrochlorid: Weiße Kristalle, Fp: 225-230°C, FAB-MS:
412 (M+H⁺)

### Beispiel 27:

### Boc-(D)-Phe(4-Cl)-Pro-NH-pAmb:

Die Verbindung wurde hergestellt analog Beispiel 3 aus Boc-(D)-Phe(4-Cl)-OH; Fp: 124-137°C (Hydroacetat)

### Beispiel 28:

### H-(D)-Phe(4-Cl)-Pro-NH-pAmb:

Die Abspaltung der Boc-Gruppe von Beispiel 27 wurde analog A.I.c. durchgeführt. Man erhielt die Verbindung als Dihydrochlorid;
Fp: 221-234°C

### Beispiel 29:

### Boc-(D,L)-Phe(4-Br)-Pro-NH-pAmb:

Die Verbindung wurde ausgehend von Boc-(D,L)-Phe(4-Br)-OH und H-Pro-p-cyano-benzylamid x HCl analog Beispiel 3 hergestellt.
¹H-NMR (D₆-DMSO, δ in ppm): 8,4/8,1 (t, 1H, NH); 7,78 (2d, 2H, Ar-H); 7,2 (m, 2H, Ar-H); 7,0 (2d, 1H, NH); 4,5 - 4,2 (m, 4H, CH2/2 α-H); 3,6 (m, 2H, Pro); 3.0 - 2,6 (m, 2H, CH2-Ph); 2,15 - 1,7 (m, 4H, β/γ-Pro); 1,3/1,2 (2d, 9H, Boc)
FAB-MS: 575/574 (M+H⁺); mp: 171°C (Zers.) (Hydroacetat)

### Beispiel 30:

### H-(D,L)-Phe(4-Br)-Pro-NH-pAmb:

Die Verbindung wurde durch Boc-Abspaltung aus Beispiel 29 hergestellt.
¹H-NMR (d₆-DMSO, δ in ppm): 9,2 (b, 4H); 8,6/8,5 (2t, 1H, NH); 7,8 (2d, 2H, Ar-H); 7,4 (m, 4H, Ar-H); 7,2 (m, 2H, Ar-H); 7,2 (b, 3H, NH); 4,38 (2dd, 3H, CH2/α-H); 4,2 (m, 1H, α-H); 3,8 - 3,6 (m, 2H, Pro); 3,1 - 2,8 (m, 2H, CH2); 2,2 - 1,7 (m, 4H, Pro);
FAB-MS: 474 (M+H⁺); mp: 56°C (Zers.) (Dihydroacetat)

### Beispiel 31:

### H-(D)-Phe(4-OH)-Pro-NH-pAmb:

Die Verbindung erhielt man durch hydrogenolytische Abspaltung der Benzylgruppe mit Pd/C analog der üblichen Z-Schutzgruppenspaltung (A.I.b.) aus Beispiel 37. Nach Abfiltrieren des Katalysators und Abziehen des Lösungsmittels erhielt man das Dihydrochlorid durch Fällung mit etherischer HCl.
Fp: 129-140°C

### Beispiel 32:

### Boc-(D)-Phe(4-MeO)-Pro-NH-pAmb:

Die Herstellung erfolgte analog Beispiel 3 aus
Boc-(D)-Phe(4-MeO)OH;
Fp: 67-83°C (Hydroacetat)

### Beispiel 33:

### H-(D)-Phe(4-MeO)-Pro-NH-pAmb:

Die Abspaltung der Boc-Gruppe von Beispiel 32 wurde analog A.I.C. durchgeführt.
Fp: 215-227°C (Dihydrochlorid)

### Beispiel 34:

### Boc-(D,L)-Phe(4-EtO)-Pro-NH-pAmb:

Die Herstellung erfolgte analog Beispiel 3 aus
Boc-(D,L)-Phe(4-EtO)OH;
Fp: 115-145°C (Hydroacetat)

### Beispiel 35:

### H-(D,L)-Phe(4-EtO)-Pro-NH-pAmb:

Die Abspaltung der Boc-Gruppe von Beispiel 34 wurde analog A.I.c. durchgeführt.
Fp: 218-230°C (Dihydrochlorid)

### Beispiel 36:

### Boc-(D)-Phe(4-BzlO)-Pro-NH-pAmb:

Die Herstellung erfolgte analog Beispiel 3 aus
Boc-(D)-Phe(4-BzlO)OH:
Fp: 111-125°C (Hydroacetat)

### Beispiel 37:

### H-(D)-Phe(4-BzlO)-Pro-NH-pAmb:

Die Abspaltung der Boc-Gruppe von Beispiel 36 wurde analog A.I.c. durchgeführt;
Fp: 201-210°C (Dihydrochlorid)

### Beispiel 38:

### Boc-(D,L)-Phe(4-Et)-Pro-NH-pAmb:

Die Herstellung erfolgte analog Beispiel 3 aus Boc-(D,L)-Phe(4-Et)OH zum Produkt - Hydroacetat.
¹H-NMR (DMSO-d₆, δ in ppm): 9,25 (2H, Amidin), 8,85 (2H, Amidin), 8,42 und 8,09 (zusammen 1H, NH), 7,80 - 6,95 (9H, Aromaten-H und NH), 4,45 - 4,20 (4H, 2 x CH und 1 x CH₂), 3,75 - ca. 3,0 (2H, CH₂), 3,0 - 2,6 (2H, CH₂), 2,6 - ca. 2,5 (2H, CH₂), 2,3 - 1,5 (4H, 2 x CH₂, 1,3 - ca. 1,2 (9H, Boc), ca. 1,2 - 1,05 (3H, CH₃)

### Beispiel 39:

### H-(D,L)-Phe(4-Et)-Pro-NH-pAmb:

Das Dihydrochlorid erhielt man durch Abspaltung der Boc-Gruppe aus Beispiel 38 analog A.I.c.
¹H-NMR (DMSO-d₆, δ in ppm): 9,55 - 9,40 (2H, Amidin), 9,32 - 9,20 (2H, Amidin), 8,90 und 8,70 (zusammen 1H, NH), 8,85 - 8,75 und 8,40 - 8,30 (zusammen 3H, NH₃⁺), 7,90 - 7,05 (8H, Aromaten-H), 4,50 - 4,10 (4H, 1 x CH₂ und 2 x CH), 2,6 - ca. 2,5 (2H, CH₂), 1,9 - 1,3 (4H, 2 x CH₂), 1,2/1,1 (3H, CH₃)

### Beispiel 40

### Boc-(D,L)-Phe(4-iPr)-Pro-NH-pAmb:

Man erhielt das Hydroacetat durch Umsetzung von
Boc-(D,L)-Phe(4-iPr)OH analog Beispiel 3.
¹H-NMR (DMSO-d₆, δ in ppm): 9,25 (2H, Amidin), 8,50 (2H, Amidin), 8,43 und 8,09 (zusammen 1H, NH), 7,80 - 6,95 (9H, Aromaten-H und NH), 4,45 - 4,20 (4H, 2 x CH und 1 x CH₂), 3,7 - ca. 3,2 (2H, CH₂), 3,0 - 2,6 (3H, CH₂ und CH), 2,2 - 1,5 (4H, 2 x CH₂), 1,3-ca. 1,2 (9H, Boc), ca. 1,2 - 1,05 (6H, 2 x CH₃)

### Beispiel 41:

### H-(D,L)-Phe(4-iPr)-Pro-NH-pAmb:

Das Dihydrochlorid erhielt man durch Abspaltung der Boc-Gruppe aus Beispiel 40 analog A.I.c.
¹H-NMR (DMSO-d₆, δ in ppm): 9,50 - 9,40 (2H, Amidin), 9,30 - 9,20 (2H, Amidin), 8,90 und 8,70 (zusammen 1H, NH), 8,75 und 8,30 (zusammen 3H, NH₃⁺), 7,85 - 7,10 (8H, Aromaten-H), 4,50 - 4,10 (4H, 1 x CH₂ und 2 x CH), ca. 3,8 - 3,3 (2H, CH₂), 3,3 - 2,8 (3H, CH₂ und CH), 2,4 - 1,3 (4H, 2 x CH₂), 1,20 (6H, 2 x CH₃)

### Beispiel 42:

### Z-(D)-Phe(4-tBuO)-Pro-NH-pAmb:

Man erhielt das Hydroacetat durch Umsetzung von
Z-(D)-Phe(4-tBuO)OH analog Beispiel 3.
Fp: 92-104°C

### Beispiel 43

### H-(D)-Phe(4-tBuO)-Pro-NH-pAmb:

Die Z-Schutzgruppe aus Beispiel 42 wurde hydrogenolytisch mit Pd/C analog A.I.b. abgespalten. Man erhielt das Produkt als Dihydroacetat;
Fp: 94-102°C

### Beispiel 44:

### Boc-(D,L)-Phe(4-tBu)-Pro-NH-pAmb:

Das Hydroacetat erhielt man durch Umsetzung von
Boc-(D,L)-Phe(4-tHu)OH analog Beispiel 3.
Fp: 151-158°C

### Beispiel 45:

### H-(D,L)-Phe(4-tBu)-Pro-NH-pAmb:

Das Hydrochlorid erhielt man durch Abspaltung der Boc-Gruppe aus Beispiel 44 analog A.I.c.
Fp: 96-110°C

### Beispiel 46:

### H-(D,L)-Phe(4-Ph)-Pro-NH-pAmb:

Analog Beispiel 3 wurde Boc-(D,L)-Phe(4-Ph)OH zum
Boc-(D,L)-Phe(4-Ph)-Pro-NH-pAmb Hydroacetat umgesetzt und die Boc-Gruppe anschließend analog A.I.c. abgespalten.
¹H-NMR (DMSO-d₆, δ in ppm): 9,50 - 9,40 (2H, Amidin), 9,30 - 9,20 (2H, Amidin), 8,90 und 8,70 (zusammen 1H, NH), 8,80 und 8,35 (zusammen 3H, NH₃⁺), 7,85 - 7,25 (13H, Aromaten-H), 4,50 - 4,15 (4H, 1 x CH₂ und 2 x CH), ca. 3,8 - 3,2 (2H, CH₂), 3,10 - 2,95 (2H, CH₂), 2,6 - 1,3 (4H, 2 x CH₂)

### Beispiel 47:

### Boc-(D,L)-Phe(4-nBu)-Pro-NH-pAmb:

Die Verbindung 47 wurde ausgehend von Boc-(D,L)-Phe(4-Bu)-OH und H-Pro-p-cyano-benzylamid x HCl analog Beispiel 3 synthetisiert.
¹H-NMR (d₆-DMSO, δ in ppm): 10,0 - 9,2 (b, NH); 8,4/8,0 (2t, 1H, NH); 7,78 (2d, 2H, Ar-H); 7,42 (m, 3H, Ar-H); 7,25 - 7,0 (m, 4H, Ar-H/NH); 4,4 - 4,2 (m, 4H, CH2/α-Phe/α-Pro); 3,7 - 3,0 (m, 2H, δ-Pro/CH2); 3,0 - 2,6 (m, 4H, 2 CH2-Ph); 2,2 - 1,7 (m, 5H, β/γ-Pro); a,5 (m, 3H, β/γ-Pro/CH2); 1,25 (m, 9H, Boc); 0,95 (t, 3H, CH3) - (Hydroacetat)
MS: 550 (M+H⁺); 4,50 (-Boc); 247, 185, 134

### Beispiel 48:

### H-(D,L)-Phe(4-nBu)-Pro-NH-pAmb:

Die Verbindung wurde durch Boc-Abspaltung aus Beispiel 47 hergestellt.
¹H-NMR (d₆-DMSO, δ in ppm): 9,4 (d, 2H, NH); 9,2 (d, 2H, NH); 8,9/8,6 (2t, 1H, NH); 7,75 (2d, 2H, Ar-H); 7,45 (d, 2H, Ar-H); 7,23 (d, 1H, NH); 7,15 (m, 4H, Ar-H); 4,4 - 4,2 (3m, 4H,
CH2/2 α-H); 3,6 (m, 2H, γ-Pro/CH2); 3,1/2,9 (2m, 2H, CH2-Ph); 2,56/2,4 (2m,2H, CH2); 2,1/1,9/1,6/1,3 (4m, 11H, β/γ-Pro/CH2); 0,95 (2t, 3H, CH3)
MS: 450 (M+H⁺); 247, 176; Beispiel 48 liegt als Dihydroacetat vbr.

### Beispiel 49:

### Boc-(D)-Phe(4-COOMe)-Pro-NH-pAmb:

a) Boc-(D)-Phe(4-COOMe)-Pro-NH-p-cyano-benzyl:
   12,5 g Boc-(D)-Tyr(Bzl)OH wurden analog Beispiel 3 zu 18,9 g Boc-(D)-Tyr(Bzl)-Pro-p-cyanobenzylamid umgesetzt, in 780 ml MeOH gelöst und mit Pd/C bei Raumtemperatur hydriert. Nach 6 H wurde der Katalysator abfiltriert und das Lösungsmittel abgezogen. Man erhielt in quantitativer Ausbeute Boc-(D)-Tyr-Pro-p-cyanobenzylamid.
   12,5 g dieser Verbindung wurden in 50 ml Pyridin gelöst und bei 0°C mit 4,7 ml Trifluormethansulfonsäureanhydrid 1 h gerührt. Nach einer weiteren Stunde bei Raumtemperatur wurde das Gemisch auf Wasser gegossen und mit Essigester extrahiert. Die organische Phase wurde mit Wasser gewaschen und über Na₂SO₄ getrocknet. Man erhielt 14 g
   Boc-(D)-Tyr(SO₂CF₃)-Pro-p-cyanobenzylamid. 4 g des Triflats wurden in 1,93 ml TEA, 134 mg Bisdiphenylphosphinopropan, 73 mg Pd-II-acetat, 1 ml MeOH und 40 ml DMF vorgelegt und bei 60 - 70°C mit Kohlenmonoxid begast, bis kein Gas mehr aufgenommen wurde. Das Gemisch wurde auf gesättigte Kochsalzlösung gegossen und mit Essigester extrahiert. Nach dem Waschen der organischen Phase mit 10 %-iger Zitronensäurelösung, Trocknen über Na₂SO₄ und Abziehen des Lösungsmittels erhielt man 3,3 g Boc-(D)-Tyr(4-COOMe)-Pro-p-cyanobenzylamid.
b) Boc-(D)-Phe(4-COOMe)-Pro-NH-pAmb:
   1,7 g des vorstehenden Nitrils wurde analog Beispiel 3 zum Amidin-Hydrojodid umgesetzt. Man erhielt nach säulenchromtographischer Reinigung über Kieselgel 650 mg.
   ¹H-NMR (DMSO-d₆, δ in ppm): 9,4 - 8,7 (4H, Amidin), 8,9/8,1 (1H, NH (2 Rotamere)), 7,9 - 7,2 (9H, Aromaten-H und NH), 3,85 (3H, COOMe), 1,3 - 1,2 (9H, Boc)

### Beispiel 50:

### H-(D)-Phe(4-COOMe)-Pro-NH-pAmb:

1,3 g der Verbindung aus Beispiel 49 wurden über einen Acetationaustauscher zum Amidin-Hydroacetat umgewandelt und die Boc-Gruppe analog A.I.c. abgespalten. Man erhielt 1,0 g des Produkts als Dihydrochlorid; Fp: 204-207°C (Zersetzung)

### Beispiel 51:

### H-(D)-Phe(4-NO₂)-Pro-NH-pAmb:

a) Boc-prolin-(p-amidinobenzyl)-amid
   Boc-prolin-(p-cyanobenzyl)amid (Herstellung s. Beispiel 5) wurde analog Vorschrift A.III.1. mittels H₂S in das Thioamid und anschließend in das Amidin überführt. Das Amidin wurde in Form weißer Kristalle, Fp. 237-239°C, erhalten.
   FAB-MS (M+H⁺) = 347.
b) N-(p-Ammidinobenzyl)-prolinamid-dihydrochlorid:
   Analog A.I.c. wurde aus vorstehender Verbindung die Boc-Schutzgruppe abgespalten. Das Dihydrochlorid wurde als sehr hygroskopisches Pulver, Fp. 130 bis 140°C, isoliert.
   FAB-MS (M+H⁺) = 247.
c) Boc-(D)-Phe(4-NO₂)-Pro-NH-pAmb:
   Eine Lösung von 3,9 g (12,6 mMol) Boc-(D)-Phe(4-NO₂)OH in 40 ml THF wurde nach Zugabe von 1,9 g (12,6 mMol) 1-Hydroxybenzotriazol und 3,3 g (25 mMol) Dicyclohexylcarbodiimid 4 h bei Raumtemperatur gerührt. Der ausgefallene Harnstoff wurde abgesaugt und mit wenig THF nachgewaschen.
   Zu diesem Filtrat wurde bei 5°C eine Lösung von 4,1 g (12,6 mMol) N-(p-Amidinobenzyl)prolinamid-dihydrochlorid und 1,6 g Natriumhydrogencarbonat in 6 ml Wasser zugefügt. Nach 48 h Rühren bei Raumtemperatur wurde das Losungsmittel weitgehend abdestilliert, der Rückstand in Ethanol aufgenommen, von ungelöstem abfiltriert und erneut eingeengt.
   Der Rückstand wurde über eine Kieselgelsäule mit einem CH₂Cl₂MeOH/50 %iger Essigsäure-Gemisch (45/5/1,5) gereinigt. Das Eluat der einheitlichen Fraktionen wurde abdestilliert, gegen Ende mit Toluol als Zusatz, und der Rückstand aus 50 ml Aceton unter Zusatz von wenig Wasser umkristallisiert. Man isolierte 3,3 g (48 % d.Th.) Amidinacetat in Form weißer Kristalle, Fp. 162 bis 165°C. FAE-MS: = 539.5 (M+H⁺)
d) H-(D)-Phe(4-NO₂)-Pro-NH-pAmb:
   Die Abspaltung der Boc-Gruppe erfolgte analog A.I.c. Dihydrochlorid: weiße Kristalle, Fp. 218-225°C (Zersetzung),
   FAB-MS: 439 (M+H⁺).

### Beispiel 52:

### Boc-(D,L)-Phe(3-F)-Pro-NH-pAmb:

Die Verbindung wurde ausgehend von Boc-(D,L)-Phe(3-F)-OH und H-Pro-p-cyano-benzylamid x HCl analog Beispiel 3 hergestellt.
¹H-NMR (d₆-DMSO, δ in ppm): 9,4 - 9,0 (b, N-H); 8,9/8,4/8,15 (3t, 1H, NH); 7,8 (sb, 2H, Ar-H); 7,45 (2d, 2H, Ar-H); 7,35 - 6,9 (2m, 5H, Ar-H/NH); 4,5 - 4,2 (m, 4H, NCH2/α-Phe/α-Pro); 3,7/3,5/3,2 (3m, 2H, δ-Pro); 3,0 - 2,7 (2m, 2H, Ar-CH2); 2,2 - 1,7 (3m, 4H, β/γ-Pro); 1,25 (2s, 9H, Boc)
FAB-MS: 511 (M+H⁺)

### Beispiel 53:

### H-(D,L)-Phe(3-F)-Pro-NH-pAmb:

Die Verbindung wurde durch Boc-Abspaltung aus Beispiel 52 hergestellt.
¹H-NMR (d₆-DMSO, δ in ppm): 9,4/9,2 (2d, 4H, N-H); 8,9/8,55 (1H, NH); 8,75/8,3 (2sb, 3H, NH3); 7,8 (sb, 2H, Ar-H); 7,45 (2d, 2H, Ar-H); 7,4 - 7,05 (m, 4H, Ar-H); 4,45 - 4,2 (m, 4H, NCH2/α-Phe/α-Pro); 3,9 - 3,3 (2H, δ-Pro); 3,2 - 2,8 (m, 2H, Ar-CH2); 2,2 - 1,8 (4H, β/γ-Pro)
FAB-MS: 411 (M+H⁺) - (Dihydrochlorid)

### Beispiel 54:

### Boc-(D,L)-Phe(3-Cl)-Pro-NH-pAmb:

Das Hydroacetat wurde analog Beispiel 3 aus Boc-(D,L)-Phe(3-Cl)OH hergestellt;
¹H-NMR (d₆-DMSO, δ in ppm): 9,4 - 8,6 (4H, Amidin), 8,45/8,15 (1H, NH), 7,8 - 7,0 (9H, Aromaten-H und NH), 1,3 - 1,2 (9H, Boc)

### Beispiel 55:

### H-(D,L)-Phe(3-Cl)-Pro-NH-pAmb:

Die Spaltung der Boc-Gruppe von 54 erfolgte analog A.I.c. Man erhielt das Produkt als Dihydrochlorid.
¹H-NMR (d₆-DMSO, δ in ppm): 9,5 - 9,2 (4H, Amidin), 8,95 (1H, NH), 8,8/8,4 (3H, NH₃⁺), 7,9 - 7,2 (8H, Aromaten-H), 4,50 - 4,15 (4H, CH₂ und 2 x CH), 3,8 - ca. 3,3 (2H, CH₂), 3,25 - 2,95 (2H, CH₂), 2,2 - 1,5 (4H, 2 x CH₂)

### Beispiel 56:

### H-(D,L)-Phe(3-OH)-Pro-NH-pAmb:

Die Verbindung wurde durch Boc-Abspaltung aus
Boc-(D,L)-Phe(3-OH)-Pro-NH-pAmb hergestellt.
¹H-NMR (d₆-DMSO, δ in ppm): 9,6/9,0 (mb/N-H); 8,8/8,5 (2t, 1H, NH); 8,2 (sb, N-H); 7,8 (2d, 2H, Ar-H); 7,5 (2d, 2H, Ar-H); 7,1 (m, 1H, Ar-H); 6,8 - 6,6 (m, 3H, Ar-H), 4,4 - 4,1 (m, 4H, CH2/2 α-H); 3,8 - 3,6 (m, 2H, Pro); 3,0/2,8 (2m, 2H, CH2); 2,1 - 1,5 (m, 4H, β/γ-Pro)
FAB-MS: 410 (M+H⁺; mp: 168°C (Zers.) - (Dihydroacetat)

### Beispiel 57:

### Boc-(D,L)-Phe(3-MeO)-Pro-NH-pAmb:

Das Hydroacetat wurde hergestellt aus Boc-(D,L)-Phe(3-MeO)OH analog Beispiel 3
¹H-NMR (d₆-DMSO, δ in ppm): 9,4 - 8,7 (4H, Amidin), 8,4/8,1 (1H, NH), 7,8 - 6,7 (9H, Aromaten-H und NH), 3,7 (3H, OCH₃, 1,3 - 1,2 (9H, Boc)

### Beispiel 58:

### H-(D,L)-Phe(3-MeO)-Pro-NH-pAmb:

Die Spaltung der Boc-Gruppe von 57 erfolgte analog A.I.c. Man erhielt das Produkt als Dihydrochlorid.
¹H-NMR (DMSO-d₆, δ in ppm): 9,55 - 9,15 (4H, Amidin), 8,9/8,7 (1H, NH), 8,75/8,30 (3H, NH₃⁺), 7,9 - 6,7 (8H, Aromaten-H), 4,5 - 4,1 (4N, CH₂ und 2 x CH), 3,75/3,72 (3H, OCH₃), 3,3 - 2,9 (2H, CH₂), 2,2 - 1,4 (4H, 2 x CH₂)

### Beispiel 59:

### Boc-(D,L)-Phe(3-PhO)-Pro-NH-pAmb:

Die Verbindung wurde hergestellt aus Boc-(D,L)-Phe(3-PhO)OH. Man erhielt das Amidin-Hydroacetat analog Beispiel 3.

### Beispiel 60:

### H-(D,L)-Phe(3-PhO)-Pro-NH-pAmb:

Das Dihydrochlorid erhielt man durch Boc-Spaltung aus Beispiel 59.
¹H-NMR (d₆-DMSO, δ in ppm): 9,5 - 9,2 (4H, Amidin), 8,9/-8,2 (1H, NH), 8,75/8,35 (3H, NH₃⁺), 7,85 - 6,80 (8H, Aromaten-H), 4,50 - 4.10 (4H, CH₂ und 2 x CH), 3,8 - 2,9 (4H, 2 x CH₂), 2,8 - 1,5 (4H, CH₂)

### Beispiel 61:

### Boc-(D,L)-Phe(3-Me)-Pro-NH-pAmb:

Die Verbindung wurde ausgehend von Boc-(D,L)-Phe(3-Me)-OH und H-Pro-p-cyano-benzylamid x HCl analog Beispiel 3 hergestellt.
¹H-NMR (d₆-DMSO, δ in ppm): 8/8,4/8,05 (3t, 1H, NH); 7,74 (2d, 2H, Ar-H); 7,45 (2d, 2H, Ar-H); 7,2 - 6,9 (m, 5H, Ar-H/NH); 4,4 - 4,2 (m, 4H, NCH2/α-Pro/α-Phe); 3,7 - 3,1 (2m, 2H, δ-Pro); 2,9 - 2,7 (2H, Ar-CH2); 2,5 (2s, 3H, CH3); 2,1 - 1,6 (m, 4H, β/γ-Pro); 1,25 (2s, 9H, Boc)
MS: 508 (M+H⁺), 408 (-Boc), 277, 247 - (Hydroacetat)

### Beispiel 62:

### H-(D,L)-Phe(3-Me)-Pro-NH-pAmb:

Die Verbindung wurde durch Boc-Abspaltung aus Beispiel 61 hergestellt.
MS: 408 (M+H⁺), 247, 185, 134, 93 - (Dihydroacetat)

### Beispiel 63:

### H-(D,L)-Phe(3-Ph)-Pro-NH-pAmb:

Die entsprechende Boc-geschutzte Verbindung wurde analog Beispiel 3 aus Boc-(D,L)-Phe(3-Ph)OH hergestellt und anschließend analog A.I.c. zum Dihydrochlorid gespalten.
¹H-NMR (DMSO-d₆, δ in ppm): 9,5 - 9,2 (4H, Amidin), 8,9/ca. 8,7 (1H, NH), 8,8/8,35 (3H, NH₃⁺), 7,85 - 7,25 (13H, Aromaten-H), 4,5 - 4,15 (4H, CH₂ und 2 x CH), 3,2 - 3,00 (2H, CH₂), 2,2 - 1,4 (4H, 2 x CH₂)

### Beispiel 64:

### Boc-(D,L)-Phe(3-CF₃)-Pro-NH-pAmb:

Die Verbindung wurde ausgehend von Boc-(D,L)-Phe(3-CF₃)-OH und H-Pro-p-cyano-benzylamid x HCl analog Beispiel 3 hergestellt.
¹H-NMR (d₆-DMSO, δ in ppm): 10,0 - 9,2 (b, NH); 8,9/8,4/8.15 (3t, 1H, NH); 7,8 - 7,4 (8H, Ar-H); 7,22/7,05 (2d, 1H, NH); 4,6 - 4,2 (4H, N-CH2/α-Pro/α-H); 3,8 - 3,4 (2H, δ-Pro); 3,1/2,8 (2H, Ar-CH2); 2,1 - 1,6 (4H, β/γ-Pro); 1,2 (2s, 9H, Boc)
MS: 562 (M+H⁺), 462 (-Boc), 247, 188, 134 - (Hydroacetat)

### Beispiel 65:

### H-(D,L)-Phe(3-CF₃)-Pro-NH-pAmb:

Die Verbindung wurde durch Boc-Abspaltung aus Beispiel 64 hergestellt.
¹H-NMR (d₆-DMSO, δ in ppm): 9,4 (2s, 2H, NH); 9,2 (2s, 2H, NH); 8,9/8,8 (2t, 1H, NH); 8,8/8,6/8,4 (3sb, 3H, NH3); 7,8 - 7,4 (8H, Ar-H); 4,42 - 4,1 (4H, N-CH2/α-Pro/α-H); 3,8 (m 1H, δ-Pro/Ar-CH2); 2,2 - 1,5 (4H, β/γ-Pro)
Fp: 195-7°C - (Dihydroacetat)

### Beispiel 66:

### Boc-(D,L)-Phe(2-F)-Pro-NH-pAmb:

Die Verbindung wurde ausgehend von Boc-(D,L)-Phe(2-F)-OH und H-Pro-NH-pCNb x HCl analog Beispiel 3 hergestellt.
¹H-NMR (d₆-DMSO, δ in ppm): 9,8 - 9,2 (b, N-H); 8,5/8,2 (2t, 1H, NH); 7,75 (2d, 2H, Ar-H); 7,45 (2d, 2H, Ar-H); 7,4 - 7,0 (m, 5H, Ar-H/NH); 4,6 - 4,2 (m, 4H, NCH2/α-Phe/α-Pro); 3,6 - 3,0 (4m, 2H, δ-Pro); 2,9 - 2,7 (2m, 2H, Ar-CH2); 2,2 - 1,7 (3m, 4H, β/γ-Pro); 1,2 (2s, 9H, Boc)
MS: 512 (M+H⁺), 412 (-Boc), 247, 134 - (Hydroacetat)

### Beispiel 67:

### H-(D,L)-Phe(2-F)-Pro-NH-pAmb:

Die Verbindung wurde durch Boc-Abspaltung aus Beispiel 66 hergestellt.
¹H-NMR (d₆-DMSO, δ in ppm): 9,4 (2s, 2H, N-H); 9,15 (2s, 2H, N-H), 8,9/8,6 (2sb, 3H, NH); 7,8 (2d, 2H, Ar-H); 7,45 (2d, 2H, Ar-H); 7,4 - 7,1 (m, 4H, Ar-H); 4,5 - 4,2 (3m, 4H, NCH2/α-Phe/α-Pro); 3,6 - 3,2 (2m, 2H, δ-Pro); 3,0/2,7 (2m, 2H, Ar-CH2); 2,2 - 1,5 (5m, 4H, β/γ-Pro)
MS 412 (M+H⁺), 247, 134 - (Dihydrochlorid)

### Beispiel 68:

### Boc-(D,L)-Phe(2-Cl)-Pro-NH-pAmb:

Das Hydroacetat wurde hergestellt aus Boc-(D,L)-Phe(2-Cl)-OH analog Beispiel 3.

### Beispiel 69:

### H-(D,L)-Phe(2-Cl)-Pro-NH-pAmb:

Das Dihydrochlorid erhielt man aus Beispiel 68 analog A.I.c.
¹H-NMR (d₆-DMSO, δ in ppm): 9,5 - 9,2 (4H, Amidin), 8,9/8,7 (1H, NH); 8,85/8,45 (3H, NH₃⁺), 7,9 - 7,2 (8H, Aromaten-H), 4,5 - 4,1 (4H, CH₂ und 2 x CH), 3,8 - 3,0 (4H, 2 x CH₂), 2,2 - 1,4 (4H, 2 x CH₂)

### Beispiel 70:

### Boc-(D,L)-Phe(2-OH)-Pro-NH-pAmb:

Die Verbindung wurde ausgehend von Boc-(D,L)-Phe(2-OH)-OH und H-Pro-p-cyano-benzylamid x HCl analog Beispiel 3 hergestellt.
¹H-NMR (d₆-DMSO, δ in ppm): 8,4/8,0 (2t, 1H, NH); 7,7 (2d, 2H, Ar-H); 7,4 (2d, 2H, Ar-H); 7,2/7,15 (2d, 1H, NH); 7,0 - 6,6 (4H, Ar-H); 4,45 - 4,2 (m, 4H, H-CH2/α-Pro/α-H); 3,8 - 3,2 (2H, δ-Pro); 3,0/2,8 (2m, 2H, Ar-CH2); 2,1 - 1,6 (4H, β/γ-Pro); 1,2 (2s, 9H, Boc)
MS: 510 (M+H⁺), 410 (-Boc), 247, 134 - (Hydroacetat)

### Beispiel 71:

### H-(D,L)-Phe(2-OH)-Pro-NH-pAmb

Die Verbindung wurde durch Boc-Abspaltung aus Beispiel 70 hergestellt.
¹H-NMR (d₆-DMSO, δ in ppm): 9,4 - 9,0 (b, NH); 8,85/8,5 (2t, 1H, NH); 7,8 (2d, 2H, Ar-H); 7,5 (2d, 2H, Ar-H); 7,2 - 6,7 (4H, Ar-H); 4,4 - 3,7 (m, 4H, N-CH2/α-Pro/α-H); 3,4 - 3,2 (2H, δ-Pro); 3,1 - 2,75 (2H, Ar-CH2); 2,1 - 1,4 (4H, β/γ-Pro)
MS: 410 (M+H⁺), 369, 277, 247 - (Dihydroacetat)

### Beispiel 72:

### Hoc-(D,L)-Phe(2-MeO)-Pro-NH-pAmb:

Das Hydroacetat erhielt man aus Boc-(D,L)-Phe(2-MeO)OH analog Beispiel 3.

### Beispiel 73:

### H-(D,L)-Phe(2-MeO)-Pro-NH-pAmb:

Das Dihydrochlorid erhielt man aus Beispiel 72 analog A.I.c.
¹H-NMR (d₆-DMSO, δ in ppm): 9,55 - 9,25 (4H, Amidin), 8,90/8,65 (1H, NH) ; 8,7/8,2 (3H, NH₃⁺), 7,9 - 6,8 (8H, Aromaten-H), 4,5 - 4,1 (4H, CH₂ und 2 x CH), 3,80 (3H, OCH₃), ∼3,8 - 3,3 (2H, CH₂), 3,2 - 2,6 (2H, CH₂), 2,2 - 1,4 (4H, 2 x CH₂)

### Beispiel 74:

### Boc-(D,L)-Phe(2-Me)-Pro-NH-pAmb:

Die Verbindung wurde ausgehend von Boc-(D,L)-Phe(2-Me)-OH und H-Pro-p-cyano-benzylamid x HCl analog Beispiel 3 hergestellt.
¹H-NMR (d₆-DMSO, δ in ppm): 8.4/8,05 (2t, 1H, NH); 7,75 (2d, 2H, Ar-H); 7,4 (2d, 2H, Ar-H) ; 7,2 - 7,0 (m, 5H, Ar-H) /NH) ; 4,6 - 4,2 (m, 4H, CH2/2 α-H) ; 3,7 - 3,55 (2m, 2H, δ-Pro) ; 3,0 - 2,6 (m, 2H, CH2); 2,3 (2s, 3H, CH3); 2,2 - 1,6 (m, 4H, β/γ-Pro) ; 1,35 - 1,2 (1s, 9H, Boc)
FAB-MS: 508 (M+H⁺) - (Hydroacetat)

### Beispiel 75:

### H-(D,L)-Phe(2-Me)-Pro-NH-pAmb:

Die Verbindung wurde durch Boc-Abspaltung aus Beispiel 74 hergestellt.
¹H-NMR (d₆-DMSO, δ in ppm): 9,35 (s, 2H, N-H); 9,05 (s, 2H, N-H); 8,8/8,5 (2t, 1H, NH); 7,8/7,75 (2d, 2H, Ar-H); 7,5/7,45 (2d, 2H, Ar-H); 7,2 - 7,0 (m, 4H, Ar-H); 4,4 - 4,2 (3m, 4H, CH2/2 α-H); 3,6/,3 (2m, 2H, δ-Pro); 3,1/3,0 (2m, 2H, CH2); 2,38 (m, 3H, CH3); 2,2 - 1,3 (4H, β/γ-Pro)
MS: 408 (M+H⁺), 247, 185, 134 - (Dihydrochlorid)

### Beispiel 76:

### Boc-(D,L)-Phe(2-iPr)-Pro-NH-pAmb:

Das Hydroacetat wurde hergestellt aus Boc-(D,L)-Phe(2-iPr)OH analog Beispiel 3.

### Beispiel 77:

### H-(D,L)-Phe(2-iPr)-Pro-NH-pAmb:

Das Dihydrochlorid erhielt man aus Beispiel 76 analog A.I.c.
Fp: 220-221°C

### Beispiel 78:

### Boc-(D,L)-Phe(2-Ph)-Pro-NH-pAmb:

Das Hydroacetat wurde hergestellt aus Boc-(D,L)-Phe(2-Ph)OH analog Beispiel 3.

### Beispiel 79:

### H-(D,L)-Phe(2-Ph)-Pro-NH-pAmb:

Das Dihydrochlorid erhielt man aus Beispiel 78 analog A.I.c.
¹H-NMR (DMSO-d₆, δ in ppm): 9,5 - 9,2 (4H, Amidin), 8,85/8,67 (1H, NH), 8,6/8,2 (3H, NH₃⁺), 7,85 - 7,15 (13H, Aromaten-H), 4,4 - 3,0 (8H, 3 x CH₂ und 2 x CH), 2,2 - 1,4 (4H, 2 x CH₂)

### Beispiel 80:

### Boc-(D,L)-Phe(3,4-(F)₂)-Pro-NH-pAmb:

Das Hydroacetat wurde hergestellt aus Boc-(D,L)-Phe(3,4-(F)₂)OH (J. Med. Chem. 1967, 10, 64) analog Beispiel 3; weiße Kristalle; Fp: 110-114°C;
FAB-MS: 530 (M+H⁺)

### Beispiel 81:

### H-(D,L)-Phe(3,4-(F)₂)-Pro-NH-pAmb:

Das Dihydrochlorid erhielt man aus Beispiel 80 durch Boc-Abspaltung analog A.I.c.; weiße Kristalle; Fp: 190-195°C (Zersetzung);
FAB-MS: 430 (M+H⁺)

### Beispiel 82:

### Boc-(D,L)-Phe(3,4-(Cl)₂)-Pro-NH-pAmb:

Das Hydroacetat erhielt man durch Umsetzung von
Boc-(D,L)-Phe(3,4-(Cl)₂)-OH (Int. J. Pept. Protein Res. 1987, 30, 13) analog Beispiel 3; weiße Kristalle; Fp:135-138°C;
FAB-MS: 562 (M+H⁺)

### Beispiel 83:

### H-(D,L)-Phe(3,4-(Cl)₂)-Pro-NH-pAmb:

Das Dihydrochlorid erhielt man aus Beispiel 82 durch Boc-Abspaltung analog A.I.c.; weiße Kristalle; Fp: 208-212°C;
FAB-MS: 462 (M+H⁺)

### Beispiel 84:

### Boc-(D,L)-Phe(3-C1-4-MeO)-Pro-NH-pAmb:

Das Hydroacetat wurde hergestellt aus Boc-(D,L)-Phe(3-C1-4-MeO)OH analog Beispiel 3.

### Beispiel 85:

### H-(D,L)-Phe(3-Cl-4-MeO)-Pro-NH-pAmb:

Das Dihydrochlorid erhielt man aus Beispiel 84 durch Boc-Abspaltung analog A.I.c.
¹H-NMR (DMSO-d₆, δ in ppm): 9,58 - 9,30 (4H, Amidin), 8,98/8,85 (1H, NH), 8,8/8,35 (3H, NH₃⁺), 7,9-7,0 (7H, Aromaten-H), 4,50 - 4,20 (4H, CH₂ und 2 x CH), 3,85/3,82 (3H, OCH₃), 3,2 - 2,9 (2H, CH₂), 2,2 - 1,5 (4H, 2 x CH₂)

### Beispiel 86:

### Boc-(D,L)-Phe(3-Cl, 4-OEt)-Pro-NH-pAmb:

Die Verbindung wurde ausgehend von Boc-(D,L)-Phe(3-Cl, 4-OEt)-OH und H-Pro-p-cyano-benzylamid x HCl analog Beispiel 3 als Hydroacetat hergestellt.
FAB-MS: 573 (M+H⁺)

### Beispiel 87:

### H-(D,L)-Phe(3-Cl, 4-OEt)-Pro-NH-pAmb:

Die Verbindung wurde durch Boc-Abspaltung aus Beispiel 86 als Dihydrochlorid hergestellt.
¹H-NMR (d₆-DMSO, δ in ppm): 9,4 (d, 2H, NH); 9,2 (d, 2H, NH); 8,9/8,7 (2t, 1H, NH); 8,4 - 8.2 (b, 3H, NH3); 7,8 (2d, 2H, Ar-H); 7,45 (2d, 2H, Ar-H); 7,3 (m, 1H, Ar-H); 7,2/7,0 (2H, Ar-H); 4,5 - 4,2 (4H, N-CH2/α-Pro/α-Phe); 4,1 (m, 2H, OCH2); 3,7 - 3,1 (m, 2H, δ-Pro); 3,1 - 2,7 (m, H, Ar-CH2); 2,2 - 1,6 (m, 4H, β/γ-Pro); 1,35 (q, 3H, CH3)
MS: 472 (M+H⁺), 247, 134; 70

### Beispiel 88:

### H-(D,L)-Phe(3,4-(MeO)₂)-Pro-NH-pAmb:

Die entsprechende Boc-geschützte Verbindung wurde analog Beispiel 3 aus Boc-(D,L)-Phe(3,4-(MeO)₂)OH hergestellt und anschließend analog A.I.c. zum Dihydrochlorid gespalten.
¹H-NMR (DMSO-d₆, δ in ppm): 9,55 - 9,25 (4H, Amidin), 8,95/ca. 8,8 (1H, NH), 8,8/8,35 (3H, NH₃⁺) 7,9 - 6,7 (7H, Aromaten-H), 4,50 - 4,15 (4H, CH₂ und 2 x CH), 3,75 - 3,68 (6H, 2 x OCH₃), 3,2 - 2,8 (2H, CH₂, 2,2 - 1,4 (4H, 2 x CH₂)

### Beispiel 89:

### Boc-(D,L)-Phe(3,4-(Me)₂)-Pro-NH-pAmb:

Das Hydroacetat wurde hergestellt aus Boc-(D,L)-Phe(3,4-(Me)₂)OH analog Beispiel 3; weiße Kristalle; Fp: 108-112°C;
FAB-MS: 522 (M+H⁺)

### Beispiel 90:

### H-(D,L)-Phe(3,4-(Me)₂)-Pro-NH-pAmb:

Das Dihydrochlorid erhielt man aus Beispiel 89 durch Boc-Abspaltung analog A.I.c.; weiße Kristalle; Fp: 195-200°C,
FAB-MS: 422 (M+H⁺)

### Beispiel 91:

### Boc-(D,L)-Phe(3-Me-4-iPr)-Pro-NH-pAmb:

Das Hydroacetat wurde hergestellt aus Boc-(D,L)-Phe(3-Me-4-iPr)OH analog Beispiel 3.

### Beispiel 92:

### H-(D,L)-Phe(3-Me-4-iPr)-Pro-NH-pAmb:

Das Dihydrochlorid erhielt man aus Beispiel 91 durch Boc-Abspaltung analog A.I.c.
¹H-NMR (DMSO-d₆, δ in ppm): 9,5 - 9,2 (4H, Amidin), 8,9/9,6 (1H, NH), 8,85/8,40 (3H, NH₃⁺), 7,9 - 6,85 (7H, Aromaten-H), 4,5 - 4,0 (4H, CH₂ und 2 x CH), 3,2 - 2,9 (2H, CH₂), 2,32/2,30 (3H, CH₃), 2,2 - 1,4 (4H, 2 x CH₂), 1,2 - 1,1 (6H, 2 x CH₃)

### Beispiel 93:

### Boc-(D,L)-Phe(2,3-(MeO)₂)-Pro-NH-pAmb:

Das Hydroacetat wurde hergestellt aus Boc-(D,L)-Phe(2,3-(MeO)₂)OH analog Beispiel 3.

### Beispiel 94:

### H-(D,L)-Phe(2,3-(MeO)₂)-Pro-NH-pAmb:

Das Dihydrochlorid erhielt man aus Beispiel 93 durch Boc-Abspaltung analog A.I.c.
Das 1.3:1-Diastereomerengemisch zeigte einen Schmelzbereich von 138-140°C (Zersetzung).

### Beispiel 95:

### Boc-(D,L)-Phe(2,5-(MeO)₂)-Pro-NH-pAmb:

Das Hydroacetat wurde hergestellt aus Boc-(D,L)-Phe(2,5-(MeO)₂)OH analog Beispiel 3.

### Beispiel 96:

### H-(D,L)-Phe(2,5-(MeO)₂)-Pro-NH-pAmb:

Das Dihydrochlorid erhielt man aus Beispiel 95 durch Boc-Abspaltung analog A.I.c.
¹H-NMR (DMSO-d₆, δ in ppm): 9,55 - 9,25 (4H, Amidin), 8,95/ca. 8,7 (1H, NH), 8,7/8,2 (3H, NH₃⁺), 7,9 - 7,4 und 7,0 - 6,7 (7H, Aromaten-H), 4,50 - 4,1 (4H, CH₂ und 2 x CH), 3,8/3,7 (6H, 2 x OCH₃), 3,25 - 2,65 (2H, CH₂), 2,2 - 1,5 (4H, 2 x CH₂)

### Beispiel 97:

### Boc-(D,L)-Phe(3,5-(MeO)₂)-Pro-NH-pAmb:

Das Hydroacetat wurde hergestellt aus Boc-(D,L)-Phe(3,5-(MeO)₂)OH analog Beispiel 3.

### Beispiel 98:

### H-(D,L)-Phe(3,5-(MeO)₂)-Pro-NH-pAmb:

Das Dihydrochlorid erhielt man aus Beispiel 97 durch Boc-Abspaltung analog A.I.c.
¹H-NMR (DMSO-d₆, δ in ppm): 9,5 - 9,2 (4H, Amidin), 8,95/ca. 8,7 (1H, NH), 8,7/8,3 (3H, NH₃⁺), 7,85 - 7,40 und 6,60 - 6,35 (7H, Aromaten-H), 4,50 - 4,15 (4H, CH₂ und 2 x CH), 3,75/3,72 (6H, 2 x OCH₃), 3,2 - 2,8 (2H, CH₂), 2,2 - 1,4 (4H, 2 x CH₂)

### Beispiel 99:

### Boc-(D,L)-Phe(3,4,5-(MeO)₃)-Pro-NH-pAmb:

Die Darstellung erfolgte analog Beispiel 3, wobei das Edukt Boc((D,L)-Phe(3,4,5-(MeO)₃)OH durch Alkylierung von Benzophenoniminglycinester mit Trimethoxybenzylchlorid, anschließender Boc-Schutzgruppeneinführung und Esterverseifung hergestellt wurde.
Fp: 109-121°C (Dihydroacetat)

### Beispiel 100:

### H-(D,L)-Phe(3,4,5-(MeO)₃-Pro-NH-pAmb

Die Darstellung erfolgte aus Beispiel 99.
Fp: 180-239°C (Dihydrochlorid)

### Beispiel 101:

### Boc-(D,L)-Phe(2,4,6-(Me)₃)-Pro-NH-pAmb:

Das Hydroacetat wurde hergestellt aus Boc-(D,L)-Phe(2,4,6-(Me)₃)OH analog Beispiel 3.

### Beispiel 102:

### H-(D,L)-Phe(2,4,6-(Me)₃)-Pro-NH-Amb:

Das Dihydrochlorid erhielt man aus Beispiel 101 durch Boc-Abspaltung analog A.I.c.
¹H-NMR (DMSO-d₆, δ in ppm): 9,5 - 9,15 (4H, Amidin), 8,85/ca. 8,7 (1H, NH), 8,8/8,5 (3H, NH₃⁺), 7,9 - 7,4 und 6,9 - 6,75 (6H, Aromaten-H), 4,5 - 4,0 (4H, CH₂ und 2 x CH), ca. 3,7 - 3,3 (2H, CH₂), 3,2 - 3,0 (2H, CH₂), 2,25 - 2,10 (6H, 3 x CH₃), ca. 2,1 - 1,4 (4H, 2 x CH₂)

### Beispiel 103:

### Boc-(D)-α-Nal-Pro-NH-pAmb:

Die Darstellung erfolgte analog Beispiel 3.
Fp: 136-178°C (Hydroacetat)

### Beispiel 104:

### H-(D)-α-Nal-Pro-NH-pAmb:

Die Darstellung erfolgte aus Beispiel 103.
Fp: 228 - 234°C (Dihydrochlorid)

### Beispiel 105:

### H-(D)-β-Nal-Pro-NH-pAmb:

Die Darstellung erfolgte aus Boc-(D)-β-Nal-Pro-NH-pAmb durch Boc-Abspaltung; Fp: 223-229°C (Dihydrochlorid)

### Beispiel 106:

### Boc-(D,L)-α-Ngl-Pro-NH-pAmb:

Die Verbindung wurde ausgehend von Boc-(D,L)-α-Ngl-OH und H-Pro-p-cyano-benzylamid x HCl analog Beispiel 3 hergestellt.
¹H-NMR (d₆-DMSO, δ in ppm): 10,0 (b, NH); 8,7/8,5 (2t, 1H, NH); 8,3 - 7,3 (12H, Ar-H/NH); 6,2/6,1 (2d, 1H, α-Ngl); 4,4 (3H, N-CH2/α-Pro); 3,8 - 2,8 (2H, δ-Pro); 2,2 - 1,7 (4H, β/γ-Pro); 1,3 (2s, 9H, Boc)
MS: 530 (M+H⁺), 430 (-Boc), 247, 134; mp: 183-5°C (Zers.) - (Hydroacetat)

### Beispiel 107:

### H-(D,L)-α-Ngl-Pro-NH-pAmb:

Die Verbindung wurde durch Boc-Abspaltung aus Beispiel 106 hergestellt.
¹H-NMR (d₆-DMSO, δ in ppm): 9,5/9,3 (2d, 4H, NH); 9,0/8,8 (2t, 1H, NH); 8,7 (b, 3H, NH3); 8,4 (m, 1H, Ar-H); 8,1 (2d, 2H, Ar-H); 7,9 (2d, 2H, Ar-H); 7,7 - 7,7 (6H, Ar-H); 6,25/6,18 (2s, 1H, α-Ngl); 4,6 - 4,35 (m, 3H, N-CH2/α-Pro); 3,85/3,6/3,4 (3m, 2H, δ-Pro); 2,2 - 1,6 (4H, β/γ-Pro)
MS: 430 (M+H⁺), 3,69, 277 - (Dihydrochlorid)

### Beispiel 108:

### Boc-(D,L)-β-Ngl-Pro-NH-pAmb:

Die Verbindung wurde ausgehend von Boc-(D,L)-β-Ngl-OH und H-Pro-p-cyano-benzylamid x HCl analog Beispiel 3 hergestellt.
¹H-NMR (d₆-DMSO, δ in ppm): 9,8 - 9,2 (b, NH); 8.6/8,4 (2sb, 1H, NH); 8,0 - 7,75 (6H, Ar-H); 7,6 - 7,5 (5H, Ar-H); 7,35/7,18 (2sb, 1H, NH); 5,6/5,45/5,35 (3sb, 1H, α-Ngl); 4,4 (3H, N-CH2/α-Pro); 3,9/3,7 (2sb, 1H, δ-Pro); 3,2 (sb, 1H, δ-Pro); 2,2 - 1,85 (4H, β/γ-Pro); 1,4 (2s, 9H, Boc)
MS: 530 (M+H⁺), 430 (-Boc), 2,47, 185, 134; Fp. 183-5°C (Zers.) - (Hydroacetat)

### Beispiel 109:

### H-(D,L)-β-Ngl-Pro-NH-pAmb:

Die verbindung wurde durch Boc-Abspaltung aus Beispiel 108 als Dihydroacetat hergestellt.
¹H-NMR (d₆-DMSO, δ in ppm): 9,6 - 9,0 (b, NH); 8,75/8,6 2t, 1H, NH); 8,0 - 7,8 (6H, Ar-H); 7,6 - 7,4 (m, 5H, Ar-H); 5,2 (s, 1H, α-Ngl); 4,5 - 4,3 (m, 3H, N-CH2/α-Pro); 3,9 - 3,0 (2H, δ-Pro); 2,2 - 1,7 (4H, β/γ-Pro)
MS: 430 (M+H⁺), 247

### Beispiel 110:

### H-(D,L)-1-Tic-Pro-NH-pAmb:

Die verbindung wurde ausgehend von Beispiel 255 durch Boc-Abspaltung als Dihydroacetat hergestellt.
¹H-NMR (d₆-DMSO, δ in ppm): 9,4/9,0 (2sb, 4H, NH); 8,7 (b, 1H, NH); 7,75 (2d, 2H, Ar-H); 7,45 (2d, 2H, Ar-H); 7,4 - 6,8 (4H, Ar-H); 5,2 (2s, 1H, α-Tic); 4,8 - 4,4 (3H, N-CH2/α-Pro); 3,6 - 3,2 (4H, Ar-CH2/δ-Pro); 3,0 - 2,7 (2H, N-CH2); 2,2 - 1,8 (4H, β/γ-Pro)

### Beispiel 111:

### Boc-(D)-3-Tic-Pro-NH-pAmb:

Die Verbindung wurde ausgehend von Boc-(D)-3-Tic-PH und H-Pro-p-cyano-benzylamid x HCl analog Beispiel 3 hergestellt.
¹H-NMR (d₆-DMSO, δ in ppm): 8,44/8,2 (2sb, 1H, NH); 7,8/7,65 (2d, 2H, Ar-H); 7,5 (2d, 2H, Ar-H); 7,4/7,2 (m, 4H, Ar-H); 4,8 - 4,6 (m, 2H, CH2); 4,4 - 4,2 (m, 4H, CH2/2 α-H); 3,62 (m, 2H, Pro); 3,1 - 2,6 (m, 2H, CH2-Ph); 2,2 - 1,75 (m, 4H, Pro); 1,3 (2s, 9H, Boc)
MS: 506 (M+H⁺); 406 (-Boc); Fp: 143°C - (Hydroacetat)

### Beispiel 112:

### H-(D)-3-Tic-Pro-NH-pAmb:

Die Verbindung wurde durch Boc-Abspaltung aus Beispiel 111 hergestellt.
FAB-MS: 406 (M+H⁺): Fp: 204°C - (Dihydroacetat)

### Beispiel 113:

### 1-ICC-Pro-NH-pAmb:

Die Verbindung wurde ausgehend von 1-Icc-OH und H-Pro-p-cyano-benzylamid x HCl analog Beispiel 3 hergestellt.
FAB-MS: 402 (M+H⁺)

### Boc-(D,L)-2-Tgl-Pro-NH-pAmb:

Die Verbindung wurde als Hydroacetat ausgehend von Boc-(D,L)-2-Tgl-OH und H-Pro-p-cyano-benzylamid x HCl analog Beispiel 3 hergestellt.
¹H-NMR (d₆-DMSO, δ in ppm): 8,6(8,4 (2t, 1H, NH); 7,75 (2d, 2H, Ar-H) ; 7,45 - 6.9 (6H, Ar-H); 5,7 - 5,4 (1H, α-Tgl); 4,4 (m, 3H, N-CH₂/α-Pro); 3,8 - 3,2 (2H, δ-Pro): 2,1 - 1,7 (4H, β/γ-Pro); 1,35 (2s, 9H, Boc)
MS: 486 (M+H⁺), 386 (-Boc), 247, 185, 134

### Beispiel 115:

### H-(D,L)-2-Tgl-Pro-NH-pAmb:

Die Verbindung wurde durch Boc-Abspaltung aus Beispiel 114 hergestellt.
¹H-NMR (d₆-DMSO, δ in ppm): 9,4/9,2 (2sb,4H, NH); 8,9/8,75 (2t, 1H, NH); (sb, 3H, NH); 7,8 (2d, 2H, Ar-H); 7,62 (2d, 2H, Ar-H); 7,5 (2d, 2H, Ar-H); 7,4, sb, 1H, Ar-H; 7,1 (m, 1H, Ar-H); 5,65/5,6 (2s, 1H, α-Tgl); 4,5 - 4,4 (m, 3H, N-CH₂/α-Pro); 3,95 - 3,75 (2m, 1H, δ-Pro); 3,2/3,0 (2dd, 1H, δ-Pro); 2,2 - 2,0 (1H, β-Pro); 1,9 - 1,7 (3H, β/γ-Prol)
FAB-MS: 386 (M+H⁺) - (Dihydroacetat)

### Beispiel 116:

### Boc-(D)-2-Tal-Pro-NH-pAmb:

Die Verbindung wurde ausgehend von Boc-(D)-2-Tal-OH und H-Pro-p-cyano-benzylamid x HCl analog Beispiel 3 hergestellt.
¹H-NMR (d₆-DMSO, δ in ppm): 8,85/8,15 (2t, 1H, NH); 7,75 (2d, 2H, Ar-H); 7,45 (2d, 2H, Ar-H); 7,35 (d, 1H, Ar-H); 7,25 (sb, 1H, Ar-H); 7,0 - 6,7 (2H, Ar-H); 4,82 - 4,3 (4H, N-CH₂/α-Pro/α-Tal); 4,05/3,6 (2m, 1H, δ-Pro); 3,5 - 2,9 (m, 3H, Ar-CH₂/δ-Pro); 2,2 - 1,7 (4H, β/γ-Pro); 1,25 (2s, 9H, Boc)
MS: 500 (M+H⁺), 400 (-Boc), 247, 134 - (Hydroacetat)

### Beispiel 117:

### H-(D-)-2-Tal-Pro-NH-pAmb:

Die Verbindung wurde durch Boc-Abspaltung aus Beispiel 116 hergestellt.
¹H-NMR (d₆-DMSO, δ in ppm): 9,4 - 9,0 (4H, NH); 8,85 (b, 3H, NH₃); 7,8 (d, 2H, Ar-H); 7,5 (d, 2H, Ar-H); 7,45 (d, 1H, Ar-H); 7,0 (dd(s, 2H, Ar-H); 4,4 - 4,15 (4H, N-CH₂//α-Pro/α-Tal); 3,8 - 2,9 (3H, Ar-CH/δ-Pro); 2,8 (dd, Ar-H); 1,8 (m, 2H, β-Pro); 1,75 - 1,55 (2m, 2H, γ-Pro)
FAB-MS: 400 (M+H⁺) - (Dihydroacetat)

### Beispiel 118:

### Boc(D)-Phg-Pro-NH-pAmb:

Das Hydroacetat wurde hergestellt aus Boc-(D)-Phg-OH analog Beispiel 3.

### Beispiel 119:

### H-(D)-Phg-Pro-NH-pAmb:

Das Dihydrochlorid erhielt man aus Beispiel 118.
¹H-NMR (d₆-DMSO, δ in ppm): 9,6 - 9,3 (4H, Amidin); 9,1 - 8,7 (4H, NH und NH₂⁺); 8,0 - 7,3 (9H, Aromaten-H); 5,4 (1H, CH); 4,6 - 4,3 (3H, CH₂ und CH); 3,1 - 2,7 (2H, CH₂); 2,2 - 1,6 (4H, 2 x CH₂)

### Beispiel 120:

### Boc-(D,L)-Phg(4-MeO)-Pro-NH-pAmb:

Das Hydroacetat wurde hergestellt aus Boc-(D,L)-Phg(4-MeO)OH analog Beispiel 3.

### Beispiel 121:

### H-(D,L)-Phg(4-MeO)-Pro-NH-pAmb:

Das Dihydrochlorid erhielt man aus Beispiel 120.
¹H-NMR (d₆-DMSO, δ in ppm): 9,6 - 9,3 (4H, Amidin), 9,0(8,9 (1H, NH); 8,8/8,6 (3H, NH₃⁺); 7,9 - 7,8 und 7,55 - 7,45 und 7,05 - 6,90 (8H, Aromaten-H); 5,3 (1H, CH); 4,5 - 4,3 (3H, CH₂ und CH); 3,75 (3H, OCH₃); 2,2 - 1,6 (4H, 2 x CH₂)

### Beispiel 122:

### Boc(D)-Chg-Pro-NH-pAmb:

a) 8 g (31,1 mMol) Boc-(D)-Chg-OH, 9,88 g (37,3 mMol) H-Pro-p-cyanobenzylamid x HCl, 32 ml (186,54 mMol) DIPEA und 108 ml PPA (50%ig in Essigester) wurden bei 0°C in einem Kolben vereinigt und 18 h bei 0°C - RT gerührt. Anschließend wurde das Reaktionsgemisch in Essigester verdünnt und mit 20%iger NaHSO₄-Lsg. (5x), 5%iger NaHCO₃-Lsg. und ges. NaCl-Lsg. extrahiert. Nach Trocknen und Einengen der organischen Lösung verblieben 13,8 g reines Boc(D)-Chg-Pro-p-cyano-benzylamid..
b) 13,8 g Boc(D)-Chg-Pro-p-cyano-benzylamid wurden in 113 ml Pyridin und 53 ml TEA gelöst. Die Lösung wurde mit H₂S-Gas gesättigt und über Nacht bei RT stehen gelassen. Zur Aufarbeitung wurde das Reaktonsgemisch zunächst mit Stickstoff gespült und dann auf 1 1 5%ige Zitronesäurelösung gegossen. Der ausgefallene Niederschlag wurde abfiltriert und das Filtrat mit Essigester extrahiert (3x). Der Niederschlag wurde hierauf in Essigester gelöst und mit den organischen Extrakten vereinigt. Die vereinigten Phasen wurden mit 5%iger Zitronensäure gewaschen, mit NaSO₄ getrocknet und eingeengt. Das Rohprodukt wurde in der Folgereaktion ohne weitere Reinigung eingesetzt.
c) Das rohe Thioamid wurde in 120 ml Aceton und 21 ml Mel gelöst und über Nacht bei RT gerührt. Anschließend wurde das Reaktionsgemisch im Vakuum zur Trockene eingeengt und der Rückstand bei 48 ml MeOH gelost. Nach Zugabe von 48 ml einer methanolischen Ammoniumacetatlösung (10%ig) wurde die Lösung 18 h bei RT gerührt. Zur Aufarbeitung des Amidins wurde das Lösungsmittel am Rotationsverdampfer entfernt, der Rückstand in DCM aufgenommen, der Niederschlag abgesaugt und das Filtrat im Vakuum eingeengt. Man erhielt 24,6 g des Rohproduktes. Dieses wurde mittels einer Reversed-Phase HPLC-Chromatographie gereinigt. Ausbeute 7 g - (Hydroacetat)
   ¹H-NMR (d₆-DMSO, δ in ppm): 9,6 - 9,2 (b, N-H); 8,7/8,1 (2t, 1H, NH); 7,75 (2d, 2H, Ar-H); 7,45 (2d, 2H, Ar-H); 7,0/6,9 (2d, 1H, NH); 4,4 (m, 3H, CH₂/α-Pro); 4,0 (t, 1H, α-Chg); 3,6 - 3,0 (2H, γ-Pro); 2,1 - 1,5 (m, 11H, β,γ-Pro/Ch₂); 1,4/1,3 (2s, 9H, Boc); 1,1 - 0,9 (m, 4H)
   MS: 486 (M+H⁺), 386 (-Boc), 247, 134

### Beispiel 123:

### H-(D-)-Chg-Pro-NH-pAmb:

Die Verbindung wurde durch Boc-Abspaltung aus Beispiel 122 hergestellt.
¹H-NMR (d₆-DMSO, δ in ppm): 9,4 - 9,0 (b, NH); 8,9 (t, 1H, NH); 8,4 (b, NH); 7,8 (d, 2H, Ar-H); 7,5 (d, 2H, Ar-H); 4,4 (m, 3H, N-CH₂/α-Pro); 3,9 - 3,6 (2m, 2H, α-Pro); 3,8 (d, 1H, α-Pro); 2,0 - 1,5 (m, 10H, Ch/β/γ-Pro); 1,2 - 1,0 (m, 4H, Ch)
MS: 386 (M+H⁺), 247, 185; mp: 133°C - (Dihydrochlorid)

### Beispiel 124:

### EtOOC-(D)-Chg-Pro-NH-pAmb:

Zunächst wurde aus Boc-(D)-Chg-OH und H-Pro-p-cyanobenzylamid x HCl analog Beispiel 3 H-(D)-Chg-Pro-p-cyanobenzylamid x HCl hergestellt.
Dann wurden 2,5 g (6,17 mMol) H-(D)-Chg-Pro-p-cyanobenzylamid x HCl, 2,33 ml (13,58 mMol) DIPEA, 0,075 g (0,617 mMol) DMAP und 0,652 ml Chlorameisensäureethylester in Folge zu 25 ml DCM bei RT gegeben und die Reaktionslösung 18 h bei RT gerührt. Anschließend wurde das Reaktionsgemisch mit DCM verdünnt, mit 20%iger NaHSO₄-Lösung gewaschen, getrocknet und eingeengt. Rohausbeute von EtOOC-H-(D)-Chg-Pro-p-cyanobenzylamid: 2,51 g. Die so erhaltene Zwischenstufe wurde analog A.III.1. in das entsprechende Amidin überführt. Das Rohprodukt wurde mittels Reversed-Phase HPLC Chromatographie gereinigt (Acetonitril/Wasser). Ausbeute: 0,483 g.
¹H-NMR (d₆-DMSO, δ in ppm): 8,7/8,1 (2t, 1H, NH); 7,8 (2d, 2H, Ar-H); 7,4 (2d, 2H, Ar-H); 7,4 (2d, 2H, Ar-H); 7,39/7,3 (2d, 1H, NH); 4,9/4,4 (2m, 3H, CH₂/α-Pro); 4,0 (2t, 1H, α-Chg); 3,8 (t, 2H, OCH₂); 3,7 - 3,3 (3m, 2H, δ-Pro); 2,1 (m, 1H, β-Pro); 1,9 - 1,5 (m, 11H, CH₂/β/γ-Pro); 1,2 - 0,9 (m, 9H, CH₂/CH₃)
MS: 458 (M+H⁺), 247, 134, 70 - (Hydroacetat)

### Beispiel 125:

### HOOC-CH₂-(D)-Chg-Pro-NH-pAmb:

Die Verbindung wurde durch t-Butylesterspaltung aus Beispiel 126 hergestellt.
¹H-NMR (d₆-DMSO, δ in ppm): 8,5/8,3 (2t, 1H, NH); 7,8 (2d, 2H, Ar-H); 7,6/7,45 (2d, 2H, Ar-H); 4,4 - 4,2 (m, 3H, N-CH₂/α-Pro); 4,1 (m, 1H, α-Chg); 3,8 - 3,2 (4H, HOOCCH₂/δ-Pro); 2,1 - 1,4 (m, 11H); 1,2 - 0,9 (m, 4H)
MS: 444 (M+H⁺), 386, 247 - (Hydrochlorid)

### Beispiel 126:

### tBuOOC-CH₂-(D)-Chg-Pro-NH-pAmb:

Die Verbindung wurde analog der tert.-Butylestervorstufe von Beispiel 246 hergestellt.
¹H-NMR (d₆-DMSO, δ in ppm): 8,4 (t, 1H, NH); 7,75 (d, 2H, Ar-H); 7,4 (d, 2H, Ar-H); 4,4 (m, 4H, N-CH₂/α-Pro/α-Chg); 3,8 - 2,9 (4H, HOOCCH₂/δ-Pro); 2,1 - 0,9 (m, 15H); 1,3 (s, 9H, tBu)
MS: 500 (M+H⁺), 444, 247, 170 - (Hydroacetat)

### Beispiel 127:

### Boc-(D)-Cha-Pro-NH-pAmb:

Die Verbindung 127 wurde analog Beispiel 3 synthetisiert.
¹H-NMR (d₆-DMSO, δ in ppm): 9,4 (b, 4H, NH); 8,8/8,15 (2t, 1H, NH); 7,75 (2d, 2H, Ar-H); 7,45 (2d, 2H, Ar-H); 7,05 (d, 1H, NH); 4,8/4,35 (d/m, 3H, N-CH₂/α-Pro/α-Chg); 3,75/3,5 - 3,2 (2H, α-Pro); 2,1 - 1,85 (4H, β/γ-Pro); 1,7 - 1,3 (m, 6H); 1,3 (2d, 9H, Boc); 1,4 - 0,9 (m, 7H)
MS: 500 (M+H⁺), 400 (-Boc), 247, 134; Fp: 125 - 7°C (Hydroacetat)

### Beispiel 128:

### Me-(D)-Cha-Pro-NH-pAmb:

Die Verbindung wurde durch Cbz-Abspaltung aus Beispiel 129 synthetisiert.
¹H-NMR (d₆-DMSO, δ in ppm): 9,3/8,9 (2s, 4H, NH); 8,85/8,8 (2sb, 2H, NH); 8,7 (t, 1H, NH); 7,8 (2d, 2H, Ar-H); 7,5 (2d, 2H, Ar-H); 4,4 (m, 3H, N-CH₂/α-Pro); 4,25 (db, 1H; α-Chg); 3,9/3,4 (2m, 2H, δ-Pro); 2,5 (s, 3H, NCH₃); 2,2 (m, 1H, β-Pro); 2,5 (s, 3H, NCH₃); 2,2 (m, 1H, β-Pro); 2,0 - 1,8 (m, 4H); 1,8 - 1,5 (m, 6H); 1,4 - 0,9 (6H)
MS: 414 (M+H⁺), 247, 140 - (Hydroacetat)

### Beispiel 129:

### Me-(Z)-(D)-Cha-Pro-NH-pAmb:

Die Verbindung wurde ausgehend von Me-(Z)-(D)-Cha-OH und H-Pro-p-cyanobenzylamid x HCl analog Beispiel 3 hergestellt.
¹H-NMR (d₆-DMSO, δ in ppm): 9,8 - 9,2 (b, 4H, NH); 8,8/8,5 (2t, 1H, NH); 7,8 (2d, 2H, Ar-H); 7,5 (2d, Ar-H); 7,4 (m, 5H, Ph-H); 5,2 - 5,0 (2H, OCH₂); 4,95 - 4,5 (1H, α-Pro); 4,4 (m, 3H, N-CH₂/α-Cha); 3,6 - 3,0 (2H, δ-Pro); 2,82/2,75/2,7 (3s, 3H, NCH₃); 2,1 (m, 1H, β-Pro); 1,9 - 1,4 (m, 11H, β/γ-Pro/CH₂); 1,2 - 0,8 (m, 5H)
FAB-MS: 548 (M+H⁺) - (Hydroacetat)

### Beispiel 130:

### N,N-Me-(D)-Cha-Pro-NH-pAmb:

Die Verbindung wurde ausgehend von N,N-Dimethylcyclohexylalanin und H-Pro-p-cyanobenzylamid x HCl analog Beispiel 3 synthetisiert.
¹H-NMR (d₆-DMSO, δ in ppm): 8,8/8,4 (2t, 1H, NH); 7,8 (2d, 2H, Ar-H); 7,45 (2d, 2H, Ar-H); 4,45 - 4,3 (d/m, 3H, N-CH₂/α-Pro); 3,9 (m, 1H, α-Cha); 3,6 - 3,2 (2H, δ-Pro); 2,2 (2s, 6H, NCH₃); 2,1 - 1,5 (m, 13H); 1,3 - 0,8 (m, 4H)
FAB-MS: 428 (M+H⁺) - (Hydroacetat)

### Beispiel 131:

### Boc-(D)-Trp(Boc)-Pro-NH-pAmb:

Die Verbindung wurde augehend von Boc-(D)-Trp(Boc)-OH und H-Pro-p-cyanobenzylamid x HCl analog Beispiel 3 synthetisiert.
¹H-NMR (d₆-DMSO, δ in ppm): 9,8 - 9,2 (b, N-H); 8,8 - 8,5 (2sb, 1H, NH); 8,25(8,0/7,8-7,2 (m, 10H, Ar-H/NH); 4,85/4,5 - 4,2 (d/m, 4H, CH₂-H); 3,6/3,5 (2m, 2H, CH₂, Pro); 3,1 - 2,8 (m, 2H, CH₂); 2,2 - 1,6 (m, 4H, Pro), 1,3 (2s, 18H, Boc)
FAB-MS: 633 (M+H⁺) - (Hydroacetat)

### Beispiel 132:

### H-(D)-Trp-Pro-NH-pAmb:

Die Verbindung wurde durch Boc-Abspaltung aus Beispiel 131 hergestellt.
¹H-NMR (d₆-DMSO, δ in ppm): 11,1 (s, 1H, NH); 9,4/9,15 (2s, 4H, N-H); 8,8 (t, 1H, NZ); 8,6 (s, 3H, N-H); 7,75 (d, 2H, Ar-H); 7,45 (d, 3H, Ar-H); 7,35 (d, 1H, Ar-H); 7,25 (s, 1H, Ar-H); 7,0 (2t, 2H, Ar-H); 4,3 (m, 2H, CH₂), 4,18 (sb, 1H, α-H); 3,5 (m, 2H, CH₂, Pro); 3,3 - 3,1 (m, 2H, CH₂), 2,15 (dd, 1H, Pro); 1,6/1,4 (2m, 3H, β/γ-Pro)
FAB-MS: 433 (M+H⁺) - (Dihydrochlorid)

### Beispiel 133:

### Boc-(D,L)-Dpa-Pro-NH-pAmb:

Die Verbindung wurde ausgehend von Boc-(D)-Dpa-OH und H-Pro-p-cyanobenzylamid x HCl analog Beispiel 3 synthetisiert.
¹H-NMR (d₆-DMSO, δ in ppm): 8,6/8,1 (2t, 1H, NH); 7,75 (2d, 2H, Ar-H); 7,45 - 7,0 (m, 13H, Ar-H/NH); 5,25/5,1 (2t, 1H, α-Dpa); 4,4 - 4,1 (3H, N-CH₂/α-Pro); 3,75 (m, 1H, CH); 3,6 - 2,95 (2H, δ-Pro); 2,0 - 1,5 (4H, β/γ-Pro); 1,2 (2ds, 9H, Boc)
MS: 570 (M+H⁺), 470 (-Boc), 247, 196, 134, mp: 156°C - (Hydroacetat)

### Beispiel 134

### H-(D oder L)-Dpa-Pro-NH-pAmb/a:

Die Verbindung 134 wurde durch Boc-Abspaltung aus Beispiel 133 und anschließender Diastereomerentrennung mittels einer Reversed-Phase HPLC-Trennung synthetisiert.
¹H-NMR (d₆-DMSO, δ in ppm): 9,3 (2s, 4H, NH); 8,9/8,2 (2t, 1H, NH); 8,4 (b, 3H, NH); 7,8 (2d, 2H, Ar-H); 7,6 (2d, 2H, Ar-H); 7,5 - 7,1 (10H, Ar-H); 5,1/4,6 (2d, 1H, α-Dpa); 4,4 - 4,1 (4H, N-CH₂/α-Pro/CH); 3,8 - 3,0 (2H, δ-Pro); 2,1 - 1,1 (4H, β/γ-Pro)
FAB-MS: 470 (M+H⁺) - (Dihydroacetat)

### Beispiel 135:

### H-(D oder L)-Dpa-Pro-NH-pAmb/b:

¹H-NMR (d₆-DMSO, δ in ppm): 9,3/9,2 (2s, 4H, NH); 8,4 (t, 1H, NH); 8,35 (sb, 3H, NH); 7,8/7,65 (2d, 4H, Ar-H); 7,4 - 7,1 (10H, Ar-H); 5,0 (d, 1H, α-Dpa); 4,4/3,9 (M,4H, n-CH₂/α-Pro/CH); 3,6/2,9 (2m, 2H, δ-Pro); 1,7 - 1,3 (4H, β/γ-Pro)
FAB-MS: 470 (M+H⁺) - (Dihydroacetat)

### Beispiel 136:

### EtOOC-(D oder L)-Dpa-Pro-NH-pAmb/a:

Zur Darstellung der oben genannten Verbindung wurde Boc-(D,L)-Dpa-Pro-p-cyanobenzylamid (Zwischenprodukt für die Synthese von Beispiel 133) zunächst mittels Dioxan/HCl in das entsprechende Hydrochlorid H-(D,L)-Dpa-Pro-p-cyanobenzylamid x HCl überführt. Anschließend wurde das Salz analog Beispiel 124 in das diastereomere Produktpaar überführt. Die beiden Diastereomeren wurden durch eine Reversed-Phase HPLC-Chromatographie (Acetonitril/Wasser) voneinander getrennt.
¹H-NMR (d₆-DMSO, δ in ppm): 8,6/6,6 (2t, 1H, NH); 7,8 - 7,0 (m, 15H, Ar-H, NH); 5,3/5,1 (2t, 1H, α-Dpa); 4,4 (2d, 1H, α-Pro); 4,3/4,1 (2t, 2H, CH₂); 4,0 (m, 1H, CH); 3,85 (t, 2H, OCH₂); 3,6/3,3/3,0 (3m, 2H, δ-Pro); 2,0 - 1,4 (m, 4H, β/γ-Pro): 1,0 (m, 3H, CH₃)
MS: 542 (M+H⁺), 268, 134, 70 - (Hydroacetat)

### Beispiel 137:

### EtOOC-(D oder L)-Dpa-Pro-NH-pAmb/b:

Die Verbindung wurde analog Beispiel 3 hergestellt.
¹H-NMR (d₆-DMSO, δ in ppm): 8,2 (2t, 1H, NH); 7,75 (d, 2H, Ar-H); 7,6 (d, 2H, Ar-H); 7,4 - 7,2 (m, 12H, Ar-H); 5,15 (m, 1H, α-Dpa); 4,4 (m, 3H, NCH₂/α-Pro); 3,95 (m, 1H, CH); 3,8/3,1 (2m, 2H, δ-Pro); 3,7 (m, 2H, OCH₂); 1,8 - 1,4 (m, 4H, β/γ-Pro); 1,0 (m, 3H, CH₃)
MS: 542 (M+H⁺), 268, 134, 70 - (Hydroacetat)

### Beispiel 138:

### HOOC-CH₂-(D oder L)-Dpa-Pro-NH-PAmb/a:

Zunächst wurde die Boc-Gruppe von Boc-(D,L)-Dpa-Pro-p-Cyanobenzylamid (Zwischenprodukt der Synthese von Beispiel 133) mittels Dioxan/HCl abgespalten. 3,42 g (7 mMol) des so erhaltenen Hydrochlorids wurden in 20 ml MeOH gelöst und nach Zugabe von 0,6 g (6,65 mMol) Glyoxylsäurehydrat und 1,75 g (28 mMol) NaCNBH₃ über Nacht gerührt. Zur Aufarbeitung wurde das Reaktiongemisch eingeengt, der Rückstand in DCM aufgenommen und die so erhaltene organische Lösung mit Wasser extrahiert. Nach Trocknen und Einengen der organischen Phase wurde der Rückstand in 5 ml MeOH gelöst und das gewünschte Produkt durch Eintropfen in Diisopropylether ausgefällt. Rohausbeute: 3,7 g. Das Rohprodukt wurde ohne weitere Reinigung analog Beispiel A.III.1. in das entsprechende Amidin überführt. Das Diastereomerengemisch wurde mittels Reversed-Phase HPLC (Acetonitril/Wasser) getrennt.
MS: 528 (M+H⁺), 254 - (Hydroacetat)

### Beispiel 139:

HOOC-CH₂-(D oder L)-Dpa-Pro-NH-pAmb/b:
MS: 528 (M+H⁺), 254, 134, 83 - (Hydroacetat)

### Beispiel 140:

### Boc(D oder L)-Dpa(4,4'-(Cl)₂)-ProNH-pAmb/a:

Die Verbindung wurde ausgehend Boc-(D.L)-Dpa(4,4'-(Cl)₂)-OH und H-Pro-p-cyanobenzylamid x HCl analog Beispiel 3 hergestellt. Die synthetisierte Diastereomerenpaar wurde mittels einer Reversed-Phase HPLC-Chromatographie getrennt.
MS: 638 (M+H⁺), 538 (-Boc), 303, 277, 247 - (Hydroacetat)

### Beispiel 141:

### Boc(D oder L)-Dpa-(4,4'-(Cl)₂)-Pro-NH-pAmb/b:

MS: 638 (M+H⁺), 538 (-Boc), 303, 247, 134, 70 - (Hydroacetat)

### Beispiel 142:

### H-(D oder L)-Dpa(4,4'-(Cl)₂)-Pro-NH-pAmb/a:

Die Verbindung wurde durch Boc-Abspaltung aus Beispiel 140 hergestellt.
¹H-NMR (d₆-DMSO, δ in ppm):
MS: 538 (M+H⁺), 303, 247, 134, 70 - (Hydroacetat)

### Beispiel 143:

### H-(D oder L)-Dpa(4,4'-(Cl)₂)-Pro-NH-pAmb/b:

Die Verbindung wurde durch Boc-Abspaltung aus Beispiel 141 hergestellt.
MS: 538 (M+H⁺), 303, 264, 247, 134, 70 - (Hydroacetat)

### Beispiel 144:

### EtOOC-(D oder L)-Dpa(4,4'-(Cl)₂)-Pro-NH-pAmb/a:

Zur Darstellung der oben genannten Verbindung wurde Boc-(D,L)-Dpa(4,4'-(Cl)₂)-Pro-p-cyanobenzylamid (Zwischenprodukt für die Synthese von Beispiel 141) zunächst mittels Dioxan/HCl in das entsprechende Hydrochlorid H-(D,L)-Dpa(4,4'-(Cl)₂)-Prop-cyanobenzylamid x HCl überführt. Anschließend wurde das Salz analog Beispiel 124 in das diastereomere Produktgemisch überführt. Die beiden Diastereomeren wurden durch eine Reversed-Phase HPLC-Chromatographie (Acetonitril/Wasser) voneinander getrennt.
¹H-NMR (d₆-DMSO, δ in ppm): 8,6 (2t, 1H, NH); 7,75 (2d, 2H, Ar-H); 7,6 - 7,1 (11H, Ar-H/NH); 5,2/5,0 (2t, 1H, α-Dpa); 4,4/4,38 (2d, 1H, CH); 4,3 (m, 1H, α-Pro); 4,0 (m, 2H, NCH2); 3,75 (m, 2H, OCH2); 3,7 - 3,3 (2H, δ-Pro); 2,0 (m, 1H, β-Pro); 1,95 - 1,4 (m, 4H, β/γ-Pro); 1,0 (2t, 3H, CH3)
MS: 610 (M+H⁺), 247, 134, 70 - (Hydroacetat)

### Beispiel 145:

### EtOOC-(D oder L)-Dpa(4,4'-(Cl)₂)-Pro-NH-pAmb/b:

¹H-NMR (d₆-DMSO, δ in ppm): 8,2 (2t, 1H, NH); 7,75 (2d, 2H, Ar-H); 7,6 - 7,1 (11H, Ar-H/NH); 5,1 (2t, 1H, α-Dpa); 4,4 (2d, 1H, CH); 4,3 (m, 2H, NCH2); 4,0/4,39 (m, 1H, α-Pro); 3,85 (m, 2H, OCH2); 3,7 (2H, δ-Pro); 1,9 - 1,5 (4H, β/γ-Pro); 1,0 (2t, 3H, CH3)
MS: 610 (M+H⁺), 247, 185, 134, 93 - (Hydroacetat)

### Beispiel 146:

### HOOC-CH₂-(D oder L)-Dpa(4,4'-(Cl)₂)-Pro-NH-pAmb/a:

Zunächst wurde die Boc-Gruppe von Boc-(D,L)-Dpa(4,4-Cl)-Prop-cyanobenzylamid (Zwischenprodukt der Synthese von Beispiel 140) mittels Dioxan/HCl abgespalten. Anschließend wurde analog Beispiel 138 das gewünschte Produkt hergestellt.
¹H-NMR (d₆-DMSO, δ in ppm): 11,2 (b, COOH); 8,9/8,6 (2sb, 1H, NH); 7,8 - 7,2 (14H, Ar-H/NH); 4,4 - 4,0 (5H, CH7 N-CH2/α-Dpa/α-Pro); 3,8 - 3,0 (2H, δ-Pro); 2,8 (2d, 2H, HOOC-CH2); 2,0 - 1,4 (4H, β/γ-Pro)
MS: 596 (M+H⁺), 247, 134, 93, 70 - (Hydroacetat)

### Beispiel 147:

### HOOC-CH₂-(D oder L)-Dpa(4,4'-(Cl)₂)-Pro-NH-pAmb/b:

FAB-MS: 596 (M+H⁺)

### Beispiel 148:

### H-(D oder L)-Dch-Pro-NH-pAmb/a:

Die Verbindung 148 wurde ausgehend von Boc-(D,L)-Dch-OH und H-pro-p-cyanobenzylamid x HCl analog Beispiel 3 synthetisiert. Das synthetisierte Diastereomerenpaar wurde mittels einer Reversed-Phase HPLC-Chromatographie getrennt.
¹H-NMR (d₆-DMSO, δ in ppm): 9,3 - 9,0 (b, NH); 8,9/8,5 (2t, 1H, NH); 7,75/7,5 (2d, 4H, Ar-H); 4,5 - 4,0 (4H, N-CH2/α-Pro/α-Dch); 3,7 - 3,0 (2H, δ-Pro); 2,2 - 1,0 (4H, β/γ-Pro)
FAB-MS: 481 (M+H⁺); mp: 127°C - (Dihydroacetat)

### Beispiel 149:

### H-(D oder L)-Dch-Pro-NH-pAmb/b

FAB-MS: 481 (M+H⁺); mp: 127°C - (Dihydroacetat)

### Beispiel 150:

### Boc-(D)-Val-Pro-NH-pAmb:

Die Darstellung erfolgte analog Beispiel 3.
Fp: 132-145°C - (Hydroacetat)

### Beispiel 151:

### H-(D)-Val-Pro-NH-pAmb:

Die Darstellung erfolgte aus Beispiel 150.
Fp: 60-80°C - (Dihydrochlorid)

### Beispiel 152:

### Boc-(D)-Leu-Pro-NH-pAmb:

Die Darstellung erfolgte analog Beispiel 3.
Fp: 68-82°C - (Hydroacetat)

### Beispiel 153:

### H-D-Leu-Pro-NH-pAmb:

Die Darstellung erfolgte aus Beispiel 152.
Fp: 228-233°C - (Dihydrochlorid)

### Beispiel 154:

### Boc-(D)-Gly(α-tBu)-Pro-NH-pAmb:

Die Darstellung erfolgte analog Beispiel 3.
Fp: 211-220°C - (Hydroacetat)

### Beispiel 155:

### H-(D)-Gly(α-tBu)-Pro-NH-pAmb:

Die Darstellung erfolgte aus Beispiel 154.
Fp: 236-239°C - (Dihydrochlorid)

### Beispiel 156:

### Boc-(D)-Ala(β-tBu)-Pro-NH-pAmb:

Die Darstellung erfolgte analog Beispiel 3.
Fp: 185-192°C (Hydroacetat)

### Beispiel 157:

### H-(D)-Ala(β-tBu)-Pro-NH-pAmb:

Die Darstellung erfolgte aus Beispiel 156.
Fp: 225-231°C (Dihydrochlorid)

### Beispiel 158:

### H-(D bzw. L)-Msu-Pro-NH-pAmb/a:

Das Dihydrochlorid wurde analog Beispiel 3 aus Boc-(D,L)-Msu-OH hergestellt und dann analog A.I.c. die Boc-Gruppe abgespalten. Die Diastereomeren wurden über HPLC getrennt.
¹H-NMR (DMSO-d₆, δ in ppm): 9,40/9,20 (4H, Amidin), 8,9 (1H, NH), 8,55 (3H, NH₃⁺), 7,85/7,50 (4H, Aromaten-H), 4,50 - 4,35 (4H, CH₂ und 2 x CH), 3,85 - ca. 3,3 (4H, 2 x CH₂), 2,95 (3H, CH₃), 2,3 - 1,8 (6H, 3 x CH₂)

### Beispiel 159:

### H-(D bzw. L)-Msu-Pro-NH-pAmb/b:

(Dihydrochlorid);
¹H-NMR (DMSO-d₆, δ in ppm): 9,45/9,30 (4, Amidin), 8,95 (1H, NH), 8,85 (3H, NH₃⁺), 7,80/7,45 (4H, Aromaten-H), 4,4 - 4,2 (4H, CH₂ und 2 x CH), 3,85 - ca. 3,3 (4H, 2 x CH₂), 3,00 (3H, CH₃), 2,3 - 1,7 (6H, 3 x CH₂)

### Beispiel 160:

### Boc-(Cyclo)Leu-Pro-NH-pAmb:

Die Verbindung 160 wurde ausgehend von Boc-(Cyclo)Leu-OH und H-Pro-p-cyanobenzylamid x HCl analog Beispiel 3 synthetisiert.
MS: 472 (M+H⁺), 372 (-Boc); 247, 185, 140 - (Hydroacetat)

### Beispiel 161:

### H-(Cyclo)Leu-Pro-NH-pAmb:

Die verbindung wurde durch Boc-Abspaltung aus Beispiel 160 synthetisiert.
FAB-MS: 372 (M+H⁺) - (Dihydroacetat)

### Beispiel 163:

### H-Gly-Pro-NH-pAmb:

Das Dihydrochlorid erhielt man durch Boc-Spaltung aus Boc-Gly-Pro-NH-pAmb, welches ausgehend von Boc-Gly-OH analog Beispiel 3 hergestellt wurde.
¹H-NMR (DMSO-d₆, δ in ppm): 9,50/9,25 (4H, Amidin), 8,85 (1H, NH), 8,30 (3H, (NH₃⁺), 7,80/7,45 (4H, Aromaten-H), 4,5 - 4,2 (3H, CH₂ und CH), 3,9 - ca. 3,3 (4H, 2 x CH₂), 2,2 - 1,7 (4H, 2 x CH₂)

### Beispiel 166:

### Ph-CH₂-Gly-Pro-NH-pAmb:

Das Dihydrochlorid erhielt man durch Boc-Spaltung von Ph-CH₂-(Boc)Gly-Pro-NH-pAmb Hydroacetat.
¹H-NMR (DMSO-d₆, δ in ppm): 9,6 (2H, NH₂⁺), 9,4/9,2 (4H, Amidin), 8,80 (1H, NH), 7,.80 - 7,35 (9H, Aromaten-H), 4,40 - 4,25 (3H, CH₂ und CH), 4,10 (2H, CH₂), 3,95 (2H, CH₂), 3,6 - 3,4 (2H, CH₂, 2,2 - 1,8 (4H, 2 x CH₂)

### Beispiel 176:

### β-Naphtyl-SO₂-Pro-NH-pAmb:

Die Darstellung erfolgte durch Kupplung von β-Naphthyl-SO₂Cl mit H-Pro-OCH₃, anschließender Esterverseifung, Kupplung mit p-Cyanobenzylamin und Überführung der Nitrilfunktion in die Amidingruppe.
Fp: 66-72°C (Hydroacetat)

### Beispiel 177:

### p-Tol-SO₂-Pro-NH-pAmb:

Die Darstellung erfolgte analog Beispiel 176.
Fp: 89-95°C (Hydroacetat)

### Beispiel 178:

### Ph-CH₂-CH₂-SO₂-Pro-NH-pAmb:

Die Darstellung erfolgte analog Beispiel 176.
Fp: 61-69°C (Hydroacetat)

### Beispiel 179:

### H-Asp-Pro-NH-pAmb:

Von Boc-Asp(OBzl)-Pro-NH-pAmb wurde die Boc-Gruppe analog A.I.c. abgespalten und der Benzylester mit Pd/C zur Säure hydriert. Das Dihydrochlorid erhielt man durch Behandeln mit etherischer HCl.
¹H-NMR (DMSO-d₆, δ in ppm): 9,4/9,2 (4H, Amidin), 8,6 (1H, NH), 8,45 (3N, NH₃⁺), 7,80/7,45 (4H, Aromaten-H), 4,45 - 4,30 (4H, CH₂ und 2 x CH), 3,8 - ca. 3,5 (2H, CH₂), 3,2 - ca. 2,6 (2H, CH₂), 2,2 - 1,7 (4H, 2 x CH₂)

### Beispiel 191:

### H-(D)-Asp-Pro-NH-pAmb:

Das Dihydrochlorid wurde analog Beispiel 179 hergestellt.
¹H-NMR (DMSO-d₆, δ in ppm): 9,45/9,30 (4H, Amidin), 9,05 (1H, NH), 8,9 (3H, NH₃⁺), 7,80/7,45 (4H, Aromaten-H), 4,45 - 4,15 (4H, CH₂ und 2 x CH), 2,2 - 1,7 (4H, 2 x CH₂);
FAB-MS: 362 (M+H⁺)

### Beispiel 193:

### H-(D)-Asp(OtBu)-Pro-NH-pAmb:

Das Dihydroacetat wurde hergestellt aus Z-(D)-Glu(OtBu)-Pro-NHpAmb durch Hydrierung über Pd/C.
¹H-NMR (DMSO-d₆, δ in ppm): 9,3 (4H, Amidin), 8,5 (1H, NH), 8,3 (3H, NH₃⁺), 7,75/7,25 (4H, Aromaten-H), 4,4 - 4,3 (4H, CH₂ und 2 x CH), 2,9 - 2,6 (2H, CH₂, 2,2 - 1,8 (4H, 2 x CH₂), 1,4 (9H, tBu);
FAB-MS: 418 (M+H⁺)

### Beispiel 199:

### (D)-Ph-CH₂-CHOH-CO-Pro-NH-pAmb:

(a) 3-Phenyl-D-Lactyl-prolin-(p-cyanobenzyl)amid:
   5,5 g (20,4 mmol) O-Tetrahydropyranyl-3-phenyl-D-milchsäure (WO 93/18060) wurden in 30 ml DMF gelöst und nacheinander mit 5,4 g (20,9 m Mol), N-(p-Cyanobenzyl)prolinamid, 3,3 g (20,4 nMol) N-Hydroxy-benzotriazol, 3,0 g DIPEA und 4,33 g (20,6 mMol) Dicyclohexylcarbodiimid versetzt. Man ließ 48 h bei Raumtemperatur nachrühren. Nach Absaugen des ausgefallenen Harnstoffs wurde das Lösungsmittel im Vakuum weitgehend entfernt, der Rückstand mit 50 ml Wasser versetzt und mit Essigester extrahiert. Nach Waschen mit Wasser, NaHCO₃-Lösung und Trocknen über Na₂SO₄ wurde der Essigester abdestilliert, der verbleibende ölige Rückstand in Methanol gelöst und mit p-Toluolsulfonsäure auf pH 2 eingestellt. Diese Lösung blieb 6 h bei Raumtemperatur stehen. Danach wurde das Methanol abdestilliert, der Rückstand in Essigester aufgenommen, mit Wasser, 5 %iger Zitronensäure und NaHCO₃-Lösung gewaschen. Der nach Trocknen über Na₂SO₄ und Abdestillieren des Lösungsmittels erhaltene Rückstand wurde säulenchromatographisch gereinigt (Eluent: Methylenchlorid/Aceton/Methanol, 45/5/2). Man erhielt 2,5 g weiße Kristalle, die nach Kristallisation aus einem Ether-Hexan-Gemisch bei 108°C - 110°C schmolzen.
(b) 3-Phenyl-D-Lactyl-prolin-(p-amidinobenzyl)amid-acetat:
   2,0 g der vorstehenden Verbindung und 3 ml Triethylamin wurden in 30 ml Pyridin gelöst, bei 0°C mit H₂S gesättigt und über Nacht bei Raumtemperatur stehen gelassen. Gemäß DC-Kontrolle (CH₂Cl₂/MeOH, 9/1) war die Umsetzung zum Thioamid vollständig. Zur Isolierung wurde das Pyridin im Vakuum weitgehend abdestilliert, der Rückstand in 250 ml Essigester aufgenommen und mit Kochsalz-, 5 %iger Zitronensaure- und NaHCO₃-Losung gewaschen. Nach Trocknen und Abdestillieren des Lösungsmittels erhielt man 2,3 g amorphes Thioamid.
   Das Thioamid wurde in 40 ml Aceton gelöst und bei Raumtemperatur nach Zusatz von 4 ml Methyliodid 6 h stehen gelassen. Nach Abziehen des Lösungsmittels wurde der amorphe Rückstand mit trockenem Ether ausgerührt und anschließend getrocknet. Das S-Methyl-thioimidsäuremethylester-hydroiodid wurde in 50 ml Ethanol gelöst, mit 15 ml 10 %iger Ammoniumacetatlösung versetzt und 3 h auf 60°C erwärmt. Zur Isolierung wurde das Lösungsmittel abgezogen, der Rückstand in 100 ml CH₂Cl₂ gelöst, die unlöslichen Bestandteile abfiltriert und anschließend das CH₂Cl₂ abdestilliert. Durch Digerieren mit einem Essigester-Diethylether-Gemisch wurden die darin löslichen Verunreinigungen abgetrennt. Das verbleibende Iodid-Acetat-Mischsalz wurde in Aceton/Wasser (3/2) gelöst und mittels eines IRA-Acetat-Ionenaustauschers in das reine Acetat überführt und anschließend säulenchromatographisch gereinigt (Eluent: Methylenchlorid/Methanol/50 %ige Essigsäure 40/10/1,5). Die einheitlichen Fraktionen wurden nach Entfernen des Eluenten gefriergetrocknet. Es verblieben 1,1 g weißes Pulver, Fp: 185°C - 187°C, FAB-MS: 395 (M+H⁺).

### Beispiel 200:

### (D)-Man-Pro-NH-pAmb:

Das Hydroacetat wurde hergestellt analog Beispiel 199 ausgehend von O-Tetrahydropyranyl-(D)-mandelsäure (WO 93/18060); weiße Kristalle;
Fp: 211-213°C; FAB-MS: 381 (M+H⁺)

### Beispiel 202:

### H-(D)-Phe-Aze-NH-pAmb:

Das Hydrojodid/Hydrochlorid-Mischsalz wurde hergestellt durch Umsetzung von Boc-(D)-Phe-OH mit H-Aze-p-cyanobenzylamid analog Beispiel 3 bis zum Amidin und anschließender Boc-Spaltung.
¹H-NMR-(DMSO-d₆, δ in ppm): 9,3/9,1 (4H, Amidin), 9,0 (1H, NH, 8,7 (3H, NH₃⁺), 7,8-7,2 (9H, Aromaten-H, 4,5- ca. 3,3 (6H, 2 x CH₂ und 2 x CH), 3,2-2,8 (2H, CH₂), 2,2-1,8 (2H, CH₂)

### Beispiel 204 und Beispiel 205:

### H-(D)-Phe-(D bzw. L)-Pic-NH-pAmb/a und H-(D)-Phe-(D bzw. L)-Pic-NH-PAmb/b:

Das Dihydrochlorid des Diastereomerenpaars wurde hergestellt aus Boc-(D)-Phe-OH und H-(D,L)-Pic-p-cyanobenzylamid bis zum Amidin analog Beispiel 3. Anschließend wurde die Boc-Gruppe abgespalten.
¹H-NMR-(DMSO-d₆, δ in ppm): 9,6-9,3 (4H, Amidin), 9,1-8,7 (4H, NH und NH₃⁺), 7,8-7,2 (9H, Aromaten-H), 4,6-4,3 (4H, CH₂ und 2 x CH), 3,3-2,8 (2H, CH₂), 2,3-0.9 (6H, 3 x CH₂);
FAB-MS: 408 (M+H⁺)

Das Diastereomerenpaar wurde anschließend uber HPLC-Chromatographie in die Beispiele 204 und 205 getrennt.

### Beispiel 207:

### H-(D)-Phe-(D,L/trans)-Pic(4-Me)-NH-pAmb:

Das Dihydrochlorid wurde ausgehend von Boc-(D)-Phe-OH und H-(D,L/trans)-Pic(4-Me)-p-cyanobenzylamid analog Beispiel 204/205 aufgebaut ;
Fp: 160-170°C

### Beispiel 208:

### Boc-(D)-Phe-Pyr-NH-pAmb:

Die Verbindung 160 wurde ausgehend von Boc-(D)-Phe-OH und H-Pyrp-cyanobenzylamid x HCI analog Beispiel 3 synthetisiert.
MS: 492,5 (M+H⁺), 392, 245, 133

### Beispiel 209:

### H-(D)-Phe-Pyr-NH-pAmb:

Die Verbindung wurde durch Boc-Abspaltung aus Beispiel 208 synthetisiert. ¹H-NMR (d₆-DMSO, δ in ppm): 9,4-9,2 (b, N-H); 8,8-8,2 (b, N-H); 8,6 (2t, 1H, NH); 7,75 (2d), 2H, Ar-H); 7,45 (2d, 2H, Ar-H; 7,35-7,1 (m, 5H, Ar-H); 6,15/6,0/5,85/5,75 (4sb, 2H, CH=CH); 5,5/4,9 (sb, 1H, α-Pyr); 4,4-4,2 (m, 4H, CH₂/α-Phe/δ-Pyr); 3,6 (d, 1H, δ-Pyr); 3,1-3,0 (m, 2H, CH₂-Ph)
FAB-MS: 392 (M+H⁺)

### Beispiel 210:

### Boc-(D)-Phe-Hyp(OtBu)-NH-pAmb:

Die Verbindung wurde ausgehend von Boc-(D)-Phe-Hyp(OtBu)-OH und p-cyanobenzylamin x HCI analog Beispiel 1 synthetisiert.
MS: 566 (M+H⁺), 466(-Boc), 319 (466-Phe

### Beispiel 211:

### H-(D)-Phe-Hyp-NH-pAmb:

Die Verbindung wurde durch Boc- und tButyl-Abspaltung aus Beispiel 210 synthetisiert. ¹H-NMR (DMSO-d₆, δ in ppm): 9,35 (s, 2H, N-H); 9,1 (s, 2H, N-H); 8,8 (t, 1H, NH); 8,5 (sb, 3H, N-H); 7,75 (2d, 2H, Ar-H); 7,45 (2d, 2H, Ar-H); 7,35-7,2 (m, 5H, Ar-H); 4,4-4,2 (m, 5H, CH₂/2 α-H/CHOH; 3,8 (m, 1H, Pro); 3,0 (m, 2H, CH₂); 2,75 (m, 1H, Pro); 1,95 (m, 1H, Pro); 1,8 (m, 3H, Pro)
FAB-MS: 410 (M+H⁺)

### Beispiel 213:

### H-(D)-Phe-(Me)Val-NH-pAmb:

Das Dihydrochlorid wurde ausgehend von Boc-(D)-Phe-OH und H-(Me)Val-p-cyanobenzylamid analog Beispiel 3 aufgebaut.
¹H-NMR(DMSO-d₆, δ in ppm): 9,45/9,25 (4H, Amidin), 8,8 (1H, NH), 8,6 (3H, NH₃⁺), 7,8/7,5/7,3 (9H, Aromaten-H), 4,65 (1H, CH), 4,60 (2H, CH₂), 4,45-4,20 (2H, CH₂), 3,20-2,95 (2H, CH₂), 2,85 (3H, N-CH₃), 2,0 (1H, CH), 0,8/0,45 (6H, 2 x CH₃)

### Beispiel 216:

### Boc-(D)-Phe-Tia-NH-pAmb:

Die Verbindung wurde ausgehend von Boc-(D)-Phe-Tia-OH und p-Cyanobenzylaminhydrochlorid analog Beispiel 1 synthetisiert.
MS: 512 (M+H⁺), 412 (-Boc), 265, 204, 133

### Beispiel 217:

### H-(D)-Phe-Tia-NH-pAmb:

Die Verbindung wurde durch Boc-Abspaltung aus Beispiel 216 synthetisiert. ¹H-NMR (DMSO-d₆, δ in ppm): 9,4/9,2 (2sb, 4H, N-H); 9,0 (t, 1H, NH; 7,75 (2d, 2H, Ar-H); 7,45 (2d, 3H, Ar-H); 7,4-7,2 (m, 5H, Ar-H); 4,8 (d, 1H, α-Tia); 4,7/3,7 (2d, 2H, NCH2S); 4,4-4,2 (m, 3H, CH₂/α-Phe); 3,2/3,1 (2m, 2H, SCH₂); 3,0/2,7 (m, 3H, CH₂-Ph)
FAB-MS: 412 (M+H⁺)

### Beispiel 218:

### H-(D)-Phe-Pro-NH-3-(6-am)-pico:

a) 2-Cyano-5-(azidomethyl)pyridin:
   Zu einer Lösung von 8,8 g (0,07 mMol) 2-Cyano-5-(hydroxymethyl)pyridin (WO 83/01446) und 6,9 g Triethylamin in 200 ml Methylenchlorid wurden bei Raumtemperatur 14,5 g (0,07 Mol) Trifluoressigsäureanhydrid gelöst in 20 ml Methylenchlorid zugetropft und anschließend 2 h nachgerührt. Nach Abdestillieren des Methylenchlorids wurde der Rückstand in einem Gemisch von 50 ml Toluol und 50 ml Dimethylsulfoxid gelöst, mit 11,2 g (0,17 Mol) Natriumazid und 0,7 g Tetrabutylammonium-bromid versetzt und über Nacht bei Raumtemperatur nachgerührt.
   Das Reaktionsgemisch wurde in 300 ml Wasser gegossen und mehrmals mit Ether extrahiert. Nach Trocknen mit Na₂SO₄ und Abdestillieren des Ethers verblieben 6,8 g gelbliche Kristalle (Fp: 62-64°C), die ohne weitere Reinigung in die Folgereaktion eingingen.
b) 2-Cyano-5-(aminomethyl)pyridin:
   Die nach a) erhaltene Verbindung wurde in 45 ml Tetrahydrofuran und 1,2 ml Wasser gelöst und unter Rühren portionsweise mit 11,2 g Triphenylphosphin versetzt. Das Reaktionsgemisch blieb über Nacht bei Raumtemperatur stehen.
   Nach Abdestillieren des Lösungsmittels wurde der Rückstand in 100 ml Ether aufgenommen, das ausgefallene Triphenylphosphinoxid abgesaugt und das Filtrat mit etherischer Salzsäure auf pH2 eingestellt. Das ausgefallene Hydrochlorid wurde abgesaugt, mit Ether gewaschen und nacheinander mit Toluol und heißem Isopropanol digeriert. Man isolierte 4,7 g (40 %) Hydrochlorid, Fp.: 253-256°C (Zersetzung).
c) Boc-D-phenylalanyl-prolin-(6-cyano-3-picolyl)amid:
   Zu einer Lösung von 2,11 g (12,5 mMol) 2-Cyano-5-(aminomethyl)pyridin und 4,5 g (12,5 mMol) Boc-D-Phe-Pro-OH in 70 ml CH₂Cl₂ tropfte man bei -5°C 8,12 g Diisopropylethylamin und anschließend 11 ml (15 mMol) Propan-phosphonsäureanhydrid (50 %ige Lösung in Essigester). Es wurde 2 h nachgerührt, wobei man die Temperatur von -5° auf 20°C ansteigen ließ. Die organische Phase wurde mit Wasser, 5%iger Natriumbicarbonatund 5%iger Zitronensäurelösung gewaschen, über Na₂SO₄ getrocknet und zur Trockene eingeengt. Man erhielt einen schwach gelblichen kristallinen Rückstand, Fp.: 167-170°C, der ohne weitere Reinigung in die Folgereaktion einging.
d) Boc-D-phenylalanyl-prolin-(6-amidino-3-picolyl)amid:
   1,15 g (16,5 mMol) Hydroxylamin-hydrochlorid wurden in 5 ml Ethanol suspendiert, mit 1,2 g 25%iger Ammoniaklösung versetzt und 10 min. gerührt. Nach Zugabe von 45 ml Ethanol wurde das ausgefallene Salz abgesaugt und zur Lösung 3,14 g (6.6 mMol) der vorstehenden Verbindung (Stufe c) hinzugefügt. Nach kurzer Zeit schied sich die Hydroxyamidin-Verbindung ab, wurde nach 30 minütigem Nachrühren abgesaugt und mit wenig kaltem Wasser und Ethanol gewaschen. Der ethanolfeuchte Rückstand wurde in 40 ml Ethanol und 8 ml Eisessig gelöst, mit 250 mg 10%iger Pd/C versetzt und bei ca. 50°C hydriert. Nach 5 Stunden war lt. DC (CH₂Cl₂/MeOH/50%ige Essigsäure, 20/5/1) kein Ausgangsmaterial mehr nachweisbar.
   Nach Absaugen des Katalysators über eine Cellitschicht wurde das Lösungsmittel gegen Ende unter Zusatz von Toluol abdestilliert. Nach Zugabe von 50 ml Aceton kristallisierte das Amidinacetat aus und wurde abfiltriert. weiße Kristalle,
   Fp 130-4°C, FAB-MS: 495 (M+H⁺).
e) H-(D)-Phe-Pro-NH-3-(6-am)-pico:
   Aus Verbindung d) wurde nach Standardbedingungen die Boc-Gruppe abgespalten. Dihydrochlorid: Weiße Kristalle,
   Fp 235-240°C,
   FAB-MS: 395 (M+H⁺)

### Beispiel 219:

### Boc-(D)-Chg-Pro-NH-3-(6-Am)-pico:

a) Boc-D-Cyclohexylglycyl-prolin:
   29 g (0,113 Mol) Hoc-(D)-Cyclohexylglycin und 18,7 g (0,113 Mol) Prolinmethylester-hydrochlorid wurden in 300 ml CH₂Cl₂ suspendiert und durch Zutropfen von 58,3 g (0,45 Mol) Diisopropylethylamin in Lösung gebracht. Nach Abkühlen auf -15°C wurden 113 ml (0,147 Mol) Propanphosphonsäureanhydrid (50%ige Lösung in Essigester) zugetropft und 1 Stunde nachgerührt.
   Nach Zugabe von 200 ml Wasser wurde die organische Phase abgetrennt und mit wäßriger K₂CO₃-Lösung, 0,5 N Salzsäure und 5%iger Bicarbonatlösung gewaschen. Nach Trocknen mit Na₂SO₄ wurde das Lösungsmittel abdestilliert, der ölige Rückstand (41 g) in 400 ml Ethanol gelöst, mit 120 ml 1 N NaOH versetzt und 2 Stunden bei Raumtemperatur gerührt.
   Nach Abdestillieren des Alkohols wurde die wäßrige Phase mit Wasser verdünnt und mehrmals mit Methyl-tert.butylether extrahiert. Die wäßrige Phase wurde mit KHSO₄-Lsg. angesäuert und 3 x mit CH₂Cl₂ extrahiert. Nach Trocknen und Abdestillieren des Methylenchlorids wurde der ölige Rückstand aus Diisopropylether/n-Hexan (1/3) kristallisiert. Man isolierte 28 g weiße Kristalle, Fp 145-148°C.
b) Boc-(D)-Cyclohexylglycyl-prolin-(6-cyano-3-picolyl)-amid:
   26,6 g (0,075 Mol) Boc-(D)-Cyclohexylglycyl-prolin und 12,7 g (0,075 Mol) 6-Cyano-3-picolylamin-hydrochlorid wurden in 300 ml CH₂Cl₂ suspendiert und mit 47 g (0,364 Mol) Diisopropyl-ethylamin versetzt. Anschließend wurden bei -10°C 66 ml Propanphosphonsäureanhydrid (50%ige Essigesterlösung) zugetropft, 1 Stunde bei 0°C nachgerührt, mit 200 ml Wasser versetzt und die CH₂Cl₂-Phase abgetrennt. Nach Waschen der organischen Phase mit 0,1 N Natronlauge und Wasser wurde getrocknet und das Lösungsmittel abdestilliert. Der Rückstand wurde in 100 ml Essigester aufgenommen, wobei rasch Kristallisation einsetzte, die durch Zugabe von 150 ml n-Hexan vervollständigt wurde. Nach Absaugen und Trocknen wurden 31,4 g (89 % d. Th.) weiße Kristalle, Fp. 150-151°C, isoliert.
c) Boc-(D)-Cyclohexylglycyl-prolin-(6-amidino-3-picolyl)amid:
   Die Amidinbildung erfolgte analog Beispiel 218, Stufe d. Acetat: weiße Kristalle, Fp. 160-8°C (Zersetzung);
   FAB-MS: 487 (M+H⁺)

### Beispiel 220:

### H-(D)-Chg-Pro-NH-3-(6-Am)-pico:

Die Abspaltung der Boc-Gruppe aus Stufe c der vorstehenden Verbindung erfolgte nach Standardbedingungen. Dihydrochlorid: Weiße Kristalle, Fp. 235-238°C (Zersetzung);
FAB-MS: 387 (M+H⁺).

### Beispiel 221:

### HOOC-CH₂-(D)-Chg-Pro-NH-3-(6-Am)-pico:

a) H-(D)-Cyclohexylglycylprolin-(6-cyano-3-picolyl)amid:
   46,9 g (0,1 Mol) Boc-(D)-Cyclohexylglycylprolin-(6-cyano-3-picolyl)amid (Verbindung 219, Stufe b) wurden in 300 ml Ether suspendiert. unter Rühren bei Raumtemperatur mit 600 ml HCL-gesättigtem Ether versetzt und über Nacht nach nachgerührt. Danach ließ man die Suspension unter Rühren und Eiskühlung in 1,5 1 einer 15 %igen Natronlaufe einlaufen. Nach Zugabe von 80 ml CH₂CH₂ wurde die organische Phase abgetrennt und die alkalische Phase 6x mit einem Ether/CH₂CH₂-Gemisch (7/3) extrahiert. Die vereinigten organischen Phasen wurden über Na₂SO₄ getrocknet und zur Trockene eingeengt. Es verblieben 27,2 g amorphes, weißes Pulver, das lt.DC (CH₂Cl₂/MeOH, (4/1) noch ca. 5-10 % der durch Hydrolyse der Cyanogruppe entstandenen Amidverbindung enthielt.
b) N-(t-Butoxycarbonylmethyl)-(D)-cyclohexylglycylprolin-(6-cyano-3-picolyl)amid:
   Zu einer Lösung von 27,2 g (0,074 Mol) der vorstehenden Verbindung (Stufe a) und 28,6 g (0,22 Mol) Diisopropylethylamin in 150 ml Methylenchlorid tropfte man unter Rühren bei Raumtemperatur 14 g (0,072 Mol) Bromessigsäure-t-butylester und ließ über Nacht rühren.
   Die Reaktionslösung wurde mit Wasser gewaschen, über Na₂SO₄ getrocknet und der Rückstand nach Abdestillieren des Lösungsmittels über eine Kieselgelsäule mit einem CH₂Cl₂/Aceton/MeOH (45/5/1)-Eluenten chromatographiert. Man isolierte 28,6 g (80 % d. Th.) amorphes, weißes Pulver. Eine Probe kristallisierte aus Diisopropylether unter Zusatz von wenig Ether und schmolz bei 89-91°C.
c) N-t-Butoxycarbonylmethyl-(D)-cyclohexylglycylprolin-(6-amidino-3-picolyl)amid:
   Die vorstehende Verbindung wurde analog Beispiel 218, Stufe d), in das Amidin überführt. Acetat: Weißes, amorphes Pulver,
   FAB-MS: 501 (M+H⁺)
d) N-(Carboxymethyl)-(D)-cyclohexylglycylprolin-(6-amidino-3-picolyl)amid:
   2,4 g des vorstehenden Amidin-acetats wurden in 50 ml eines CH₂Cl₂/CF₃COOH-Gemisches (1/1) gelöst und über Nacht bei Raumtemperatur stehen gelassen.
   Die Losung wurde im Vakuum eingeengt, der Rückstand in Methylenchlorid aufgenommen, nochmals unter Zusatz von Toluol abdestilliert und anschließend über eine Kieselgelsaule mit Methanol/25%igem wäßrigen Ammoniak (50/2) chromatographiert. Nach Abdestillieren des Eluenten wurde das Produkt in Wasser aufgenommen und nach Behandeln mit Aktivkohle lyophylisiert. Das Lyophylisat (1,95 g) zeigte einen Schmelzpunkt von 202-205°C,
   FAB-MS: 445 (M+H⁺)

### Beispiel 222:

### HOOCCH₂-(D)-Chg-Pyr-NH-3-(6-Am)-pico:

a) 5,2 g (14,75 mMol) Boc-(D)-Chg-Pyr-OH, 2,88 g (17 mMol) 6-Cyano-3-aminomethylpyridin, 12,2 ml DIPEA und 17 ml PPA (50%ig in Essigester) wurden bei 0°C in 50 ml DCM vereinigt. Man ließ anschließend unter Rühren innerhalb 1,5 h das Reaktionsgemisch auf Raumtemperatur kommen. Zur Aufarbeitung wurde die Lösung in 250 ml Essigester verdünnt und mit ges. NaHCO₃-Lsg. (3x), 20%iger NaHSO₄ (3x) und ges. NaCl-Lsg. (1x) gewaschen. Nach Trocknen der Lösung mit MgSO₄ wurde Essigester am Rotationsverdampfer entfernt. Rohausbeute: 7,8 g. Das Rohprodukt wurde ohne weitere Reinigung in der Folgereaktion eingesetzt.
b) Boc-(D)-Chg-Pyr-NH-3-(6-CN)-pico wurden in 10 ml DCM vorgelegt. Nach Kühlen der Lösung auf 0°C wurden 20 ml TFA (50%ig in DCM) zugefügt. Anschließend ließ man das Reaktionsgemisch innerhalb 3 h auf Raumtemperatur erwärmen und engte dann die Lösung am Rotationsverdampfer ein. Der Rückstand wurde in Toluol aufgenommen und die Lösung nochmals im Vakuum eingeengt. Dieser Vorgang wurde noch einmal wiederholt. Rohausbeute 13,5 g.
c) 13,5 g H-(D)-Chg-Pyr-NH-3-(6-CN)-pico x TFA wurden in 100 ml Acetonitril vorgelegt. Nach Zugabe von 2,69 g KI, 6,11 g K₂CO₃ und 2,87 g Bromessigsäure-t-butylester wurde die Suspension bei Raumtemperatur 5 h gerührt. Anschließend wurde K₂CO₃ und KI abfiltriert, Acetonitril im Vakuum am Rotationsverdampfer entfernt und der Rückstand in Essigester aufgenommen. Die Lösung wurde mit Wasser (2x) und ges. NaCl-Lsg. (1x) gewaschen, mit Na₂SO₄ getrocknet und eingeengt. Rohausbeute: 6,4 g.
d) 6 g tBuOOCCH₂-(D)-Chg-Pyr-NH-3-(6-CN)-pico wurden in 42 ml Pyridin und 19,4 ml TEA gelöst und mit H₂S-Gas gesättigt. Nach 18-stündigem Stehen bei Raumtemperatur wurde die Lösung zunächst mit Stickstoff gespült und dann auf 2 L Eiswasser gegossen. Die wäßrige Lösung wurde mit Essigester (6x) extrahiert und die vereinigten organischen Extrakte mit 5 %iger NaHSO₄-Lsg. gewaschen. Nach Trocknen und Einengen verblieben 6,1 g tBuOOCCH₂-(D)-Chg-Pyr-NH-3-(6-CSNH₂)-pico Rohprodukt.
e) 6,1 g rohes tBuOOCCH₂-(D)-Chg-Pyr-NH-3-(6-CSNH₂)-pico wurden in 7,4 ml MeI und 70 ml Aceton gelöst und 4,5 h bei Raumtemperatur gerührt. Anschließend wurde die Lösung eingeengt, in Toluol aufgenommen und nochmals am Rotationsverdampfer zur Trockne eingedampft. Rohausbeute: 6,1 g.
f) 6,1 g tBuOOCCH₂-(D)-Chg-Pyr-NH-3-(6-CSMe=NH)-pico wurden in einem Einhalskolben mit 30 ml MeOH und 30 ml methanolischer Ammoniumacetatlösung (20 %ig) vereinigt und 18 h bei Raumtemperatur stehengelassen. Die Lösung wurde eingeengt und der Rückstand in DCM aufgenommen. Die organische Lösung wurde mit Wasser (3 x 20 ml) gewaschen, mit Na₂SO₄ getrocknet und am Rotationsverdampfer eingeengt. Nach Umfällen des Rohproduktes aus Essigester/Diisopropylether erhielt man 2,7 g Rohprodukt. Das Rohprodukt wurde durch eine Reversed-Phase-HPLC-Chromatographie gereinigt. Ausbeute: 0,364 g.
g) 0,28 g tBuOOCCH₂-(D)-Chg-Pyr-NH-3-(6-am)-pico wurden bei 0°C in 5 ml Dioxan vorgelegt und nach Zugabe von 5 ml Dioxan/HCl 48 h bei Raumtemperatur gerührt. Das Rohprodukt wurde nach Einengen der Lösung mittels einer Säulenchromatographie gereinigt (MeOH/3% konz. NH₃-Lösung). Ausbeute: 180 mg.
   FAB-MS: 443 (M+H⁺)

### Beispiel 223:

### HOOCCH₂-(D)-Chg-2-Phi-NH-3-(6-Am)-pico:

Ausgehend von Boc-(D)-Chg-2-Phi-OH und 3-Aminomethyl-6-cyanopyridin wurde analog Beispiel 222 tBuOOC-CH₂-(D)-Chg-2-Phi-NH-3-(6-CN)-pico dargestellt. Dieses Zwischenprodukt wurde wie folgt in das Beispiel 223 überführt:
a) 8 g (14,9 mMol) tBuOOC-CH₂-(D)-Chg-2-Phi-NH-3-(6-CN)-pico wurden zusammen mit 8 ml TEA, 2,58 g Hydroxylaminhydrochlorid und 90 ml EtOH bei 70°C 18 h gerührt. Anschließend wurde die Suspension eingeengt, der Rückstand in DCM gelöst und die Lösung 3x mit je 5 ml HOAc (30%ig) gewaschen. Nach Trocknen uber Na₂SO₄ wurde DCM am Rotationsverdampfer entfernt. Das N-Hydroxyamidin wurde ohne weitere Reinigung in der Folgereaktion eingesetzt.
b) 5 g des N-Hydroxyamidins wurden in einem Reaktionskolben zusammen mit 6 g Raney-Nickel, 40 ml EtOH und 9 ml HOAc vereinigt und bei 60°C unter wasserstoffatmosphäre reduziert. Das Rohprodukt wurde mittels einer Reversed Phase HPLC-Chromatographie (Acetonitril/Wasser) getrennt. Ausbeute 0,7 g.
c) 0,7 g tBuOOC-CH₂-(D)-Chg-2-Phi-NH-3-(6-Am)-pico wurden analog Beispiel 222 in die freie Säure überführt.
   FAB-MS: 499 (M+H⁺)

### Beispiel 224:

### HOOC-CH(Me)- (D)-Chg-Pro-NH-3-(6-Am)-pico:

a) 7,4 g (15,22 mMol) H-(D)-Chg-Pro-NH-3-(6-CN)-pico x TFA, 6,3 g K₂CO₃ und 3,69 g 2-Brompropionsäurebenzylester wurde in 100 ml Acetonitril 12 h bei 50°C gerührt. Nach vollständigem Umsatz des Eduktes wurde der Feststoff abfiltriert und das Filtrat eingeengt. Hierauf wurde der Rückstand in Essigester gelöst und 2 x mit Wasser gewaschen. Nach Trocknen der organischen Lösung wurde der Essigester am Rotationsverdampfer entfernt. Rohausbeute: 5 g. Nach einer Saulenchromatographie über Kieselgel verblieben 3 g des Produktes.
b) 3 g des BzlOOC-CH(Me)-(D)-Chg-Pro-NH-3-(6-CN)-pico wurden analog Beispiel 223 in das entsprechende Amidin überführt.
   Ausbeute: 0,8 g.
c) Die freie Säure wurde durch Hydrieren des Benzylesters unter Standardbedingungen gewonnen. Das Rohprodukt wurde mittels einer Reversed Phase HPLC-Chromatographie gereinigt. Ausbeute: 0,4 g.
   FAB-MS 459 (M+H⁺)

### Beispiel 225:

### Boc-(D)-Phe-Pro-NH-3-(2-Me-6-Am)-pico:

Die Darstellung erfolgte analog Beispiel 227; Fp: 130 - 140°C; (Hydroacetat)

### Beispiel 226:

### H-(D)-Phe-Pro-NH-3-(2-Me-6-Am)-pico:

Die Darstellung erfolgte analog Beispiel 228; (Dihydrochlorid) ¹³CNMR d⁶-DMSO δ in ppm:
170,79, 167,62, 161,85, 156,34, 140,72, 138,41, 135,87, 134,53, 129,30, 128,49, 127,29, 120,70, 60,23, 52,18, 46,61, 39,1, 36,48, 29,22, 23,33, 21,72

### Beispiel 227:

### Boc-(D)-Chg-Pro-NH-3-(2-Me-6-Am)-pico:

a) Darstellung von Boc-(D)Chg-Pro-NH-3-(2-Me)-pico:
   6,4 g Boc-(D)-Chg-Pro-OH (18,05 mMol) wurden zusammen mit 4,0 g 2-Methyl-3-picolylamin (20,5 mMol, Herstellung s. Arch. Pharm 308 (1975) 969-76) und 14 ml DIPEA (81,8 mMol) in 200 ml DCM vorgelegt, auf 5°C abgekühlt und bei dieser Temperatur 18,8 ml 50 %ige Propanphosphonsäureanhydrid-Lösung in Essigester (23,92 mMol) zugetropft. Nach Erwärmen auf Raumtemperatur ließ man 1 h nachreagieren und konzentrierte anschließend im Vakuum ein. Der Rückstand wurde in Essigester aufgenommen, die Essigesterphase ca. 10 mal mit Wasser extrahiert, über Magnesiumsulfat getrocknet und einrotiert. Durch Ausrühren des Rückstandes mit Diisopropylether erhielt man 7,2 g (87 %) Boc-(D)-Chg-Pro-NH-3-(2-Me)-pico als weiße Festsubstanz.
b) Darstellung von
   Boc-(D)-Chg-Pro-NH-3-(2-Me-1-Oxo)-pico:
   5,3 g Boc-(D)-Chg-Pro-NH-3-(2-Me)-pico (11,58 mMol) wurden zusammen mit 3,1 g 98 %iger m-Chlorperbenzoesäure (18,14 mMol) in 150 ml DCM 2 h bei Raumtemperatur gerührt. Anschließend wurde Ammoniakgas bis zur sättigung eingeleitet, 1 h bei Raumtemperatur gerührt, der Niederschlag abgesaugt, mit DCM gewaschen und das Filtrat nochmals mit Ammoniak gesättigt. Danach wurde die DCM-Phase 3 mal mit Wasser gewaschen, über Magnesiumsulfat getrocknet und im Vakuum eingeengt.
   Man erhielt 5,5 g Boc-(D)-Chg-Pro-NH-3-(2-Me-1-Oxo)-pico als weiße Festsubstanz.
c) Darstellung von
   Boc-(D)-Chg-Pro-NH-3-(2-Me-1-MeO)-pico:
   3,6 g Boc-(D)-Chg-Pro-NH-3-(2-Me-1-Oxo)-pico (7,58 mMol) wurden in 10 ml DCM gelöst, mit 2,0 ml Dimethylsulfat (21,1 mMol) in 20 ml DCM versetzt, über Nacht bei Raumtemperatur gerührt, die Losung im Vakuum eingeengt und der Rückstand 3 mal mit Ether ausgerührt.
   Man erhielt 4,55 g (100 %) Boc-(D)-Chg-Pro-NH-3-(2-Me-1-MeO)-pico^{⊕} CH₃OSO₃^{⊖} als weiße Festsubstanz, die ohne weitere Aufreinigung in der nachfolgenden Umsetzung eingesetzt wurde.
d) Darstellung von
   Boc-(D)-Chg-Pro-NH-3-(2-Me-6-CN)-pico:
   4,55 g Boc-(D)-Chg-Pro-NH-3-(2-Me-1-MeO)-pico^{⊕} CH₃OSO₃^{⊖} (7,58 mMol) wurden in 10 ml DMF gelöst und bei Raumtemperatur 0,5 g Natriumcyanid (10,02 mMol) gelöst in 30 ml DMF zugetropft (leicht exotherme Reaktion). Nach einer Stunde Rühren bei Raumtemperatur wurde DMF im Vakuum (1 mbar) abdestilliert, der Rückstand in 1 M Kaliumhydrogensulfatlösung aufgenommen, mit Ether extrahiert, die organischen Phasen über Magnesiumsulfat getrocknet und im Vakuum einrotiert.
   Man erhielt 2,8 g (76 %) Boc-(D)-Chg-Pro-NH-3-(2-Me-6-CN)-pico als weißen Schaum.
e) Darstellung von
   Boc-(D)-Chg-Pro-NH-3-(2-Me-6-Ham)-pico:
   3,63 g Boc-(D)-Chg-Pro-NH-3-(2-Me-6-CN)-pico (7,51 mMol) wurden zusammen mit 1,9 g Hydroxylammoniumchlorid (18,76 mMol) und 6,4 ml DIPEA (37,525 mMol) in 50 ml DCM 4 h bei Raumtemperatur gerührt, anschließend im Vakuum einrotiert, der Rückstand in Essigester aufgenommen, 6 mal mit verd. Salzsäure (pH 4) gewaschen, die organische Phase über Magnesiumsulfat getrocknet und im Vakuum einrotiert.
   Man erhielt 3,8 g (98 %) Boc-(D)-Chg-Pro-NH-3-(2-Me-6-Ham)-pico als weiße Festsubstanz.
f) Darstellung von
   Boc-(D)-Chg-Pro-NH-3-(2-Me-6-Am)-pico:
   3,8 g Boc-(D)-Chg-Pro-NH-3-(2-Me-6-Ham)-pico (7,35 mMol) wurden mit zwei Spatelspitzen 10 % Pd/c in 80 ml Ethanol und 15 ml Essigsäure bei 60°C 8 h unter leichtem Überdruck hydriert, der Katalysator über einen Glasfaserfilter abfiltriert, mit Ethanol gewaschen und das Filtrat im Vakuum (1 mbar) eingeengt. Nach zweimaligem Ausrühren des Rückstandes mit Ether erhielt man 4,0 g (97 %) Boc-(D)-Chg-Pro-NH-3-(2-Me-6-Am)-pico als weiße Festsubstanz. Fp: 144-153; (Hydroacetat)

### Beispiel 228:

### H-(D)-Chg-Pro-NH-3-(2-Me-6-Am)-pico:

2,8 g Boc-(D)-Chg-Pro-NH-3-(2-Me-6-Am)-pico x CH₃COOH (4,99 mMol) wurden in 10 ml DCM und 15 ml Methanol mit 25 ml etherischer Salzsäure (> 3 M) 4 h bei Raumtemperatur gerührt. Die Lösung wurde im Vakuum eingeengt, mehrfach mit DCM, Methanol kodestilliert und der Rückstand aus Ether/DCM und Ether/Methanol ausgerührt. Man erhielt 2,5 g H-(D)-Chg-Pro-NH-3-(2-Me-6-Am)-pico x 2 HCl als weiße Festsubstanz.
Fp: 128-135°C: (Dihydrochlorid)
¹³C-NMR d₆-DMSO, δ in ppm:
170,96, 167,72, 161,86, 156,30, 140.76, 138,53, 135,85, 120,72, 60,56, 55,17, 47,43, 39,20, 38,78, 29,66, 27,75, 25,40, 25,31, 25,20, 23,71, 21,76

### Beispiel 229:

### tBuOOC-CH₂-(D)-Chg-Pro-NH-3-(2-Me-6-Am)-pico:

a) Darstellung von
   H-(D)-Chg-Pro-NH-3-(2-Me)pico:
   7,8 g Boc-(D)-Chg-Pro-NH-3-(2-Me)-pico (17,0 mMol) wurden in 35 ml DCM und 35 ml etherischer Salzsäure (> 3 M) 2 h bei Raumtemperatur gerührt, im Vakuum einrotiert, mehrfach mit Methanol/DCM kodestilliert und der Rückstand aus Ether ausgerührt. Man erhielt 7,3 g (100 %) H-(D)-Chg-Pro-NH-3-(2-Me)-pico x 2 HCl als weiße Festsubstanz.
b) Darstellung von
   tBuOOC-CH₂-(D)-Chg-Pro-NH-3-(2-Me)-pico:
   9,4 g H-(D)-Chg-Pro-NH-3-(2-Me)-pico x 2HCl (21,79 mMol) wurden zusammen mit 11,26 g (14,9 ml) DIPEA (81,16 mMol) und 4,89 g (3,69 ml) Bromessigsäure-tert.-butylester (25,0 mMol) in 150 ml DCM (getrocknet üher Molsieb) 16 h bei Raumtemperatur gerührt. Da laut DC noch Edukt vorhanden war, wurden noch 0,4 ml Bromessigsaure-tert.-butylester und 1,5 m DIPEA zugegeben und weitere 3 h bei Raumtemperatur gerührt. Anschließend wurde die Reaktionsmischung zunächst im Wasserstrahlvakuum dann bei 1 mbar bei max. 40°C einkonzentriert. Der Ruckstand wurde mit Ether ausgerührt, abfiltriert und mit Ether gewaschen. Nach Aufnahme des Kristallisats in Wasser wurde bei pH 7,5 mehrfach mit Essigester extrahiert, diese Essigesterextrakte zusammen mit dem obigen Etherfiltrat vereinigt, getrocknet und im Vakuum einrotiert. Nach Aufnahme des Ruckstandes in Ether wurde etherische Salzsäure bis zu pH 3 zugesetzt, er ausgefallene Niederschlag abgesaugt, gut mit Ether gewaschen und noch 2 mal aus Ether ausgerührt. Man erhielt 9,1 g (82 %) tBuOOC-CH₂-(D)-Chg-Pro-NH-3-(2-Me)-pico x HCl als weiße Festsubstanz.
c) Darstellung von
   t-BuOOC-CH₂-(Boc)(D)-Chg-Pro-NH-3-(2-Me)-pico:
   9,5 g tBuOOC-CH₂-(D)-Chg-Pro-NH-3-(2-Me)-pico x HCl (18,66 mMol) wurden zusammen mit 18,66 g (Boc)₂O (18,66 mMol) in 160 ml DCM vorgelegt, innerhalb von 5 min. mit 5,3 g (7,03 ml) DIPEA (41,05 mMol) versetzt und anschließend über Nacht bei Raumtemperatur gerührt. Nach weiterer Zugabe von DCM wurde mit 0,5 M HCl-Lösung gewaschen, bis im DCM kein DIPEA mehr vorhanden war (DC-Kontrolle), über Magnesiumsulfat getrocknet und im Vakuum einrotiert.
   Durch Säulenchromatographie auf Kieselgel mit DCM und 0-5 % Methanol erhielt man 5,8 g (54 %) tBuOOC-CH₂-(Boc)(D)-Chg-Pro-NH-3-(2-Me)-pico als weiße Festsubstanz.
d) Darstellung von
   tBuOOC-CH₂-(Boc)-(D)-Chg-Pro-NH-3-(2-Me-1-Oxo)-pico:
   5,8 g tBuOOC-CH₂-(Boc)-(D)-Chg-Pro-NH-3-(2-Me)-pico (10,12 mMol) wurden zusammen mit 9,99 g 70 %iger m-Chlorperbenzoesaure (40,5 mMol) in 200 ml DCM 2 h bei Raumtemperatur gerührt. Anschließend wurde Ammoniakgas bis zur Sättigung eingeleitet, 1 h bei Raumtemperatur gerührt, der Niederschlag abgesaugt, mit DCM gewaschen und das Filtrat nochmals mit Ammoniak gesättigt. Danach wurde die DCM Phase 3 mal mit Wasser gewaschen, über Magnesiumsulfat getrocknet und im Vakuum eingeengt. Man erhielt 5,95 g (100 %).
e) Darstellung von
   tBuOOC-CH₂-(Boc)(D)-Chg-Pro-NH-3-(2-Me-1-MeO)-pico⊕·CH₃OSO₃⊖:
   5,95 g tBuOOC-CH₂-(Boc)-(D)-Chg-Pro-NH-3-(2-Me-1-Oxo)-pico (10,12 mMol) wurden in 25 ml DCM gelöst und mit 28 ml einer 5 %igen Dimethylsulfatlösung in DCM versetzt. Nach 5-stündigem Rühren bei 40°C und Stehenlassen über Nacht bei Raumtemperatur wurde auf 100 ml DCM verdünnt, rasch 3 mal mit Wasser gewaschen, über Magnesiumsulfat getrocknet und im Vakuum einrotiert. Das erhaltene tBuOOC-CH₂-(Boc)-(D)-Chg-Pro-NH-3-(2-Me-1-MeO)-pico^{⊕}·CH₃OSO₃^{⊖} wurde als Rohprodukt in der nachfolgenden Umsetzung eingesetzt.
f) Darstellung von
   tBuOOC-CH₂-(Boc)-(D)-Chg-Pro-NH-3-(2-Me-6-CN)-pico:
   Das aus der obigen Umsetzung erhaltene Rohprodukt von tBuOOC-CH₂-(Boc)(D)-Chg-Pro-NH-3-(2-Me-1-MeO)-pico⊕CH₃OSO₃⊖ wurde innerhalb von 20 min. zu einer Lösung von 1,1 g Natriumcyanid (21,3 mMol) in 50 ml DMF getropft, wobei die Temperatur durch Kühlung bei 23 - 25°C gehalten wurde. Nach weiteren 20 min. wurde DMF im Vakuum (1 mbar) abdestilliert, der Rückstand in Ether aufgenommen, nacheinander mit Wasser, KHSO₄-Lösung (pH 2), Wasser und gesättigter Kochsalzlösung gewaschen, die Etherphase über Magnesiumsulfat getrocknet und im Vakuum einrotiert.
   Nach säulenchromatographischer Reinigung über Kieselgel (Elutionsmittel DCM mit 0-2 % MeOH) erhielt man 4,1 g Festsubstanz, welche aus Ether ausgerührt wurde.
   Ausbeute: 4,0 g (66 %) tBuOOC-CH₂-(Boc)(D)-Chg-Pro-NH-3-(2-Me-6-CN)-pico
g) Darstellung von
   tBuOOC-CH₂-(Boc)(D)-Chg-Pro-NH-3-(2-Me-6-Ham)-pico:
   3,95 g tBuOOC-CH₂-(Boc)(D)-Chg-Pro-NH-3-(2-Me-6-CN)-pico (6,6 mMol) wurden zusammen mit 1,15 g Hydroxylaminhydrochlorid (16,52 mMol) und 5,12 g (6,78 ml) DIPEA (39,6 mMol) in 75 ml DCM (getrocknet über Molsieb) 2 h unter Rückfluß erhitzt und anschließend über Nacht bei Raumtemperatur gerührt. Nach Zugabe von weiterem DCM wurde mit verd. Salzsäure (pH 4) gewaschen, die org. Phase über Magnesiumsulfat getrocknet und im Vakuum einrotiert. Die erhaltenen 4,2 g Rohprodukt von tBuOOC-CH₂-(Boc)(D)-Chg-Pro-NH-3-(2-Me-6-Ham)-pico wurden als Rohprodukt in der nachfolgenden Umsetzung eingesetzt.
h) Darstellung von
   tBuOOC-CH₂-(Boc)-(D)-Chg-Pro-NH-3-(2-Me-6-Am)-pico:
   4,2 g Rohprodukt von tBuOOC-CH₂-(Boc)-(D)-Chg-Pro-NH-3-(2-Me-6-Ham)-pico wurden in einem Gemisch von 15 ml Essigsäure und 80 ml Ethanol über Pd/C (10 %ig) mit Wasserstoff 5 h bei 50°C hydriert. Anschließend wurde der Katalysator abfiltriert, mit Ethanol gewaschen, das Filtrat im Vakuum (1 mbar) einrotiert, der Rückstand mehrfach mit Toluol/DCM kodestilliert, in 100 ml Ether aufgenommen und 3 mal mit je 4 ml Wasser gewaschen. Die vereinigten Wasserphasen wurden im Vakuum (1 mbar) bei max. 35 - 40°C einrotiert und der Rückstand mit Ethanol kodestilliert. Man erhielt 4,2 g fast reines tBuOOC-CH₂-(Boc)-(D)-Chg-Pro-NH-3-(2-Me-6-Am)-pico x CH₃COOH (94 % über zwei Stufen) als weiße Festsubstanz.
i) Darstellung von
   tBuOOC-CH₂-(D)-Chg-Pro-NH-3-(2-Me-6-Am)-pico:
   2,0 g tBuOOC-CH₂-(Boc)(D)-Chg-Pro-NH-3-(2-Me-6-Am)-pico x CH₃COOH (2,96 mMol) wurden in 10 ml DCM zusammen mit 10 ml etherischer Salzsäure (Ether gesättigt mit HCl) 1 h 20 min. bei Raumtemperatur gerührt, anschließend im Vakuum einrotiert, der Rückstand in Wasser aufgenommen und mehrfach mit Essigester extrahiert. Die wäßrige Phase wurde im Vakuum (1 mbar) bei max. 35-40°C einrotiert und mehrfach mit Aceton kodestilliert. Nach säulenchromatographischer Trennung des erhaltenen Gemisches über Kieselgel (Elutionsmittel DCM/Methanol/Essigsäure 100/10/2 → 100/20/5) erhielt man 0,7 g tBu-OOC-CH₂-(D)-Chg-Pro-NH-3-(2-Me-6-Am)- pico x (HX)_{1,2} (X^{⊖} = Cl und/oder CH₃CO₂^{⊖}), als weiße Festsubstanz, die unter Zersetzung ab 205°C schmolz.

### Beispiel 230:

### HOOC-CH₂-(D)-Chg-Pro-NH-3-(2-Me-6-Am)-pico:

2,2 g tBuOOC-CH₂-(Boc)-(D)-Chg-Pro-NH-3-(2-Me-6-Am)-pico x CH₃COOH (3,25 mMol) wurden in 30 ml DCM zusammen mit 15 ml etherischer Salzsäure mehrere Stunden bei Raumtemperatur gerührt, währenddessen langsam ein Feststoff ausfiel. Der Feststoff wurde abgesaugt, mit heißem DCM mehrfach ausgerührt und anschließend über Kieselgel chromatographiert (Fließmittel Methanol/25 % wäßrige Ammoniaklösung im Verhältnis 95/5). Man erhielt 1,3 g (94 %) HOOC-CH₂-(D)-Chg-Pro-NH-3-(2-Me-6-Am)-pico als weiße Festsubstanz, die ab 210°C unter Zersetzung schmolz.

### Beispiel 231:

### MeOOC-CH₂-(D)-Chg-Pro-NH-3-(2-Me-6-Am)-pico:

0,45 g HOOC-CH₂-(D)-Chg-Pro-NH-3-(2-Me-6-Am)-pico (0,1 mMol) wurden in 30 ml Methanol (über Molekularsieb getrocknet) vorgelegt, tropfenweise mit 1 ml Thionylchlorid versetzt und 2 h unter Rückfluß gerührt. Nach weiterer Zugabe von 0,3 ml Thionylchlorid und 1 h Rühren unter Rückfluß wurde die Lösung im Vakuum einrotiert, mehrfach mit Methanol/DCM kodestilliert und der Rückstand säulenchromatographisch über Kieselgel gereinigt (Fließmittel: DCM/Methanol/Essigsäure 100/20/5). Nach mehrfachem Kodestillieren mit Toluol wurden 0,38 g MeOOC-CH₂-(D)-Chg-NH-3-(2-Me-6-Am)-pico·(HX)_{1,2} (X^{⊖}= Cl und/oder CH₃COO^{⊖}) als weiße Festsubstanz erhalten, die bei 155-160°C schmolz.

### Beispiel 232:

### Boc-(D)-Chg-Pro-NH-2-(5-Am)-pico:

a) 5-Carboxamido-2-picolylamin:
   Zu einer Lösung von 3,5 g (24 mMol) 2-Cyano-5-carboxamidopyridin in 80 ml Methanol und 20 ml konz. Ammoniak gab man 3 g Raney-Ni und hydrierte bei Raumtemperatur. Nach ca. 7 h war die wasserstoffaufnahme vollständig.
   Nach Absaugen des Katalysators wurde das Filtrat eingeengt und der Rückstand in 20 ml 2 N Salzsäure und 20 ml Methanol gelöst. Durch Zugabe von 150 ml Essigester kam es zur Abscheidung des Hydrochlorids, das abgesaugt und getrocknet wurde (3,7 g). Die freie Base schmolz bei 198-202°C.
b) 5-Cyano-2-picolylamin:
   41 g (0,22 Mol) 5-Carboxamido-2-picolylamin wurden in 150 ml Methanol und 300 ml Methylenchlorid suspendiert, auf 10°C abgekühlt und durch Zugabe von 150 ml Triethylamin in Lösung gebracht. Anschließend tropfte man eine Lösung von 47,6 g (0,22 Mol) (Boc)₂O zu und ließ 4 h bei Raumtemperatur nachrühren.
   Nach Abziehen des Lösungsmittels wurde der Rückstand mit einer gesättigten K₂CO₃-Lösung versetzt und 5 x mit Methylenchlorid extrahiert. Die vereinigten Extrakte wurden getrocknet und das Lösungsmittel, gegen Ende unter Zusatz von Toluol, abdestilliert.
   5,4 g des Rückstands wurden in 40 ml Dioxan und 15 ml Methylenchlorid suspendiert, mit 4,3 g Pyridin versetzt und anschließend bei 0°C 5,2 g Trifluoressigsäureanhydrid zugetropft, wobei eine klare Lösung auftrat.
   Nach Zugabe von 100 ml wasser wurde mit Essigester extrahiert, die organische Phase mit verd. Zitronensäure-, NaHCO₃-Lösung und Wasser gewaschen. Nach Trocknen und Abziehen des Lösungsmittels verblieb ein gelbes Öl (ca. 5 g), das man in 15 ml Isopropanol und 30 ml Essigester löste und mit 35 ml etherischer Salzsäurelösung versetzte. Nach Stehen über Nacht wurde das ausgefallene Hydrochlorid abgesaugt und getrocknet. Man isolierte 4 g weiße Kristalle. Fp 230-234°C.
c) Boc-(D)-Cyclohexylglycyl-prolin-(5-cyano-2-picolyl)amid:
   Herstellung analog Beispiel 219, Stufe b) durch Kupplung von Boc-(D)-Cyclohexylglycyl-prolin mit 5-Cyano-2-picolylamin. Weiße Kristalle, Fp 128-129°C
d) Boc-D-Cyclohexylglycyl-prolin-(6-amidino-2-picolyl)amid:
   Die Amidierung der vorstehenden Verbindung erfolgte analog Beispiel 218, Stufe d).
   Acetat: Weiße Kristalle, Fp 98-100°C (Zersetzung);
   FAB-MS: 487 (M+H⁺)

### Beispiel 233:

### H-(D)-Chg-Pro-NH-2-(5-Am)-pico:

Die Verbindung 233, Stufe d) wurde nach Standardbedingungen entschützt.
Dihydrochlorid: Weiße Kristalle, Fp 233-235°C (Zersetzung)
FAB-MS: 386 (MH⁺).

### Beispiel 234:

### HOOC-CH₂-(D)-Chg-Pro-NH-2-(5-Am)-pico:

Analog Beispiel 221, Stufe a), b), c) und d) wurde aus Boc-(D)-Cyclohexylglycyl-prolin-(5-cyano-2-picolyl)amid durch Abspaltung der Boc-Gruppe, wobei keine Amidbildung auftrat, N-Alkylierung mit Bromessigsäure-t-butylester, Amidinbildung und saure Verseifung des t-Butylesters die Titelverbindung erhalten. weiße Kristalle, Fp 162-4°C,
FAB-MS: 445 (MH⁺)

### Beispiel 235:

### HOOC-CH₂-(D)-Chg-Pro-NH-5-(2-Am)-pym:

a) 2-Thiomethyl-5-Aminomethylpyrimidinhydrochlorid:
   28,1 g (182,2 mMol) 2-Thiomethyl-5-formyl-pyrimidin (Z. Arnold et al. J. Heterocyclic Chem. 1991, 28, 1281) wurden in 880 ml MeOH/THF (1:1) bei -23°C vorgelegt. Nach Zugabe von 12,8 g (34,3 mMol) CeCl₃ x 7H₂O wurden portionsweise 5,19 g (137,2 mMol) Natriumborhydrid zugefügt. Nach 1,5 h Reaktionszeit wurde die Reaktionslösung mit 1,5 L ges. NaCl-Lsg. versetzt und diese Mischung mit DCM (4 x 130 ml) extrahiert. Die vereinigten organischen Phasen wurden getrocknet und im Vakuum eingeengt. Ausbeute: 26,9 g.
   26,89 g (172,14 mMol) 2-Thiomethyl-5-hydroxymethyl-pyrimidin wurden in 390 ml DCM (abs.) gelöst und nach Zugabe von 1 Tropfen DMF und 27 ml (370,37 mMol) SOCl₂ 45 min. bei 0°C gerührt. Zur Aufarbeitung wurde die Reaktionslösung zur Trockne eingeengt.
   Das so erhaltene 2-Thiomethyl-5-chlormethyl-pyrimidin wurde zusammen mit 16,79 g (258,2 mMol) NaN₃ in 84 ml DMSO über Nacht bei Raumtemperatur gerührt. Aufgrund eines unvollständigen Umsatzes wurden weitere 4,2 g NaN₃ zugefügt. Nach weiteren 2 h Reaktionszeit war das Chloridderivat vollständig umgesetzt. Zur Aufarbeitung wurde das Reaktionsgemisch in 300 ml Wasser gegossen und die wäßrige Phase mit Et₂O (5 x 100 ml) extrahiert. Die vereinigten organischen Extrakte wurden mit Wasser (3 x 25 ml) gewaschen, getrocknet und anschließend der Ether im Vakuum fast vollständig entfernt.
   Die konzentrierte etherische 2-Thiomethyl-5-azidomethylpyrimidin-Lösung wurde in 28 ml THF gelöst und vorsichtig zu einer Lösung von 45,15 g (172,1 mMol) Ph₃P in 84 ml THF unter Eiskühlung gegeben. Nach 15 min. wurde die Eiskühlung entfernt, 4,65 ml Wasser zum Reaktionsgemisch gegeben und die Reaktionslösung 18 h bei RT gerührt. Zur Aufarbeitung wurde das Reaktionsgemisch im Vakuum zur Trockne eingeengt und der erhaltene Rückstand in 70 ml 3N HCl aufgenommen. Die wäßrige Lösung wurde mit Essigester/Et₂O (1/1; 4 x 50 ml) gewaschen. Anschließend wurde die Lösung mit Na₂CO₃ auf pH 9 eingestellt und mit DCM (12 x 50 ml) extrahiert. Die vereinigten organischen Extrakte wurden getrocknet und eingeengt. Der Rückstand wurde in DCM/Essigester gelöst und das freie Amin mit Dioxan/HCl als Hydrochlorid gefällt. Ausbeute: 30,48 g.
   ¹H-NMR (d₆-DMSO, δ in ppm): 2,55 (s, 3H, CH3); 4,1 (q, 2H, N-CH2); 8,8 (s, 2H, Ar-H); 10,8 (sb, NH)
b) Boc-Pro-NH-5-(2-SMe)-pym:
   12,9 g (60 mMol) Boc-Pro-OH wurden zusammen mit 15 g (65,8 mMol) 2-Thiomethyl-5-aminomethylpyrimidinhydrochlorid und 61,4 ml (359 mMol) DIPEA in 150 ml DCM bei 0°C vorgelegt. Nach Zugabe von 63,4 ml PPA (50 %ig in Essigester) wurde das Reaktionsgemisch bei 0°C - Raumtemperatur 6 h gerührt. Nachdem das Boc-Pro-OH vollständig umgesetzt war (DC-Kontrolle: DCM/MeOH 95:5), wurde das Reaktionsgemisch in 300 ml Essigester aufgenommen. Die organische Phase wurde mit 20 %iger Natriumhydrogensulfatlosung (2x), Wasser (2x) und gesättigter NaCl-Lösung gewaschen. Nach Trocknen der organischen Phase mit Natriumsulfat wurde Essigester im Vakuum entfernt. Es verblieben 16,7 g des gewunschten Produktes.
c) Boc-Pro-NH-5-(2-SO₂Me)-pym:
   20,5 g (58,1 mMol) Boc-Pro-NH-5-(2-SMe)-pym wurden bei Raumtemperatur in 700 ml DCM vorgelegt. Anschließend wurden 42,94 g (174 mMol) m-CPBA portionsweise innerhalb 30 min. zu der Lösung gefügt. Nach insgesamt 2 h Reaktionszeit wurde das Reaktionsgemisch mit 20 %iger NaHSO₄ (2x), 5 %iger NaHCO₃-Lsg. (6x) und 20 %iger Na₂S₂O₅-Lsg. (3x) extrahiert. Nach Trocknen der Lösung und Entfernen des DCM's verblieben 21,7 g des Sulfons Boc-Pro-NH-5-(2-SO₂Me)-pym.
d) Boc-Pro-NH-5-(2-CN)-pym:
   21,7 g (56,4 mMol) Boc-Pro-NH-5-(2-SO₂Me)-pym wurden in 30 ml DMSO gelöst und nach Zugabe von 2,84 g NaCN über Nacht bei Raumtemperatur gerührt. Anschließend wurde die Lösung in 150 ml Wasser gegossen und die waßrige Lösung mit DCM (5 x 100 ml) extrahiert. Die vereinigten organischen Phasen wurden mit ges. NaCl-Lsg. (5x) und Wasser (2x) gewaschen. Nach Trocknen und Einengen der organischen Lösung erhielt man 15,3 g des gewünschten Cyanids.
e) H-Pro--NH-5-(2-CN) -pym x 3 TFA:
   13,98 g (42,1 mMol) Boc-Pro-NH-5-(2-CN)-pym wurden in DCM vorgelegt. Nach Zugabe von 13 ml (170 mMol) TFA ließ man die Lösung bis zum vollständigen Umsatz des Eduktes bei Raumtemperatur rühren (DC-Kontrolle). Nach Einengen der Lösung im Vakuum verblieb das gewünschte Salz, welches in den Folgereaktionen ohne weitere Reinigung weiter umgesetzt wurde.
f) H-(D)-Chg-Pro-NH-5-(2-CN)-pym x 3 TFA:
   10 mMol H-Pro-NH-5-(2-CN)-pym x 3 TFA, 2,44 g (9,5 mMol) Boc-D-Chg-OH und 9,8 ml (57 mMol) DIPEA wurden bei 0°C vorgelegt. Nach Zugabe von 10,1 ml PPA (50 %ig in Essigester) ließ man das Reaktionsgemisch unter Rühren innerhalb von 6 h auf Raumtemperatur kommen. Zur Aufarbeitung wurde es mit 300 ml Essigester verdünnt und die organische Phase mit 20 %iger Natriumhydrogensulfatlösung (2x), Wasser (2x) und gesättigter NaCl-Lösung gewaschen. Nach Trocknen der organischen Phase mit Natriumsulfat wurde Essigester im Vakuum entfernt. Es verblieben 4,74 g des gewünschten Produktes. Das so erhaltene Rohprodukt wurde wie oben beschrieben in das entsprechende Trifluoressigsäuresalz überführt.
g) tBuOOC-CH₂-(D)-Chg-Pro-NH-5-(2-CN)-pym:
   4,1 g (11,07 mMol) H-(D)-Chg-Pro-NH-5-(2-CN)-pym x 3 TFA wurden zusammen mit 1,68 g (12,17 mMol) Kaliumcarbonat und 1,63 ml (11,07 mMol) Bromessigsäure-t-butylester bei RT gerührt. Nach vollständigem Umsatz wurde das Kaliumcarbonat abfiltriert und das Filtrat am Rotationsverdampfer eingeengt. Der Rückstand wurde in Essigester gelöst und die organische Lösung mit Natriumhydrogencarbonatlösung (5 %ig) und ges. Natriumchloridlösung gewaschen. Anschließend wurde das Lösungsmittel im Vakuum entfernt (Rohausbeute: 3,66 g). Das Rohprodukt wurde mittels Säulenchromatographie (DCM/MeOH 98/2 + 0,5 % konz. NH₃-Lsg.) gereinigt. Man erhielt 1,3 g des reinen Produktes.
h) tBuOOC-CH₂-(D)-Chg-Pro-NH-5-(2-Am)-pym:
   1,3 g (2,68 mMol) tBuOOC-CH₂-(D)-Chg-Pro-NH-5-(2-SMe)-pym wurden in 15 ml EtOH gelöst und nach Zugabe von 0,5 g (6,71 mMol) Hydroxylammoniumchlorid und 2,5 ml DIPEA 4 h bei 60°C gerührt. Das Reaktionsgemisch wurde am Rotationsverdampfer eingeengt und in DCM aufgenommen. Nach Waschen der organischen Lösung mit wenig Wasser, Trocknen und Einengen der Lösung wurde das Rohprodukt wieder in EtOH gelöst und nach Zugabe von Raney-Nickel 4 h bei 60°C unter einer Wasserstoffatmosphäre hydriert. Nach Abfiltrieren des Raney-Nickels wurde die ethanolische Lösung eingeengt und das Rohprodukt mittels einer Säulentrennung über Kieselgel (DCM/MeOH/50 % HOAc 40/10/2) gereinigt. Ausbeute: 250 mg.
i) HOOC-CH₂-(D)-Chg-Pro-NH-5-(2-Am)-pym:
   250 mg tBuOOC-CH₂-(D)-Chg-Pro-NH-5-(2-Am)-pym wurde mit TFA/DCM zur Säure gespalten und das Rohprodukt säulenchromatographisch gereinigt (MeOH/3 % konz. NH₃). Ausbeute: 108 mg.
   MS: 446 (M+H⁺), 369

### Beispiel 236:

### (D)-Man-Pro-NH-4-(1-Am)-pip:

Eine Lösung von 4,2 g (12,6 mMol) O-Tetrahydropyranyl-(D)-2-phenyl-2-hydroxyacetyl-(L)-prolin (WO 93/18060) in 40 ml THF wurde nach Zugabe von 1,9 g (12,6 mMol) 1-Hydroxybenzotriazol und 3,3 g (25 mMol) Dicyclohexylcarbodiimid 4 h bei Raumtemperatur gerührt. Der ausgefallene Harnstoff wurde abgesaugt und mit wenig THF nachgewaschen.
Zu diesem Filtrat wurde bei 5°C eine Lösung von 2,9 (12,6 mMol) 1-Amidino-4-aminomethyl-piperidin-dihydrochlorid und 1,6 g Natriumhydrogencarbonat in 6 ml Wasser zugefügt. Nach 48 h Rühren bei Raumtemperatur wurde das Lösungsmittel weitgehend abdestilliert, der Rückstand in Ethanol aufgenommen, von Ungelöstem abfiltriert und erneut eingeengt.
Der Rückstand wurde über eine Kieselgelsäule mit einem CH₂Cl₂/MeOH/50 %igem Essigsäure-Gemisch (45/5/1,5) gereinigt. Das Eluat der einheitlichen Fraktionen wurde abdestilliert, gegen Ende mit Toluol als Zusatz, und der Rückstand aus 50 ml Aceton unter Zusatz von wenig Wasser umkristallisiert. Man isolierte 3,5 g Acetat in Form weißer Kristalle, Fp 199-202°C (Zersetzung);
FAB-MS: 388 (M+H⁺).

### Beispiel 239:

### Boc-(D)-Phe-Pro-NH-(2-MeO)-pAmb:

a) Boc-Pro-(4-cyano-2-methoxy)-benzylamid:
   16,0 g Boc-Prolin (50 mMol), gelöst in 80 ml THF, wurden mit 5,7 g Hydroxysuccinimid und 10,2 g DCC 30 min. bei 0°C gerührt. Anschließend wurden 8,0 g (50 mMol) 4-Aminomethyl-3-methoxy-benzonitril, gelöst in 50 ml THF, bei 0°C hinzugetropft und 20 h bei RT gerührt. Der Feststoff wurde abfiltriert, das Filtrat mit dem gleichem Volumen Essigester versetzt und mit kalter 5 %iger NaHSO₄-Lösung sowie gesättigter NaCl-Lösung gewaschen. Man erhielt 11,5 g (65 %) Produkt.
   ¹H-NMR (DMSO-d₆; δ in ppm): 8,38 (m, NH); 7,50 - 7,35 (m, 3H); 4,40 - 4,05 (m, 3H, N-CH₂-Ar/N-CH-CO); 3,87 (s, OCH₃); 3,50-3,25 (m, 2H, N-CH₂); 2,2,5 - 2,00 (m, 1H); 1,90 - 1,65 (m, 3H); 1,40 und 1,30 (2s; 9H)
b) H-Pro-(4-cyano-2-methoxy)-benzylamid:
   11,4 g (31,7 mMol) Boc-Prolin-(2-methoxy-4-cyano)-benzylamid wurden in 130 ml DCM gelöst und bei 0 - 5°C mit HCl-Gas gesättigt. Nach 2 h war die Boc-Gruppe vollständig abgespalten.
   Das Lösungsmittel wurde im Vakuum entfernt und das Produkt ohne weitere Reinigung in den Folgereaktionen eingesetzt.
   ¹H-NMR (DMSO-d₆, δ in ppm): 10,25 (s, 1H); 8,60 (s, 1H); 7,50 (d, 1H); 7,42 (dd, 1H); 7,39 (d, 1H); 4,40 - 4,20 (m, 3H); 3,88 (s, 3H); 3,20 (m, 2H); 2,35 (m, 1H); 2,00 - 1,80 (m, 3H)
c) Boc-(D)-Phe-Pro-(4-cyano-2-methoxy)-benzylamid:
   3,54 g (13,35 mMol) Boc-(D)-Phe-Pro-OH, 9,9 ml DIPEA und 4,80 g (13,35 mMol) H-Pro-(4-cyano-2-methoxy)-benzylamid Hydrochlorid wurden bei -5°C mit 11,1 ml (15,0 mMol) PPA (50 %ig in Essigester) in 100 ml DCM vereinigt und 2 h bei 0°C gerührt. Das Reaktionsgemisch wurde nacheinander mit 1N NaOH, 1N HCl und gesättigter Kochsalzlösung gewaschen und über Na₂SO₄ getrocknet. Nach Abziehen des Lösungsmittels erhielt man 6,5 g (96 %) des Produkts.
   ¹H-NMR (DMSO-d₆, δ in ppm): 8,75/7,88 (1H, NH (2 Rotamere)), 7,5 - 7,1 (9H, Aromaten-H und NH), 4,4 - 4,1 (4H, CH₂ und 2 x CH), 3,85 (3H, OCH₃), 3,7 - 3,4 (2H, CH₂), 3,0 - 2,7 (2H, CH₂), 2,3 - 1,5 (4H, 2 x CH₂), 1,3 - 1,1 (9H, Boc)
d) Boc-(D)-Phe-Pro-(4-amidino-2-methoxy)-benzylamid:
   Das Nitril aus der vorhergehenden Stufe wurde analog A.III.1. zu 4,6 g Amidin-Hydrojodid umgesetzt.
   ¹H-NMR (DMSO-d₆, δ in ppm): 9,25/8,85 (4H, Amidin), 8,75/7,95 (1H, NH (2 Rotamere)), 7,4 - 7,1 (9H, Aromaten-H und NH), 4,45 - 4,10 (4H, CH₂ und 2 x CH), 3,90 (3H, OCH₃), 3.65-ca. 3,4 (2H, CH₂), 3,0 - 2,7 (2H, CH₂), 1,95 - 1,55 (4H, 2 x CH₂, 1,3 - 1,2 (9H, Boc)

### Beispiel 240:

### H-(D)-Phe-Pro-NH-(2-MeO)-pAmb:

Das Amidin-Hydrojodid (Beispiel 239) wurde über einen Acetationenaustauscher (IRA 420) zum Amidin-Hydroacetat umgesetzt, dann in 50 ml DCM gelöst und bei 0°C mit HCl-Gas gesättigt. Nach 1 h wurde das Lösungsmittel abgezogen. Man erhielt 3,0 g des Amidins als Dihydrochlorid.
¹H-NMR (DMSO-d₆, δ in ppm): 9,50/9,27 (4H, Amidin), 8,80 (3H, NH₃⁺), 8,75 (1H, NH), 7,50 - 7,20 (8H, Aromaten-H), 4,35 - 4,10 (4H, CH₂ und 2 x CH), 3,90 (3H, OCH₃), ca. 1,9 - 1,35 (4H, 2 x CH₂)

### Beispiel 241:

### Boc-(D)-Phe(4-MeO)-Pro-NH-(2-MeO)-pAmb:

a) Boc-(D)-Phe(4-MeO)-Pro-(4-cyano-2-methoxy)-benzylamid:
   1,55 g (5,25 mMol) Boc-(D)-Phe(4-OMe)-OH, 3,9 ml DIPEA und 1,55 g (5,25 mMol) Prolin-(2-methoxy-4-cyano)-benzylamid-hydrochlorid wurden bei -5°C mit 4,4 ml (5,9 mMol) PPA (50 %ig in Essigester) in 35 ml DCM vereinigt und 1 h bei 0°C gerührt. Das Reaktionsgemisch wurde nacheinander mit 1N NaOH, 1N HCl und gesättigter Kochsalzlösung gewaschen und über Na₂SO₄ getrocknet. Nach Abziehen des Lösungsmittels blieben 2,4 g Feststoff zurück.
   ¹H-NMR (DMSO-d₆, δ in ppm): 8,72 und 7,87 (t, 2H); 7,42 (1H); 7,35 (m, 3H); 7,15 (d, 2H); 6,85 (d, 2H); 7,00/6,70 (2d, 1H (2 Rotamere)) 1H; 4,40 - 4,10 (m, 4H); 3,85 (s, 3H); 3,70 (s, 3H); 3,05 - 2,55 (m, 2H); 1,95 - 1,55 (m, 4H); 1,2 (s, 9H)
b) Boc-(D)-Phe(4-MeO)-Pro-NH-(2-MeO)-pAmb:
   2,4 g des Nitrils (Beispiel 241/a) wurden analog A.III.1. nach säulenchromatographischer Reinigung über Kieselgel (Laufmittel: DCM/MeOH 9:1) zu 1,7 g des Amidin-Hydrojodids umgesetzt.
   ¹H-NMR (DMSO-d₆, δ in ppm): 9,25/8,85 (4H, Amidin), 7,95 (1H, NH), 7,4 - 6,8 (8H, Aromaten-H und NH), 4,4 - 4,1 (4H, CH₂ und 2 x CH), 3,90/3,70 (6H, 2 x OCH₃), ca. 3,7 - 2,9 (2H, CH₂), 3,0 - 2,6 (2H, CH₂), 1,9 - 1,5 (4H, 2 x CH₂), 1,3 - 1,2 (9H, Boc)

### Beispiel 242:

### H-(D)-Phe(4-MeO)-Pro-NH-(2-MeO)-pAmb:

1,7 g des Amidin-Hydrojodids (Beispiel 241) wurde über einen Acetationenaustauscher (IRA 420) ins Acetatsalz umgewandelt und dann die Boc-Gruppe analog Beispiel 240 abgespalten. Man erhielt 1,0 g des Dihydrochlorids.
¹H-NMR (DMSO-d₆, δ in ppm): 9,50/0,25 (4H, Amidin), 8m85 - 8,65 (4H, NH und NH₂⁺), 7,50 - 7,30 und 7,15/6,90 (7H, Aromaten-H), 4,28 - 4,05 (4H, CH₂ und 2 x CH), 3,90/3,75 (6H, 2 x OCH₃), 3,20-2,85 (2H, CH₂), 1,95 - 1,40 (4H, 2 x CH₂);
FAB-MS: 454 (M+H⁺)

### Beispiel 243:

### HOOC-CH₂-(D)-Phe(4-MeO)-Pro-NH-(2-MeO)-pAmb:

Die Boc-Gruppe der Verbindung aus Beispiel 241 a) wurde analog Beispiel 240 gespalten. 3,5 g dieses Spaltprodukts wurden in 80 ml DCM gelöst und zusammen mit 4,45 ml DIPEA und 1,09 ml Bromessigsäure-tert-butylester bei Raumtemperatur 3 Tage gerührt. Das Produkt wurde wie in Beispiel 246 a) aufgearbeitet. 3,0 g der entstandenen Verbindung tBuOOC-CH₂-(D)-Phe(4-MeO)-Pro-(2-methoxy-4-cyano)-benzylamid wurden analog Beispiel 246 b) mit Hydroxylaminhydrochlorid umgesetzt und 3,1 g des entstandenen Hydroxyamidins mit 185 mg Raney-Nickel in 65 ml Methanol, dem 0,31 ml Eisessig zugesetzt war, bei 50°C zum Amidin-Hydroacetat hydriert. Der Katalysator wurde abfiltriert und das tBuOOC-CH₂-(D)-Phe(4-MeO)-Pro-(2-MeO)-pAmb Hydroacetat über Kieselgel säulenchromatographisch gereinigt (Laufmittel: DCM + 10 % Methanol + 2 % (50 %ige) Essigsaure). Es wurden 1,3 g des tert.-Butylesters erhalten (FAB-MS: 568 (M+H⁺)) und 1,15 g davon analog Beispiel 246 d) zu 850 mg HOOC-CH₂-(D)-Phe(4-MeO)-Pro-NH-(2-MeO)-pAmb umgesetzt.
¹H-NMR (DMSO-d₆, δ in ppm): 9,9 - 9,7 und 9,2 - 9,0 (2H, NH₂⁺), 9,60/9,35 (4H, Amidin), 7,50 - 6,73 (5H, Aromaten-H), 4,50 - 3,45 (8H, 3 x CH₂ und 2 x CH), 3,90 (3H, OCH₃), 3,73 (3H, OCH₃), 3,40 - 3,27 und 3,06 - 2,87 (2H, CH₂), 2,43 - 1,25 (4H, 2 x CH₂)

### Beispiel 244:

### Boc-(D)-Chg-Pro-NH-(2-MeO)-pAmb:

a) Boc-(D)-Cyclohexylglycyl-prolin-(2-methoxy-4-cyano)-benzylamid:
   Zu 10,0 g (28,2 mMol) Boc-(D)-Chg-Pro-OH in 70 ml absolutem Dichlormethan wurden bei -5°C 20,8 ml DIPEA (121 mMol), 4,58 g (28,2 mMol) 2-Methoxy-4-cyano-benzylamin und 25 ml PPA (50 %ige Lösung in Essigester) gegeben und 2 h bei 0°C gerührt.
   Die Lösung wurde anschließend nacheinander mit 0,5 N Natronlauge, iN HCl und gesättigte Kochsalzlösung gewaschen, mit Na₂SO₄ getrocknet und das Lösungsmittel vollständig im Vakuum abgezogen. Das in nahezu quantitativer Ausbeute angefallene Produkt wurde ohne weitere Reinigung in den Folgeschritten umgesetzt.
   ¹H-NMR (DMSO-d₆, δ in ppm): 7,9 (1H, NH), 7,45 und 7,35 (3H, Aromaten-H), 7,1 (1H, NH), 4,45 - 3,50 (6H, 2 x CH₂ und 2 x CH), 3,86 (3H, OCH₃), 2,2 - 1,0 (24H, Cyclohexyl + 2 x CH₂ + Boc)
b) Boc-(D)-Cyclohexylglycyl-prolin-(2-methoxy-4-hydroxyamidino)-benzylamid:
   12,0 g (24 mMol) des Cyano-Edukts (a) wurden analog Beispiel 246 b) mit Hydroxylaminhydrochlorid umgesetzt. Das Produkt fiel als voluminöser Niederschlag nahezu quantitativ aus.
   ¹H-NMR (DMSO-d₆, δ in ppm): 9,7 - 9,5 (1H, OH), 5,8 (2H, NH₂)
c) Boc-(D)-Cyclohexylglycyl-prolin-(2-methoxy-4-amidino)-benzylamid:
   Die Hydroxyamidino-Verbindung (b) wurde analog Beispiel 243 mit Raney-Nickel hydriert. Das Produkt wurde säulenchromatographisch über Kieselgel (Laufmittel: Dichlormethan/10 % - 20 % MeOH/2 % (50 %ige) Essigsaure) gereinigt. Man erhielt 10,5 g des Amidins als Acetat-Salz (Ausbeute: 75 % - ausgehend vom Nitril (a));
   ¹H-NMR (DMSO-d₆, δ in ppm): die Amidinogruppe zeigt als Acetat-Salz ein extrem breites Signal von ca. 10 - 8 ppm; 7,95 (1H, NH), 7,4 - 7,3 (3H, Aromaten-H), 7,05 (1H, NH), 4,4 - 3,4 (6H, 2 x CH₂ und 2 x CH), 3,89 (3H, OCH₃), 2,2 - 1,0 (24H, Cyclohexyl + 2 x CH₂ + Boc)

### Beispiel 245:

### H-(D)-Chg-Pro-NH-(2-MeO)-pAmb:

10,5 g des Boc-(D)-Chg-Pro-(2-methoxy-4-amidino)-benzylamids wurden in 200 ml/10 ml absolutem.Dichlormethaa/MeOH gelöst und bei 0 ∼ 5°C 1 h mit HCl begast. Man rührte eine weitere Stunde bei 0°C, zog das Lösungsmittel vollständig im Vakuum ab und erhielt 7,6 g (86 %) des Produkts als Dihydrochlorid.
¹H-NMR (DMSO-d₆, δ in ppm):9,60 und 9,33 (4H, Amidin), 8,87 (1H, NH), 8,62 (3H, NH₃⁺), 7,5 - 7,3 (3H, Aromaten-H), 4,45 - 4,15 (4H, CH₂ und 2 x CH), 3,95 (3H, OCH₃), 3,95 - 3,82 (1H, CH₂), 3,65 - 3,55 (1H, CH₂), 2,2 - 1,0 (15H, Cyclohexyl und 2 x CH₂)

### Beispiel 246:

### MOOC-CH₂-(D)-Chg-Pro-NH-(2-MeO)-pAmb:

(a) N-tert.-Butyloxycarbonylmethyl-(D)-cyclohexylglycyl-prolin-(2-methoxy-4-cyano)-benzylamid:
   0,72 g (1,65 mMol) H-(D)-Chg-Pro-(2-methoxy-4-cyano)-benzylamid Hydrochlorid wurden in 30 ml absolutem Dichlormethan vorgelegt. Nach Zugabe von 1 ml (5,8 mMol) DIPEA wurde bei Raumtemperatur in 40 min eine Lösung aus 1,65 mMol Bromessigsäure-tert.-butylester und 15 ml Dichlormethan hinzugetropft. Die Reaktionsmischung wurde 3 Tage bei Raumtemperatur geruhrt, dann nacheinander mit 0,5 N Natronlauge, 0,5 NHCl und gesättigter Kochsalzlösung gewaschen. Nach dem Trocknen erhielt man 0,7 g des Rohprodukts, welches ohne weitere Reinigung in den Folgeschritten eingesetzt wurde.
(b) N-tert.-Hutyloxycarbonylmethyl-(D)-cyclohexylglycyl-prolin-(2-methoxy-4-hydroxyamidino)-benzylamid:
   1,45 g (2,8 mMol) des 2-Methoxy-4-cyano-benzylamids (a) wurden in 20 ml Dichlormethan/MeOH 1:1 gelöst und zusammen mit 0,49 g (7,1 mMol) Hydroxylaminhydrochlorid und 2,8ml DIPEA 20 h bei Raumtemperatur gerührt. Nach Abziehen des Lösungsmittels wurde das Produkt in Dichlormethan aufgenommen, mit Wasser und gesättigter Kochsalzlösung gewaschen und getrocknet. Man erhielt 1,2 Rohprodukt, welches direkt weiter eingesetzt wurde.
(c) N-tert.-Butyloxycarbonylmethyl-(D)-cyclohexylglycylprolin-(2-methoxy-4-amidino)-benzylamid:
   Die Hydroxyamidino-Verbindung (b) wurde analog Beispiel 243 mit Raney-Nickel hydriert. Man erhielt nach säulenchromatographischer Reinigung über Kieselgel (Laufmittel: Dichlormethan/10 % - 20 % MeOH/2 % (50 %ige) Essigsäure) 0,5 g Produkt als Acetat-Salz.
(d) N-Hydroxycarbonylmethyl-(D)-cyclohexylglycyl-prolin-(2-methoxy-4-amidino)-benzylamid:
   0,5 g (0,85 mMol) des Amidino-Acetat-Salzes (c) wurden in 25 ml absolutem Dichlormethan gelöst. Bei 0 - 5°C leitete man bis zur Sättigung des Losungsmittels HCl-Gas ein. Nach 40 min wurde eine weitere Stunde bei Raumtemperatur gerührt. Nach Abziehen des Lösungsmittels im Vakuum erhielt man 370 mg sauberes Produkt als Dihydrochlorid.
   ¹H-NMR (DMSO-d₆, δ in ppm): 9,57 und 9,33 (4H, Amidin); 9,8 - 9,1 (2H, NH₂⁺), 7,6 - 7,3 (3H, Aromaten-H); 4,5 - 4,1 (4H, 1 x CH₂ und 2 x CH); 3,93 (3H, OCH₃), 3,9 - 3,4 (4H, 2 x CH₂), 2,3 - 1,0 (15H, Cyclohexyl und 2 x CH₂)

### Beispiel 247:

### Boc-(D)-Chg-Aze-NH-(2-MeO)-pAmb:

(a) Boc-(L)-Azetidin-2-carbonsäure-(2-methoxy-4-cyano)-benzylamid:
   Zu 2,12 g Hoc-(L)-Azetidin-2-carbonsaure (10,5 mMol) in 50 ml THF wurden bei 0 - 5°C 1,22 g (10,5 mMol) Hydroxysuccinimid und 2,18 g (10,5 mMol) DCC gegeben und 30 min gerührt. Dann gab man bei 0 - 5°C 2,10 g (10,5 mMol) 2-Methoxy-4-cyano-benzylamin Hydrochlorid und zuletzt 1,48 ml Et₃N hinzu. Die Reaktionsmischung wurde über Nacht bei Raumtemperatur gerührt. Der ausgefallene Harnstoff wurde über eine Filternutsche abgetrennt, das Filtrat in Essigester aufgenommen und nacheinander mit 0,5 N HCl, 0,5 N Natronlauge und gesättigter KochSalzlösung gewaschen. Das Lösungsmittel wurde über Na₂SO₄ getrocknet und dann vollstandig unter Vakuum abgezogen. Man erhielt 3,1 g (85 %) Produkt, welches ohne weitere Reinigung eingesetzt wurde.
   ¹H-NMR (DMSO-d₆, δ in ppm): 8,5 (1H, NH); 7,48 und 7,40 - 7,25 (3H, Aromaten-H); 4,55 (dd, 1H, CH); 4,45 - 4,15 (2H, CH₂), 3,88 (3H, OCH₃), 3,9 - 3,7 (2H, CH₂), 2,5 - 2,3 (1H, CH₂); 2,15 - 1,95 (1H, CH₂); 1,35 (9H, Boc)
(b) (L)-Azetidin-2-carbonsäure-(2-methoxy-4-cyano)-benzylamid:
   3,0 g (8,7 mMol) Boc-Aze-(2-methoxy-4-cyano)-benzylamid wurden analog Beispiel 239 (b) in nahezu quantitativer Ausbeute zum gewünschten Produkt (b) umgesetzt.
   ¹H-NMR (DMSO-d₆, δ in ppm): 10,0 - 9,85 (1H, NH₂⁺), 7,50 und 7,45 - 7,35 (3H, Aromaten-H); 5,10 - 4,95 (1H, CH); 4,35 (d, 2H, CH₂); 4,05 - 3,65 (2H, CH₂); 3,89 (3H, OCH₃); 2,8 - 2,6 (1H, CH₂); 2,5 - 2,3 (1H, CH₂)
(c) Boc-(D)-Cyclohexylglycyl-(L)-azetidin-2-carbonsäure-(2-methoxy-4-cyano)-benzylamid:
   2,2 g Boc-(D)-Chg-OH wurden analog Beispiel 241 (a) mit 2,4 g H-Aze-(2-methoxy-4-cyano-)-benzylamidhydrochlorid umgesetzt. Man erhielt 3,5 g
(d) Boc-(D)-Cyclohexylglycyl-(L)-azetetin-2-carbonsäure-(2-methoxy-4-amidino)-benzylamid:
   3,4 g des Nitrils (c) wurden analog Beispiel 246(b) mit Hydroxylaminhydrochlorid umgesetzt und das entstandene Hydroxyamidin mit Raney-Nickel analog Beispiel 243 hydriert. Man erhielt 3,1 g des Amidins als Hydroacetat.

### Beispiel 248:

### H-(D)-Chg-Aze-NH-(2-MeO)-pAmb:

3,1 g der Boc-Verbindung (Beispiel 247) wurden analog Beispiel 245 zum Dihydrochlorid gespalten.
¹H-NMR (DMSO-d₅, δ in ppm): 9,45/9,20 (4H, Amidin); 9,0 (1H, NH); 8,55 (3H, NH₃⁻); 7,45/7,40 (3H, Aromaten-H); 4,75 - 4,10 (4H, CH₂ und 2 x CH); 3,90 (3H, OCH₃), 2,7 - 1,0 (13 H, Cyclohexyl und CH₂)

### Beispiel 249:

### Boc-(D)-Chg-Pro-NH-(2-iPrO)-pAmb:

4,1 g (11,5 mMol) Boc-(D)-Chg-Pro-OH wurden analog Beispiel 239 (a) mit je einem Äquivalent Hydroxysuccinimid, DCC, 4-Aminomethyl-3-isopropoxy-benzonitril Hydrochlorid und Et₃N umgesetzt. Man erhielt 5,7 g (94 %) Rohprodukt, welches ohne weitere Reinigung in den Folgeschritten eingesetzt wurde.
¹H-NMR (DMSO-d₆, δ in ppm): 7,85 (1H, NH); 7,43 und 7,30 (3H, Aromaten-H); 7,08 (1H, NH); 4,80 - 3,50 (7H, 2 x CH₂, 3 x CH); 2.2 - 1,0 (30H, Boc + Cyclohexyl ⁺ 2 x CH₃ + 2 x CH₂)

### Beispiel 250:

### H-(D)-CHg-Pro-NH-(2-iPro)-pAmb:

5,7 g der Boc-Verbindung (Beispiel 249) wurden analog Beispiel 246 (b) mit Hydroxylaminhydrochlorid umgesetzt und das entstandene Hydroxyamidin analog Beispiel 243 mit Raney-Nickel hydriert. Das dabei entstandene Amidin-Hydroacetat wurde analog Beispiel 245 gespalten. Man erhielt 3,1 g des Produkts als Dihydrochlorid.
¹H-NMR (DMSO-d₆, δ in ppm): 9,40/9,15 (4H, Amidin); 8,75 (1H, NH); 8,55 (3H, NH₃⁺); 7,40 - 7,25 (3H, Aromaten-H); 4,80 (1H, CH); 4,4 - 3,5 (6H, 2 x CH₂ und 2 x CH); 2,3 - 1,0 (15H, Cyclohexyl und 2 x CH₂), 1,3 (6H, 2 x CH₃)

### Beispiel 251:

### Boc-(D)-Chg-Pro-NH-(2-Cl)-pAmb:

(a) Boc-Prolin-(2-chlor-4-cyano)-benzylamid:
   5,4 g (24 mMol) Boc-Pro-OH wurden analog Beispiel 244 (a) mit 20 ml PPA, 17,9 ml DIPEA und 4,0 g (24 mMol) 2-Chlor-4-cyanobenzylamin zum Boc-Pro-(2-chlor-4-cyano)- benzylamid umgesetzt. Man erhielt 7,0 g (80 %) Produkt.
   ¹H-NMR (DMSO-d₆, δ in ppm): 8,57 (1H, NH); 8,05 - 7,45 (3H, Aromaten-H); 4,50 - 4,10 (3H, CH₂ und CH); 3,4 - 3,2 (2H, CH₂); 2,25 - 1,70 (4H, 2 x CH₂); 1,4 - 1,3 (9H, Boc)
(b) Prolin-(2-chlor-4-cyano)-benzylamid-hydrochlorid:
   Die Boc-Gruppe wurde analog Beispiel 239 (b) abgespalten. Man erhielt 5,6 g (97 %) des Hydrochlorids.
   ¹H-NMR (DMSO-d₆, δ in ppm): 10,2 und 8,6 (NH₂⁺), 9,45 (1H, NH); 8,05 - 7,50 (3H, Aromaten-H); 4,45 (d, 2H, CH₂); 4,28 (1H, CH); 3,20 (2H, CH₂), 2,40 - 1,80 (4H, 2 x CH₂)
(c) Boc-(D)-Cyclohexylglycyl-prolin-(2-chlor-4-cyano)-benzylamid:
   4,76 g (18,7 mMol) Boc-(D)-Cyclohexylglycin wurden analog Beispiel 241 (a) mit 15,5 ml PPA, 14 ml DIPEA und 5,6 g (18,7 mMol) des Hydrochlorids (b) zum Boc-(D)-Chg-Pro-(2-chlor-4-cyano)-benzylamid umgesetzt. Man erhielt 8,7 g (92 %) Produkt.
(d) Boc-(D)-Cyclohexylglycyl-prolin-(2-chlor-4-hydroxyamidino)-benzylamid:
   Die Cyano-Gruppe der Substanz (c) wurde analog Beispiel 246 (b) mit Hydroxylamin in nahezu quantitativer Ausbeute zum Boc-(D)-Chg-Pra-(2-chlor-4-hydroxyamidin)-benzylamid umgesetzt Man erhielt 4,6 g Produkt.
   ¹H-NMR (DMSO-d₆, δ in ppm): 9,75 (1H, OH); 5,90 (2H, NH₂)
(e) Boc-(D)-Cyclohexyl-prolin-(2-chlor-4-amidino)-benzylamid:
   4,6 g des Hydroxyamidins (d) wurden analog Beispiel 243 mit Raney-Nickel zum Amidin hydriert. Nach säulenchromatographischer Reinigung über Kieselgel (Laufmittel: Dichlormethan/15 % MeOH/2 % (50 %ige) Essigsäure) erhielt man 5,4 g des Amidins als Acetat-Salz.
   ¹H-NMR (DMSO-d₆, ¹H-NMR (DMSO-d₆, δ in ppm): das Signal des Amidins als Acetat-Salz ließ sich wegen seiner Breite nicht lokalisieren; 8,15 (1H, NH), 7,9 - 7,5 (3H, Aromaten-H), 7,05 (1H, NH), 4,5 - 3,4 (6H, 2 x CH₂ und 2 x CH), 2,2 - 1,0 (24, Cyclohexyl + 2 x CH₂ + Boc)

### Beispiel 252:

### H-(D)-Chg-Pro-NH-(2-Cl)-pAmb:

Die Boc-Gruppe des Beispiels 251 wurde analog Beispiel 245 abgespalten. Man erhielt 3,0 g (65 %) des Produkts als Dihydrochlorid.
¹H-NMR (DMSO-d₆, δ in ppm): 9,55 und 9,34 (4H, Amidin), 9,05 (1H, NH), 8,60 (3H, NH₃⁺), 7,95 - 7,48 (3H, Aromaten-H), 4,5 - 3,5 (6H, 2 x CH₂, 2 x CH), 2,25 - 1,0 (15H, Cyclohexyl + 2 x CH₂)

### Beispiel 253:

### H-(D)-Phe-Pro-(D,L)(4-Am)-PhgOMe:

Die Verbindung wurde durch Cbz-Abspaltung aus Beispiel 18 hergestellt.
¹H-NMR (d₆-DMSO, δ in ppm): 9/9,2/8,85/8,8 (4d, 1H, NH); 7,8 (m, 2H, Ar-H); 7,6 (m, 2H, Ar-H); 7,3 - 7,0 (m 5H, Ar-H); 5,7/5,6 (2d, 1H, α-H); 4,8/4,4 (2dd, 1H, α-Phe); 3,9 (m 1H, α-Pro); 3,75 (2s, 3H, OCH3); 3,6 (2H, δ-Pro); 3,0 - 2,6 (m, 2H, CH2-Ph); 2,2 - 1,6 (m, 4H, β/γ-Pro)
MS: 452 (M+H⁺), 305, 192; Fp: 71 - 73°C (Dihydroacetat)

### Beispiel 256:

### H-(D)Chg-Pro-NH-3-(2-MeO-6-Am)-pico:

a) 2-Methoxy-3-picolylalkohol·HCl:
   20,0 g 2-Methoxynicotinsäure (130,59 mMol) wurden zusammen mit 28,7 ml N-Methylmorpholin (261,18 mMol) in THF bei -10°C vorgelegt und 25,4 ml Chlorameisensäure-isobutylester (195,89 mMol) in 50 ml THF rasch zugetropft, wobei ein Niederschlag ausfiel. Nach einstündigem Rühren bei 0°C wurden 16,3 g Natriumborhydrid (430,95 mMol) portionsweise zugegeben und anschließend 250 ml Methanol langsam zugetropft (starke Gasentwicklung und exotherme Reaktion). Nach Absaugen des ausgefallenen Salzes wurde das Filtrat im Vakuum einrotiert, der Rückstand in Essigester aufgenommen, mit Wasser, verd. Salzsäure (pH= 2), gesättigter Kochsalzlösung gewaschen (Wasserphasen wurden sehr klein gehalten), über Magnesiumsulfat getrocknet und einrotiert. Der Rückstand wurde in Ether aufgenommen, mit etherischer Salzsaure versetzt, der Niederschlag abgesaugt und mit Ether ausgerührt. Es wurden 16,3 g (71 %) 2-Methoxy-3-picolylalkohol-hydrochlorid als weißes Salz erhalten.
b) 2-Methoxy-3-picolyl-chlorid·HCl:
   Zu 17 g 2-Methoxy-3-picolylalkohol·HCl (96,76 mMol) suspendiert in 60 ml DCM wurden 51 ml Thionylchlorid (696,67 mMol) getropft, die Lösung 1 h bei Raumtemperatur gerührt, das Lösungsmittel und überschüssiges Thionylchlorid im Vakuum entfernt, der Rückstand 4 mal mit Methanol im Vakuum kodestilliert und anschließend aus Ether ausgerührt. Es wurden 15,2 g 2-Methoxy-3-picolylchloridhydrochlorid (81 %) als weißes kristallines Salz erhalten.
c) 2-Methoxy-3-picolylamin·2HCl:
   15,1 g 2-Methoxy-3-picolylchloridhydrochlorid (77,76 mMol) wurden langsam zu einer Mischung von 600 ml 30 %iger Ammoniaklosung in wasser und 250 ml Methanol bei 35°C getropft, wobei ständig gasförmiges Ammoniak eingeleitet wurde.
   Nach 1 h Rühren bei 35°C wurde die Lösung im Vakuum einrotiert, der Rückstand mit 20 %iger NaOH-Lösung alkalisiert (Wasserphasen wurden klein gehalten), mehrfach mit DCM extrahiert, die org. Phasen über Magnesiumsulfat getrocknet und im Vakuum einrotiert. Nach Aufnahme des Rückstandes in Ether Zugabe von etherischer Salzsäure, Absaugen und Waschen des Niederschlags mit Ether wurden 11,0 g (67 %) 2-Methoxy-3-picolylamin·2HCl als weißes Kristallisat erhalten.
d) Boc-(D)Chg-Pro-NH-3-(2-MeO)-pico:
   Die Darstellung erfolgte in Analogie zu Boc-(D)Chg-Pro-NH-3-(2-Me)-pico (s. Beispiel 227) Ausbeute 92 %.
e) Boc-(D)Chg-Pro-NH-3-(2-MeO-1-oxo)-pico:
   4.9 g Boc-(D)Chg-Pro-NH-(2-MeO)-pico (10,32 mMOl) wurden in 100 ml DCM gelöst, mit 3,6 g meta-Chlorperbenzoesäure (20,64 mMol) versetzt und mehrere Tage bei Raumtemperatur gerührt (laut DC nur Teilumsatz). Die Lösung wurde anschließend mit DCM verdünnt, über Magnesiumsulfat getrocknet, mit gasförmigen Ammoniak gesättigt, der ausgefallene Niederschlag abfiltriert und das Filtrat im Vakuum eingeengt. Das Produktgemisch wurde in Ether aufgenommen, mit 1 M Kaliumhydrogensulfatlösung extrahiert (pH 2), die wäßrige Phase mit Kaliumcarbonat alkalisiert, mehrfach mit DCM ausgeschüttelt, über Magnesiumsulfat getrocknet und im Vakuum einrotiert. Man erhielt 1,6 g Boc-(D)Chg-Pro-NH-3-(2-MeO)-1-oxo)-pico als festen Schaum. 3,1 g Edukt konnten aus dem sauren Etherextrakt zurückgewonnen und erneut in die N-oxid-Darstellung eingesetzt werden. Durch mehrfaches Wiederholen wurden insgesamt 4,2 g Produkt gewonnen.
f) Boc-(D)Chg-Pro-NH-3-(2-MeO-6-CN)-pico:
   4,2 g Boc-(D)Chg-Pro-NH-3-(2-MeO-6-CN)-pico (8,56 mMol) wurden zusammen mit 16 ml Trimethylsilylcyanid, 5 ml 1,2-Dichlorethan und 10 ml Dimethylcarbaminsäurechlorid versetzt, sofort auf 70°C erwärmt und 10 min. bei dieser Temperatur gerührt. Nach Einengen im Vakuum wurde das Produktgemisch (2 Hauptkomponenten, DC: Rf= 0,46 bzw. 0,26, Fließmittel DCM/MeOH= 95/5) säulenchromatographisch über Kieselgel (Eluent: DCM mit 0,5 auf 1,5 % MeOH ansteigend) getrennt.
   Die erste Hauptfraktion enthielt 1,17 g Boc-(D)Chg-Pro-NH-3-(2-MeO-6-CN)-pico.
g) Darstellung von Boc-(D)Chg-Pro-NH-3-(2-MeO-6-Ham)-pico:
   1,17 g Boc-(D)Chg-Pro-NH-3-(2-MeO-6-CN)-pico (2,34 mMol) wurden zusammen mit 0,41 g Hydroxylammoniumchlorid und 2 ml DIPEA (11,7 mMol) in 10 ml DCM 4 h bei Raumtemperatur gerührt, anschließend im Vakuum einrotiert, der Rückstand in Essigester aufgenommen, mehrfach mit verd. Salzsäure (pH 4) extrahiert, die org. Phase über Magnesiumsulfat getrocknet und im Vakuum einrotiert. Das erhaltene Produktgemisch (2 Komponenten, DC: Rf= 0,54 bzw. 0,42, Fließmittel DCM/MeOH= 9/1) wurde säulenchromatographisch über Kieselgel (Eluent: DCM mit 0,5 auf 1,5 % MeOH ansteigend) getrennt. Die erste Hauptfraktion enthielt 300 mg Boc-(D)Chg-Pro-NH-3-(2-MeO-6-Ham)-Pico.
   ¹³C-NMR (d₆-DMSO, δ in ppm) : 171,16, 170,46, 159,13, 155,69, 148,89, 145,44, 135,84, 120,76, 111,53, 77,80, 59,55, 56,66, 52,84, 46,56, 38,4, 36,32, 28,87, 28,38, 28,17, 27,72, 27,57, 25,37, 25,29, 25,09, 23,65.
h) Boc-(D)Chg-Pro-NH-3-(2-MeO-6-Am)-pico:
   300 mg Boc-(D)Chg-Pro-NH-3-(2-MeO-6-Ham)-pico (0,56 mMol) wurden in 10 ml Ethanol und 2 ml Essigsäure über Pd/C (10 %) bei 60°C 4 h hydriert. Nach Abfiltrieren des Katalysators und Einkonzentrieren der Reaktionslösung im Vakuum erhielt man 260 mg Boc-(D)Chg-Pro-NH-3-(2-MeO-6-Am)-pico-Rohprodukt, das ohne weitere Reinigung in der nachfolgenden Umsetzung eingesetzt wurde.
i) H-(D)Chg-Pro-NH-3-(2-MeO-6-Am)-pico:
   260 mg Boc-(D)Chg-Pro-NH-3-(2-MeO-6-Am)-pico Rohprodukt wurden in 5 ml DCM und 5 ml Methanol zusammen mit 5 ml etherischer Salzsäure über Nächt bei Räumtemperatur gerührt, die Reaktionsmischung im Vakuum einkonzentriert, der Rückstand mehrfach mit Toluol/Methanol kodestilliert und anschließend aus Ether ausgerührt. Man erhielt 210 mg Boc-(D)Chg-Pro-NH-3-(2-MeO-6-Am)-pico·(HCl)_{1,2} als weiße kristalline Festsubstanz. Fp: 205-212°C
   FAB-MS: (M+H)⁺= 417

## Patentansprüche

1. Verbindungen der Formel 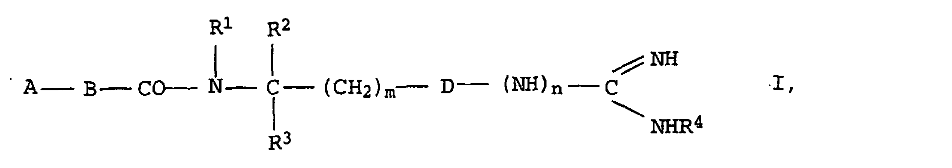 sowie deren Salze mit physiologisch verträglichen Säuren und deren Stereoisomeren, worin die Substituenten folgende Bedeutungen besitzen:
A: 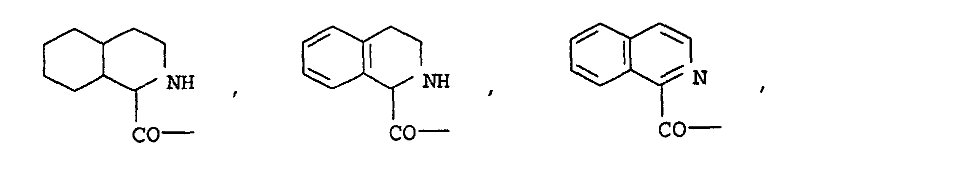 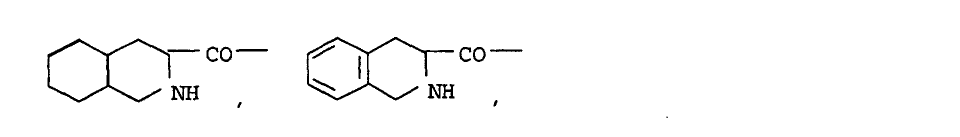 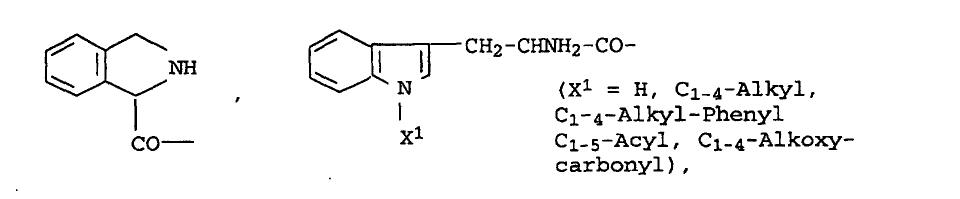 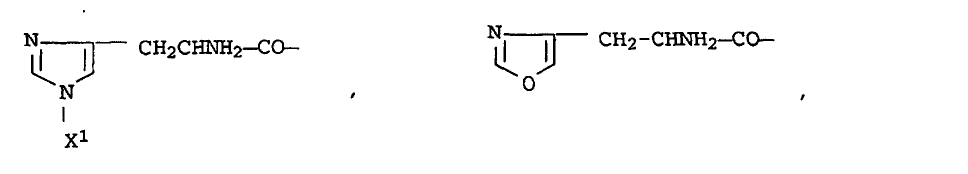 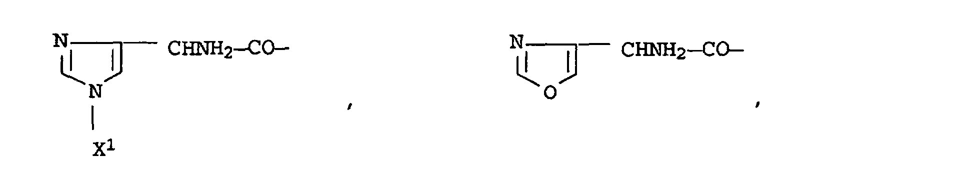 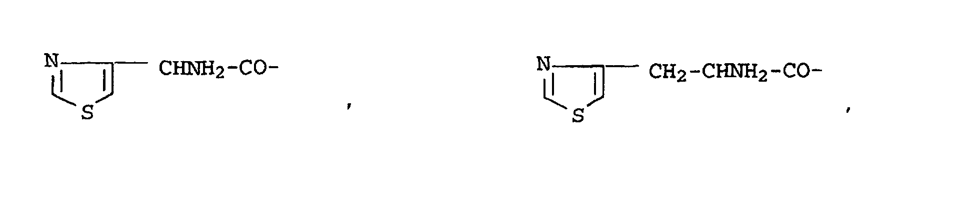 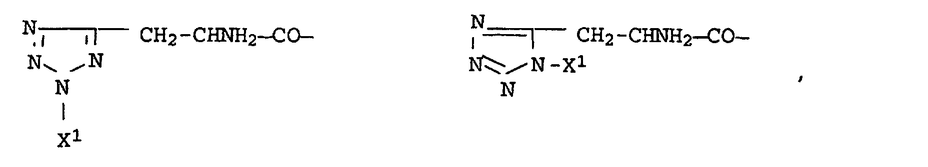 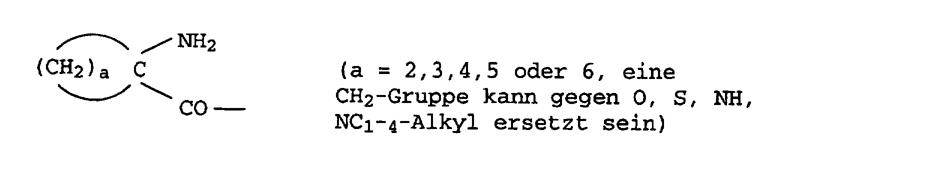 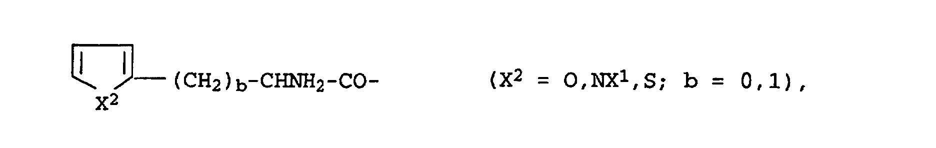 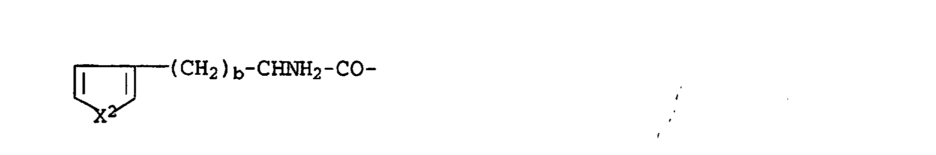 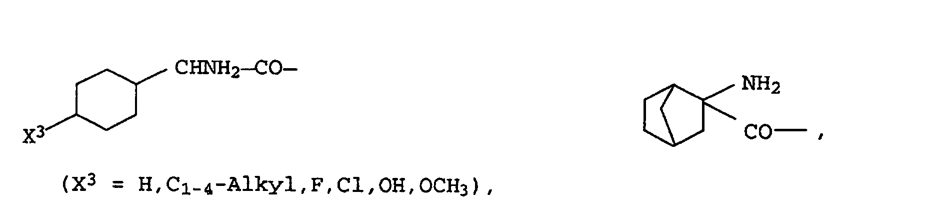 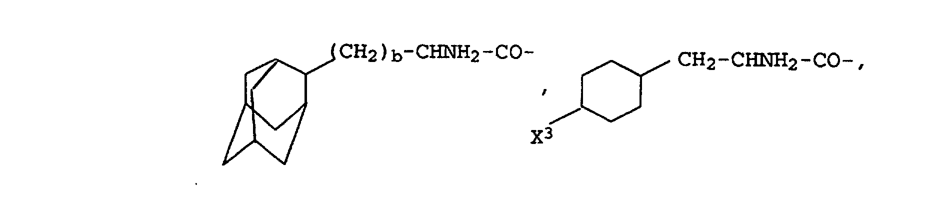 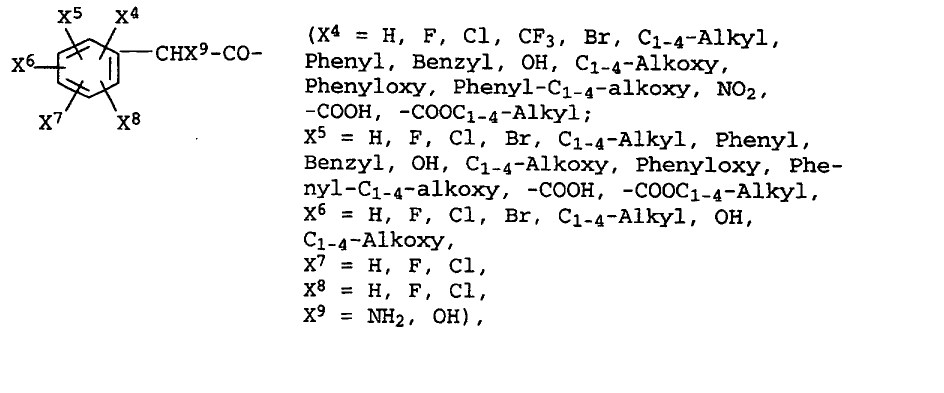 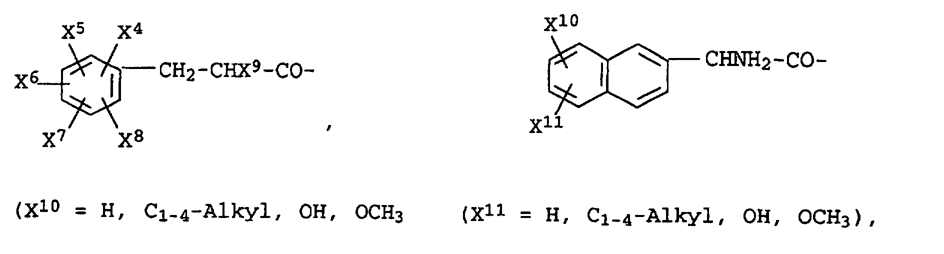 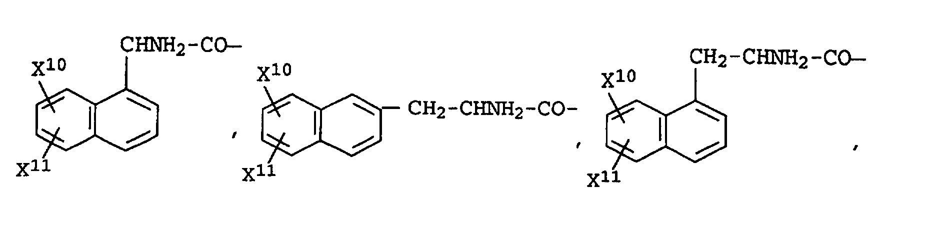 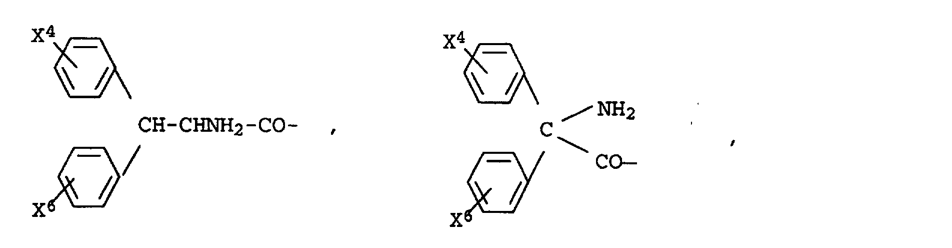 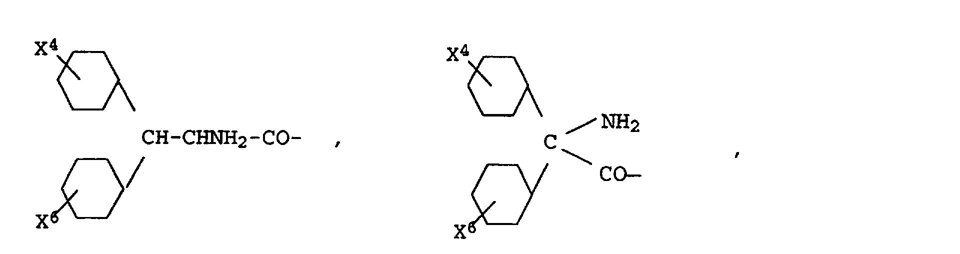 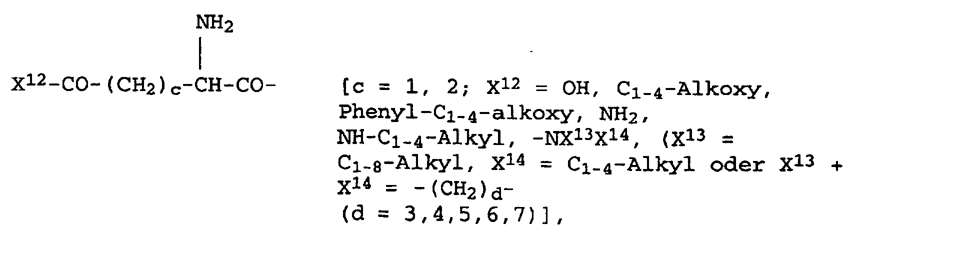
X¹³ - SO₂― CH₂ ― CH₂ ― CHNH₂ ― CO ― ,
H₂N-CH₂-CO-, H₂N-CHX¹³-CO-
- wobei in allen vorstehend genannten A-Resten die α-NH- oder α-NH₂-Gruppe mono- bzw. disubstituiert sein kann durch C₁₋₁₂-Alkyl, Phenyl-C₁₋₄-alkylen X¹²OC-C₁₋₆-alkylen, X¹²OC-C₁₋₆-alkylcarbonyl, -α- bzw. β-Naphthyl-C₁₋₄-alkylen, C₁₋₁₂-Alkylcarbonyl, Phenyl-C₁₋₄-alkylcarbonyl, C₁₋₇-Alkoxycarbonyl, Phenyl-C₁₋₅-alkoxycarbonyl, -α- bzw. β-Naphthyl-C₁₋₄-alkylcarbonyl-, C₁₋₆-Alkylaminocarbonyl oder Phenyl-C₁₋₄-alkylaminocarbonyl
noch A: X¹-NH-CH₂-CH₂-CO-, X¹-NH-CH₂-CH₂-CH₂-CO- 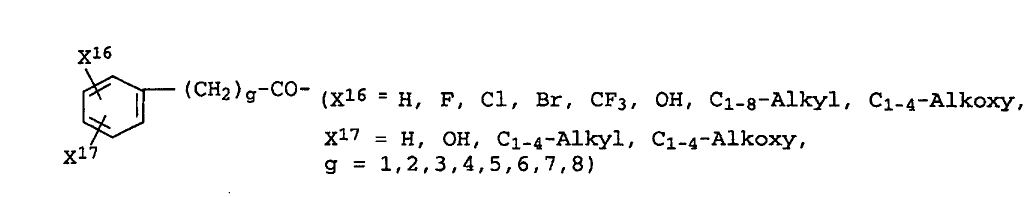 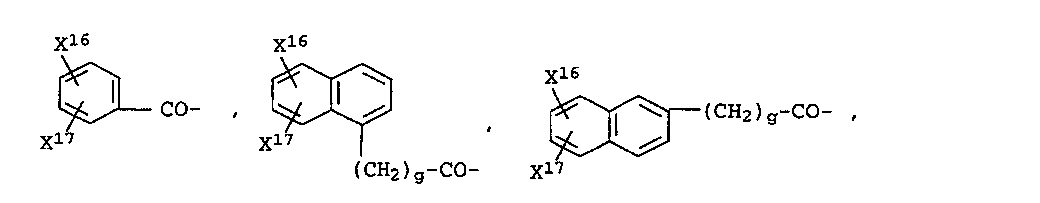 X¹⁸-O-CO-C₁₋₄-alkylen-CO- (X¹⁸ = H, C₁₋₄-Alkyl),
C₁₋₁₂-Alkyl-CO-, C₁₋₁₀-Alkyl-NH-CO-, Phenyl-C₁₋₄-alkylen-NH-CO-, α- oder β-Naphthyl-CO- oder C₃₋₇-Cycloalkyl-CO-,
B: 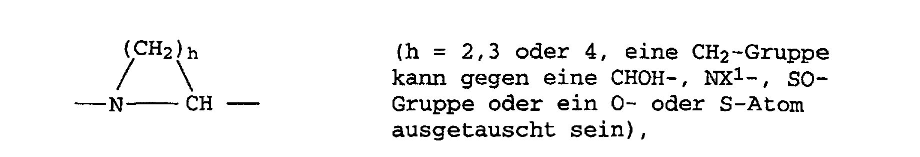 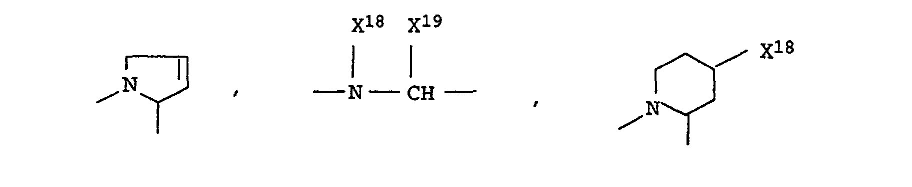 (X¹⁸ = H oder C₁₋₄-Alkyl,
X¹⁹ = H, C₁₋₆-Alkyl, Phenyl, Benzyl, Cyclohexyl oder Cyclohexylmethyl)
R¹: H oder C₁₋₄-Alkyl,
R²: H,
R³: H,
m: 0,
D: Phenylen, an welchem (CH₂)ₘ und (NH)ₙ para- oder meta-ständig zueinander gebunden sind und welches in der ortho-Stellung zu (CH₂)ₘ durch F, Cl, Br, HO-CH₂-, OH, NH₂, NO₂, C₁₋₄-Alkoxy, C₁₋₆-Alkyl oder COX²¹ (X²¹ = H, C₁₋₄-Alkyl, C₁₋₄-Alkoxy, OH, NH₂, NH-C₁₋₄-Alkyl) -O-(CH₂)₁₋₃-CO-X²¹ oder -(CH₂)₁₋₃-CO-X²¹ substituiert sein kann,
Pyridinylen, Pyrimidinylen, Pyrazinylen oder Pyridazinylen, an welchen (CH₂)ₘ und (NH)ₙ para- oder meta-ständig zueinander gebunden sind und welche in der ortho-Stellung zu (CH₂)ₘ durch F, Cl, Br, OH, NH₂, C₁₋₄-Alkoxy oder C₁₋₆-Alkyl substituiert sein können,
1,4- oder 1,3-Cyclohexylen, in dem eine CH₂-Gruppe in orthoStellung zu (CH₂)ₘ durch NH, O, S oder SO ersetzt sein kann oder
Piperidinylen, welches in 3- oder 4-Position zum Stickstoff mit (CH₂)ₘ verbunden ist, und in welchem das Stickstoffatom selbst die C(=NH)NHR⁴-Gruppe trägt,
n: 0 oder 1,
R⁴: H, -CO-C₁₋₂₀-Alkyl, -CO-O-C₁₋₂₀-Alkyl, OH oder NH₂,
wobei A, B, D, R¹, R⁴, n nicht folgende Bedeutungen gleichzeitig annehmen darf: 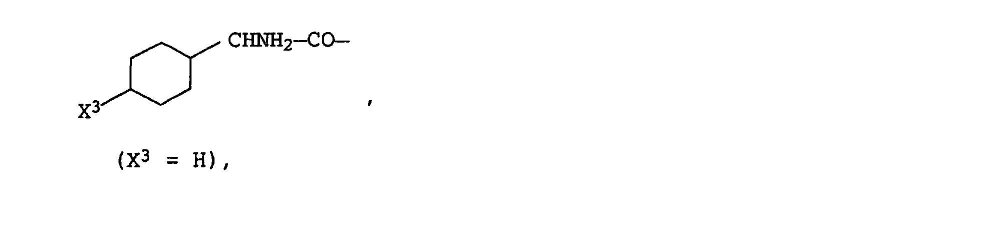 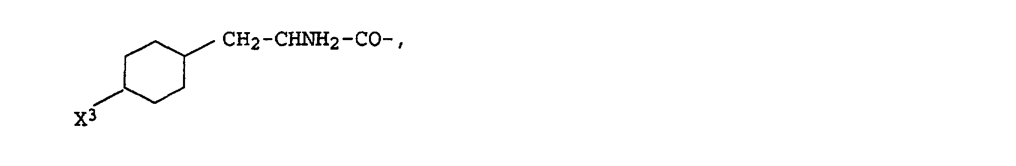 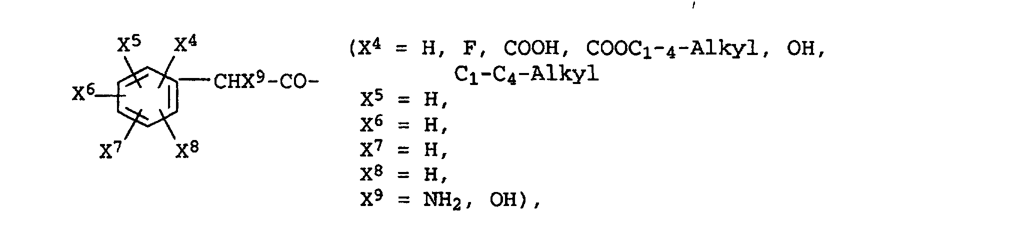 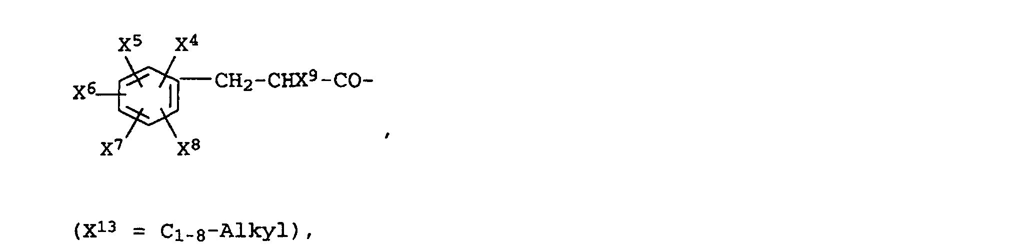
H₂N-CH₂-CO-, H₂N-CHX¹³-CO-
- wobei in allen vorstehend genannten A-Resten die α-NHoder α-NH₂-Gruppe monosubstituiert sein kann durch C₁₋₆-Alkyl, Phenyl-C₁₋₄-alkylen, C₁₋₆-Alkylcarbonyl, C₁₋₆-Alkoxycarbonyl, Phenyl-C₁₋₅-alkoxycarbonyl, 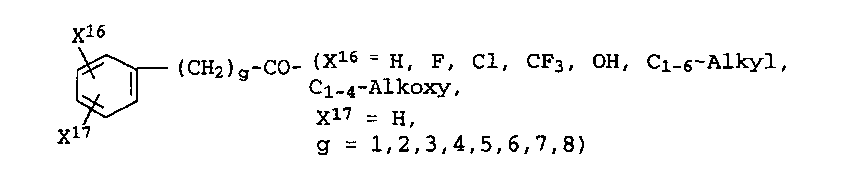 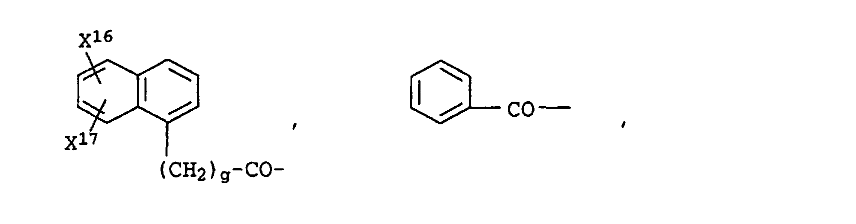
X¹⁸-O-CO-C₁₋₄-alkylen-CO- (X¹⁸ = H, C₁₋₄-Alkyl),
C₁₋₁₂-Alkyl-CO-, C₃₋₇-Cycloalkyl-CO-,
B: 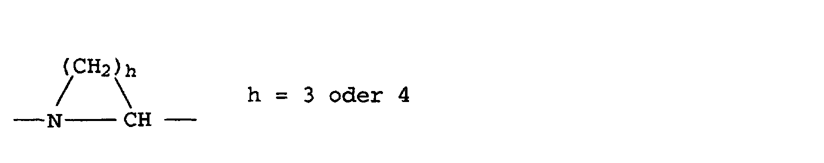
R¹: H oder C₁₋₄-Alkyl
D: Phenylen, an welchem (CH₂)ₘ, und (NH)ₙ paraständig zueinander gebunden sind
1,4-Cyclohexylen
oder
Piperidinylen, welches in 4-Position zum Stickstoff mit (CH₂)ₘ verbunden ist, und in welchem das Stickstoffatom selbst die C(=NH)NHR⁴-Gruppe trägt,
n: 0 oder 1,
R⁴: H, -CO-C₁₋₆-Alkyl, -CO-O-C₁₋₆-Alkyl oder OH
und auch nicht folgende Bedeutung annehmen darf:
A: 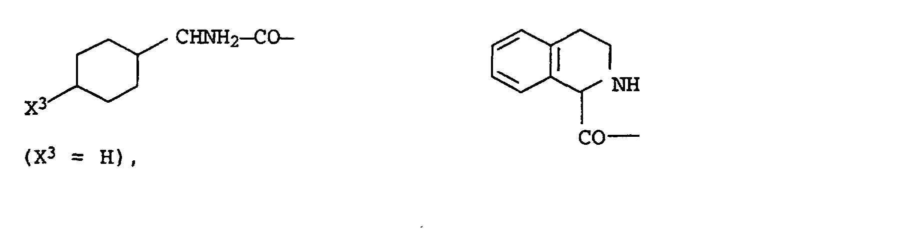 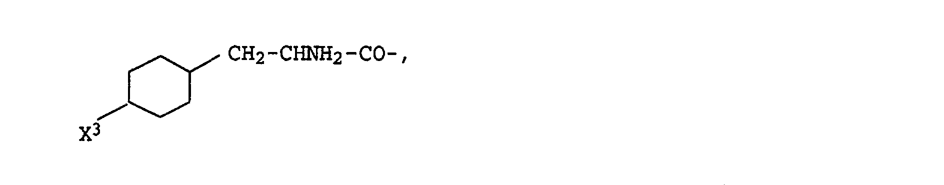 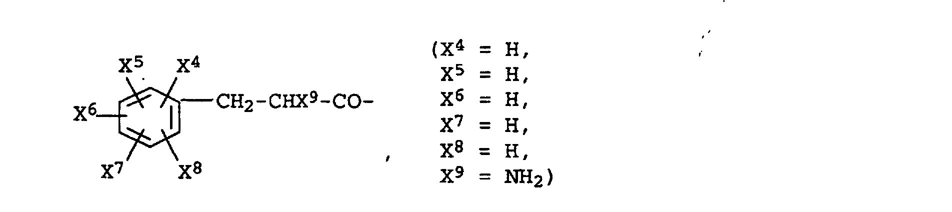 X¹² = OH, C₁₋₄-Alkoxy, NH₂,
- wobei in allen vorstehend genannten A-Resten die α-NH- oder α-NH₂-Gruppe mono- bzw. disubstituiert sein kann durch C₁₋₄-Alkyl, X¹²OC-C₁₋₄-alkylen, X¹²OC-C₁₋₂-alkylcarbonyl, C₁₋₄-Alkylcarbonyl, C₁₋₄-Alkoxycarbonyl,
B: 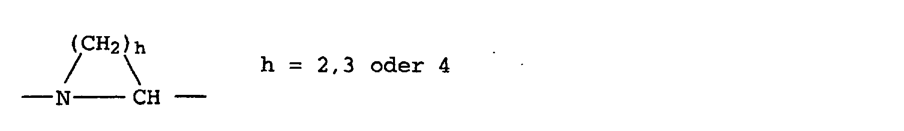 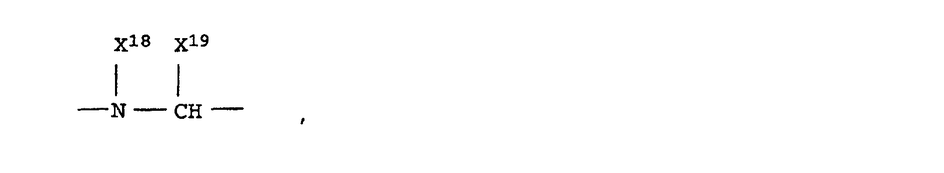
X¹⁸ = H
X¹⁹ = C₁₋₆-Alkyl, Benzyl)
R¹: H
D: Phenylen, an welchem (CH₂)ₘ und (NH)ₙ paraständig zueinander gebunden sind
1,4-Cyclohexylen,
oder
Piperidinylen, welches in 4-Position zum Stickstoff mit (CH₂)ₘ verbunden ist, und in welchem das Stickstoffatom selbst die C(=NH)NHR⁴-Gruppe trägt,
n: 0
R⁴: H
und auch nicht folgende Bedeutung annehmen darf:
A: 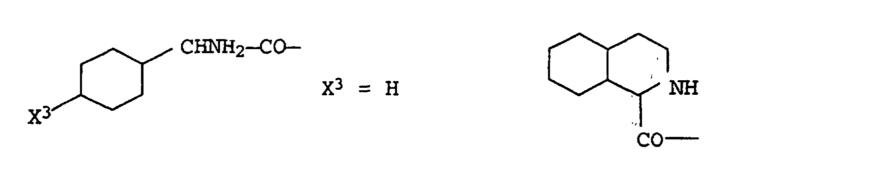 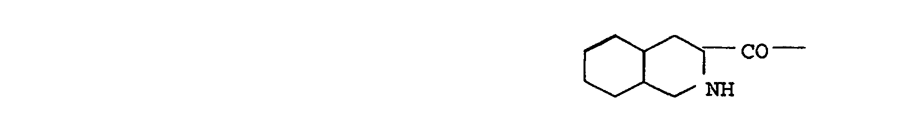 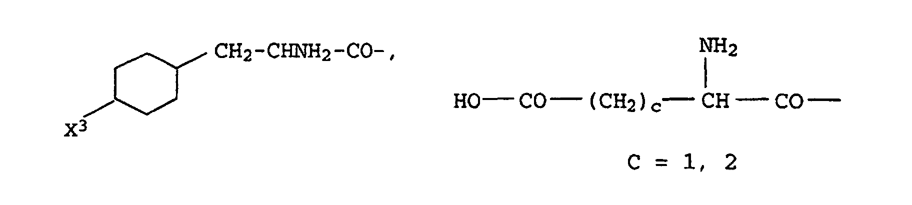 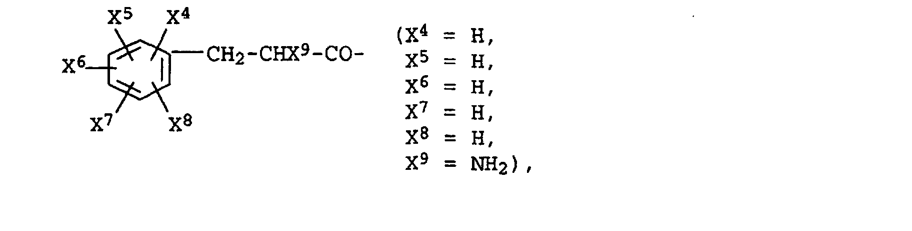
- wobei in allen vorstehend genannten A-Resten die α-NH₂-Gruppe monosubstituiert sein kann durch X¹²OC-C₁₋₃-alkylen, C₁₋₅-Alkylcarbonyl, mit X¹² = OH
B: 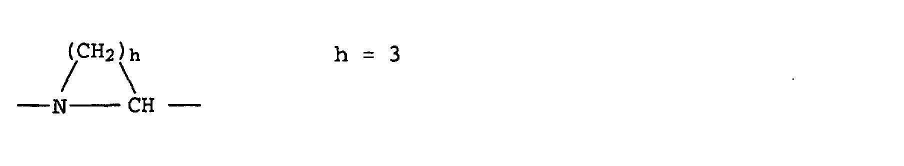
R¹: H
D: Phenylen, an welchem (CH₂)ₘ und (NH)ₙ meta- oder paraständig zueinander gebunden sind und welches in der ortho-Stellung zu (CH₂)ₘ durch F substituiert sein kann,
Pyridinylen mit der Amidingruppe in 3-Stellung oder Pyridazinylen, an welchen (CH₂)ₘ und (NH)ₙ paraständig zueinander gebunden sind
Piperidinylen, welches in 4-Position zum Stickstoff mit (CH₂)ₘ verbunden ist, und in welchem das Stickstoffatom selbst die C(=NH)NHR⁴-Gruppe trägt,
n: 0
R⁴: H
und nicht N-[[1-(Aminoiminomethyl)-3-piperidinyl]methyl]-1-Dphenylalanyl-L-prolinamid bedeutet

2. Verbindungen der Formel 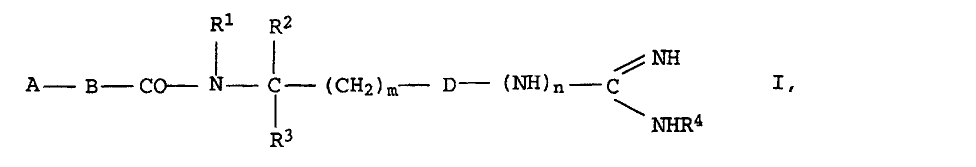 sowie deren Salze mit physiologisch verträglichen Säuren und deren Stereoisomeren, worin die Substituenten folgende Bedeutungen besitzen:
A: 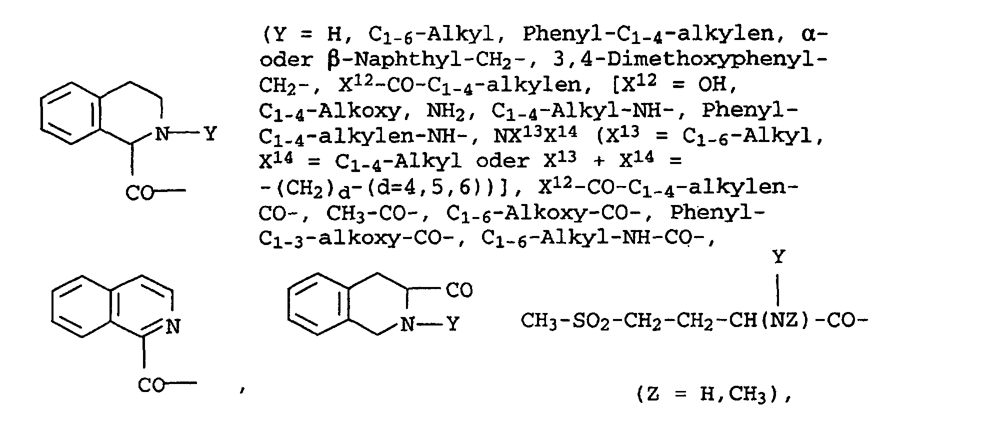 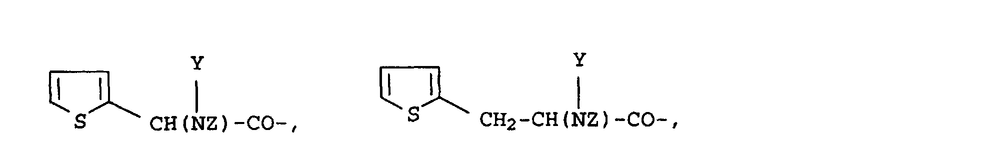 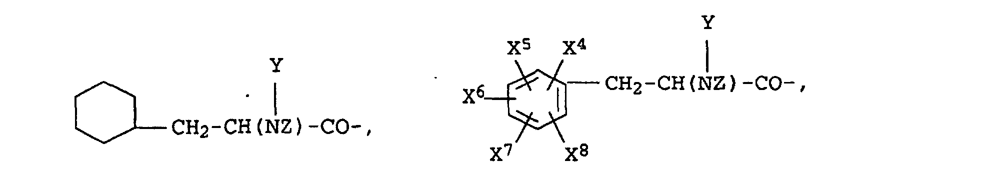 (X⁴ = H, F, Cl, Br, CF₃, C₁₋₄-Alkyl, OH, OCH₃, NO₂, Phenyl, X⁵ = H, F, Cl, Br, CH₃, OH, OCH₃, Phenyl, X⁶ = H, F, Cl, Br, CH₃, OH, OCH₃, X⁷ = H, F, X⁸ = H, F), 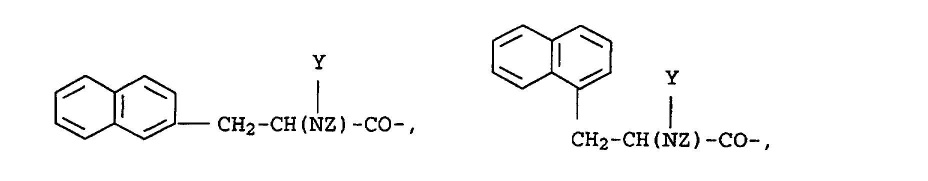 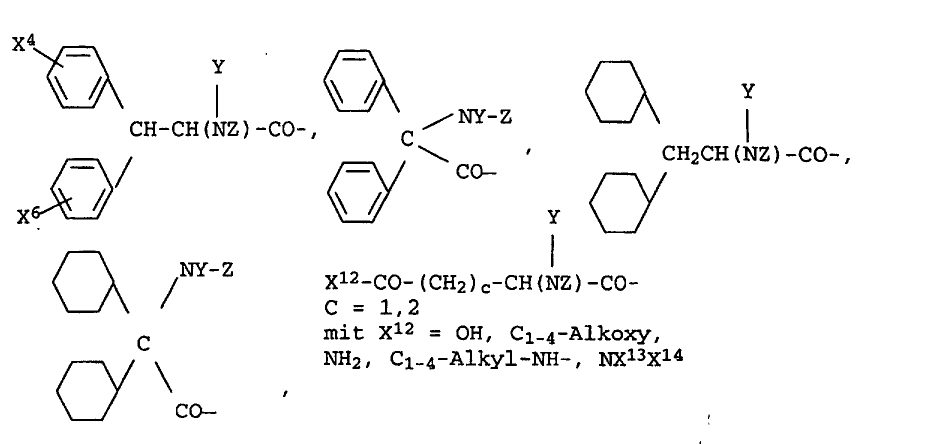 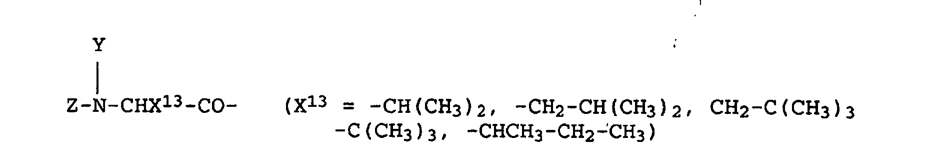 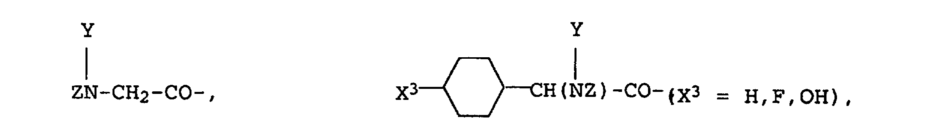 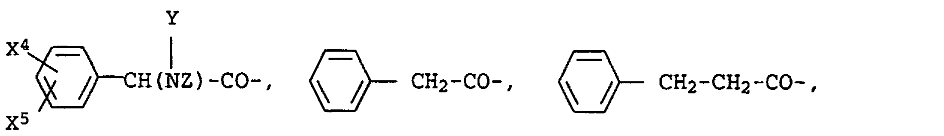 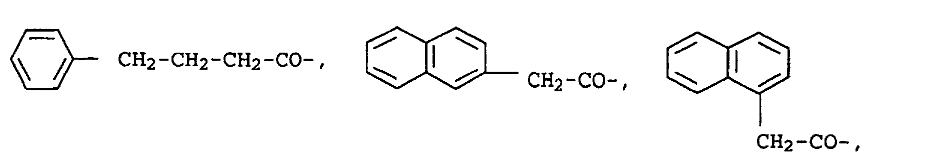 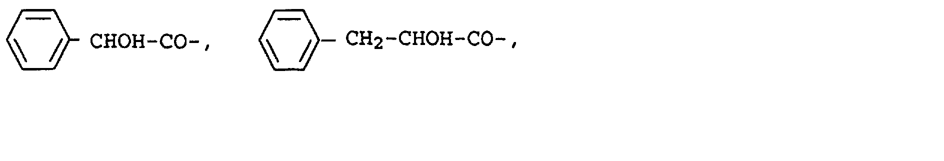 B: 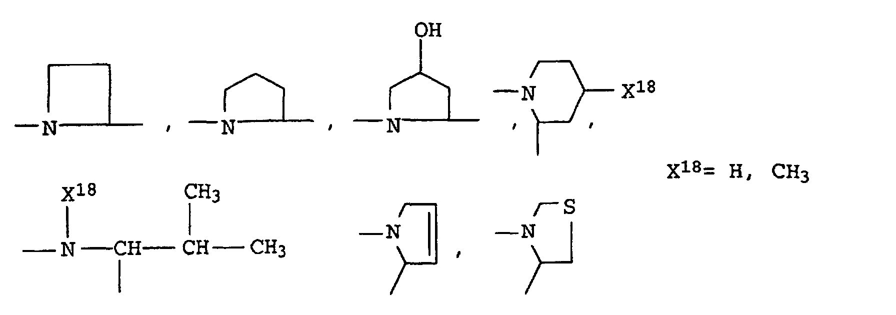
R¹: H, CH₃,
R²: H,
R³: H,
R⁴: H, OH, NH₂
m: 0,
für -(CH₂)ₘ-D-(NH)ₙ- mit n = 0 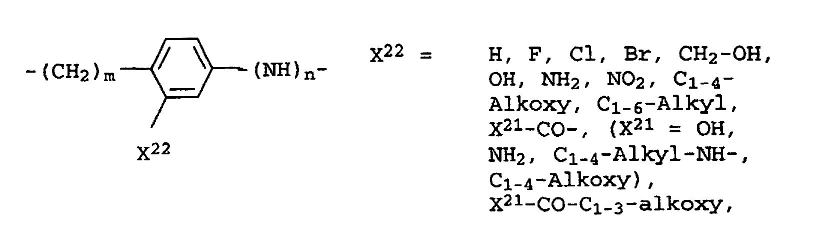 für -(CH₂)ₘ-D-(NH)ₙ- mit n = 0, 1 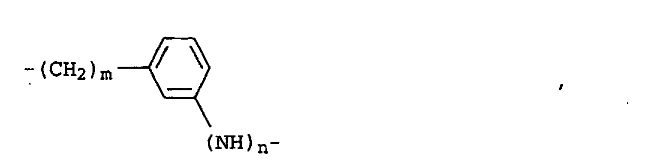 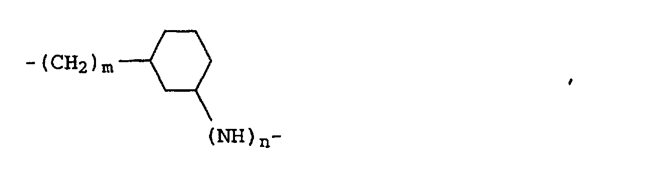 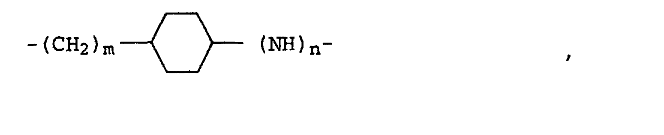 für -(CH₂)ₘ-D-(NH)ₙ- mit n = 0, 1 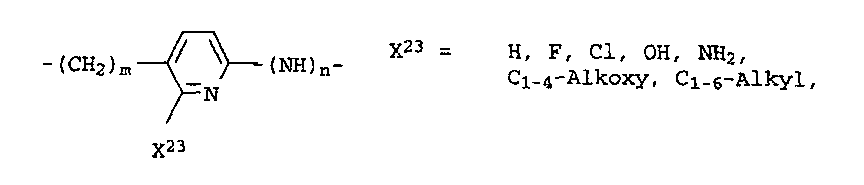 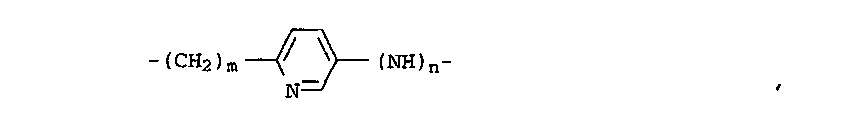 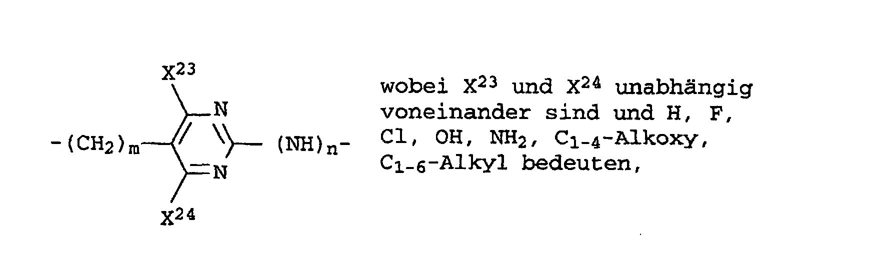 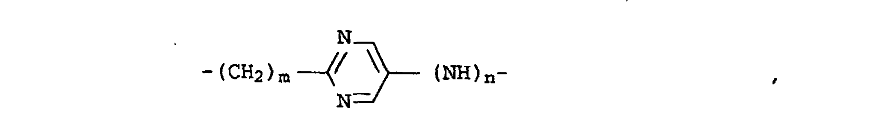 für -(CH₂)ₘ-D-(NH)ₙ- mit n = 0: 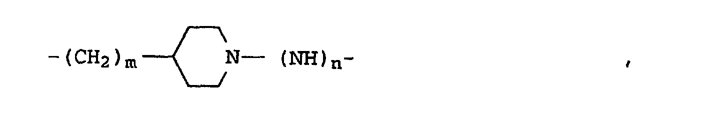 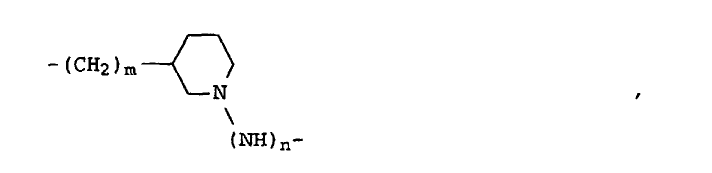 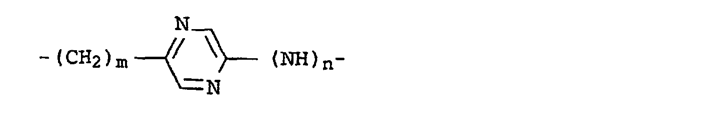 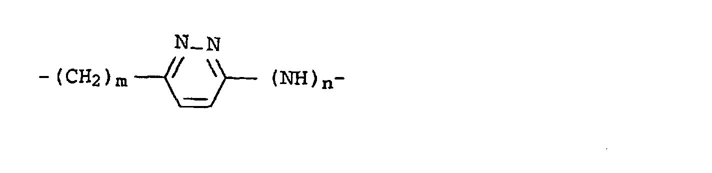 wobei R³, A, B und D, für R⁴ = OH oder H nicht gleichzeitig folgende Bedeutung annehmen dürfen
A: für R⁴ = OH oder H
Y = H, C₁₋₆-Alkyl, Phenyl-C₁₋₄-alkylen, CH₃-CO-, C₁₋₆-Alkoxy-CO-, Phenyl-C₁₋₃-alkoxy-CO-, 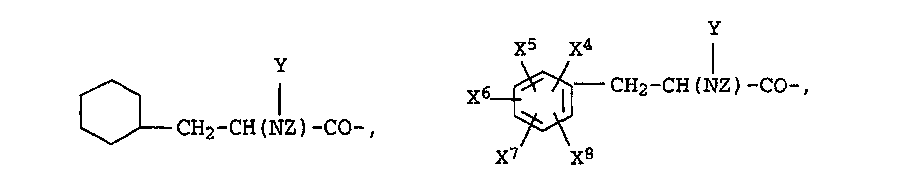
(X⁴ = H, F, C₁₋₄-Alkyl,
X⁵ = H,
X⁶ = H,
X⁷ = H,
X⁸ = H), Z = H, 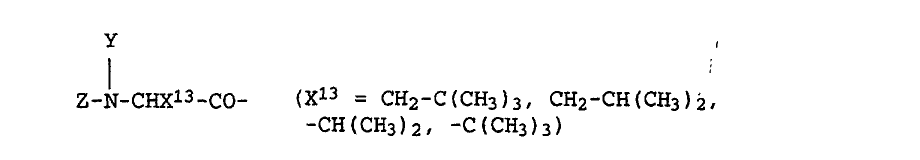 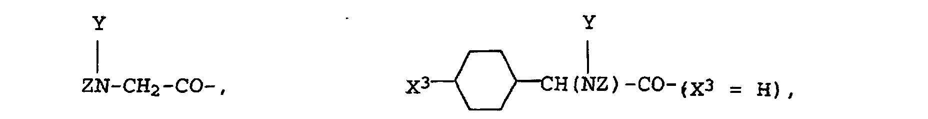 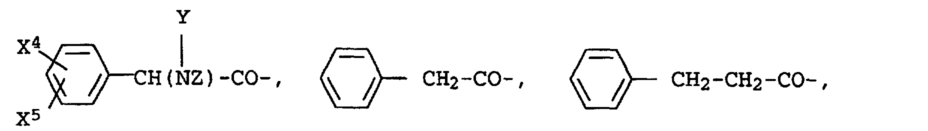 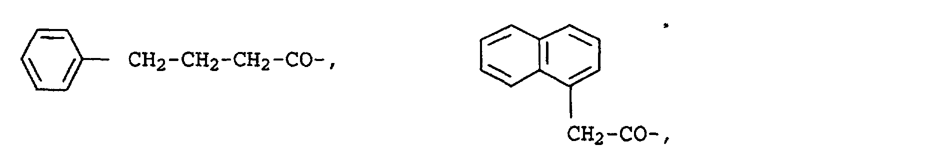 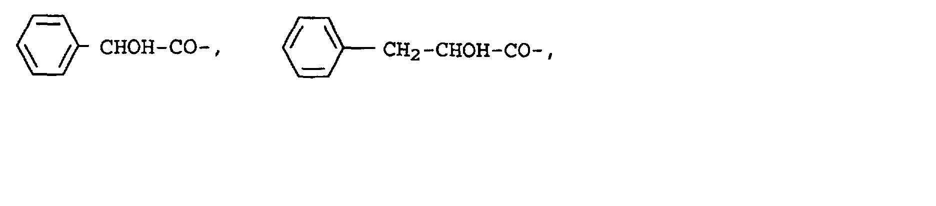 B: 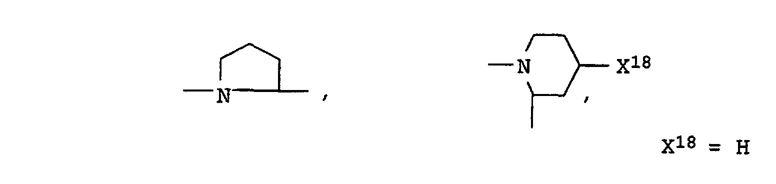
R¹: H, CH₃
für -(CH₂)ₘ-D-(NH)ₙ- mit m = 0, n = 0 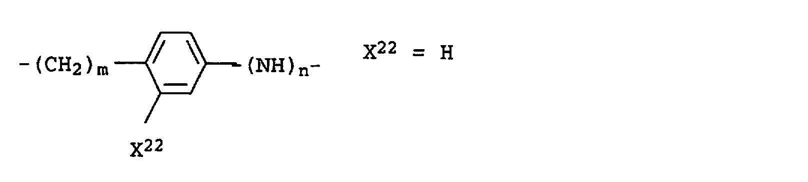 für -(CH₂)ₘ-D-(NH)ₙ- mit m = 0, n = 0,1 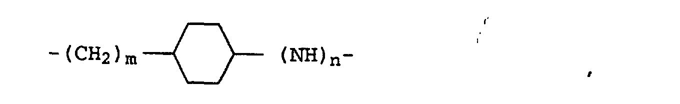 für -(CH₂)ₘ-D-(NH)ₙ- mit m = 0, n = 0: 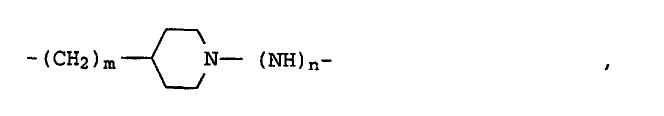 und
wobei für R⁴ = H A, B, D, R³ nicht gleichtzeitig folgende Bedeutung annehmen darf:
A: 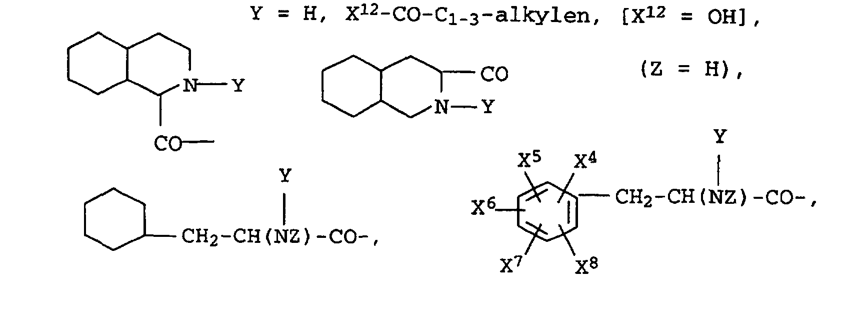
(X⁴ = H,
X⁵ = H,
X⁶ = H,
X⁷ = H,
X⁸ = H), 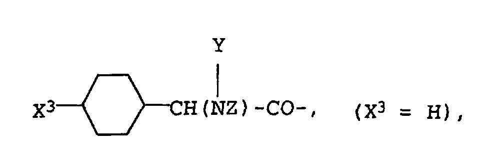 B: 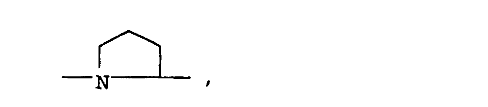
R¹: H
m: 0
für -(CH₂)ₘ-D-(NH)ₙ- mit m = 0, n = 0 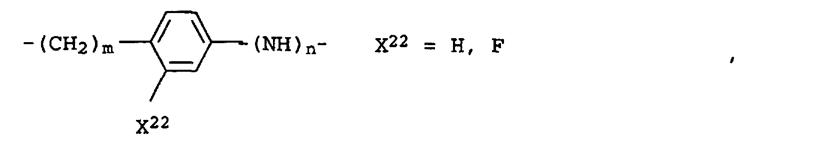 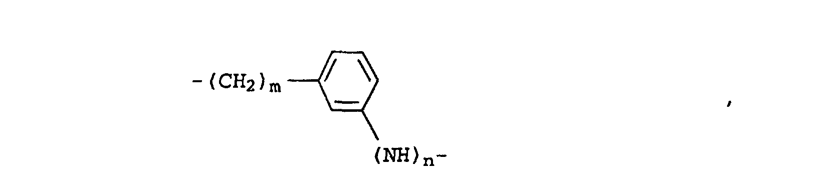 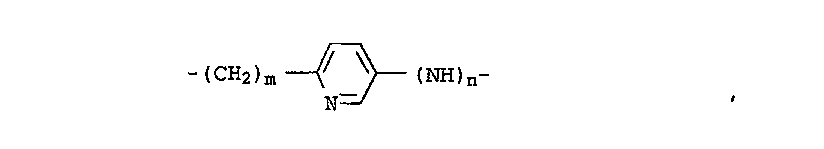 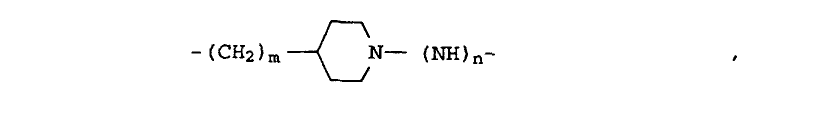 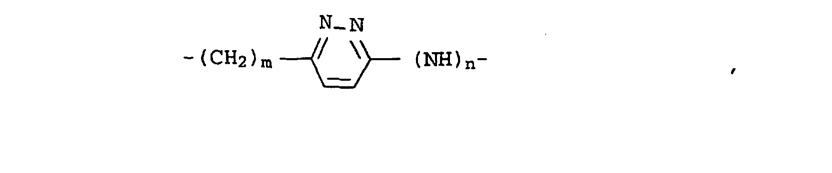 und ebenfalls dürfen A, B, D, R¹, R⁴ nicht bedeuten
A: (Y = H, X¹²-CO-C₁₋₄-alkylen, [X¹² = OH, C₁₋₄-Alkoxy, NH₂]; X¹²-CO-C₁₋₄-alkylen-CO-, CH₃-CO-), 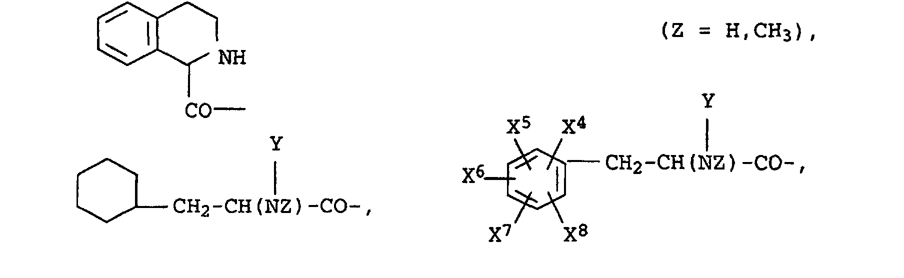
(X⁴ = H
X⁵ = H,
X⁶ = H
X⁷ = H
X⁸ = H), 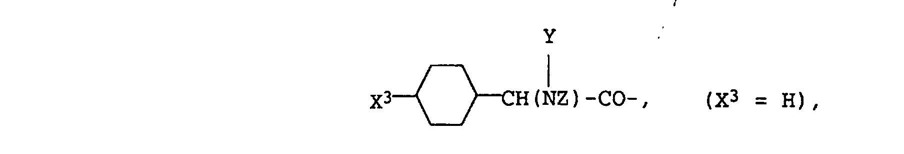
B: 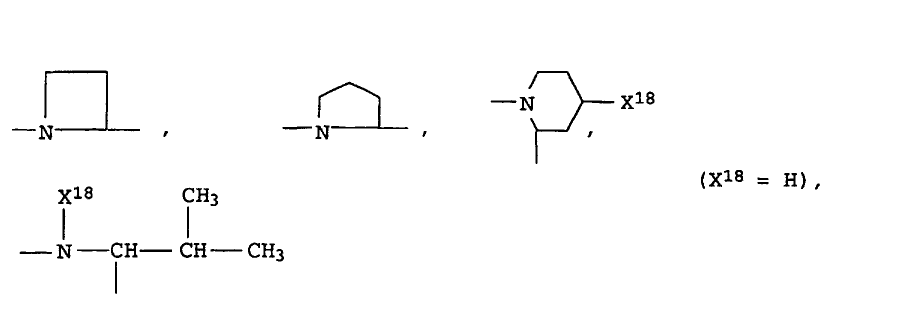
R¹: H
R⁴: H
für -(CH₂)ₘ-D-(NH)ₙ- mit n = 0 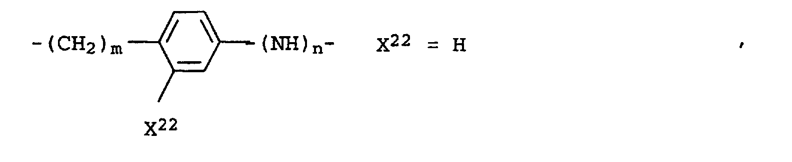 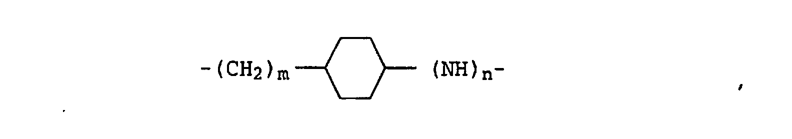 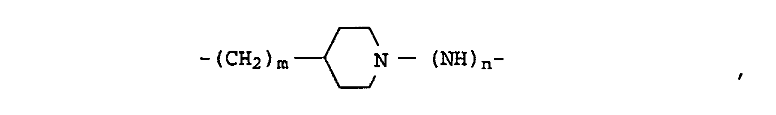 und nicht N-[[1-(Aminoiminomethyl)-3-piperidinyl]methyl]-1-Dphenylalanyl-L-prolinamid bedeutet

3. Verbindungen der Formel 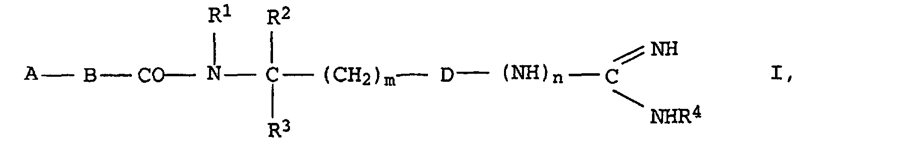 sowie deren Salze mit physiologisch verträglichen Säuren und deren Stereoisomeren, worin die Substituenten folgende Bedeutungen besitzen: 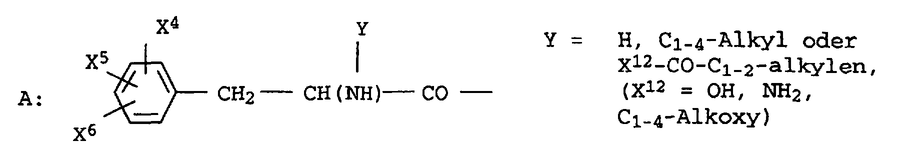
X⁴ = H, F, Cl, CH₃, CF₃, OCH₃ oder Br
X⁵ = H, Cl,, CH₃, oder OCH₃, OC₂H₅
X⁶ = H oder OCH₃, 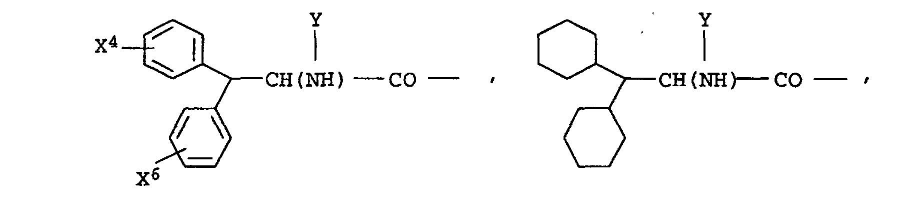 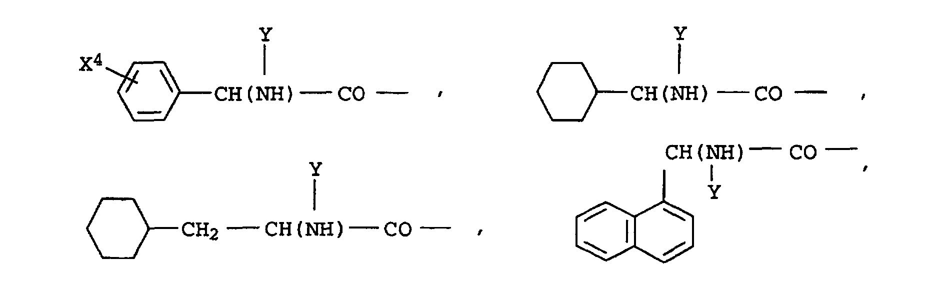 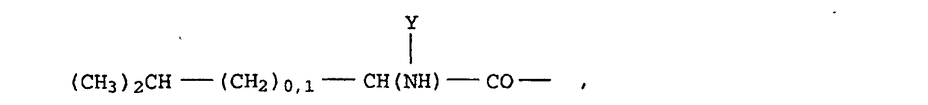 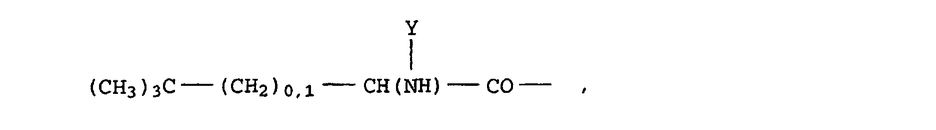 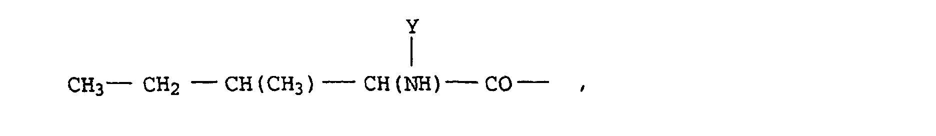 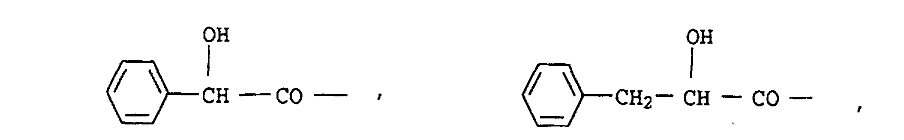 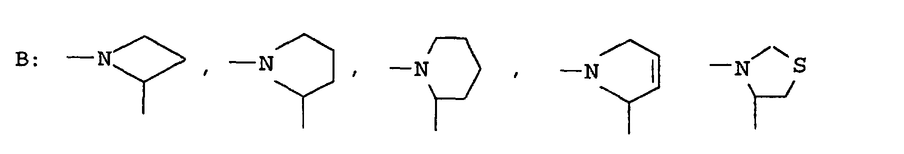
R¹ = H
R², R³ = H
für -(CH₂)ₘ-D-(NH)ₙ- mit m = 0, n = 0 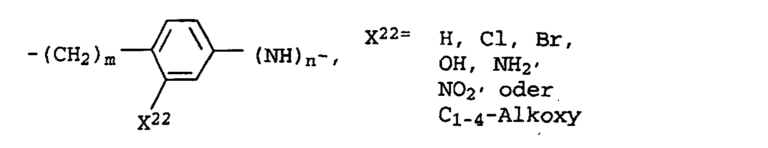 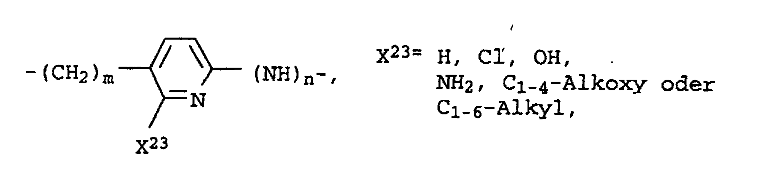 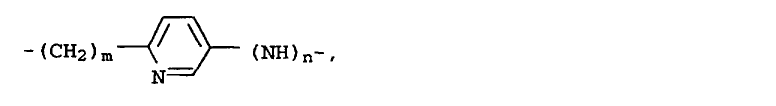 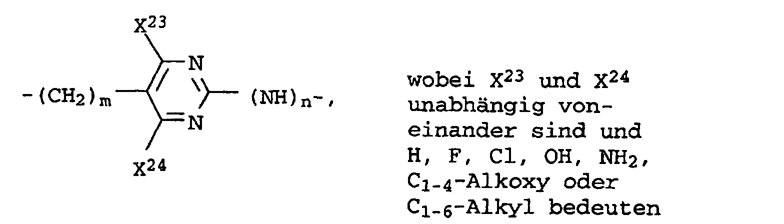 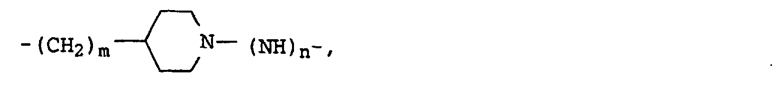 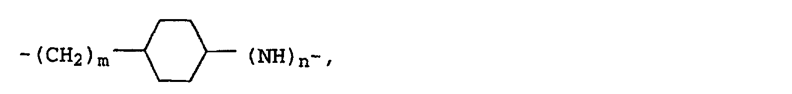 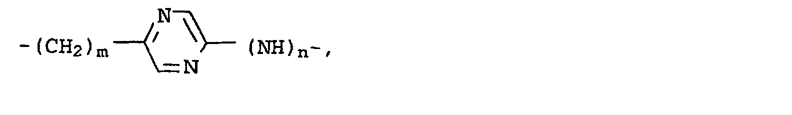 für -(CH₂)ₘ-D-(NH)ₙ- mit m = 0, n = 1 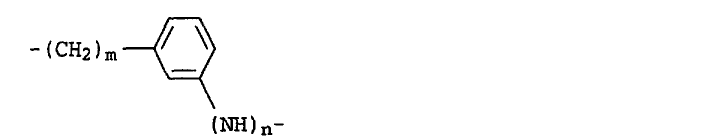 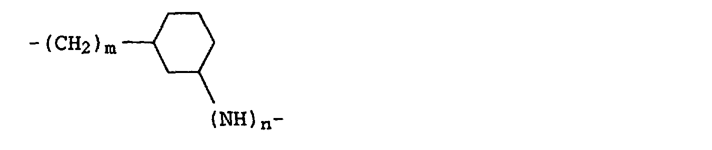
R⁴: H, OH,
wobei A, B, D und R⁴ nicht gleichzeitig folgende Bedeutung haben: 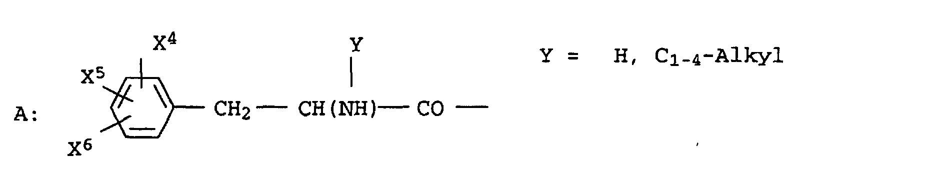
X⁴ = H, F, CH₃
X⁵ = H
X⁶ = H 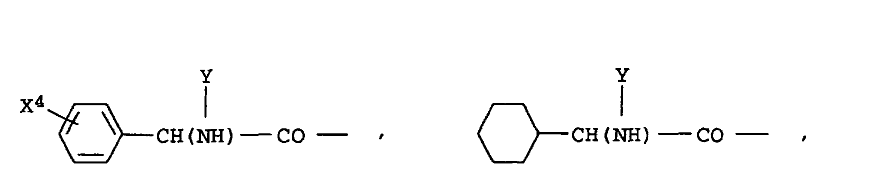 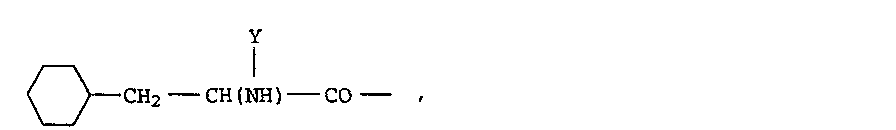 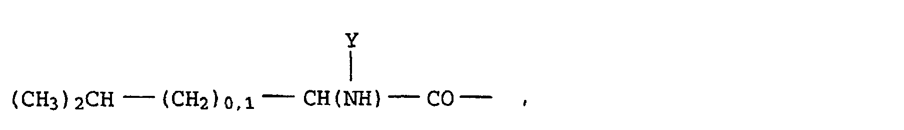 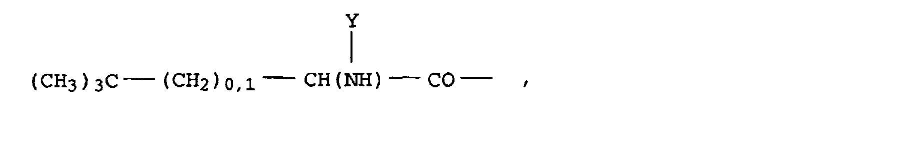 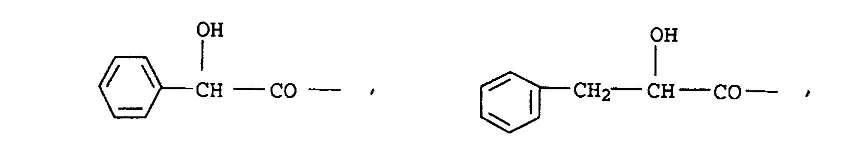
B: 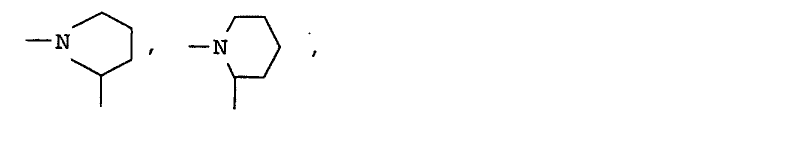 für -(CH₂)ₘ-D-(NH)ₙ- mit m = 0, n = 0 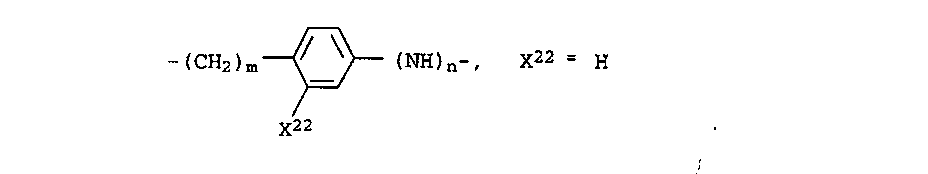 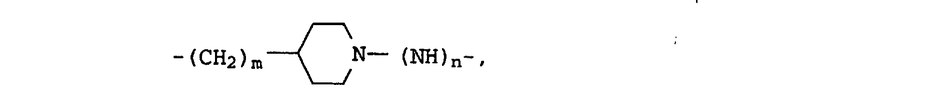 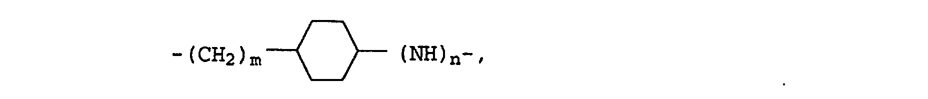
R⁴: H, OH.
und für
R⁴ = H
A, B und D nicht gleichzeitig folgende Bedeutungen annehmen darf: 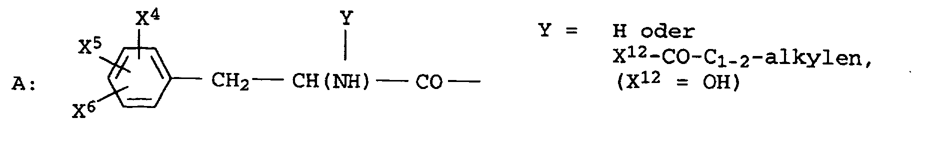
X⁴ = H
X⁵ = H
X⁶ = H 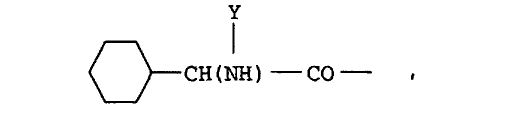 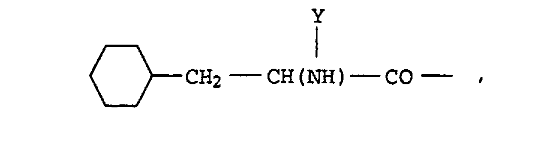
B: 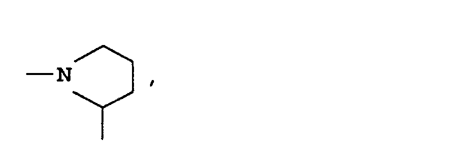 für -(CH₂)ₘ-D-(NH)ₙ- mit m = 0, n = 0 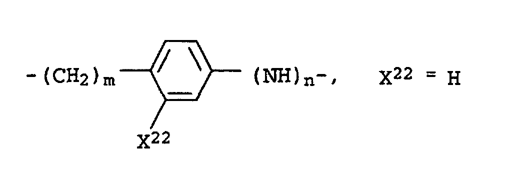 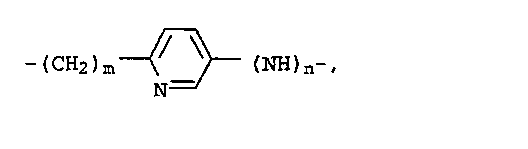 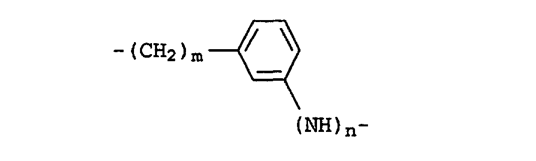 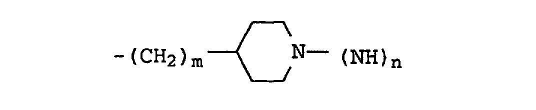 und A, B, D, R¹ ebenso für R⁴ = H nicht folgende Bedeutungen annehmen darf: 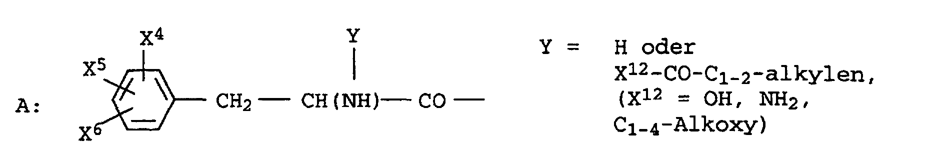
X⁴ = H
X⁵ = H
X⁶ = H 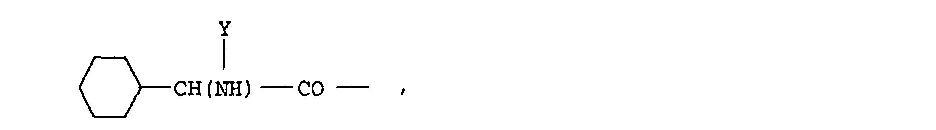 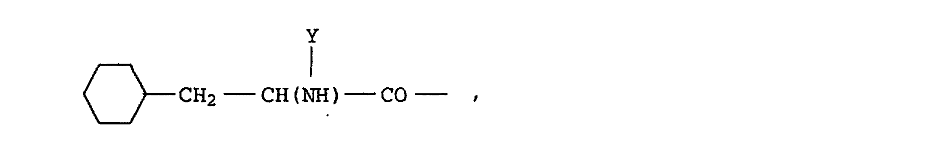 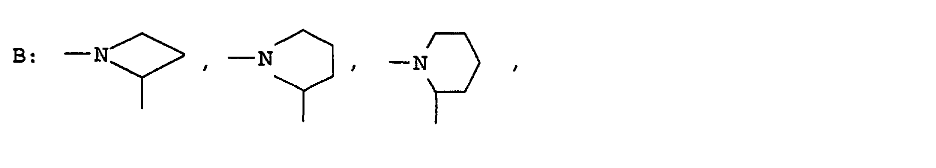
für -(CH₂)ₘ-D-(NH)ₙ- mit m = 0, n = 0

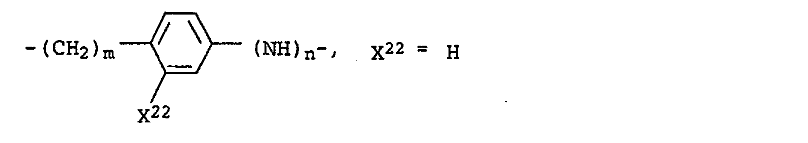

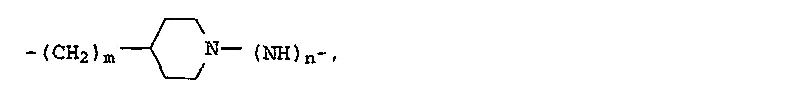

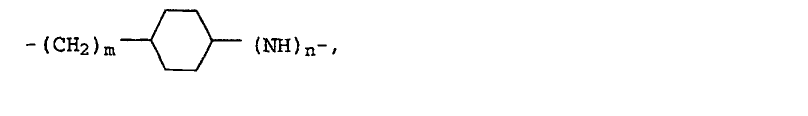

4. Verbindungen der Formel I gemäß einem der Ansprüche 1 bis 3 zur Verwendung bei der Bekämpfung von Krankheiten.

5. Verbindungen der Formel II 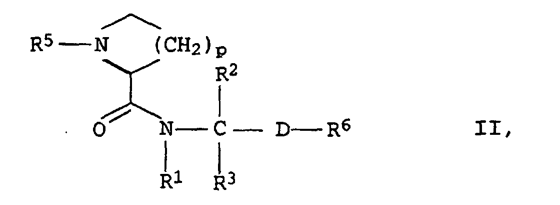 worin R¹, R², R³ und D die für Formel I angegebene Bedeutungen besitzen, und
R⁵: H, C₁₋₄-Alkoxy-CO- oder Phenyl-C₁₋₃-alkoxy-CO-,
R⁶: ein Cyano-, Amidino- oder Guanidino-Rest in der m- oder p-Stellung zu C(R², R³) und
p: 1,2 oder 3
bedeuten.

## Claims

1. Compounds of the formula 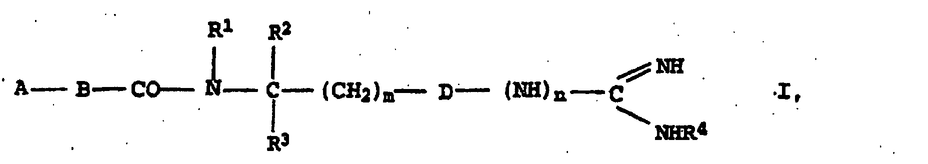 as well as their salts with physiologically compatible acids and their stereoisomers, in which the substituents have the following meanings:
A: 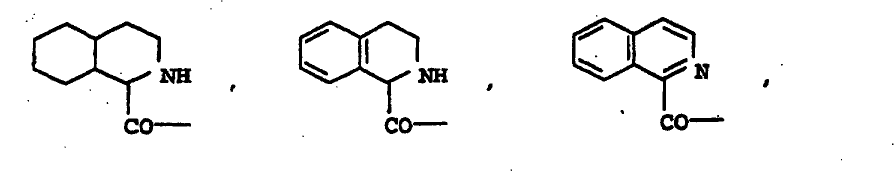 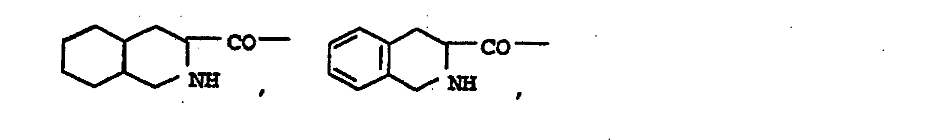 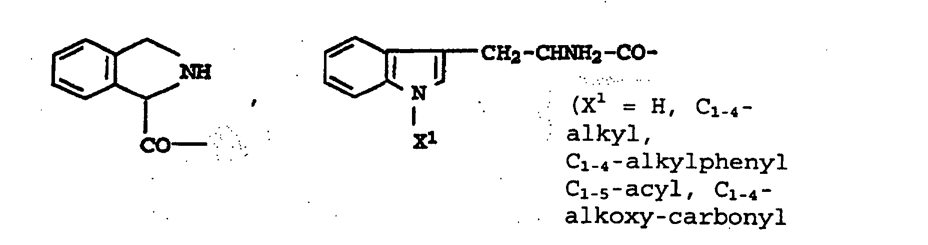 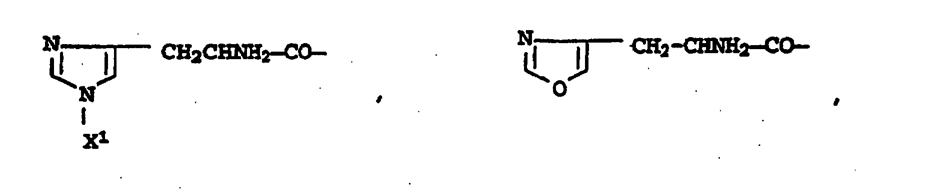 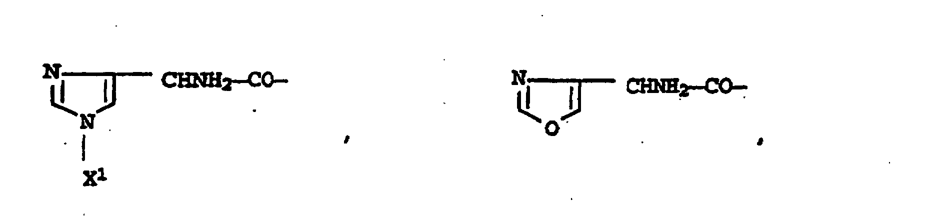 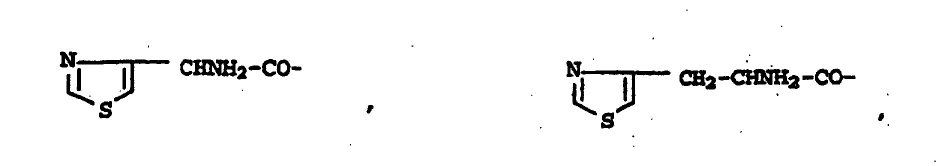 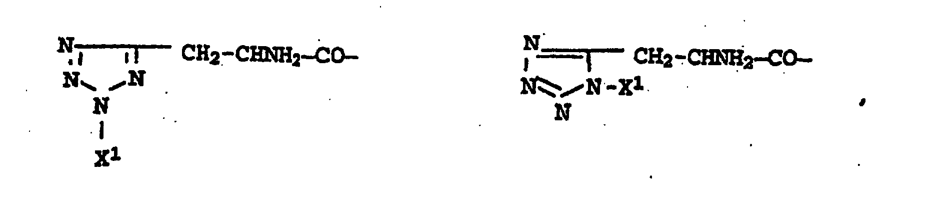 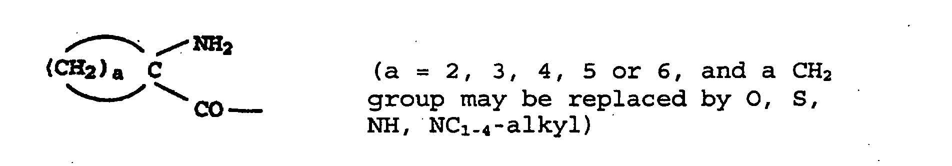 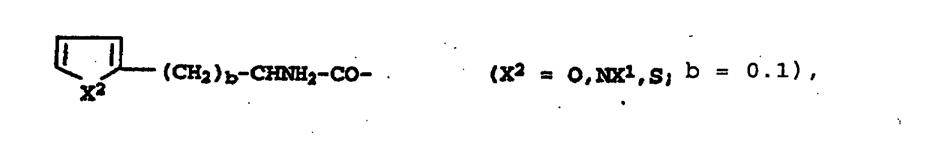 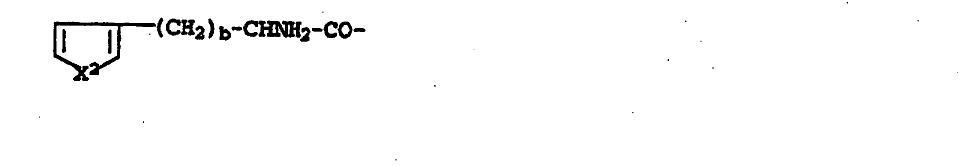 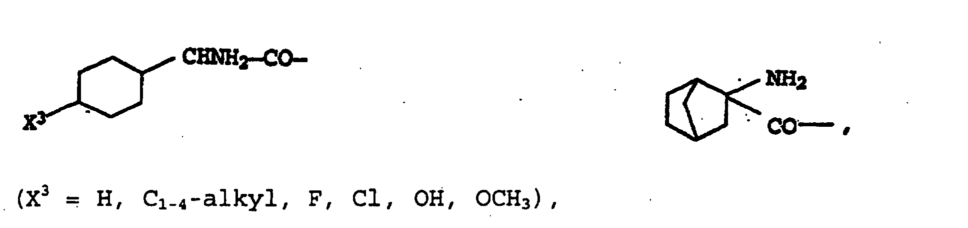 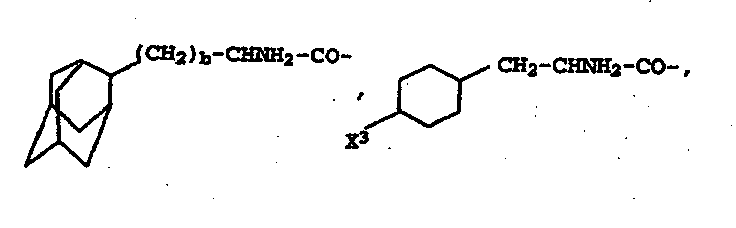 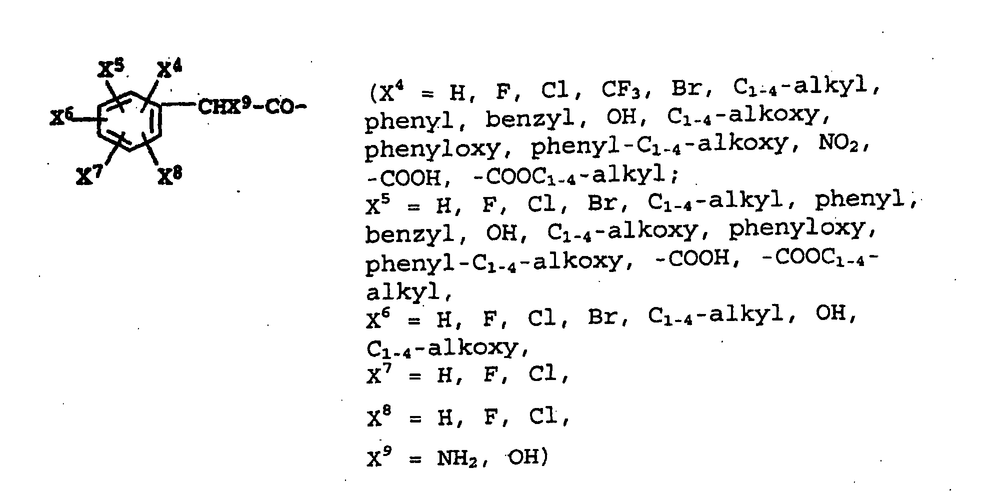 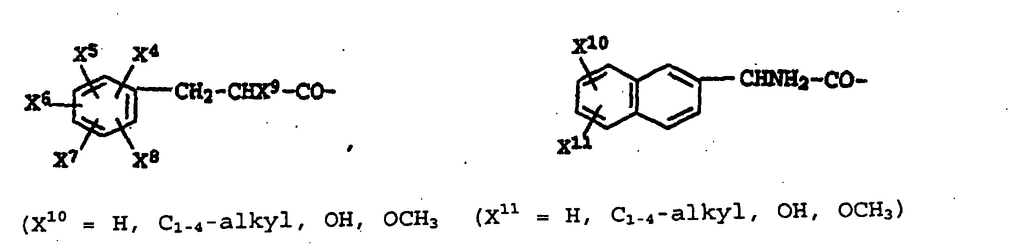 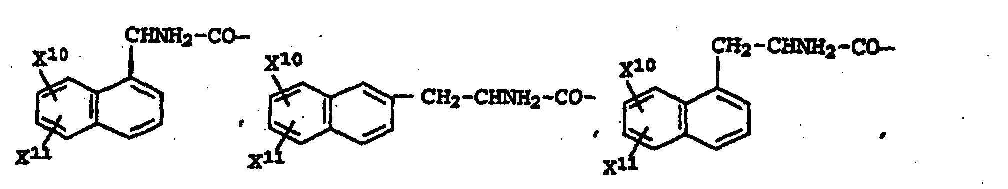 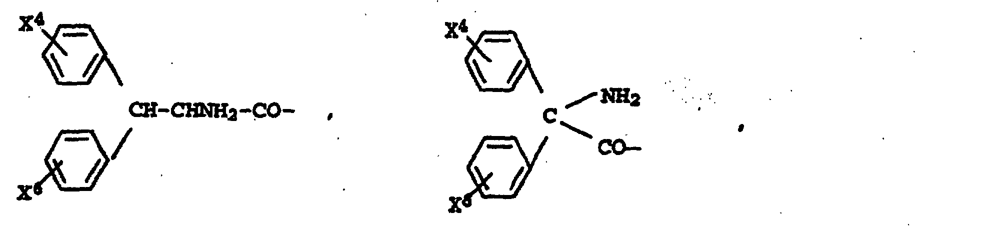 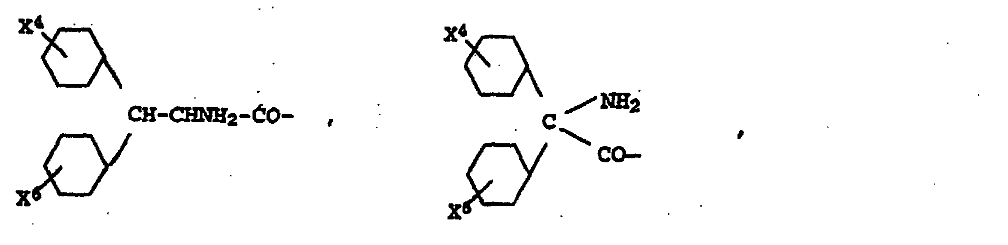 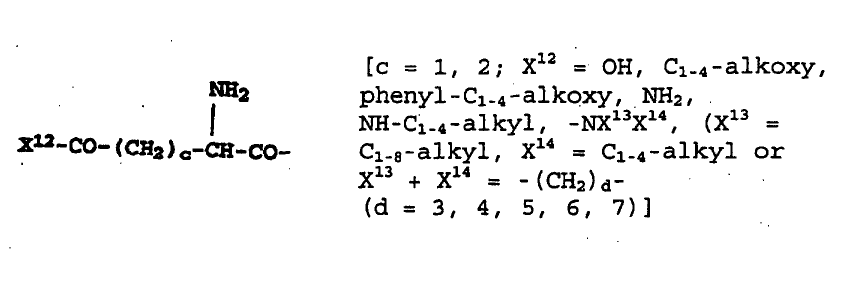
X¹³ - SO₂― CH₂ ― CH₂ ― CHNH₂ ― CO ― ,
H₂N-CH₂-CO-, H₂N-CHX¹³-CO-
- wherein in all the abovementioned A radicals the α-NH or α-NH₂ group may be monosubstituted or disubstituted by C₁₋₁₂-alkyl, phenyl-C₁₋₄-alkylene, X¹²OC-C₁₋₆-alkylene, X¹²OC-C₁₋₆-alkylcarbonyl, α- or β-naphthyl-C₁₋₄-alkylene, C₁₋₁₂-alkylcarbonyl, phenyl-C₁₋₄-alkylcarbonyl, C₁₋₇-alkoxycarbonyl, phenyl-C₁₋₅-alkoxycarbonyl, α- or β-naphthyl-C₁₋₄-alkylcarbonyl, C₁₋₆-alkylaminocarbonyl or phenyl-C₁₋₄-alkylaminocarbonyl
also A: X1-NH-CH₂-CH₂-CO-, X¹-NH-CH₂-CH₂-CH₂-CO- 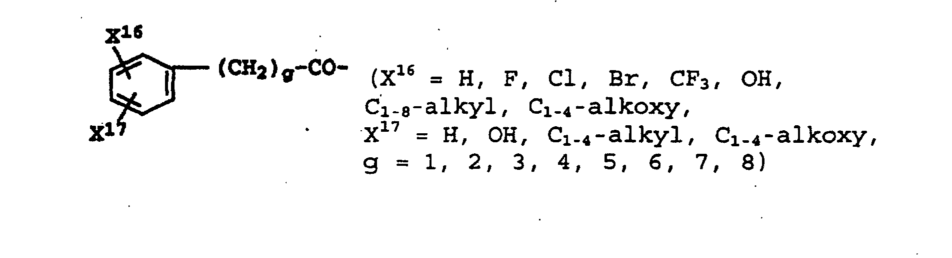 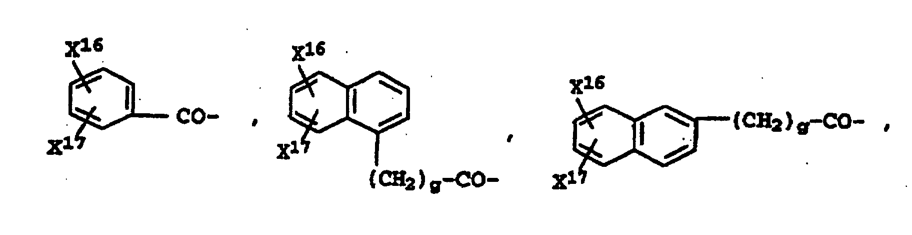 X¹⁸-O-CO-C₁₋₄-alkylene-CO- (X¹⁸ = H, C₁₋₄-alkyl),
C₁₋₁₂-alkyl-CO-, C₁₋₁₀-alkyl-NH-CO-, phenyl-C₁₋₄-alkylene-NH-CO-, α- or β-naphthyl-CO- or C₃₋₇-cycloalkyl-CO-,
B: 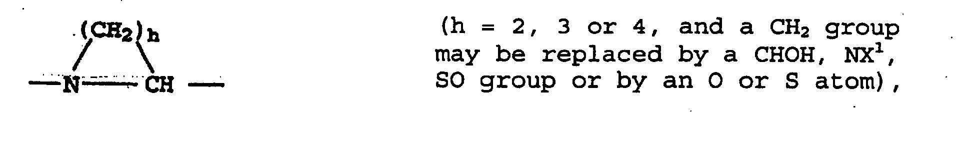 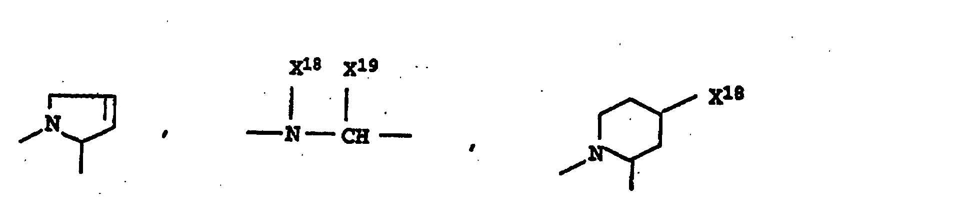 (X¹⁸ = H or C₁₋₄-alkyl,
X¹⁹ = = H, C₁₋₆-alkyl, phenyl, benzyl, cyclohexyl or cyclohexylmethyl)
R¹: H or C₁₋₄-alkyl,
R²: H,
R³: H,
m: O,
D: phenylene, on which (CH₂)ₘ and (NH)ₙ are bonded in the meta or para position to one another and which may be substituted in the ortho position to (CH₂)ₘ by F, Cl, Br, HO-CH₂-, OH, NH₂, NO₂, C₁₋₄-alkoxy, C₁₋₆-alkyl or COX²¹ (X²¹ = H, C₁₋₄-alkyl, C₁₋₄-alkoxy, OH, NH₂, NH-C₁₋₄-alkyl) -O- (CH₂)₁₋₃-CO-X²¹ or -(CH₂)₁₋₃-CO-X²¹,
pyridinylene, pyrimidinylene, pyrazinylene or pyridazinylene, on which (CH₂)ₘ and (NH)ₙ are bonded in the para or meta position to one another and which can be substituted in the ortho position to (CH₂)ₘ by F, Cl, Br, OH, NH₂, C₁₋₄-alkoxy or C₁₋₆-alkyl,
1,4- or 1,3-cyclohexylene in which a CH₂ group in the ortho position to (CH₂)ₘ may be replaced by NH, O, S, or SO
piperidinylene which is bonded in the 3 or 4 position to the nitrogen atom, to (CH₂)ₘ, and in which the nitrogen atom itself carries the C(=NH)NHR⁴ group,
n: 0 or 1,
R⁴: H, -CO-C₁₋₂₀-alkyl, -CO-O-C₁₋₂₀-alkyl, OH or NH₂,
wherein A, B, D, R¹, R⁴, n may not simultaneously have the following meanings: 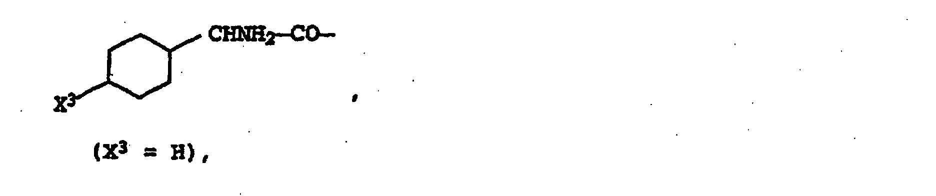 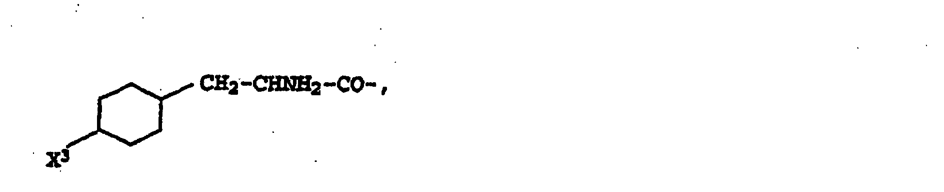 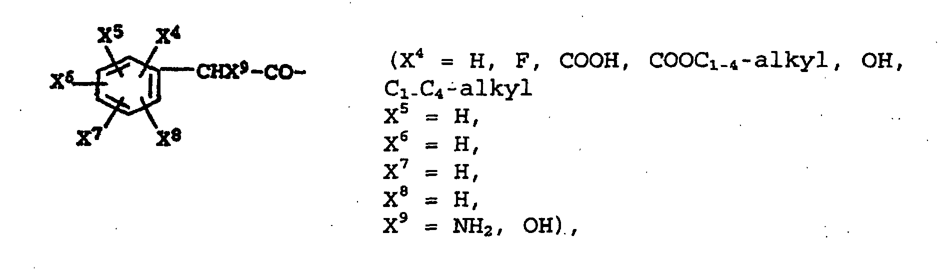 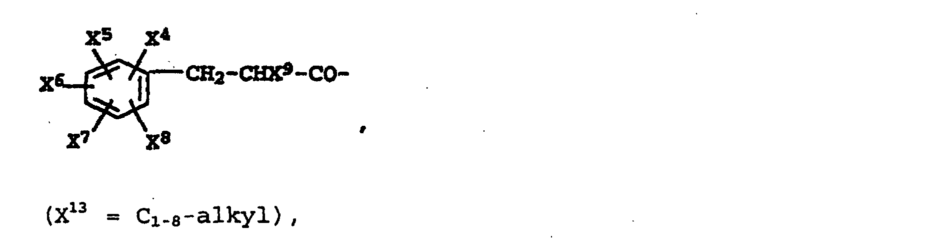
H₂N-CH₂-CO-, H₂N-CHX¹³-CO-
- wherein in all the above mentioned A radicals the α-HN group or α-NH₂ group may be monosubstituted by C₁₋₆-alkyl, phenyl-C₁₋₄-alkylene, C₁₋₆-alkylcarbonyl, C₁₋₆-alkoxycarbonyl, phenyl-C₁₋₅-alkoxycarbonyl, 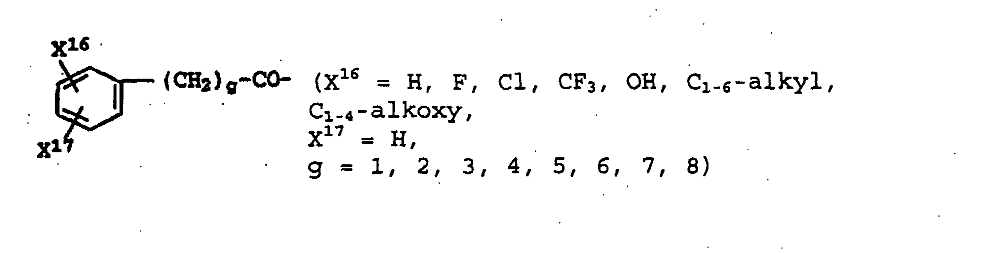 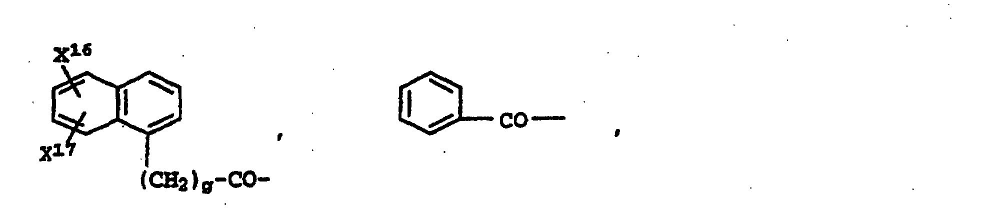 X¹⁸-O-CO-C₁₋₄-alkylene-CO-(X¹⁸ = H, C₁₋₄-alkyl),
C₁₋₁₂-alkyl-CO, C₃₋₇-cycloalkyl-CO-,
B: 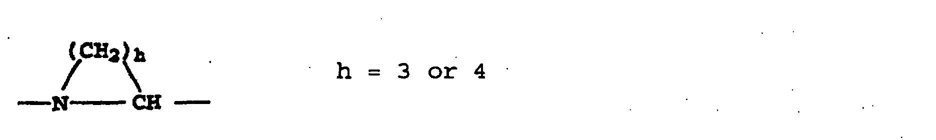
R¹: H or C₁₋₄-alkyl
D: phenylene, on which (CH₂)ₘ and (NH)ₙ are bonded in the para position to one another,
1,4-cyclohexylene
or
piperdinylene which is bonded in the 4 position to the nitrogen atom to (CH₂)ₘ, and in which the nitrogen atom itself carries the C(=NH)NHR⁴ group,
n: 0 or 1,
R⁴: H, -CO-C₁₋₆-alkyl, -CO-O-C₁₋₆-alkyl or OH,
and may also not have the following meanings:
A: 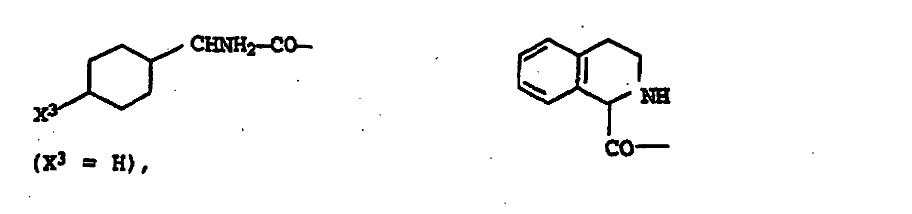 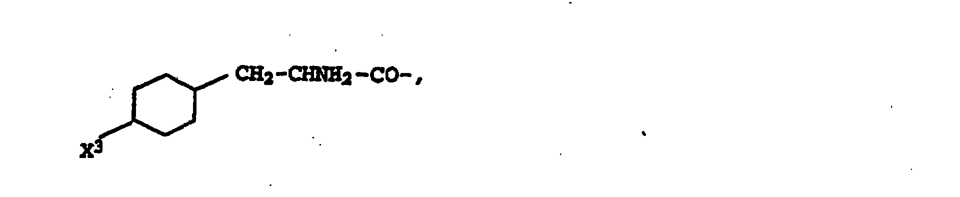 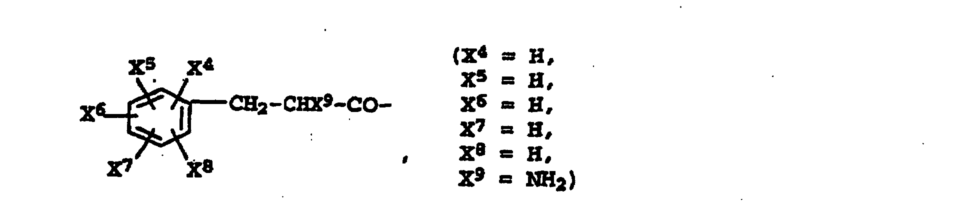 X¹² = OH, C₁₋₄-alkoxy, NH₂,
- wherein in all the abovementioned A radicals the α-NH group or α-NH₂ group may be monosubstituted or disubstituted by C₁₋₄-alkyl, X¹²OC-C₁₋₄-alkylene, X¹²OC-C₁₋₂-alkylcarbonyl, C₁₋₄-alkylcarbonyl, C₁₋₄-alkoxycarbonyl,
B: 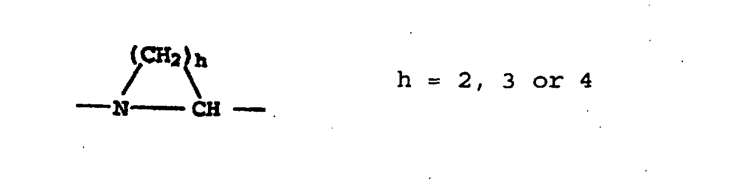 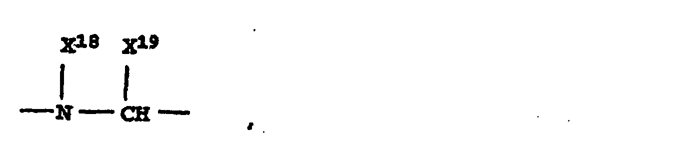 X¹⁸ = H
X¹⁹ = C₁₋₆-alkyl, benzyl)
R¹: H
D: phenylene, on which (CH₂)ₘ and (NH)ₙ are bonded in the para position to one another,
1,4-cyclohexylene,
or
piperidinylene that is bonded in the 4 position to the nitrogen atom, to (CH₂)ₘ, and in which the nitrogen atom itself carries the C(=NH)NHR⁴ group,
n: 0
R⁴: H
and may also not have the following meanings:
A: 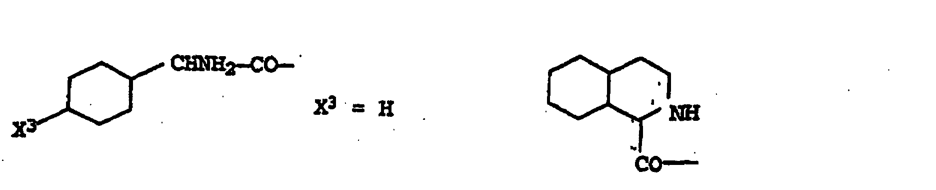 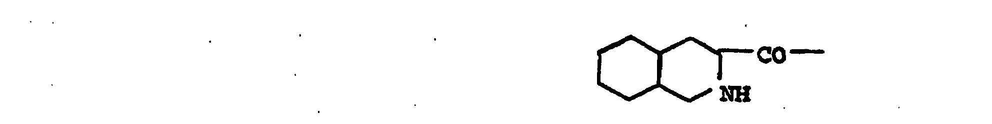 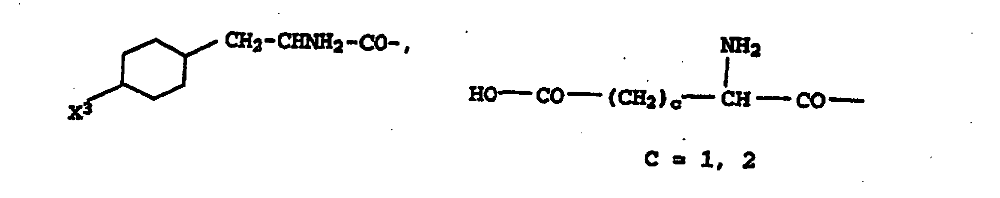 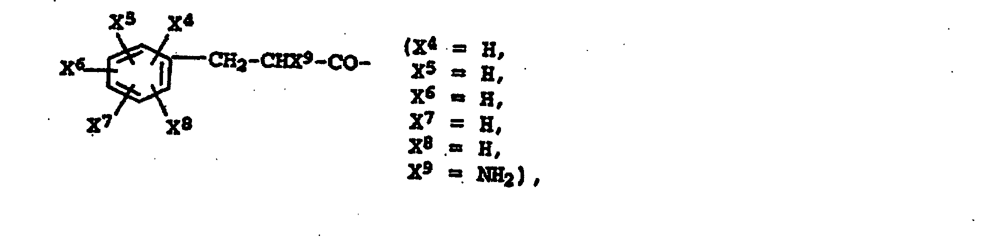
- wherein in all the abovementioned A radicals the α-NH₂ group may be monosubstituted by X¹²OC-C₁₋₃-alkylene, C₁₋₅-alkylcarbonyl, where X¹² = OH
B: 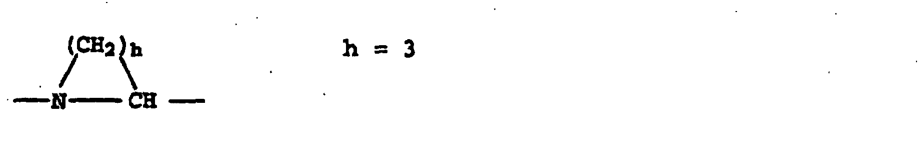
R¹: H
D: phenylene, on which (CH₂)ₘ and (NH)ₙ are bonded in the meta or para position to one another and which may be substituted in the ortho position to (CH₂)ₘ by F, pyridinylene with the amidine group in the 3 position or pyridazinylene, on which (CH₂)ₘ and (NH)ₙ are bonded in the para position to one another,
piperidinylene that is bonded in the 4 position to the nitrogen atom to (CH₂)ₘ, and in which the nitrogen atom itself carries the C(=NH)NHR⁴ group,
n: 0
R⁴: H
and does not denote N-[[1-(aminoiminomethyl)-3-piperidinyl]methyl]-1-D-phenylalanyl-L-prolinamide.

2. Compounds of the formula 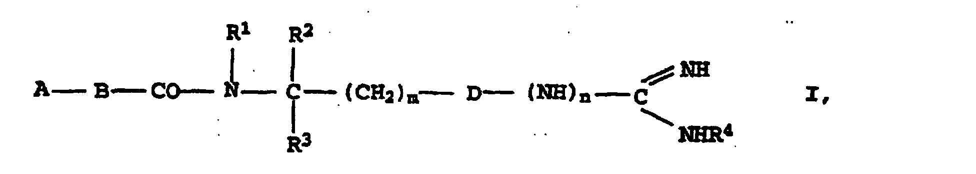 as well as their salts with physiologically compatible acids and their stereoisomers, in which the substituents have the following meanings: 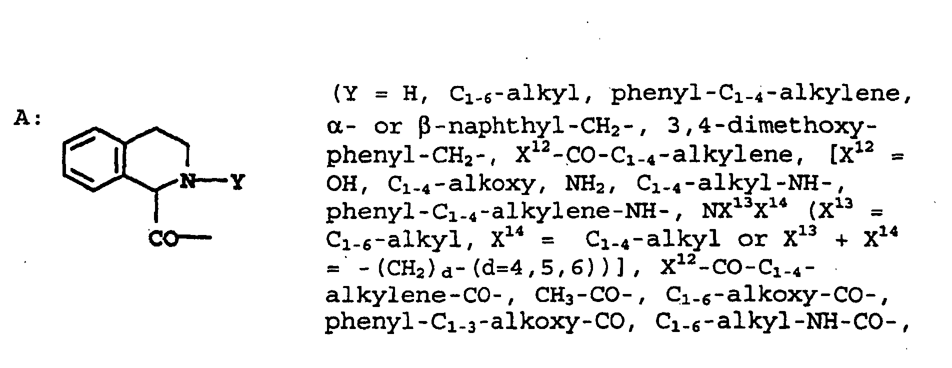 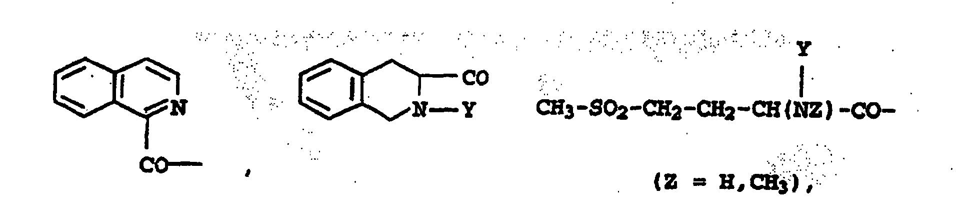 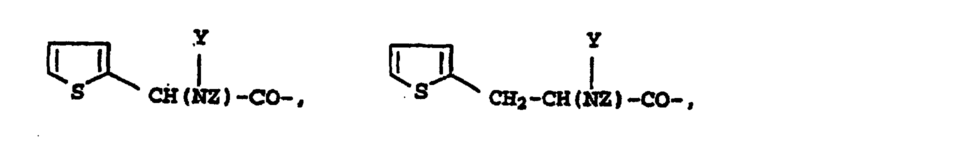 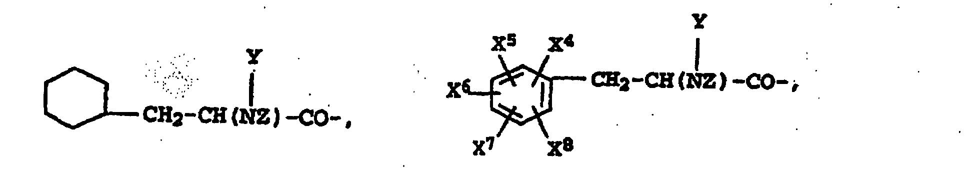 (X⁴ = H, F, Cl, Br, CF₃, C₁₋₄-alkyl, OH, OCH₃, NO₂, phenyl, X⁵ = H, F, Cl, Br, CH₃, OH, OCH₃, phenyl, X⁶ = H, F, Cl, Br, CH₃, OH, OCH₃, X⁷ = H, F, X⁸ = H, F), 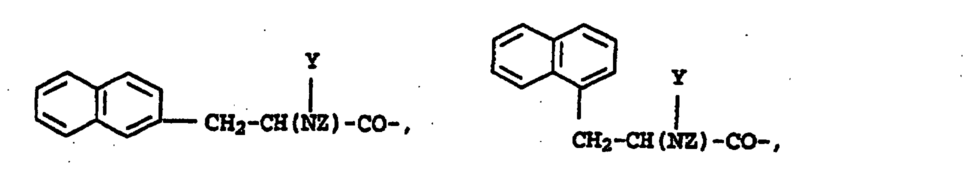 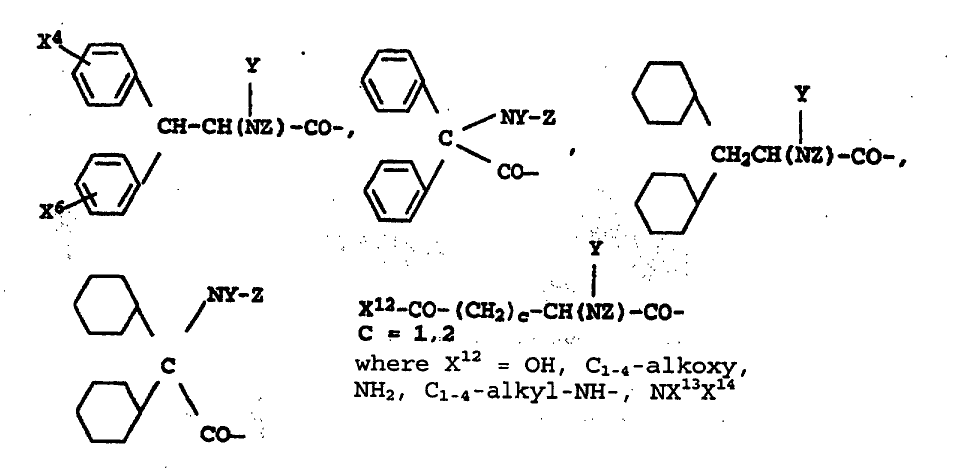 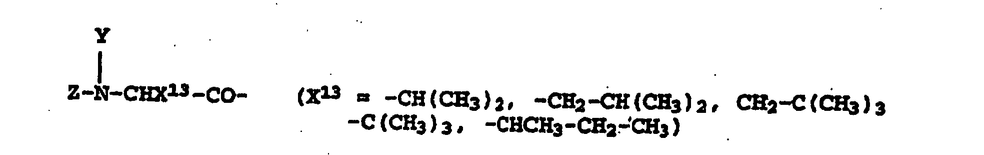 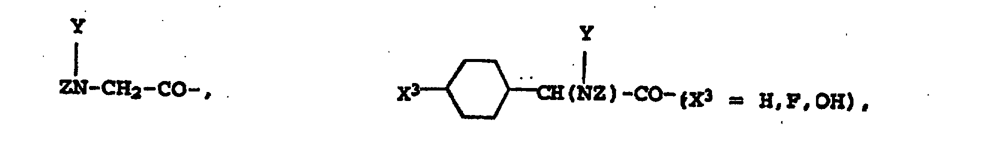 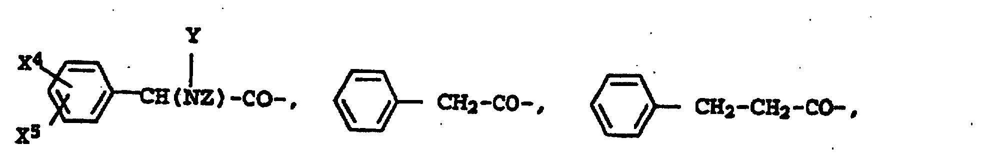 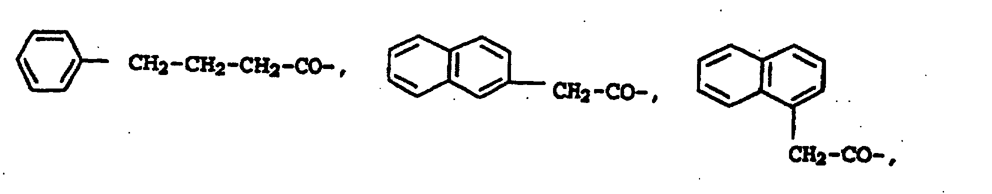 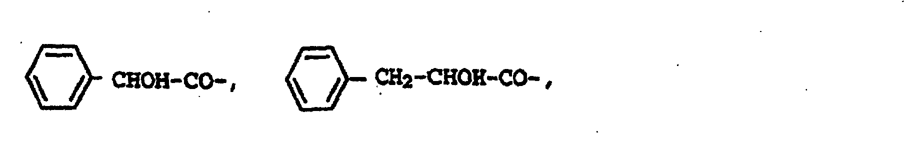 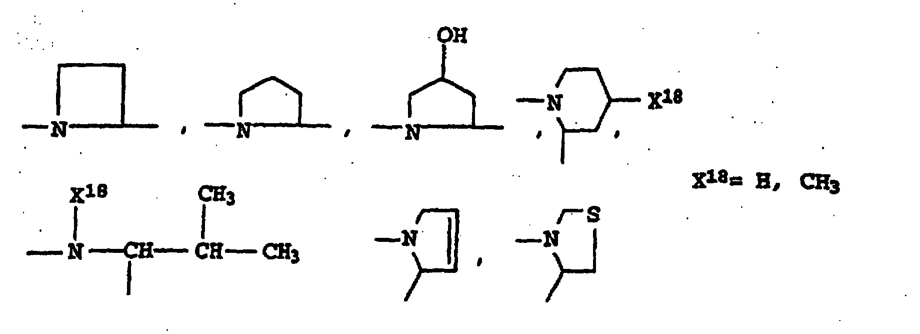
R¹: H, CH₃,
R²: H,
R³: H,
R⁴: H, OH, NH₂,
m: 0,
for -(CH₂)ₘ-D-(NH)ₙ- where n = 0 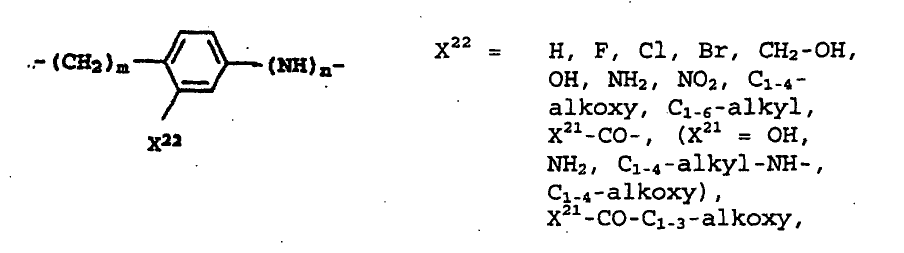 for -(CH₂)ₘ-D-(NH)ₙ- where n = 0, 1 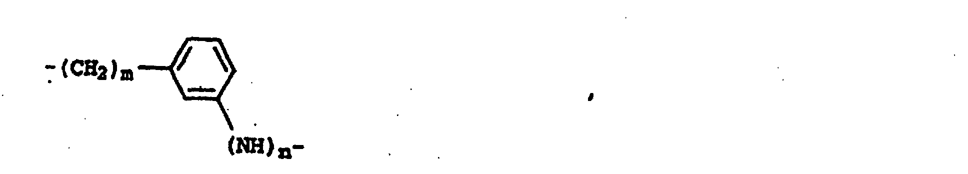 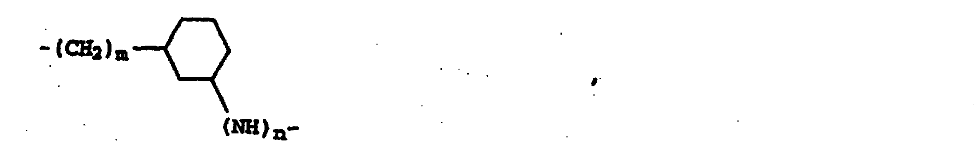 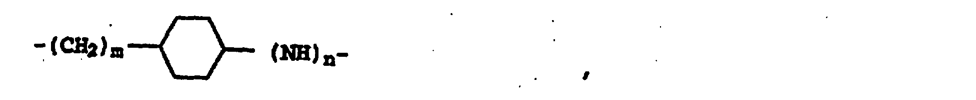 for -(CH₂)ₘ-D-(NH)ₙ- where n = 0, 1 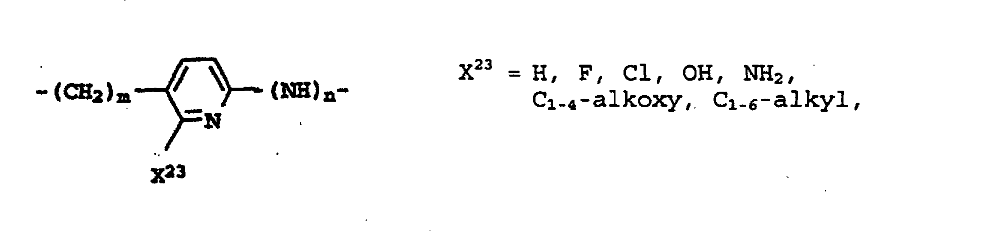 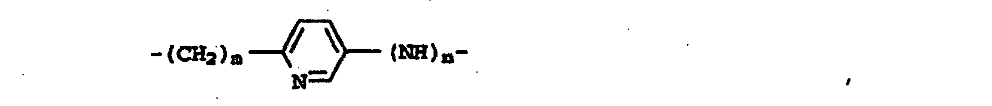 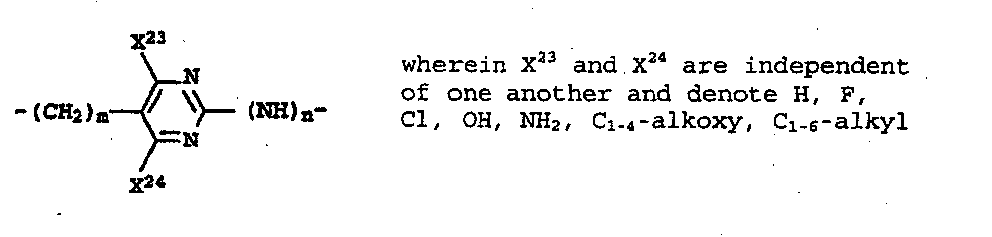 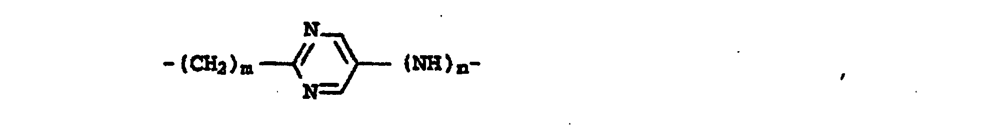 for -(CH₂)ₘ-D-(NH)ₙ- where n = 0: 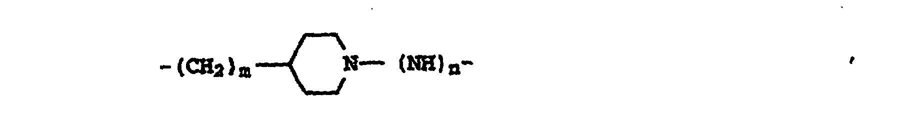 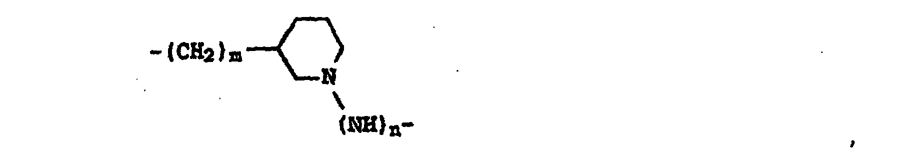 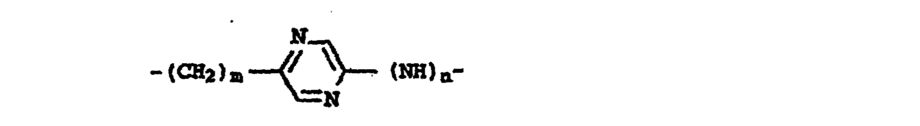 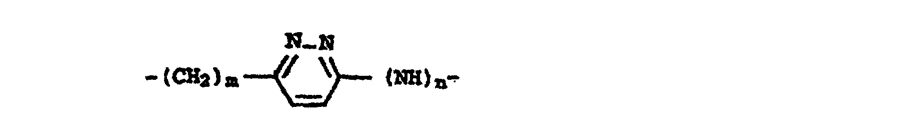 wherein R³, A, B and D, for R⁴ = OH or H may not simultaneously have the following meanings
A: for R⁴ = OH or H
Y = H, C₁₋₆-alkyl, phenyl-C₁₋₄-alkylene,
CH₃-CO-, C₁₋₆-alkoxy-CO-, phenyl-C₁₋₃-alkoxy-CO-, 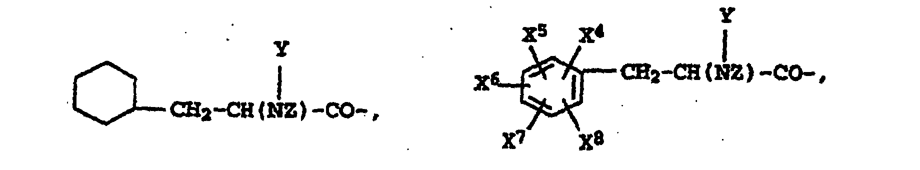
(X⁴ = H, F, C₁₋₄-alkyl,
X⁵ = H,
X⁶ = H,
X⁷ = H,
X⁸ = H), z = H 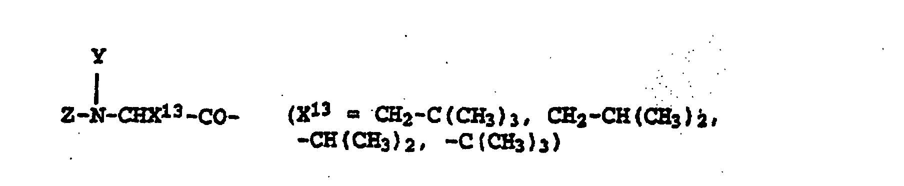 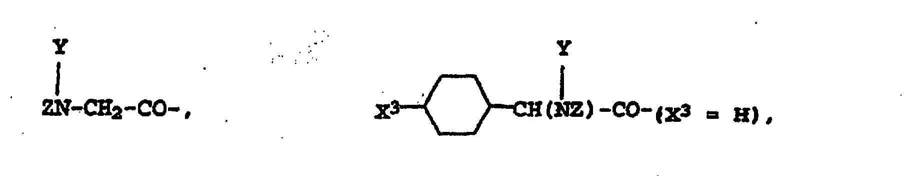 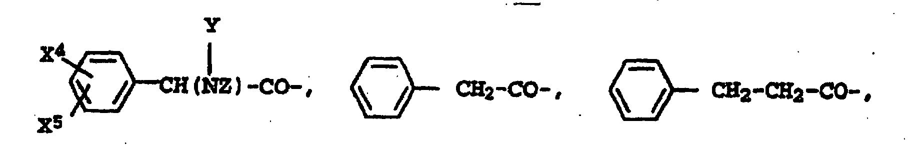 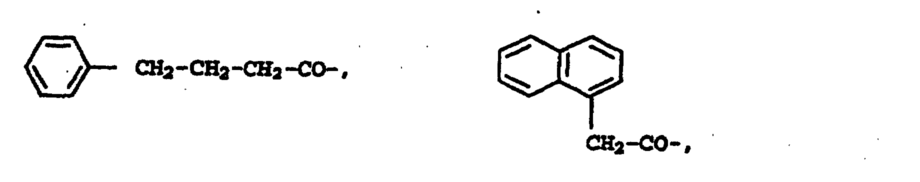 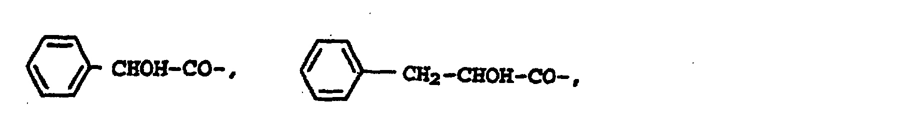 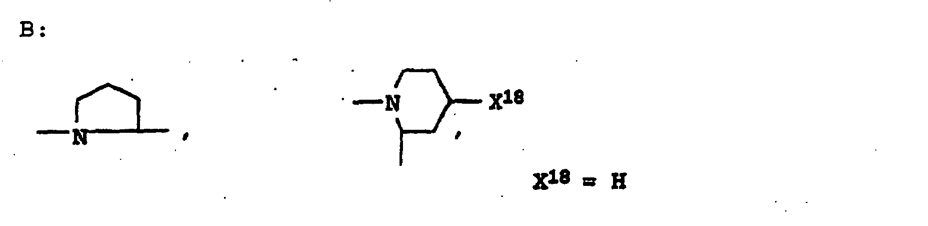
R¹: H, CH₃
for -(CH₂)ₘ-D-(NH)ₙ- where m = 0, n = 0 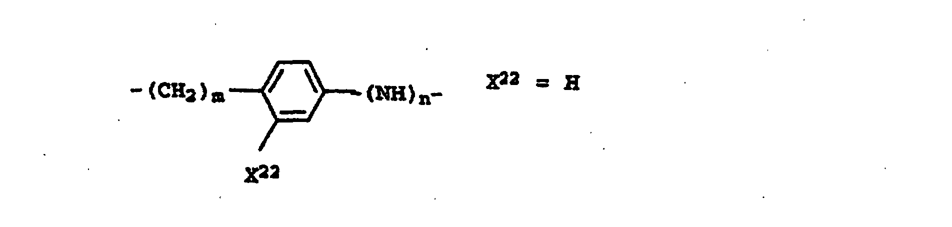 for -(CH₂)ₘ-D-(NH)ₙ- where m = 0, n = 0, 1 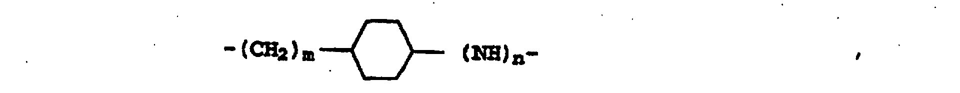 for -(CH₂)ₘ-D-(NH)ₙ- where m = 0, n = 0: 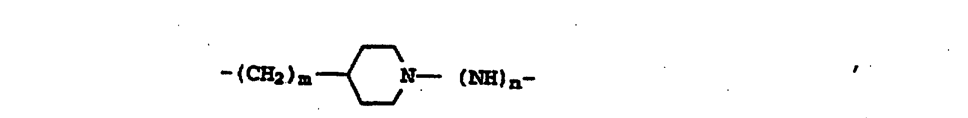 and
wherein for R⁴ = H A, B, D, R³ may not simultaneously have the following meanings:
A: 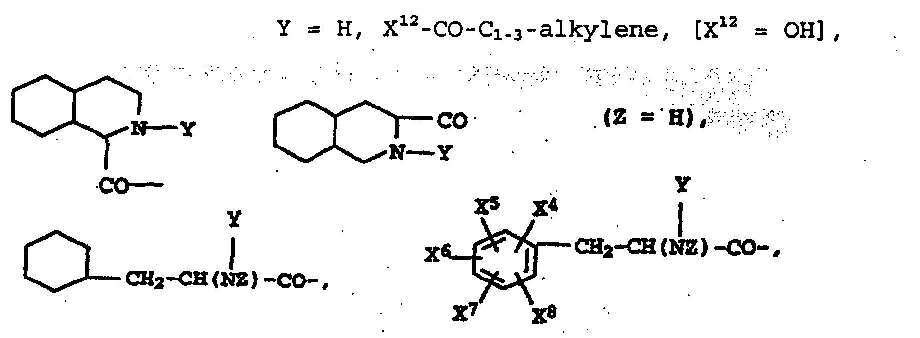
(X⁴ = H,
X⁵ = H,
X⁶ = H,
X⁷ = H,
X⁸ = H), 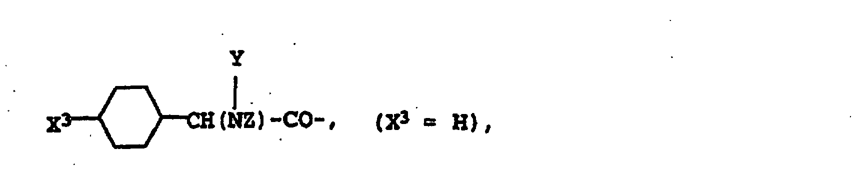 B: 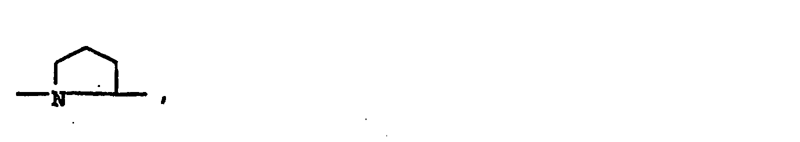
R¹: H
m: 0
for -(CH₂)ₘ-D-(NH)ₙ- where m = 0, n = 0 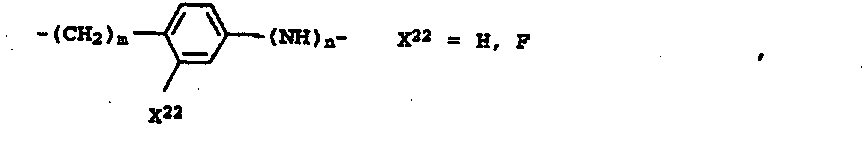 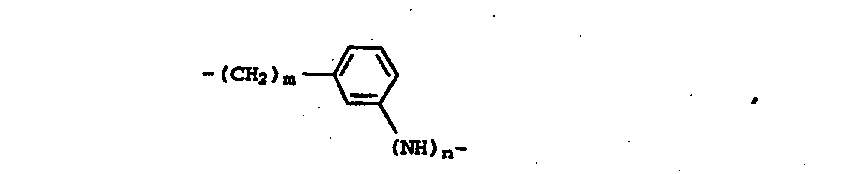 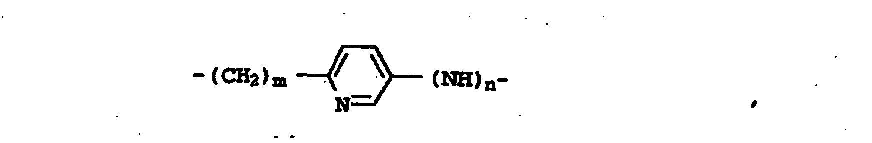 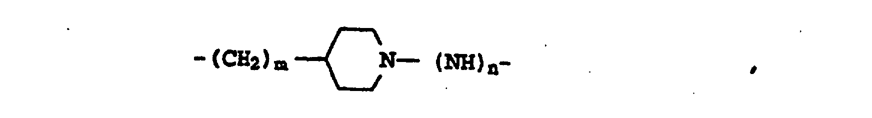 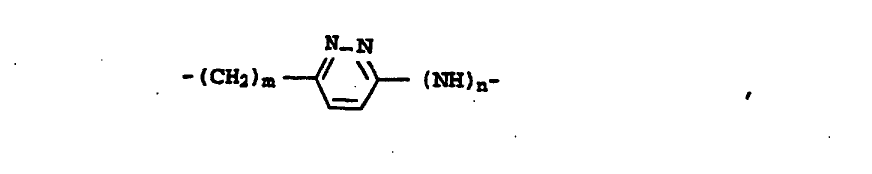 and similarly A, B, D, R¹, R⁴ may not denote
A: (Y = H, X¹²-CO-C₁₋₄-alkylene, [X¹² = OH, C₁₋₄-alkoxy, NH₂] ; X¹²-CO-C₁₋₄-alkylene-CO-, CH₃-CO-), 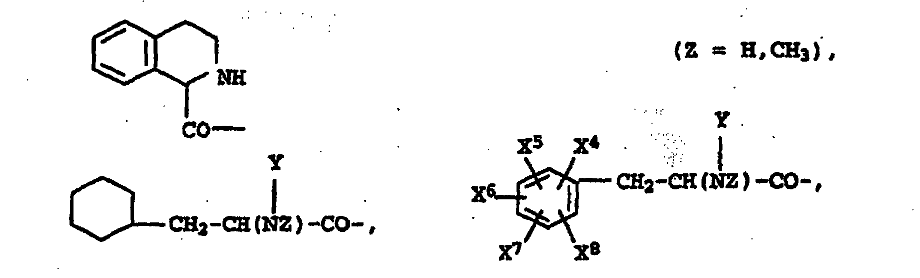
(X⁴ = H,
X⁵ = H,
X⁶ = H,
X⁷ = H,
X⁸ = H), 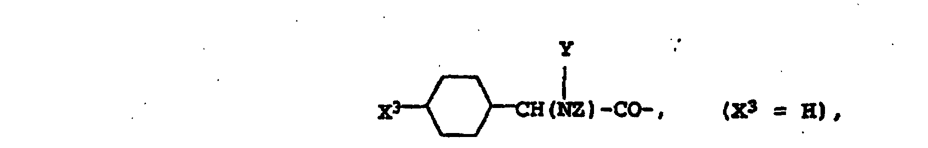
B: 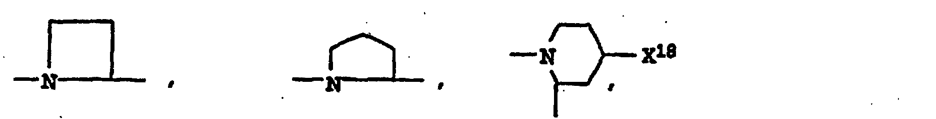 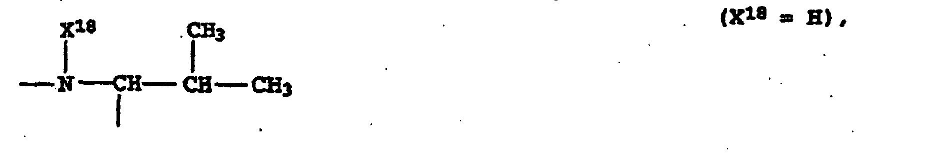
R¹: H
R⁴: H
for -(CH₂)ₘ-D-(NH)ₙ- where n = 0 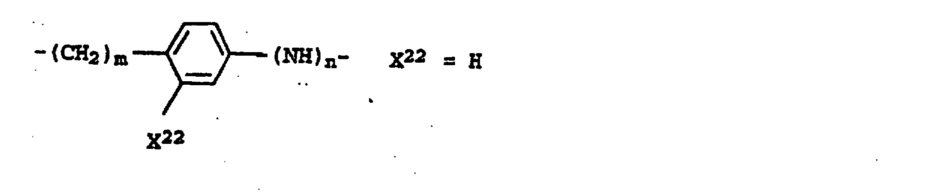 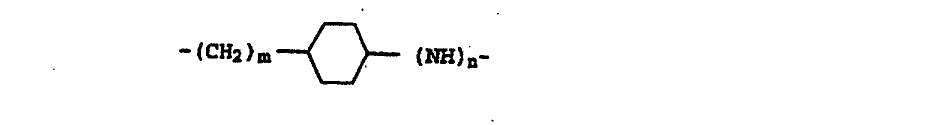 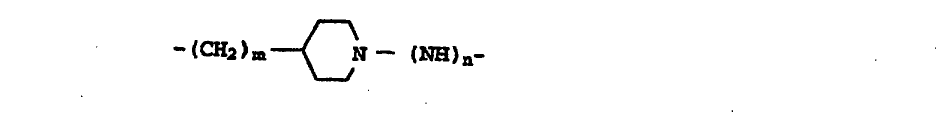 and does not denote N-[[1-(aminoiminomethyl)-3-piperidinyl]methyl]-1-D-phenylalanyl-L-prolinamide.

3. Compounds of the formula 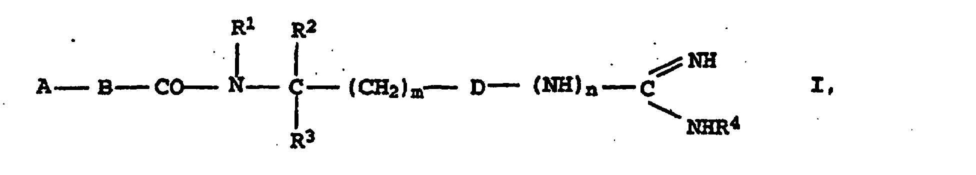 as well as their salts with physiologically compatible acids and their stereoisomers, in which the substituents have the following meanings: 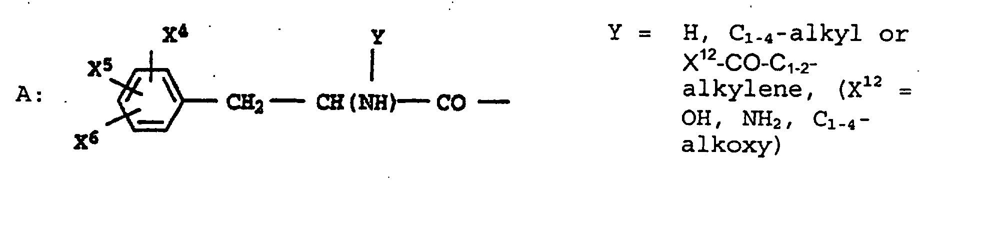
X⁴ = H, F, Cl, CH₃, OCH₃ or Br
X⁵ = H, Cl, CH₃, or OCH₃, OC₂H₅
X⁶ = H or OCH₃, 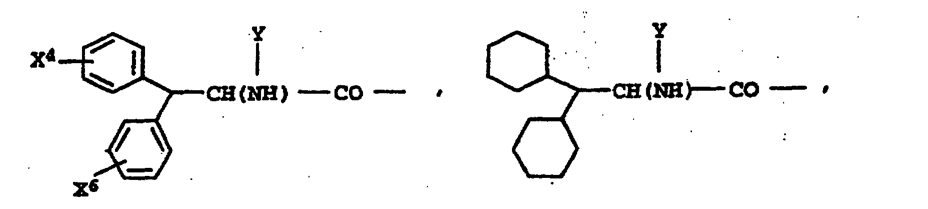 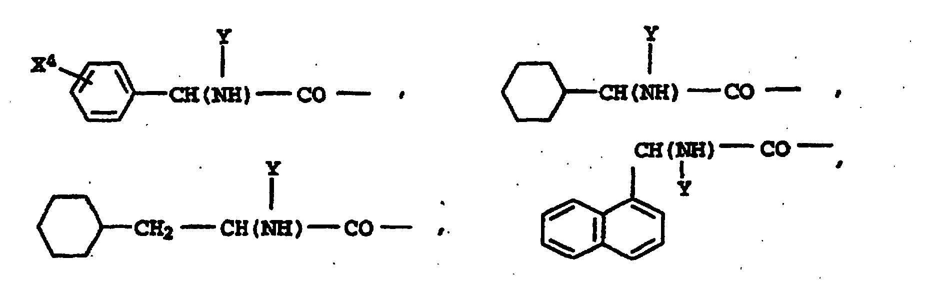 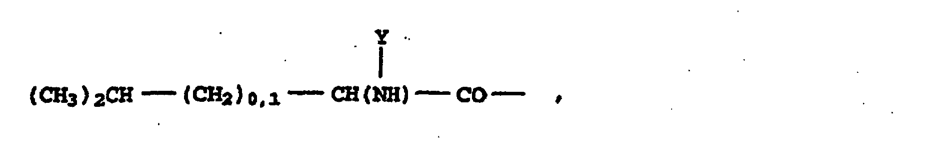 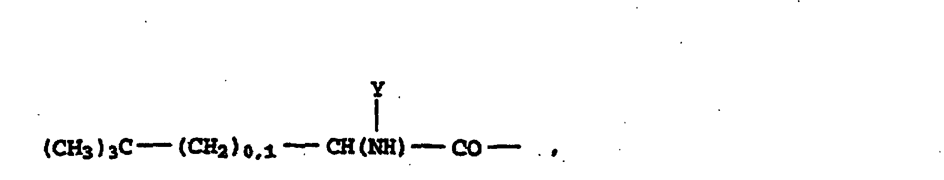 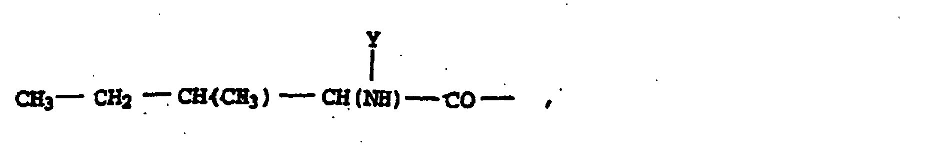 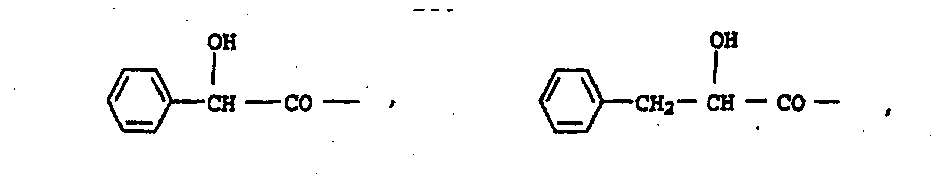 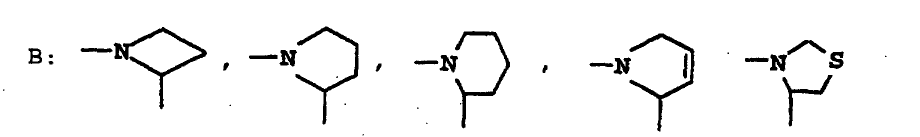
R¹ = H
R², R³ = H
for -(CH₂)ₘ-D-(NH)ₙ- where m = 0, n = 0 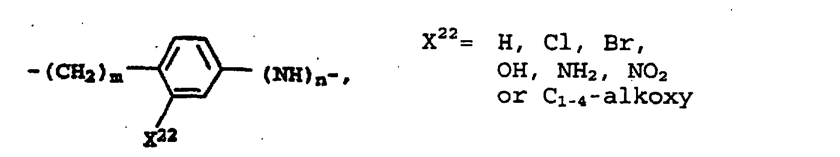 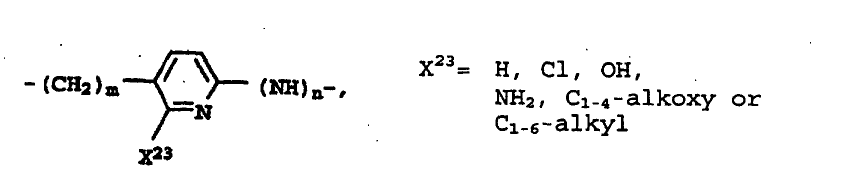 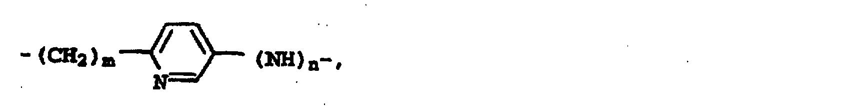 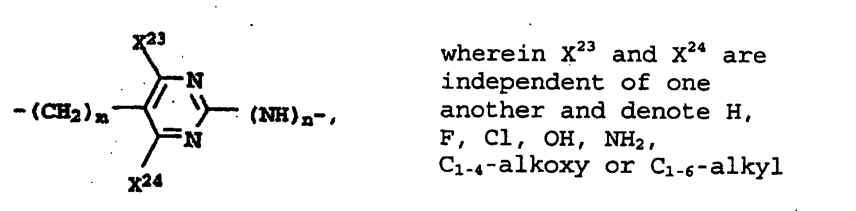 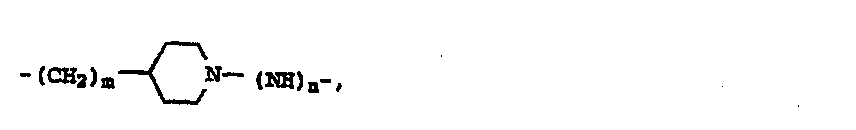 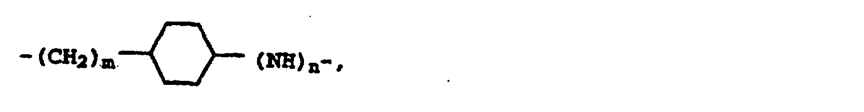 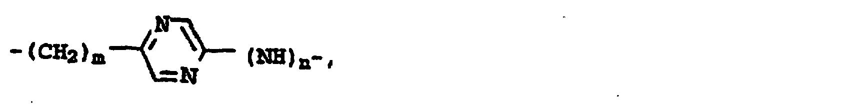 for -(CH₂)ₘ-D-(NH)ₙ- where m = 0, n = 1 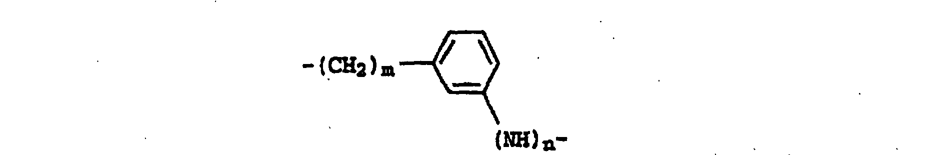 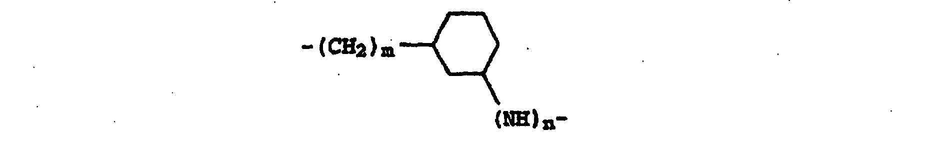
R⁴: H, OH,
wherein A, B, D and R⁴ do not simultaneously have the following meanings: 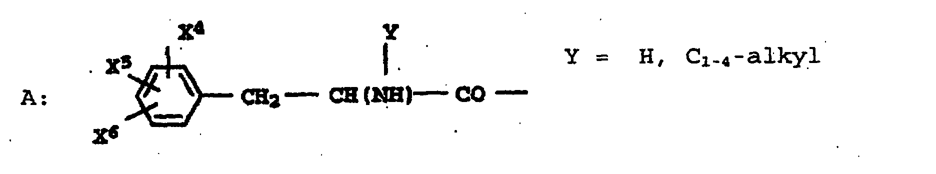
X₄ = H, F, CH₃
X₅ = H
X₆ = H 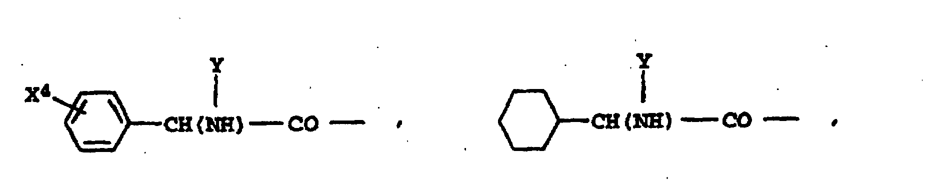 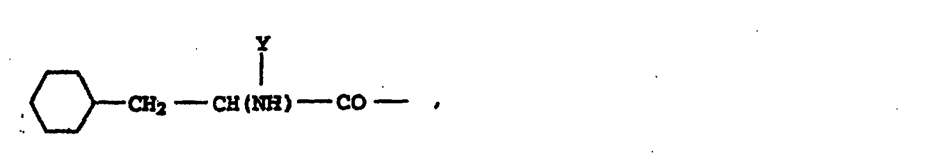 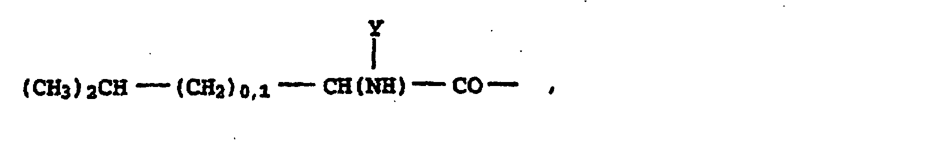 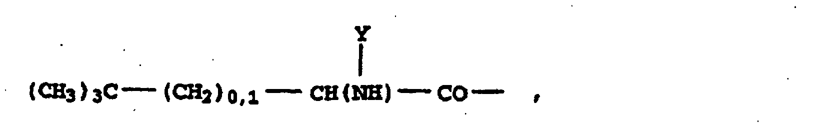 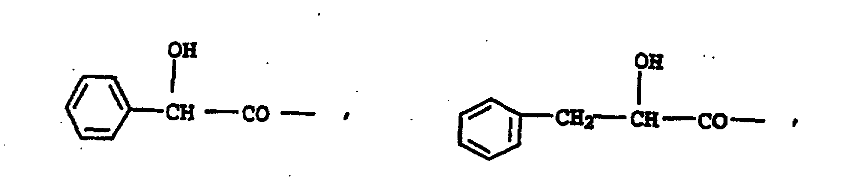 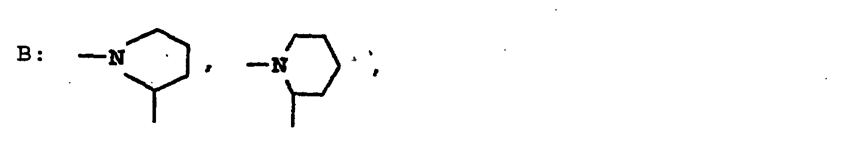
for -(CH₂)ₘ-D-(NH)ₙ- where m = 0, n = 0 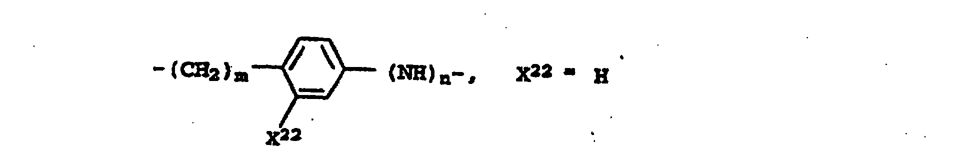 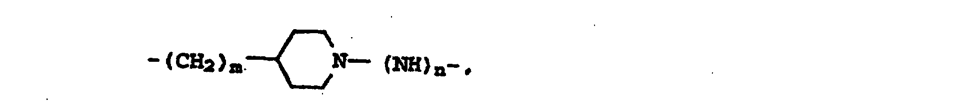 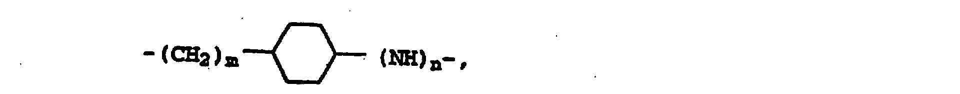
R⁴: H, OH
and for
R⁴ = H
A, B and D may not simultaneously have the following meanings: 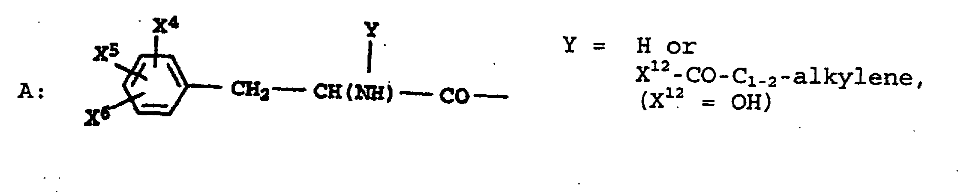
X₄ = H
X₅ = H
X₆ = H 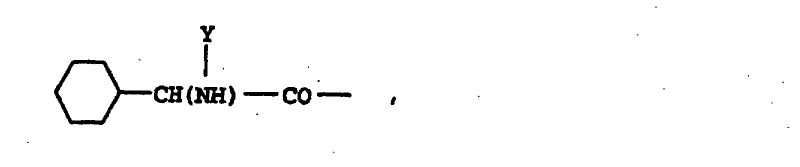 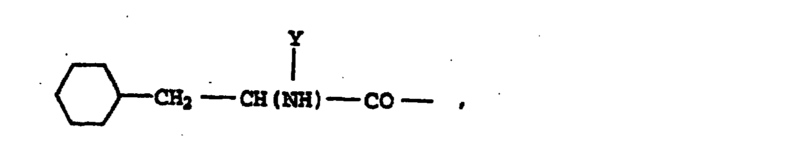 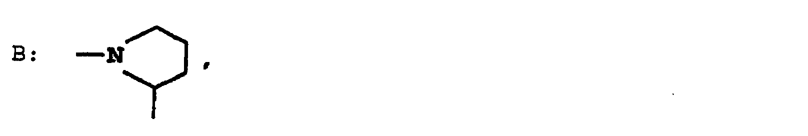
for -(CH₂)ₘ-D-(NH)ₙ- where m = 0, n = 0 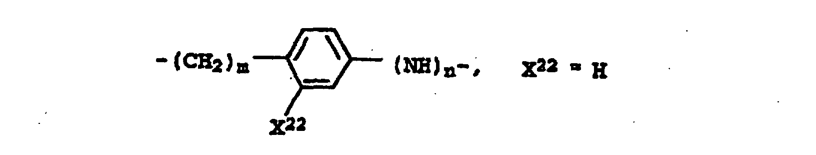 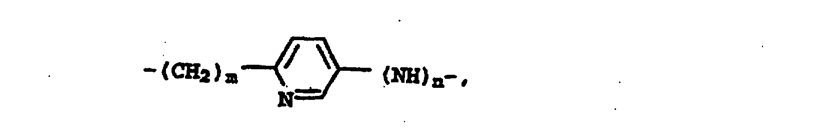 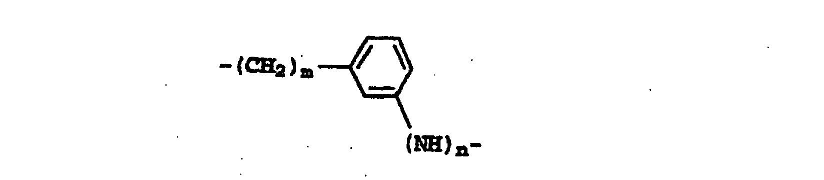 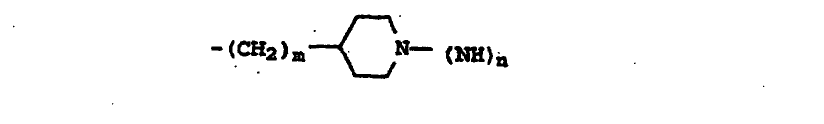 and A, B, D, R¹ as well as for R⁴ = H may not have the following meanings: 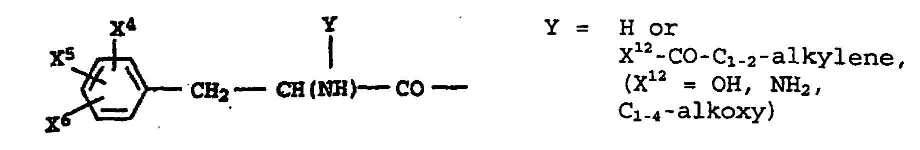
X₄ = H
X₅ = H
X₆ = H 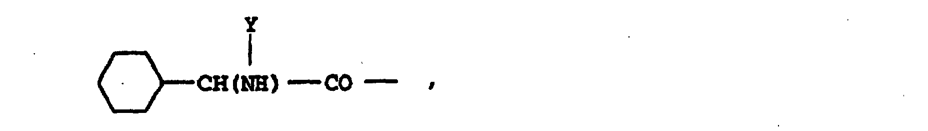 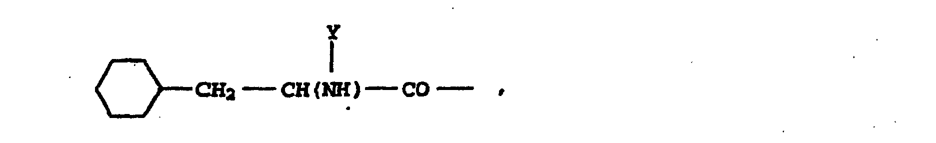 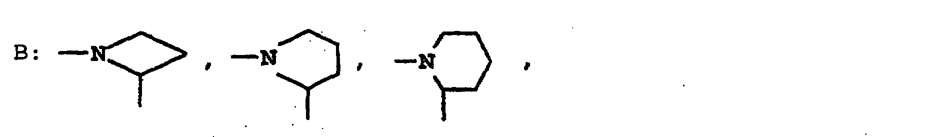
for -(CH₂)ₘ-D-(NH)ₙ- where m = 0, n = 0

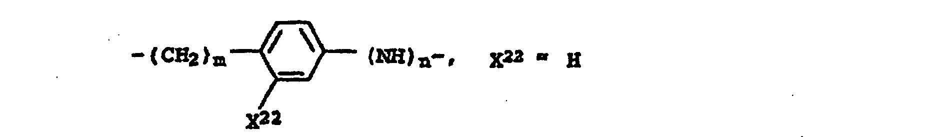

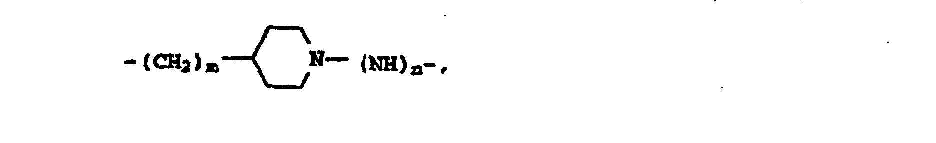

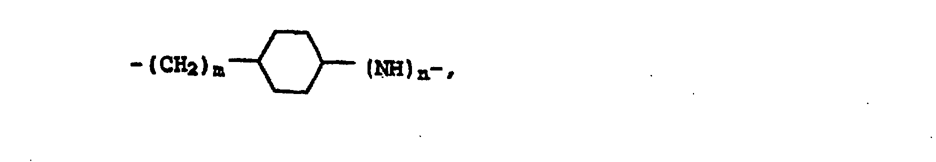

4. Compounds of the formula I according to one of claims 1 to 3 for use in controlling diseases.

5. Compounds of the formula II 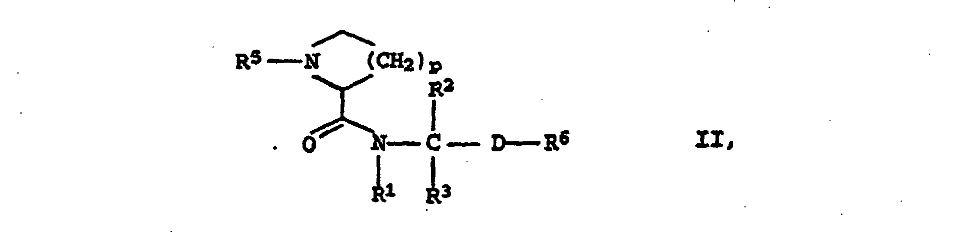 in which R¹, R², R³ and D have the meanings given for formula I, and
R⁵: denotes H, C₁₋₄-alkoxy-CO- or
phenyl-C₁₋₃-alkoxy-CO-,
R⁶: denotes a cyano, amidino or guanidino radical in the m-position or p-position to C(R², R³) and
p: denotes 1, 2 or 3.

## Revendications

1. Composés de formule 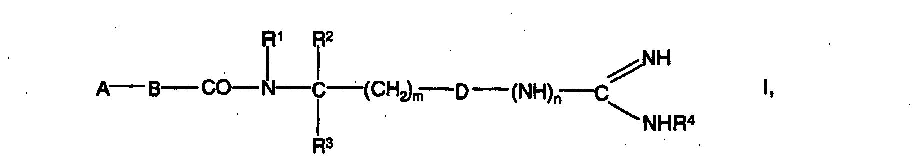 ainsi que leurs sels avec des acides physiologiquement acceptables et leurs stéréoisomères, où les substituants possèdent les significations suivantes:
A: 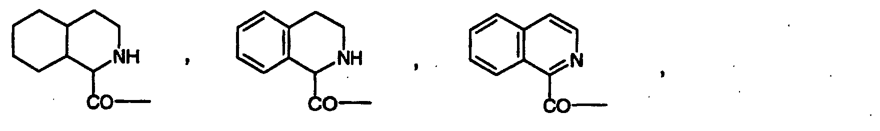 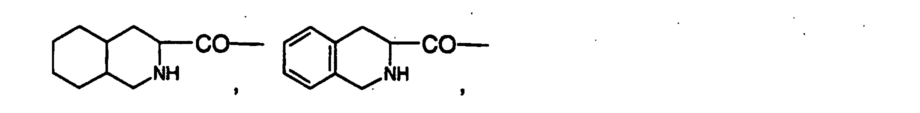 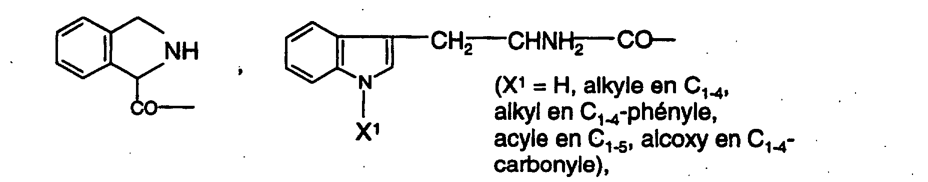 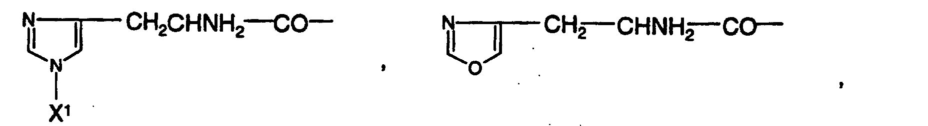 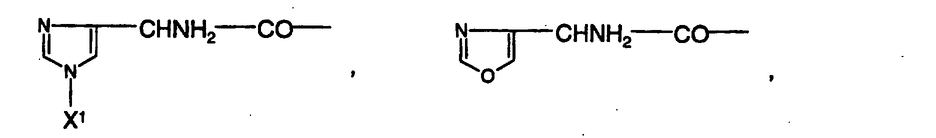 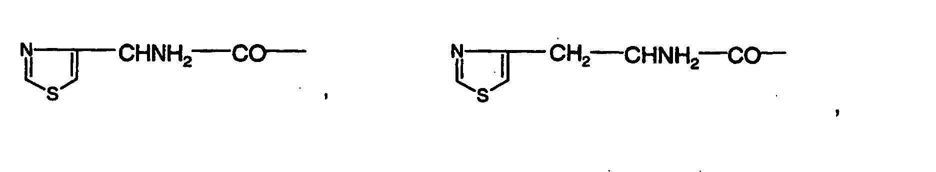 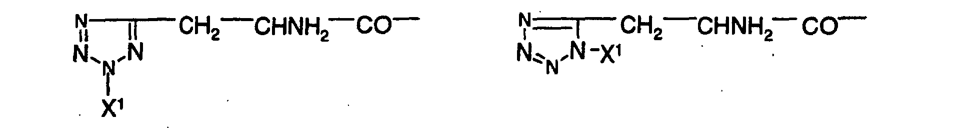 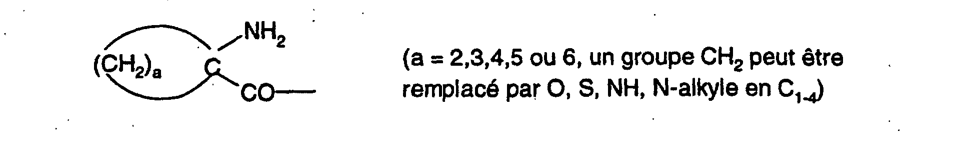 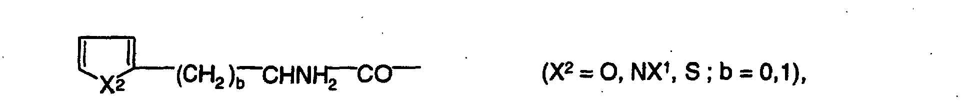 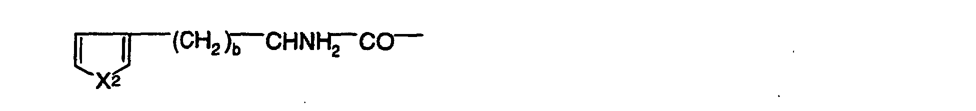 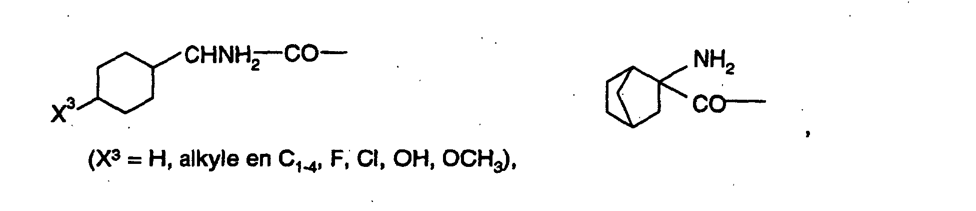 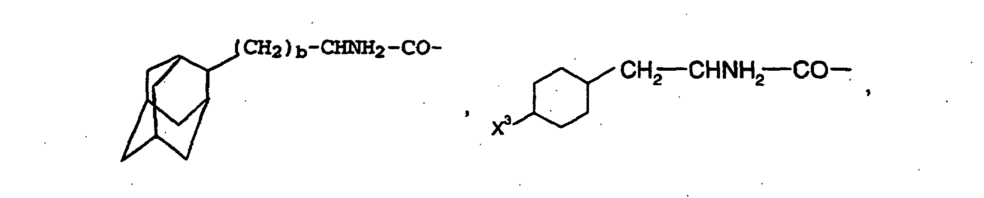 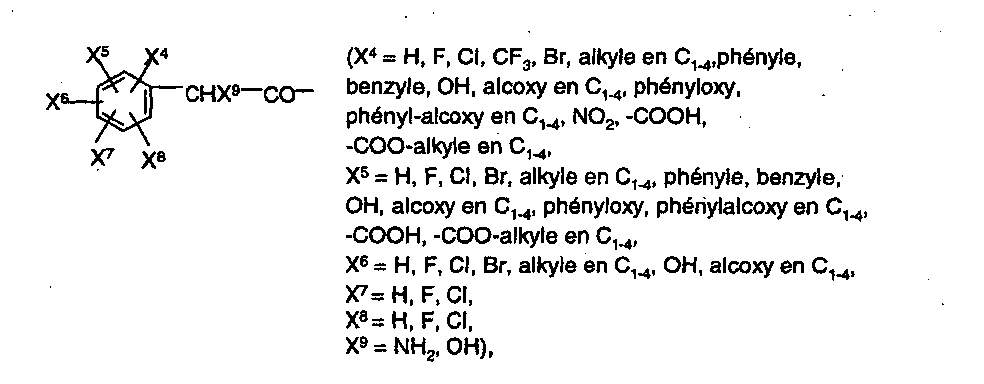 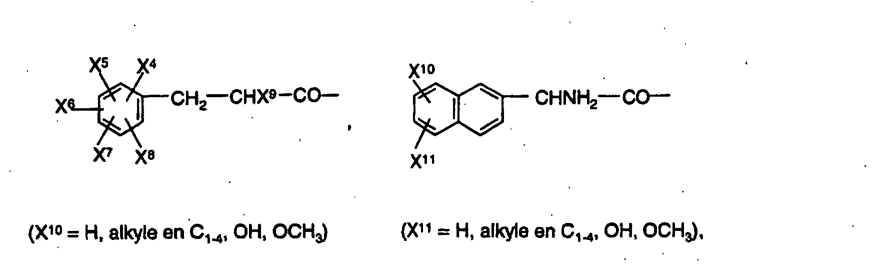 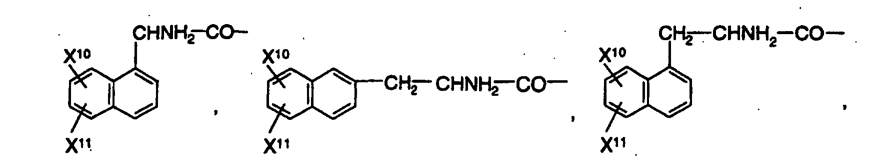 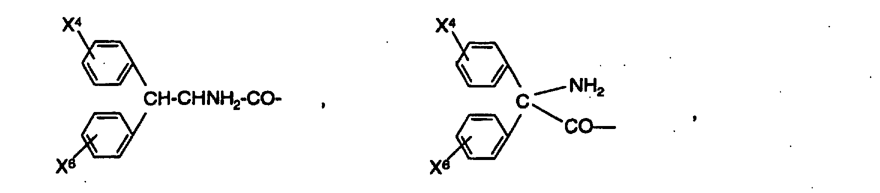 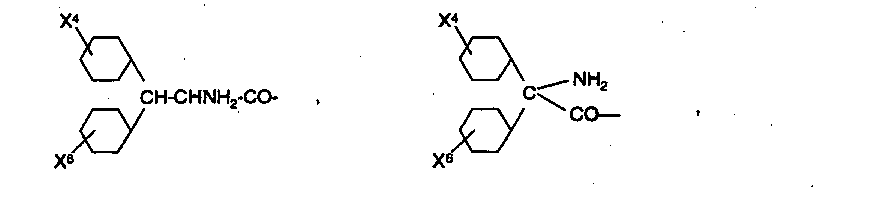 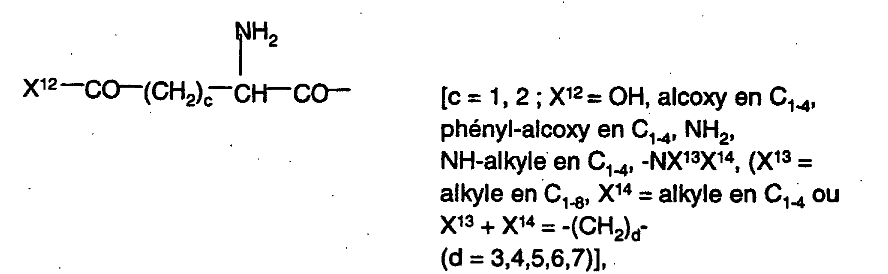
X¹³―SO₂―CH₂―CH₂―CHNH₂―CO― ,
H₂N-CH₂-CO-, H₂N-CHX¹³-CO-
où dans tous les restes A cités précédemment le groupe α-NH- ou α-NH₂-peut être mono- ou di-substitué par alkyle en C₁₋₁₂, phényl-alkylène en C₁₋₄, X¹²OC-alkylène en C₁₋₆, X¹²OC-alkyle en C₁₋₆-carbonyle, α- ou β-naphtyl-alkylène en C₁₋₄, alkyle en C₁₋₁₂-carbonyle, phényl-alkyle en C₁₋₄-carbonyle, alcoxy en C₁₋₇-carbonyle, phényl-alcoxy en C₁₋₅-carbonyle, α ou β-naphtyl-alkyle en C₁₋₄-carbonyle, alkyle en C₁₋₆-aminocarbonyle ou phényl-alkyle en C₁₋₄-aminocarbonyle,
en outre A : X¹-NH-CH₂-CH₂-CO, X¹-NH-CH₂-CH₂-CH₂-CO- 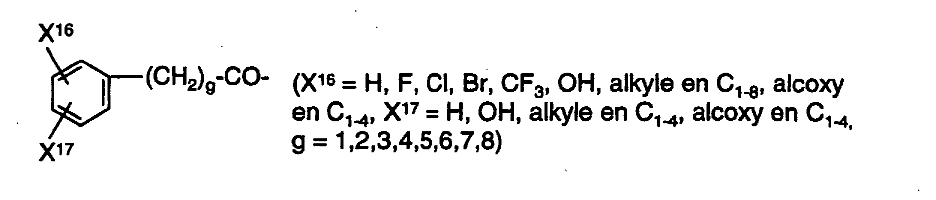 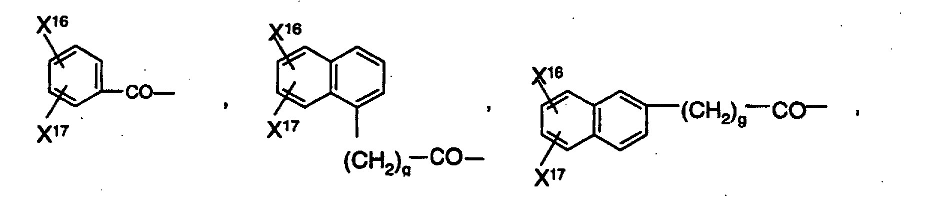 X¹⁸-O-CO-alkylène en C₁₋₄-CO- (X¹⁸ = H, alkyle en C₁₋₄),
alkyle en C₁₋₁₂-CO-, alkyle en C₁₋₁₀-NH-CO-, phényl-alkylène- en C₁₋₄-NH-CO-; α- ou β-naphtyl-CO- ou cycloalkyle en C₃₋₇-CO-, 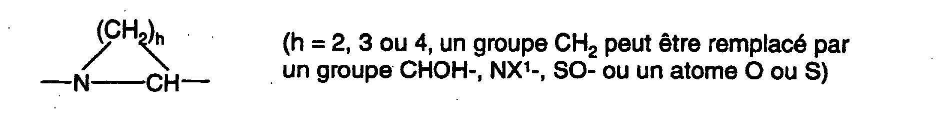 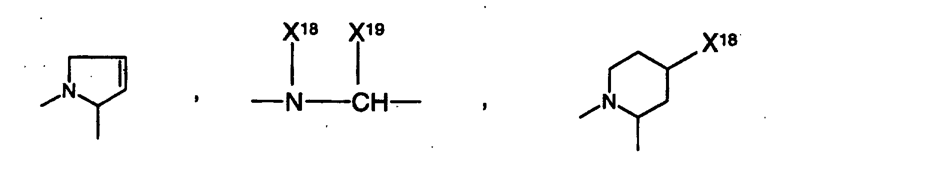 X¹⁸ = H ou alkyle en C₁₋₄,
X¹⁹ = H, alkyle en C₁₋₆, phényle, benzyle, cyclohexyle ou cyclohexylméthyle)
R¹ : H ou alkyle en C₁₋₄,
R² : H,
R³ : H,
m : 0,
D : phénylène auquel (CH₂)ₘ et (NH)ₙ sont liés en position para ou méta l'un par rapport à l'autre et qui, en position ortho par rapport à (CH₂)ₘ, peut être substitué par F, Cl, Br, HO-CH₂-, OH, NH₂, NO₂, alcoxy en C₁₋₄, alkyle en C₁₋₆ ou COX²¹ (X²¹ = H, alkyle en C₁₋₄, alcoxy en C₁₋₄, OH, NH₂, NH-alkyle en C₁₋₄), -O-(CH₂)₁₋₃-CO-X²¹ ou -(CH₂)₁₋₃-CO-X²¹, pyridinylène, pyrimidinylène, pyrazinylène ou pyridazinylène, auxquels (CH₂)ₘ et (NH)ₙ peuvent être liés en position para ou méta l'un par rapport à l'autre et qui, en position ortho par rapport à (CH₂)ₘ, peuvent être substitués par F, Cl, Br, OH, NH₂, alcoxy en C₁₋₄ ou alkyle en C₁₋₆, 1,4- ou 1,3-cyclohexylène dans lequel un groupe CH₂ peut être remplacé en position ortho par rapport à (CH₂)ₘ par NH, O, S ou SO
ou
pipéridinylène qui est lié à (CH₂)ₘ en position 3 ou 4 par rapport à l'azote, et dans lequel l'atome d'azote lui-même porte le groupe C(=NH)NHR⁴,
n : 0 ou 1,
R⁴ : H, -CO-alkyle en C₁₋₂₀, -CO-O-alkyle en C₁₋₂₀, OH ou NH₂,
où A, B, D, R¹, R⁴, n ne peuvent pas revêtir simultanément les significations suivantes : 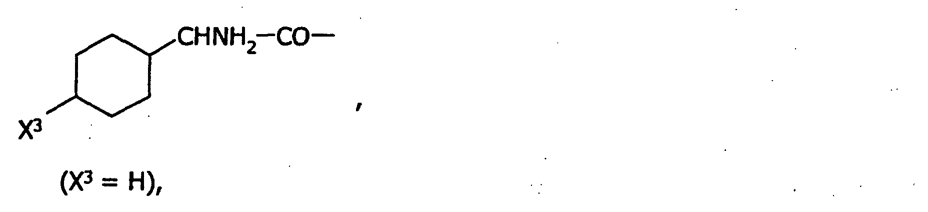 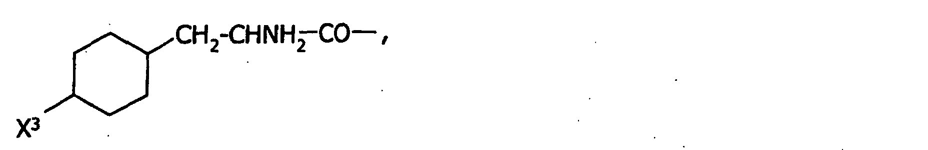 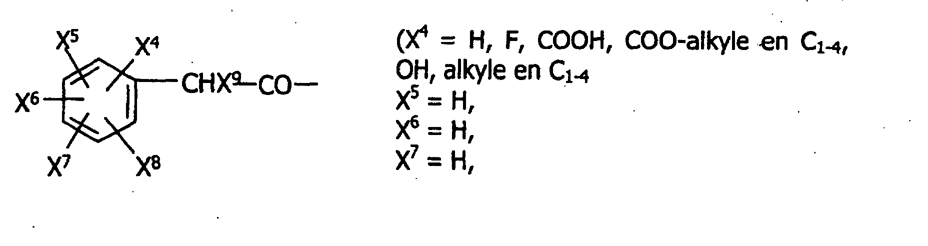 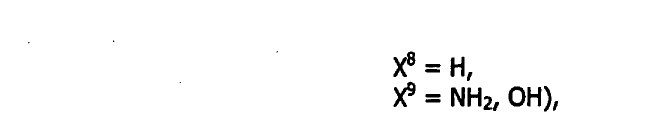 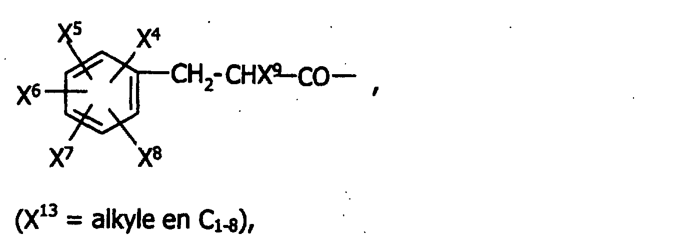
H₂N-CH₂-CO-, H₂N-CHX¹³-CO-
- où, dans tous les restes A cités précédemment, le groupe α-NH ou α-NH₂ peut être monosubstitué par alkyle en C₁₋₆, phényl-alkylène en C₁₋₄, alkyle en C₁₋₆-carbonyle, alcoxy en C₁₋₆-carbonyle, phényl-alcoxy en C₁₋₅-carbonyle, 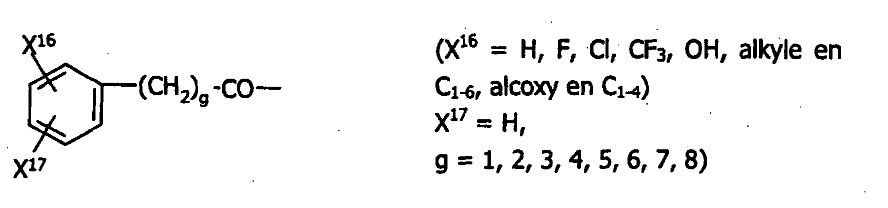 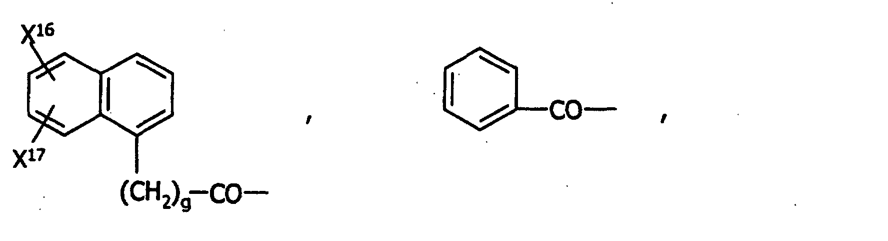 X¹⁸-O-CO-alkylène en C₁₋₄-CO- (X¹⁸ = H, alkyle en C₁₋₄),
alkyle en C₁₋₁₂-CO-, cycloalkyle en C₃₋₇-CO-, 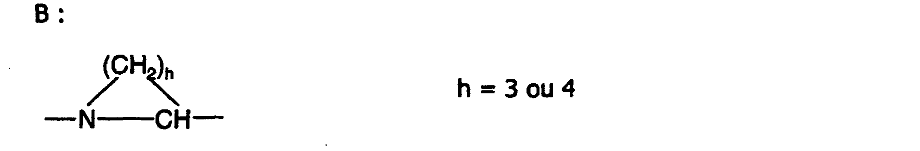 R¹ : H ou alkyle en C₁₋₄
D : phénylène, aulequel (CH₂)ₘ et (NH)ₙ sont liés en position para l'un par rapport à l'autre
1,4-cyclohexylène
ou
pipéridinylène qui est lié à (CH₂)ₘ en position 4 par rapport à l'azote et dans lequel l'atome d'azote lui-même porte le groupe C(=NH)NHR⁴,
n : 0 ou 1,
R⁴ : H, -CO-alkyle en C₁₋₆, -CO-O-alkyle en C₁₋₆ ou OH
et ne doit pas non plus revêtir la signification suivante :
A: 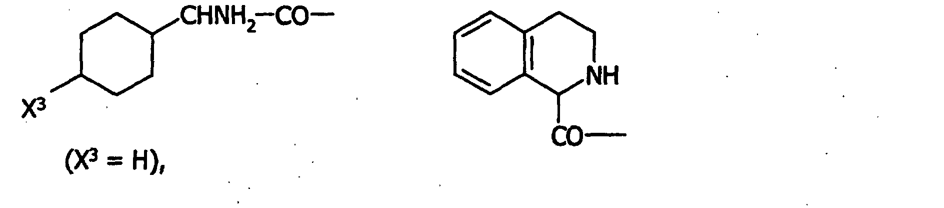 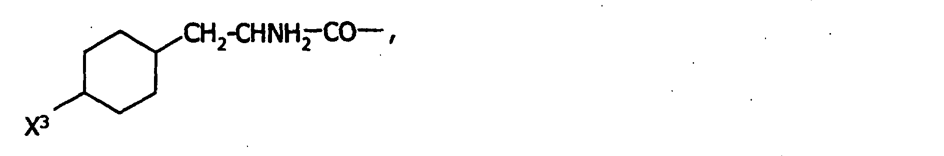 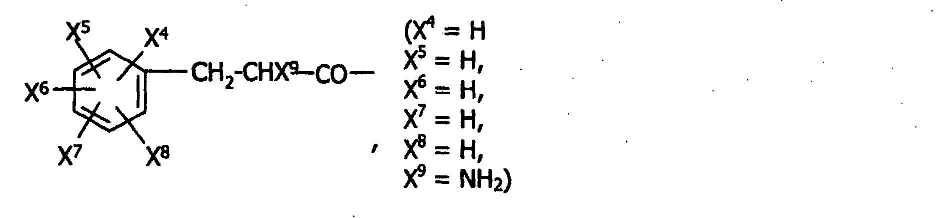 X¹² = OH, alcoxy en C₁₋₄, NH₂,
- où, dans tous les restes A cités précédemment, le groupe α-NH ou α-NH₂ peut être mono- ou di-substitué par alkyle en C₁₋₄, X¹²OC-alkylène en C₁₋₄, X¹²OC-alkyle en C₁₋₂-carbonyle, alkyle en C₁₋₄-carbonyle, alcoxy en C₁₋₄-carbonyle,
B: 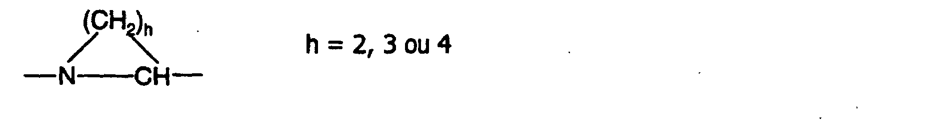 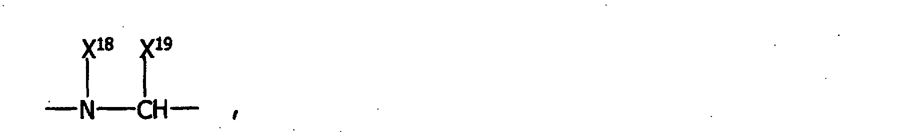 X¹⁸ = H
X¹⁹ = alkyle en C₁₋₆, benzyle)
R¹ : H
D : phénylène, auquel (CH₂)ₘ et (NH)ₙ sont liés en position para l'un par rapport à l'autre
1,4-cyclohexylène
ou
pipéridinylène qui est lié à (CH₂)ₘ en position 4 par rapport à l'azote et dans lequel l'atome d'azote lui-même porte le groupe C(=NH)NHR⁴,
n : 0
R⁴ : H
et ne doit pas non plus revêtir la signification suivante: 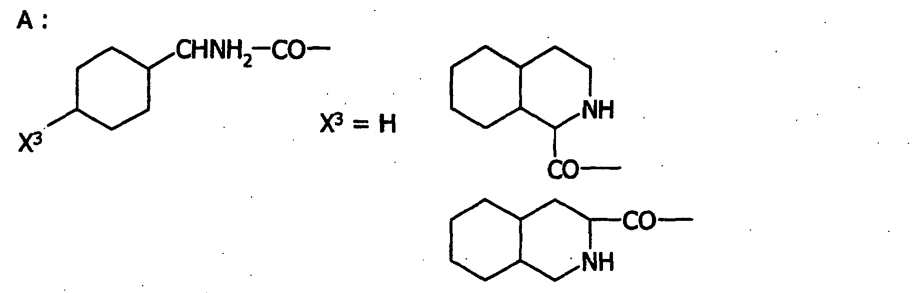 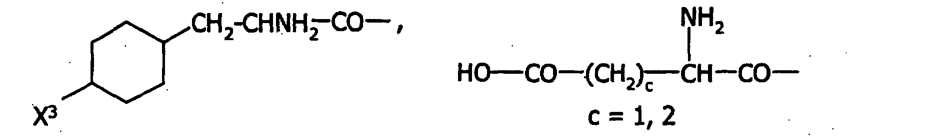 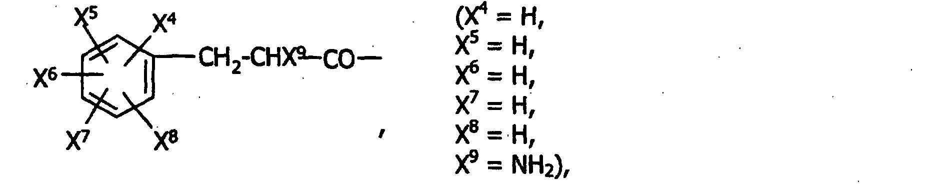
- où, dans tous les restes A cités précédemment, le groupe α-NH₂ peut être monosubstitué par X¹²OC-alkylène en C₁₋₃, alkyle en C₁₋₅-carbonyle, avec X¹² = OH,
B: 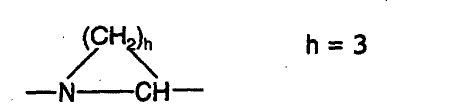 R¹ : H
D : phénylène auquel (CH₂)ₘ et (NH)ₙ sont liés en position méta ou para l'un par rapport à l'autre et qui peut être substitué par F en position ortho par rapport à (CH₂)ₘ,
pyridinylène avec le groupe amidine en position 3 ou
pyridazinylène auquel (CH₂)ₘ et (NH)ₙ sont liés en position para l'un par rapport à l'autre pipéridinylène qui est lié à (CH₂)ₘ en position 4 par rapport à l'azote et dans lequel l'atome d'azote lui-même porte le groupe C(=NH)NHR⁴,
n : 0
R⁴ : H
et ne représente pas le N-[[1-(aminoiminométhyl)-3-pipéridinyl]méthyl]-1-D-phénylalanyl-L-prolinamide.

2. Composés de formule 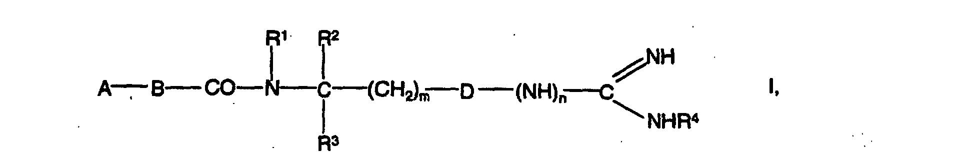 ainsi que leurs sels avec des acides physiologiquement acceptables et leurs stéréoisomères, où les substituants possèdent les significations suivantes :
A: 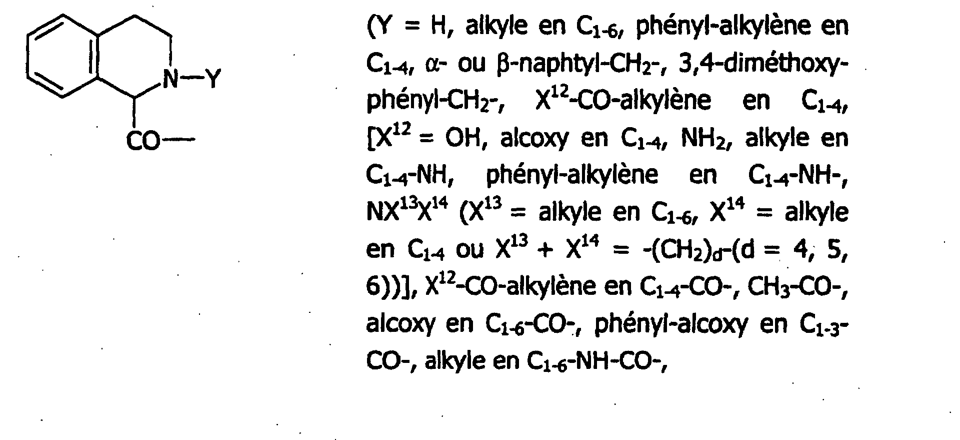 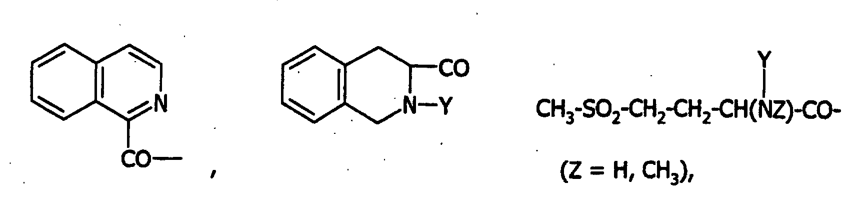 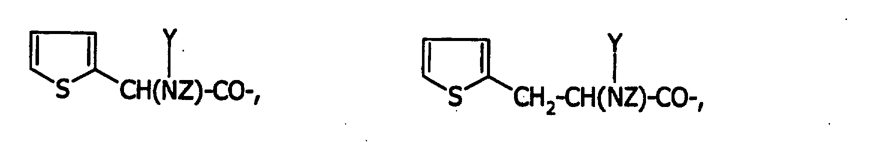 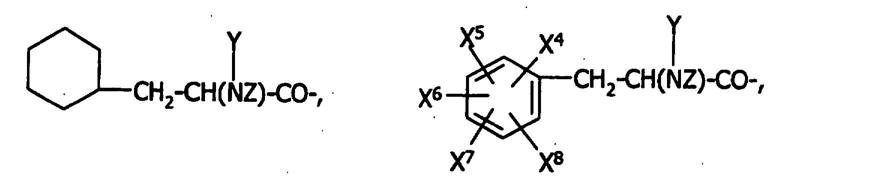 (X⁴ = H, F, Cl, Br, CF₃, alkyle en C₁₋₄, OH, OCH₃, NO₂, phényle, X⁵ = H, F, Cl, Br, CH₃, OH, OCH₃, phényle, X⁶ = H, F, Cl, Br, CH₃, OH, OCH₃, X⁷ = H, F, X⁸ = H, F), 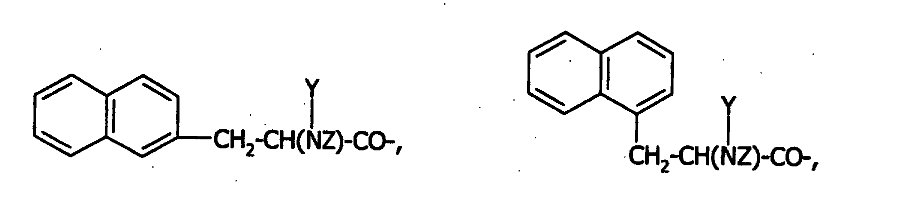 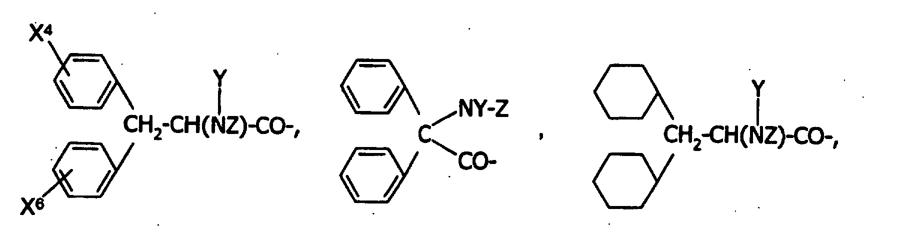 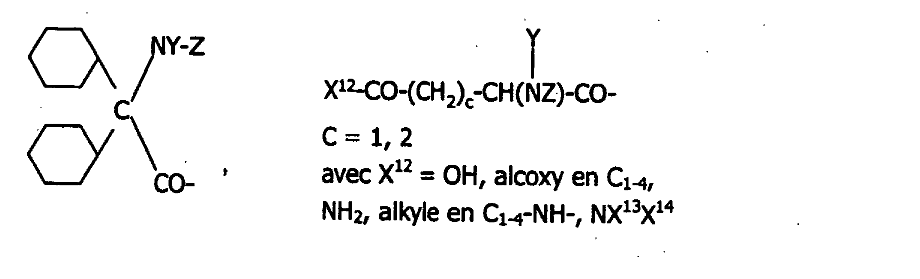 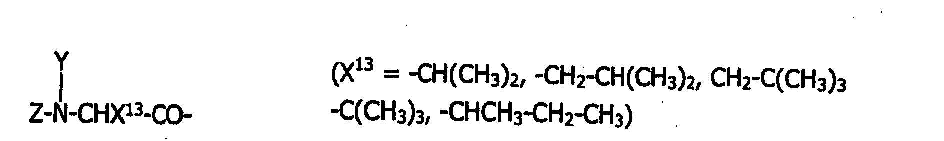 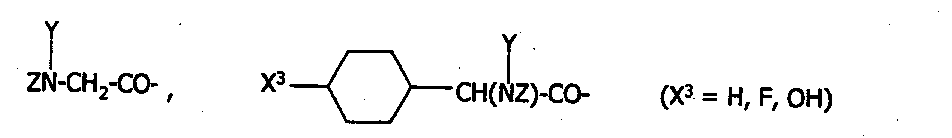 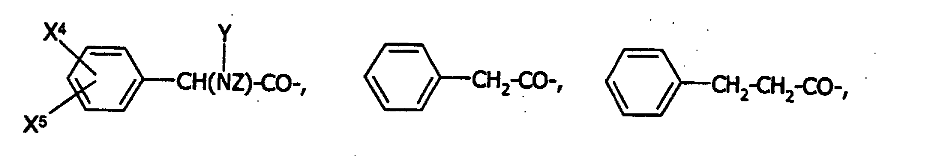 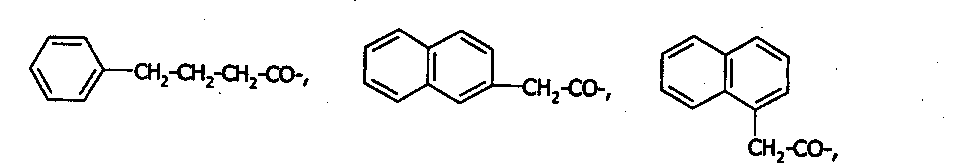 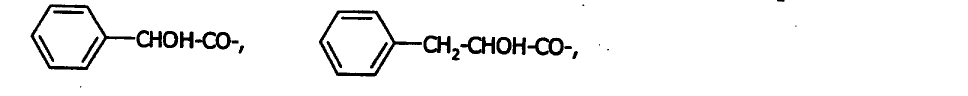 B: 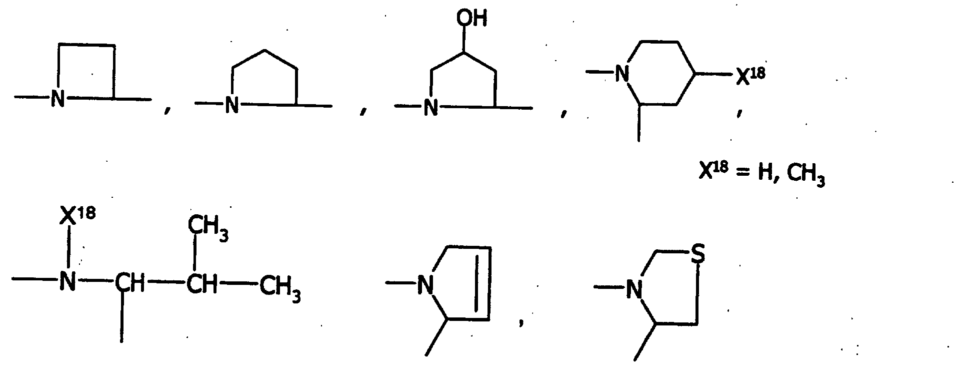
R¹ : H, CH₃,
R² : H,
R³ : H,
R⁴ : H, OH, NH₂
m : 0
pour -(CH₂)ₘ-D-(NH)ₙ- avec n = 0 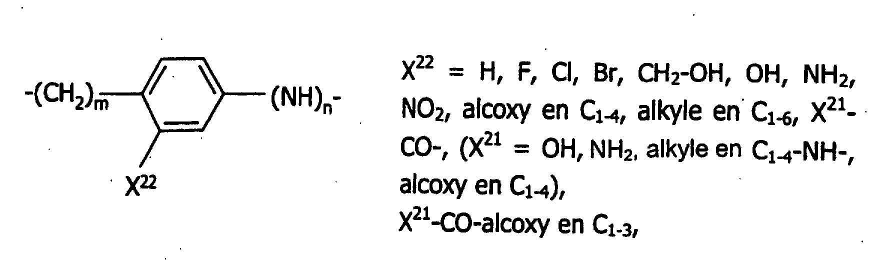 pour -(CH₂)ₘ-D-(NH)ₙ- avec n = 0, 1 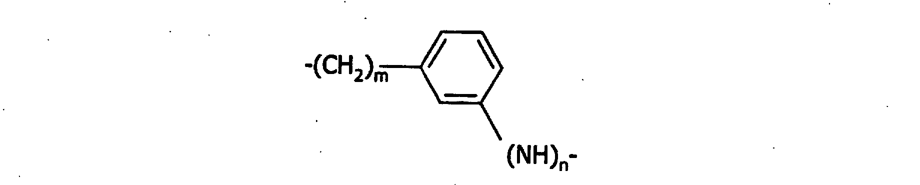 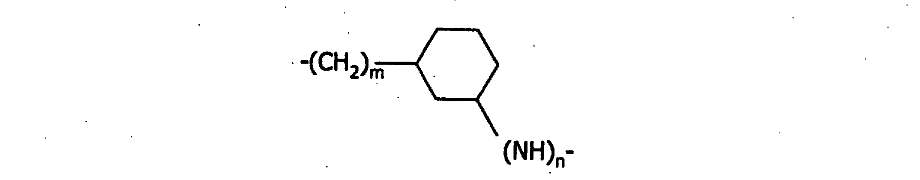 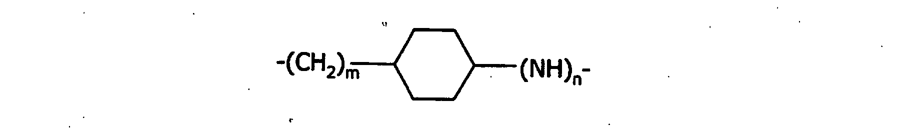 pour -(CH₂)ₘ-D-(NH)ₙ- avec n = 0, 1 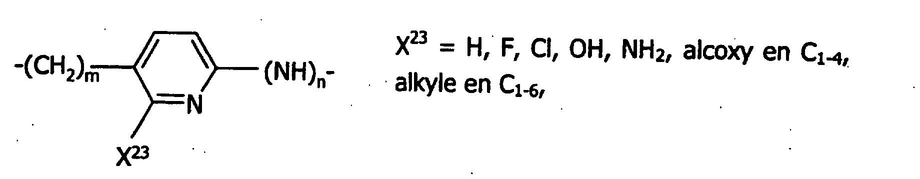 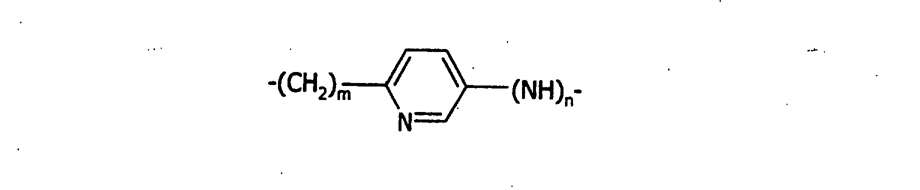 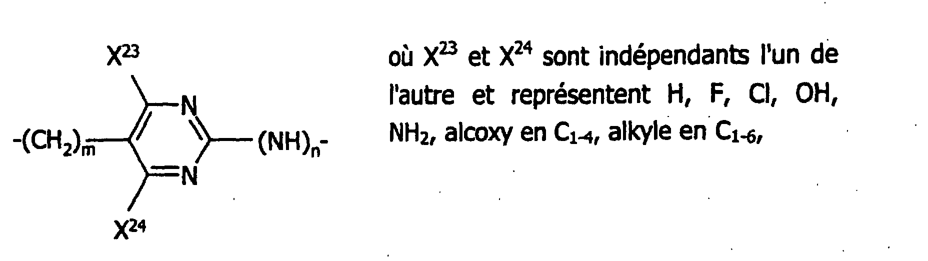 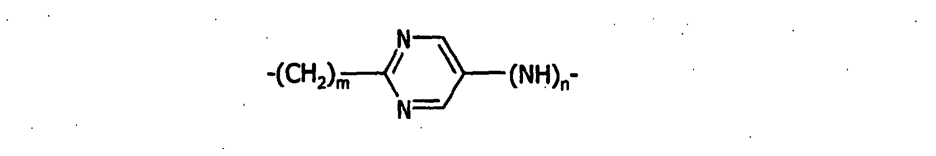 pour -(CH₂)ₘ-D-(NH)ₙ- avec n = 0 : 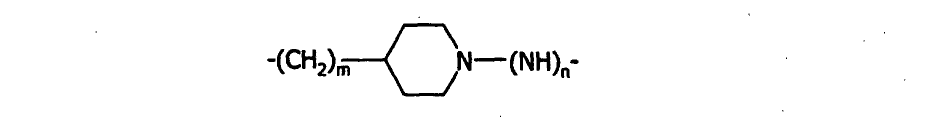 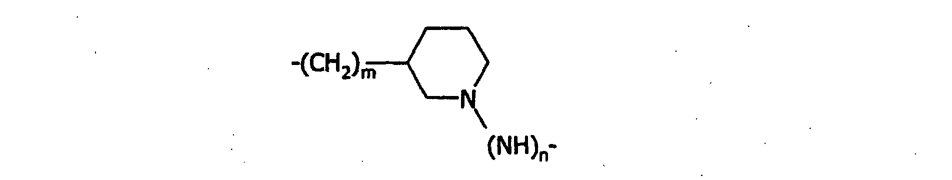 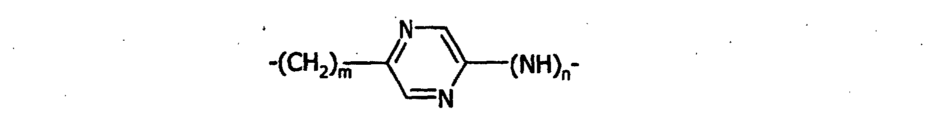 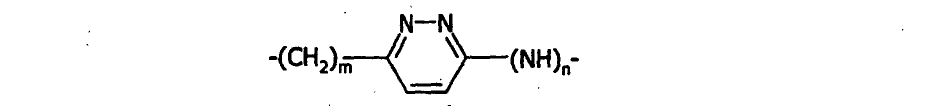 où R³, A, B et D, pour R⁴ = OH ou H, ne peuvent pas revêtir simultanément la signification suivante
A: pour R⁴ = OH ou H
Y = H, alkyle en C₁₋₆, phényl-alkylène en C₁₋₄, CH₃-CO-, alcoxy
en C₁₋₆-CO-, phényl-alcoxy en C₁₋₃-CO-, 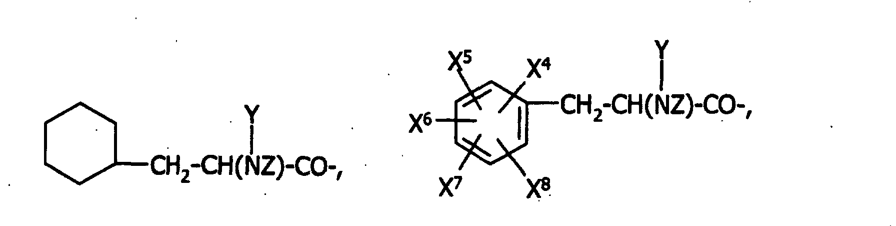
(X⁴ = H, F, alkyle en C₁₋₄,
X⁵ = H,
X⁶ = H,
X⁷ = H,
X⁸ = H), Z = H, 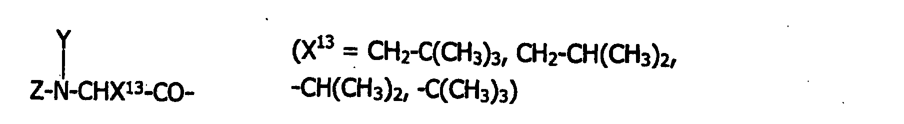 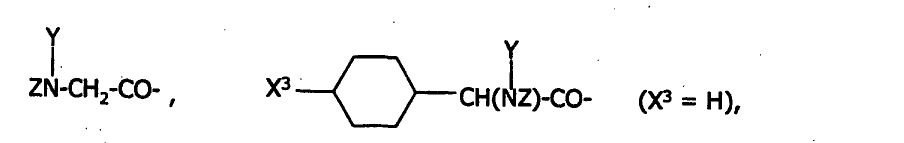 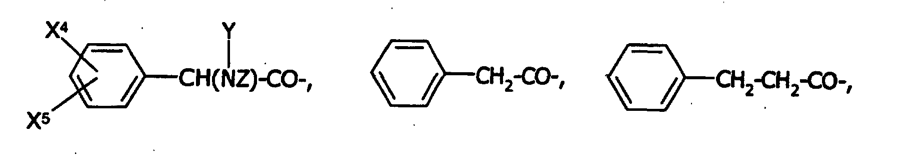 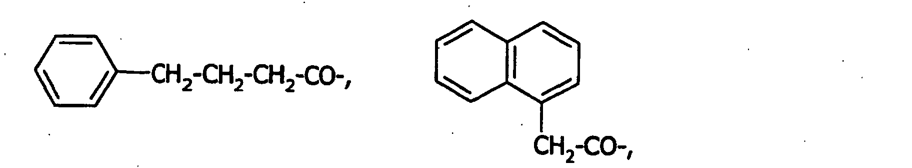 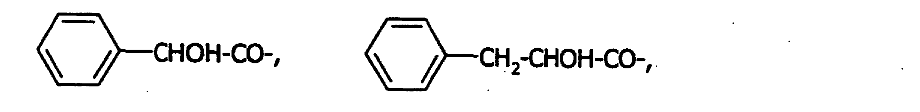 B: 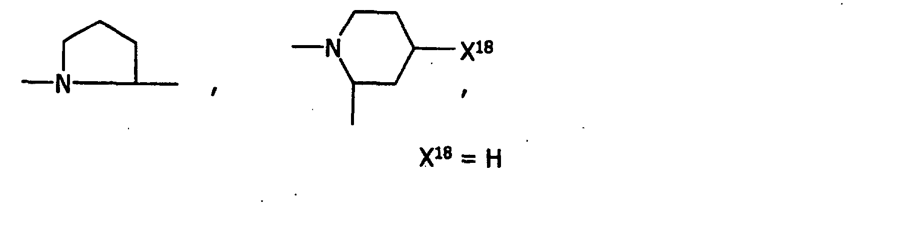
R¹ : H, CH₃,
pour -(CH₂)ₘ-D-(NH)ₙ- avec m = 0, n = 0 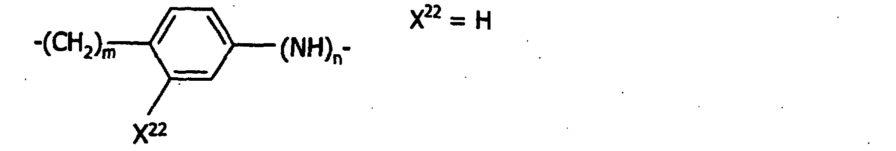 pour -(CH₂)ₘ-D-(NH)ₙ- avec m = 0, n = 0, 1 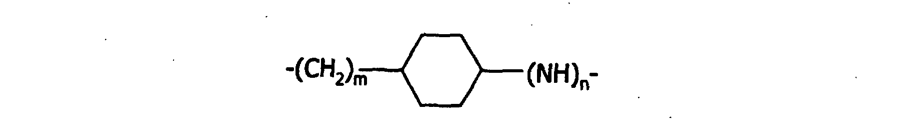 pour -(CH₂)ₘ-D(NH)ₙ- avec m = 0, n = 0 : 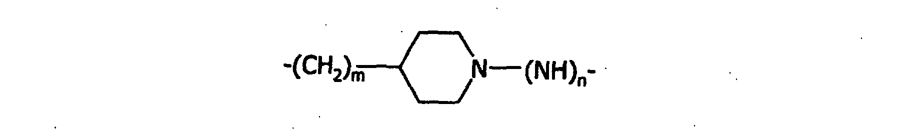 et
où, pour R⁴ = H, A, B, D, R³ ne peuvent pas revêtir simultanément la signification suivante :
A: 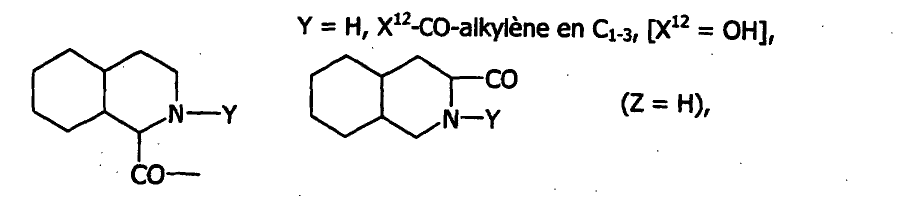 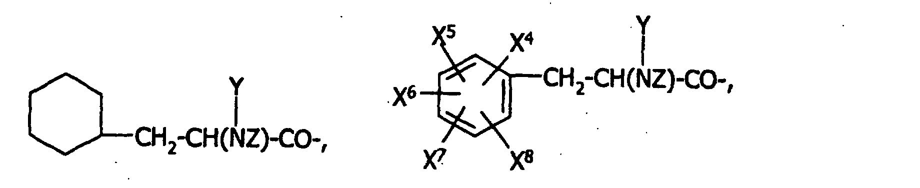
(X⁴ = H,
X⁵ = H,
X⁶ = H,
X⁷ = H,
X⁸ = H), 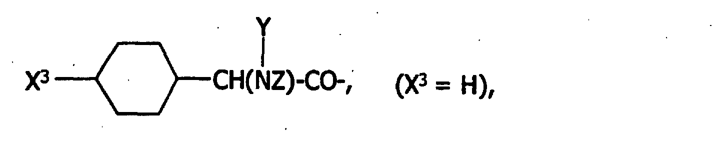 B: 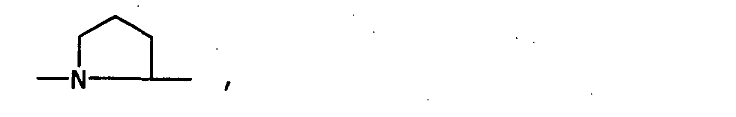
R¹ = H
m = 0
pour -(CH₂)ₘ-D-(NH)ₙ- avec m = 0, n = 0 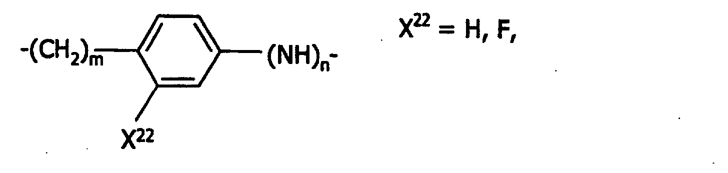 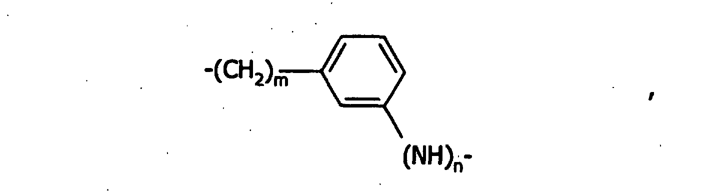 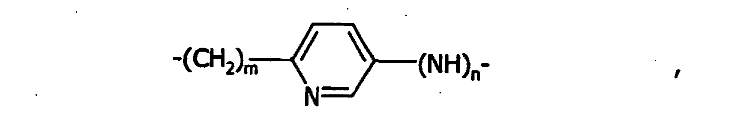 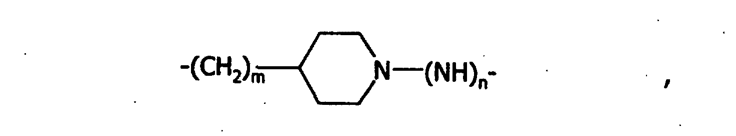 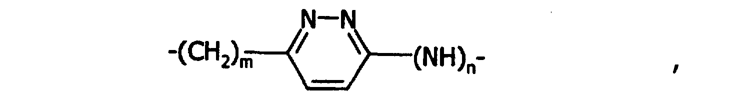 et de même A, B, D, R¹, R⁴ ne peuvent pas représenter
A : (Y = H, X¹²-CO-alkylène en C₁₋₄, [X¹² = OH, alcoxy en C₁₋₄, NH₂];
X¹²-CO-alkylène en C₁₋₄-CO-, CH₃-CO-), 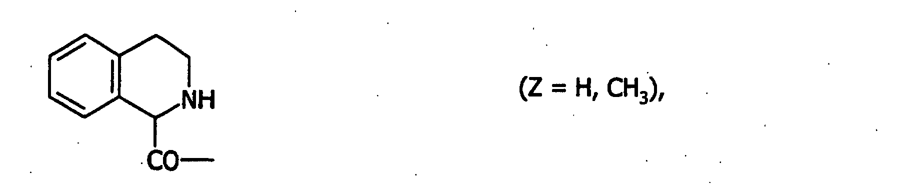 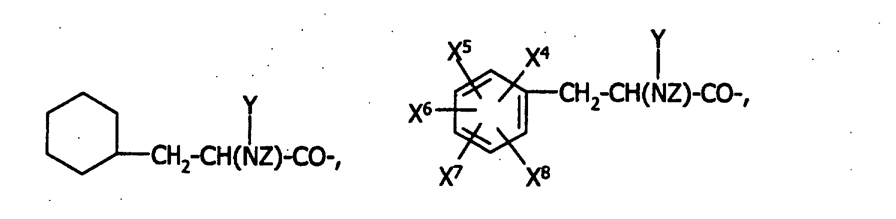
(X⁴ = H,
X⁵ = H,
X⁶ = H,
X⁷ = H,
X⁸ = H), 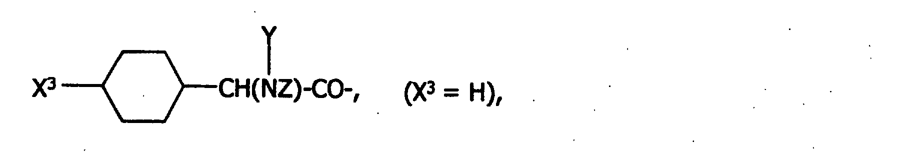 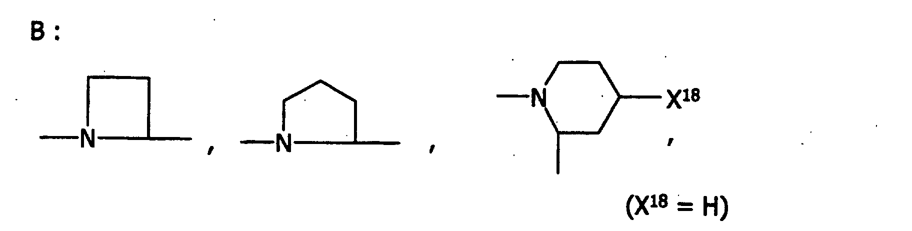 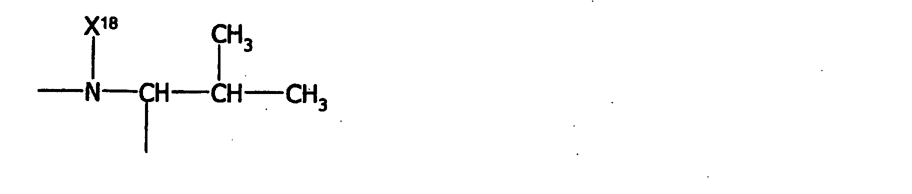
R¹ : H,
R⁴ : H
pour -(CH₂)ₘ-D-(NH)ₙ- avec n = 0 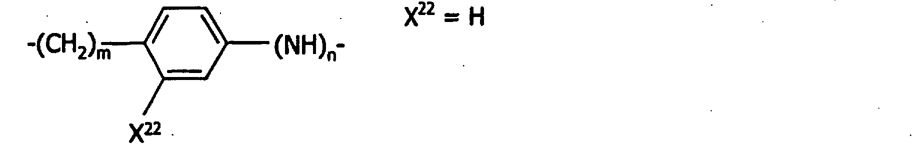 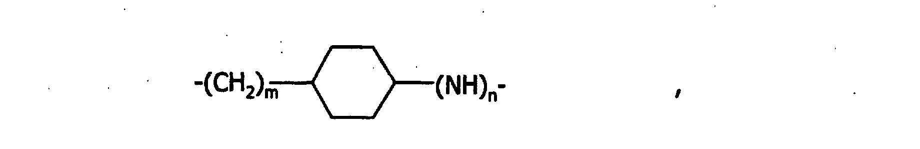 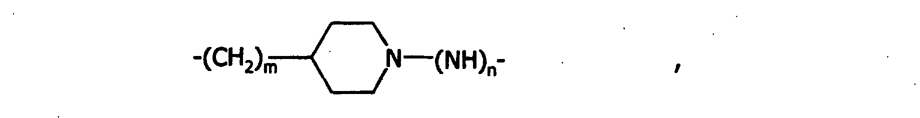 et ne représente pas le N-[[1-[aminoiminométhyl)-3-pipéridinyl]méthyl]-1-D-phénylalanyl-L-prolinamide.

3. Composés de formule 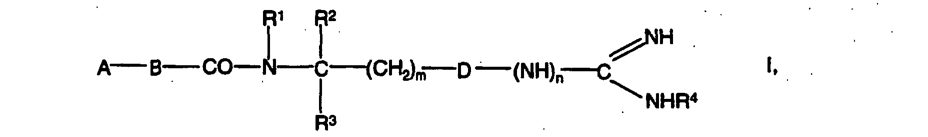 ainsi que leurs sels avec des acides physiologiquement acceptables et leurs stéréoisomères, où les substituants possèdent les significations suivantes : 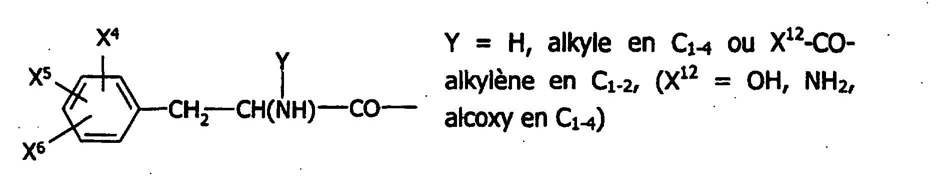
X⁴ = H, F, Cl, CH₃, CF₃, OCH₃ ou Br
X⁵ = H, Cl, CH₃ ou OCH₃, OC₂H₅
X⁶ = H ou OCH₃, 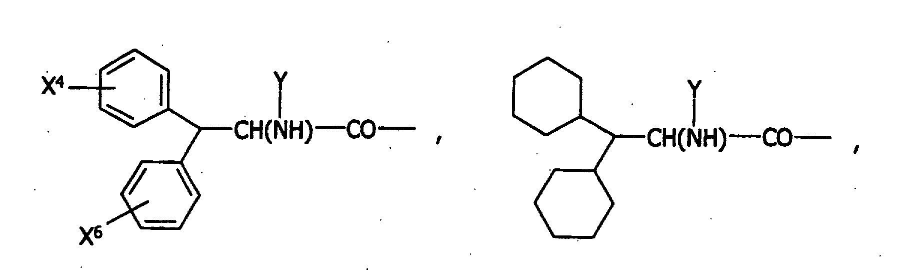 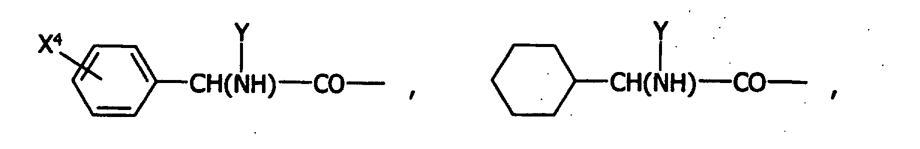 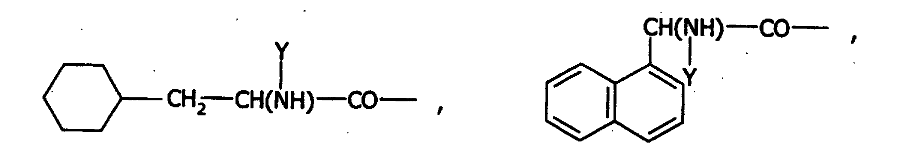 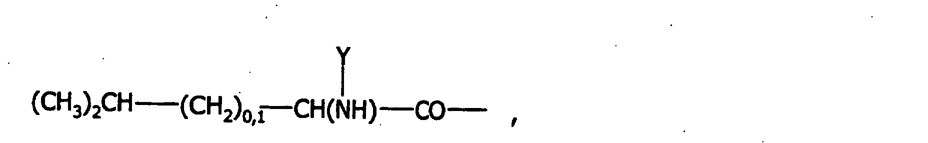 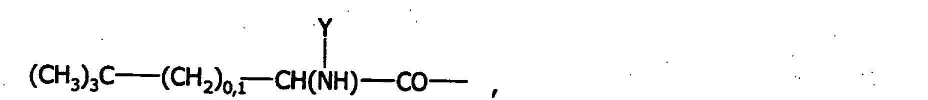 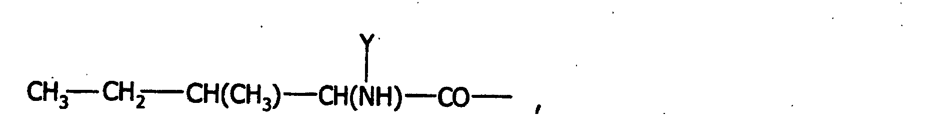 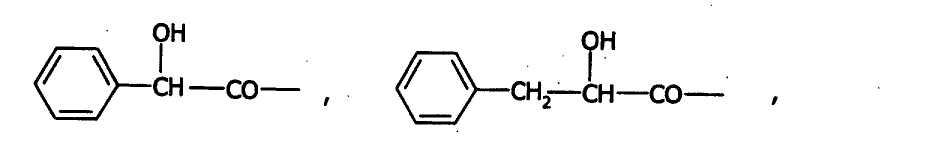
B: 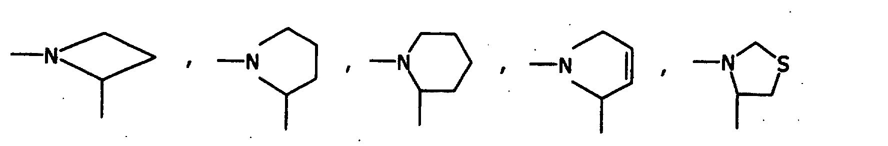
R¹ = H
R², R³ = H
pour -(CH₂)ₘ-D-(NH)ₙ- avec m = 0, n = 0 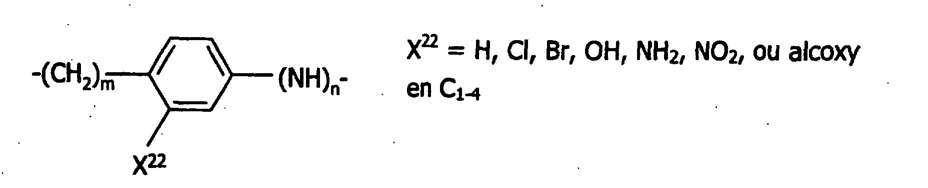 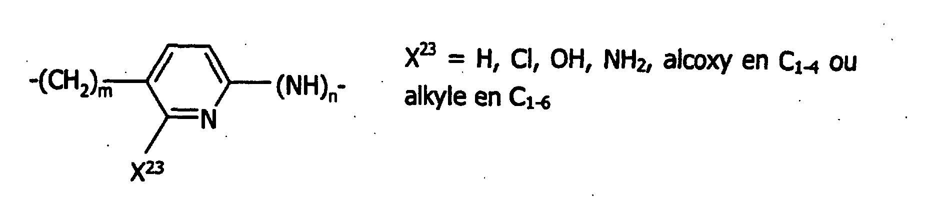 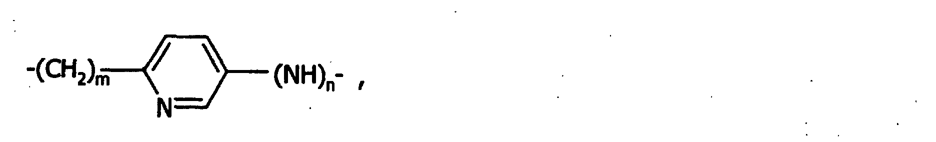 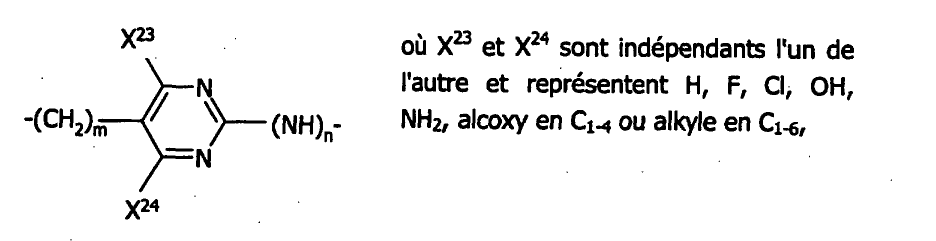 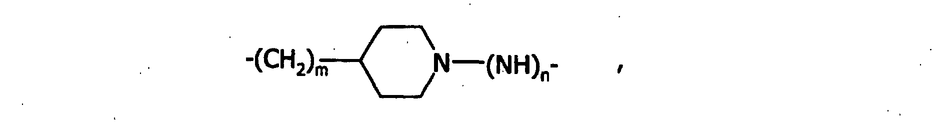 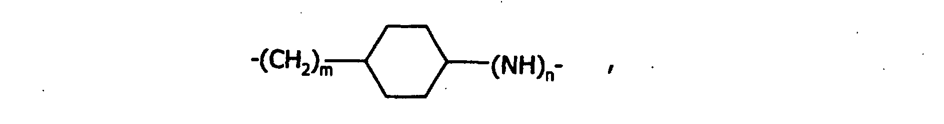 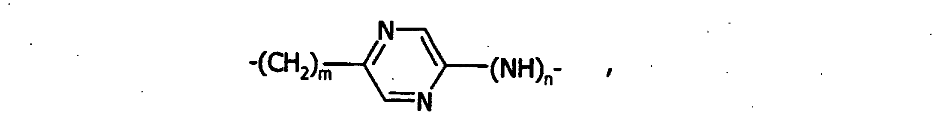 pour -(CH₂)ₘ-D-(NH)ₙ- avec m = 0, n = 1 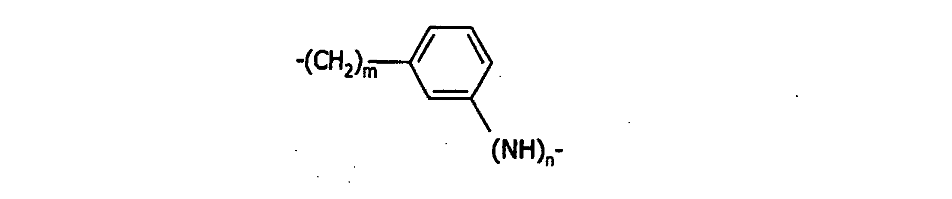 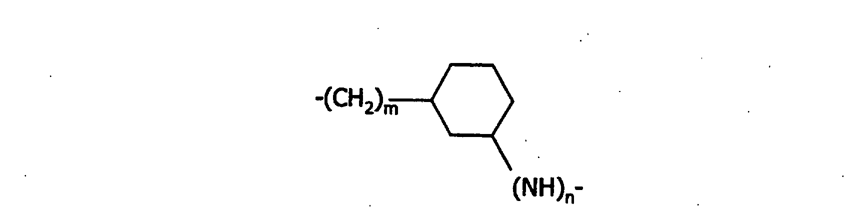 R⁴ : H, OH
où A, B, D et R⁴ n'ont pas simultanément la signification suivante :
A: 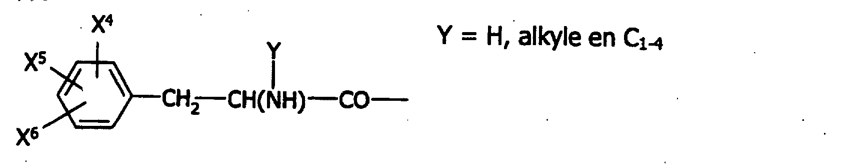
X⁴ = H, F, CH₃
X⁵ = H
X⁶ = H 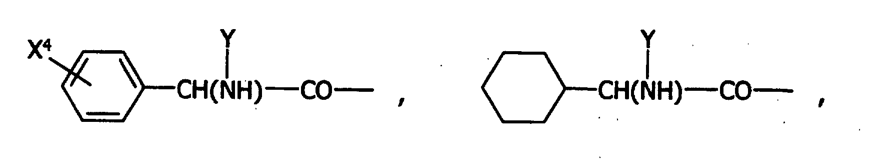 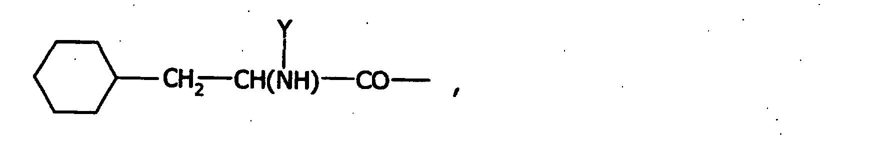 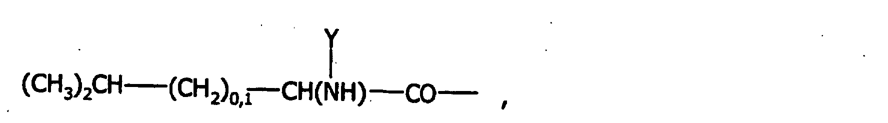 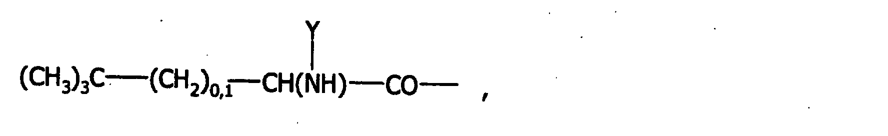 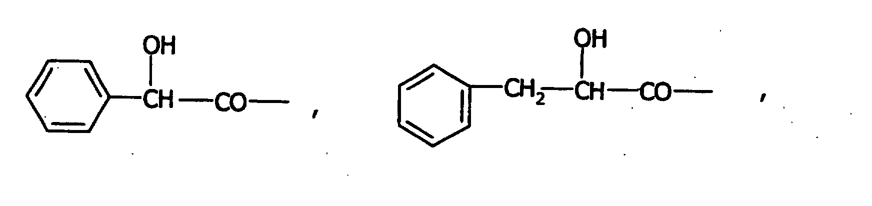
B: 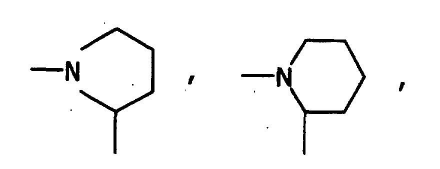 pour -(CH₂)ₘ-D-(NH)ₙ- avec m = 0, n = 0 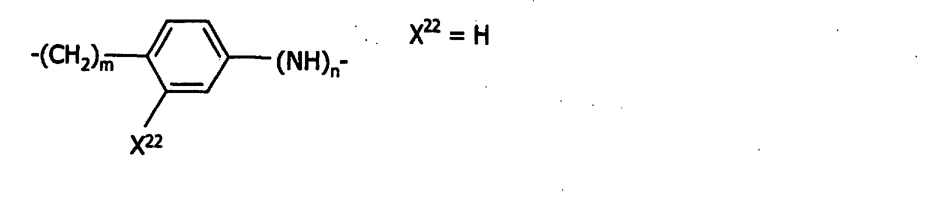 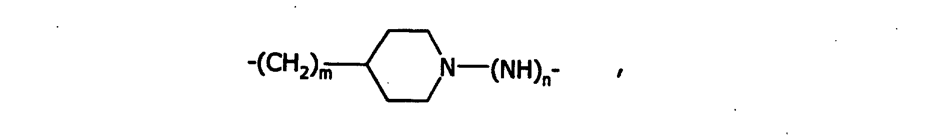 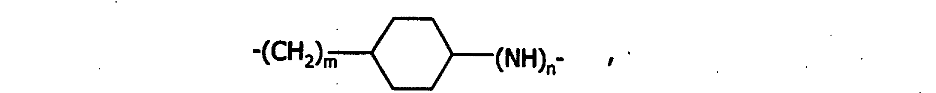 R⁴ : H, OH
et pour
R⁴ = H
A, B et D ne peuvent pas simultanément revêtir les significations suivantes :
A: 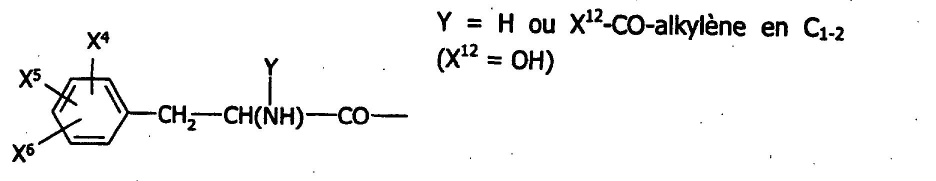
X⁴ = H
X⁵ = H
X⁶ = H 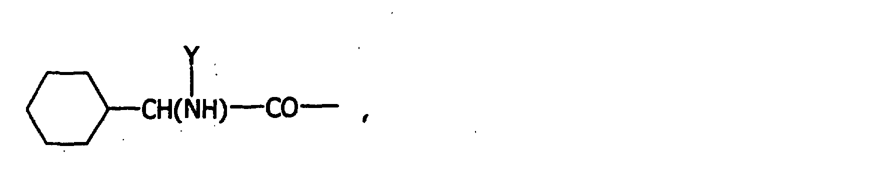 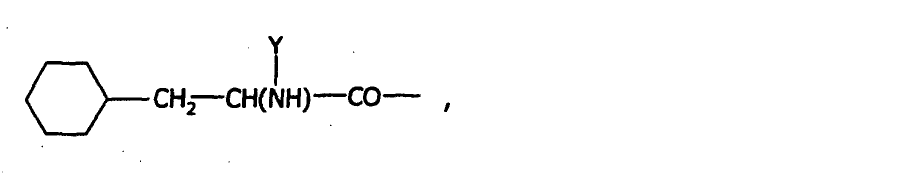 B: 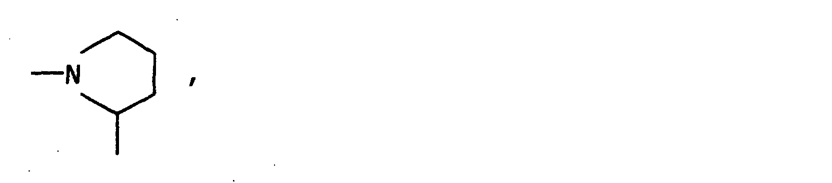
pour -(CH₂)ₘ-D-(NH)ₙ- avec m = 0, n = 0 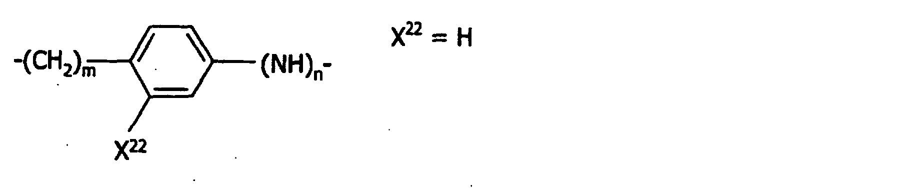 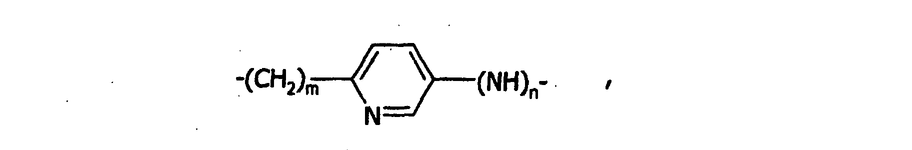 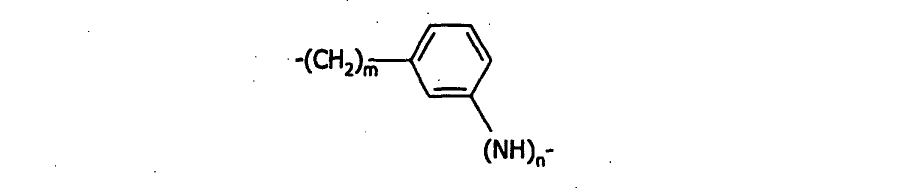 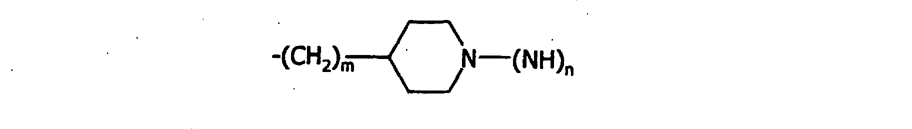 et, de même, pour R⁴ = H, A, B, D, R¹ ne peuvent pas revêtir les significations suivantes :
A : Y = H ou X¹²-CO-alkylène en C₁₋₂ 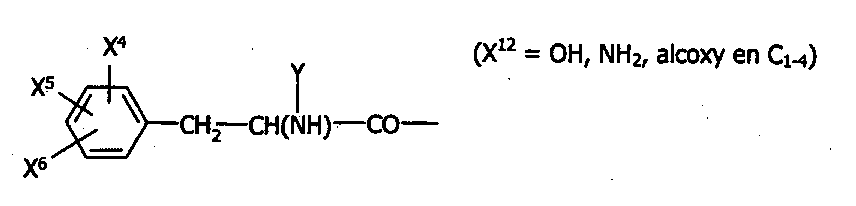
X⁴ = H
X⁵ = H
X⁶ = H 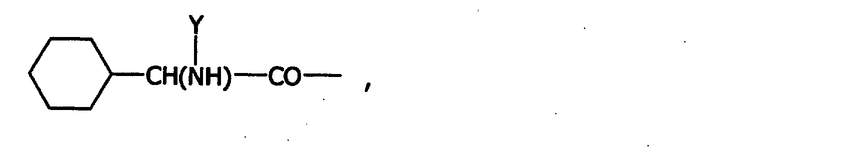 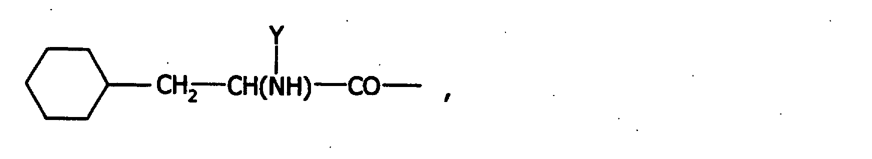 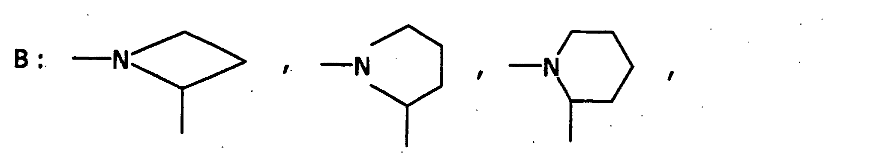
pour -(CH₂)ₘ-D-(NH)ₙ- avec m = 0, n = 0

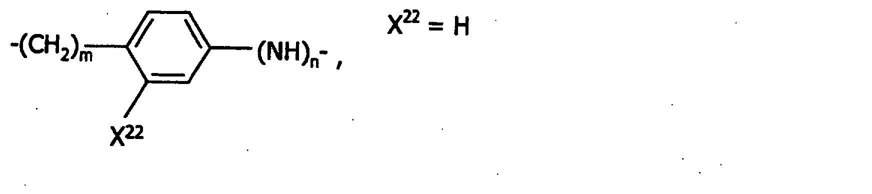

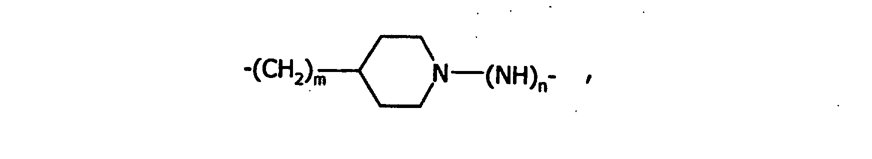

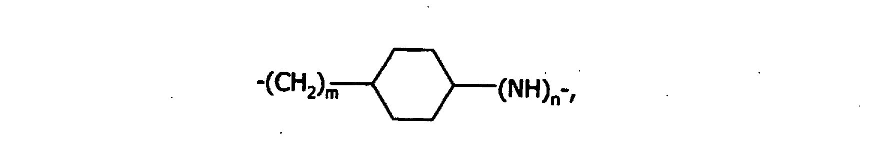

4. Composés de formula I selon l'une des revendications 1 à 3 destinés à être utilisés pour la lutte contre des maladies.

5. Composés de formule II 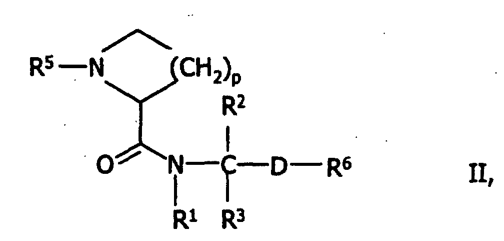 où R¹, R², R³ et D possèdent les significations indiquées pour la formule I et
R⁵ représente H, alcoxy en C₁₋₄-CO- ou phényl-alcoxy en C₁₋₃-CO-,
R⁶ représente un reste cyano, amidino ou guanidino en position m ou p par rapport à C(R², R³) et
p représente 1, 2 ou 3.
